# EUROPEAN PATENT APPLICATION

(11) **EP 1 837 332 A1**
(43) Date of publication of application: **26.09.2007**
(21) Application number: 06380059.3
(22) Date of filing: 23.03.2006
(51) Int. Cl.: C07D 217/04, C07D 217/06, C07D 217/10, C07D 409/12, C07D 513/04, C07D 401/12, A61K 31/472, A61K 31/4725, A61P 3/00

(54) **Substituted tetrahydroisoquinoline compounds, their preparation and use in medicaments**

(71) Applicant: LABORATORIOS DEL DR. ESTEVE, S.A., 08041 Barcelona (ES)
(72) Inventor: Torrens Jover, Antoni, 08221 Terrassa (Barcelona) (ES); Mas Prio, Josep, 08191 Rubí (Barcelona) (ES); Port Casamitjana, Adriana, 08202 Sabadell (Barcelona) (ES); Buschmann, Helmut H., 08960 Sant Just Desvern (Barcelona) (ES)
(74) Representative: Brosch, Oliver

(57) **Abstract**

The present invention relates to substituted tetrahydroisoquinoline compounds of general formula I, 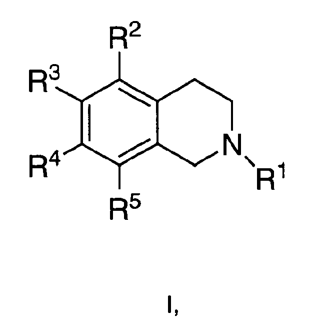 a process for their preparation, medicaments comprising said substituted tetrahydroisoquinoline compounds as well as the use of said substituted tetrahydroisoquinoline compounds for the preparation of medicaments, which are particularly suitable for the prophylaxis and/or treatment of disorders or diseases that are at least partially mediated via 5-HT₆ receptors.

## Description

The present invention relates to substituted tetrahydroisoquinoline compounds of general formula I, 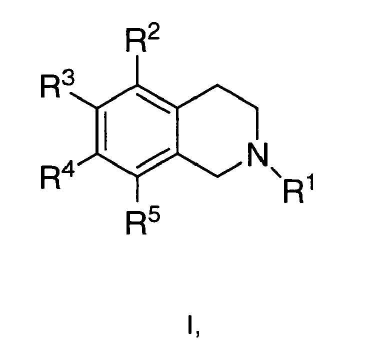 a process for their preparation, medicaments comprising said substituted tetrahydroisoquinoline compounds as well as the use of said substituted tetrahydroisoquinoline compounds for the preparation of medicaments, which are particularly suitable for the prophylaxis and/or treatment of disorders or diseases that are at least partially mediated via 5-HT₆ receptors.

The superfamily of serotonin receptors (5-HT) includes 7 classes (5-HT₁-5-HT₇) encompassing 14 human subclasses [D. Hoyer, et al., Neuropharmacology, 1997, 36, 419]. The 5-HT₆ receptor is the latest serotonin receptor identified by molecular cloning both in rats [F.J. Monsma et al., Mol. Pharmacol., 1993, 43, 320; M. Ruat et al., Biochem. Biophys. Res. Commun., 1993, 193, 268] and in humans [R. Kohen, et al., J. Neurochem., 1996, 66, 47].

Compounds with 5-HT₆ receptor affinity are useful for the treatment of various disorders of the Central Nervous System and of the gastrointestinal tract, such as irritable intestine syndrome. Compounds with 5-HT₆ receptor affinity are also useful in the treatment of anxiety, depression and cognitive memory disorders [M. Yoshioka et al., Ann. NY Acad. Sci., 1998, 861, 244; A. Bourson et al., Br. J. Pharmacol. , 1998, 125, 1562; D.C. Rogers et al., Br. J. Pharmacol. Suppl., 1999, 127, 22P; A. Bourson et al., J. Pharmacol. Exp. Ther. , 1995, 274, 173; A.J. Sleight, et al., Behav. Brain Res. , 1996, 73, 245; T.A. Branchek et al., Annu. Rev. Pharmacol. Toxicol. , 2000, 40, 319; C. Routledge et al., Br. J. Pharmacol. , 2000, 130, 1606]. It has been shown that typical and atypical antipsychotic drugs for treating schizophrenia have a high affinity for 5-HT₆ receptors [B.L. Roth et al., J. Pharmacol. Exp. Ther. , 1994, 268, 1403; C.E. Glatt et al., Mol. Med. , 1995, 1, 398; F.J. Mosma,et al., Mol. Pharmacol. , 1993, 43, 320; T. Shinkai et al., Am. J. Med. Genet. , 1999, 88, 120]. Compounds with 5-HT₆ receptor affinity are useful for treating infant hyperkinesia (ADHD, attention deficit / hyperactivity disorder) [W.D. Hirst et al., Br. J. Pharmacol. , 2000, 130, 1597; C. Gérard et al., Brain Research , 1997, 746, 207; M.R. Pranzatelli, Drugs of Today , 1997, 33, 379].

Recently, it has been shown that the 5-HT₆ receptor also plays a role in food ingestion [Neuropharmacology, 41, 2001, 210-219].

Food ingestion disorders, particularly obesity, are a serious, fast growing threat to the health of humans of all age groups, since they increase the risk of developing other serious, even life-threatening diseases such as diabetes or coronary diseases as well.

Therefore an object of the present invention was to provide compounds that are particularly suitable as active ingredients in medicaments, especially in medicaments for the prophylaxis and/or treatment of disorders or diseases related to 5-HT₆ receptors such as food intake related disorders.

Surprisingly, it has been found that the substituted tetrahydroisoquinoline compounds of general formula I given below show good to excellent affinity for 5-HT₆-receptors. These compounds are therefore particularly suitable as pharmacologically active agents in a medicament for the prophylaxis and/or treatment of disorders or diseases related to 5-HT₆-receptors such as food intake related disorders like obesity.

Thus, in one of its aspects the present invention relates to a medicament comprising a substituted tetrahydroisoquinoline compounds of general formula I, 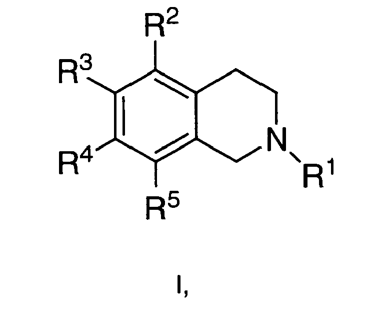 wherein
R¹ represents a hydrogen atom; a -C(=O)-OR³⁷ moiety;
a linear or branched, saturated or unsaturated C₁₋₁₀ aliphatic radical which may be unsubstituted or optionally substituted with 1, 2, 3, 4 or 5 substituent(s) independently selected from the group consisting of F, Cl, Br, I, -CN, -CF₃, -OCF₃, -SCF₃, -OH, -SH, - NH₂, -O-C₁₋₅-alkyl, -S-C₁₋₅-alkyl, -NH(C₁₋₅-alkyl) and -N(C₁₋₅-alkyl)₂;
or a saturated or unsaturated 3- to 9-membered cycloaliphatic radical, which may contain 1, 2 or 3 heteroatom(s) independently selected from the group consisting of nitrogen, oxygen and sulfur as ring member(s) and which may be unsubstituted or optionally substituted with 1, 2, 3, 4 or 5 substituent(s) independently selected from the group consisting of oxo (=O), thioxo (=S), C₁₋₅-alkyl, -O-C₁₋₅-alkyl, -S-C₁₋₅-alkyl, -C(=O)-C₁₋₅-alkyl, -O-C(=O)-C₁₋₅-alkyl, F, Cl, Br, I, -CN, -CF₃, -OCF₃, -SCF₃, -OH, -SH, -NH₂, - NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl and which may be condensed with an unsubstituted or at least mono-substituted saturated, unsaturated or aromatic mono- or bicyclic ring system and which may be bonded via a linear or branched C₁₋₆ alkylene, C₂₋₆ alkenylene or C₂₋₆ alkinylene group which may be unsubstituted or substituted with 1, 2 or 3 substituent(s) independently selected from the group consisting of F, Cl, Br, I, -CN, -CF₃, -OCF₃, -SCF₃, -OH, -SH, - NH₂, -O-C₁₋₅-alkyl, -S-C₁₋₅-alkyl, -NH(C₁₋₅-alkyl) and -N(C₁₋₅-alkyl)₂;
R², R³, R⁴ and R⁵, independently of one another, each represent a hydrogen atom; F, Cl, Br, I, -NO₂; -NH₂; -SH; -OH; -CN; -C(=O)-OH; -C(=O)-H; -S(=O)₂-OH; -C(=O)-NH₂; -S(=O)₂-NH₂; -C(=O)-R⁶; -S(=O)-R⁷; -S(=O)₂-R⁷; -OR⁸; -SR⁹; -C(=O)-OR¹⁰; -N(R¹¹)-S(=O)₂-R¹²; -NR¹³R¹⁴; -NH-R¹⁵; -C(=O)-NR¹⁶R¹⁷; C(=O)-NHR¹⁸;
a linear or branched, saturated or unsaturated, unsubstituted or at least mono-substituted aliphatic radical;
a saturated or unsaturated, unsubstituted or at least mono-substituted, optionally at least one heteroatom as a ring member containing cycloaliphatic radical, which may be bonded via a linear or branched alkylene, alkenylene or alkinylene group and which may be condensed with an unsubstituted or at least mono-substituted saturated, unsaturated or aromatic mono- or bicyclic ring system;
or an unsubstituted or at least mono-substituted aryl or heteroaryl radical, which may be bonded via a linear or branched alkylene, alkenylene or alkinylene group and which may be condensed with an unsubstituted or at least mono-substituted saturated or unsaturated, but not aromatic, mono- or bicyclic ring system;
with the proviso that at least one of the substituents R², R³, R⁴ and R⁵ represents a - N(R¹¹)-S(=O)₂-R¹² moiety;
R⁶, R⁷, R⁸, R⁹, R¹⁰, R¹³, R¹⁴, R¹⁵, R¹⁶, R¹⁷ and R¹⁸, independently of one another, each represent a linear or branched, saturated or unsaturated, unsubstituted or at least mono-substituted aliphatic radical;
a saturated or unsaturated, unsubstituted or at least mono-substituted, optionally at least one heteroatom as a ring member containing cycloaliphatic radical, which may be bonded via a linear or branched alkylene, alkenylene or alkinylene group and which may be condensed with an unsubstituted or at least mono-substituted saturated, unsaturated or aromatic mono- or bicyclic ring system;
or an unsubstituted or at least mono-substituted aryl or heteroaryl radical, which may be bonded via a linear or branched alkylene, alkenylene or alkinylene group and which may be condensed with an unsubstituted or at least mono-substituted saturated or unsaturated, but not aromatic, mono- or bicyclic ring system;
R¹¹ represents a hydrogen atom, -S(=O)₂-R¹² or a linear or branched, saturated or unsaturated, unsubstituted or at least mono-substituted aliphatic radical;
R¹² represents a phenyl radical of general formula (A), 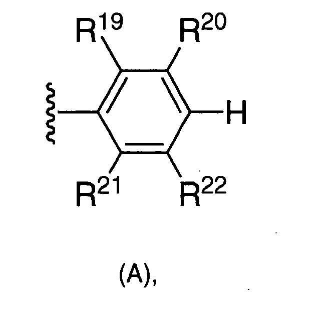 wherein
R¹⁹, R²⁰, R²¹ and R²², independently of one another, each represent a hydrogen atom; F, Cl, Br, I, -NO₂; -NH₂; -SH; -OH; -CN; -C(=O)-OH; -C(=O)-H; -S(=O)₂-OH; -C(=O)-NH₂; - S(=O)₂-NH₂; -C(=O)-R²³; -S(=O)-R²⁴; -S(=O)₂-R²⁴; -OR²⁵; -SR²⁶ ; -C(=O)-OR²⁷; -N(R²⁸)-S(=O)₂-R²⁹; -NH-S(=O)₂-R³⁰; -NR³¹R³²; -NH-R³³; -C(=O)-NHR³⁴; -C(=O)-NR³⁵R³⁶; a linear or branched, saturated or unsaturated C₁₋₁₀ aliphatic radical which may be unsubstituted or optionally substituted with 1, 2, 3, 4 or 5 substituent(s) independently selected from the group consisting of F, Cl, Br, I, -CN, -CF₃, -OCF₃, -SCF₃, -OH, -SH, - NH₂, -O-C₁₋₅-alkyl, -S-C₁₋₅-alkyl, -NH(C₁₋₅-alkyl) and -N(C₁₋₅-alkyl)₂; or a saturated or unsaturated, unsubstituted or at least mono-substituted, optionally at least one heteroatom as a ring member containing cycloaliphatic radical;
with the proviso that at least one of the substituents R¹⁹, R²⁰, R²¹ and R²² is unlike hydrogen;
an unsubstituted or at least mono-substituted 10-membered aryl radical;
a monocyclic heteroaryl radical, which may be unsubstituted or optionally substituted with 1, 2, 3, 4 or 5 substituent(s) independently selected from the group consisting of F, Cl, Br, I, -NO₂; -NH₂; -SH; -OH; -CN; -C(=O)-OH; -C(=O)-H; -S(=O)₂-OH; -C(=O)-NH₂; - S(=O)₂-NH₂; -C(=O)-R²³; -S(=O)-R²⁴; -S(=O)₂-R²⁴; -OR²⁵; -SR²⁶; -C(=O)-OR²⁷; -N(R²⁸)-S(=O)₂-R²⁹; -NH-S(=O)₂-R³⁰; -NR³¹R³²; -NH-R³³; -C(=O)-NHR³⁴; -C(=O)-NR³⁵R³⁶ ; a linear or branched, saturated or unsaturated C₁₋₁₀ aliphatic radical which may be unsubstituted or substituted with 1, 2 or 3 substituent(s) independently selected from the group consisting of F, Cl, Br, I, -CN, -CF₃, -OCF₃, -SCF₃, -OH, -SH, -NH₂, -O-C₁₋₅-alkyl, - S-C₁₋₅-alkyl, -NH(C₁₋₅-alkyl) and -N(C₁₋₅-alkyl)₂; and a saturated or unsaturated, unsubstituted or at least mono-substituted, optionally at least one heteroatom as a ring member containing cycloaliphatic radical;
an unsubstituted or at least mono-substituted monocyclic heteroaryl radical, which is condensed with an unsubstituted or at least mono-substituted saturated or unsaturated, but not aromatic, mono- or bicyclic ring system;
an unsubstituted or at least mono-substituted bi- or tricyclic heteroaryl radical, which may be condensed with an unsubstituted or at least mono-substituted saturated or unsaturated, but not aromatic, mono- or bicyclic ring system;
or a saturated or unsaturated, unsubstituted or at least mono-substituted, optionally at least one heteroatom as a ring member containing cycloaliphatic radical, which may be condensed with an unsubstituted or at least mono-substituted saturated, unsaturated or aromatic mono- or bicyclic ring system;
R²³, R²⁷, R²⁸, R²⁹ and R³⁰, independently of one another, each represent a linear or branched, saturated or unsaturated C₁₋₁₀ aliphatic radical which may be unsubstituted or optionally substituted with 1, 2, 3, 4 or 5 substituent(s) independently selected from the group consisting of F, Cl, Br, I, -CN, -CF₃, -OCF₃, -SCF₃, -OH, -SH, -NH₂, -O-C₁₋₅-alkyl, -S-C₁₋₅-alkyl, -NH(C₁₋₅-alkyl) and -N(C₁₋₅-alkyl)₂;
a saturated or unsaturated, unsubstituted or at least mono-substituted, optionally at least one heteroatom as a ring member containing cycloaliphatic radical, which may be bonded via a linear or branched alkylene, alkenylene or alkinylene group and which may be condensed with an unsubstituted or at least mono-substituted saturated, unsaturated or aromatic mono- or bicyclic ring system;
or an unsubstituted or at least mono-substituted aryl or heteroaryl radical, which may be bonded via a linear or branched alkylene, alkenylene or alkinylene group and which may be condensed with an unsubstituted or at least mono-substituted saturated or unsaturated, but not aromatic, mono- or bicyclic ring system;
R²⁴, R²⁶, R³¹, R³² and R³³, each represent a linear or branched, saturated or unsaturated C₁₋₁₀ aliphatic radical which may be unsubstituted or substituted with 1, 2, 3, 4 or 5 substituent(s) independently selected from the group consisting of F, Cl, Br, I, - CN, -CF₃, -OCF₃, -SCF₃, -OH, -SH, -NH₂, -O-C₁₋₅-alkyl, -S-C₁₋₅-alkyl, -NH(C₁₋₅-alkyl) and -N(C₁₋₅-alkyl)₂;
a saturated or unsaturated, unsubstituted or at least mono-substituted, optionally at least one heteroatom as a ring member containing cycloaliphatic radical, which may be bonded via a linear or branched alkylene, alkenylene or alkinylene group and which may be condensed with an unsubstituted or at least mono-substituted saturated, unsaturated or aromatic mono- or bicyclic ring system;
or an unsubstituted or at least mono-substituted aryl or heteroaryl radical, which may be condensed with an unsubstituted or at least mono-substituted saturated or unsaturated, but not aromatic, mono- or bicyclic ring system;
R²⁵, R³⁴, R³⁵ and R³⁶, represents a linear or branched, saturated or unsaturated C₁₋₁₀ aliphatic radical which may be unsubstituted or optionally substituted with 1, 2, 3, 4 or 5 substituent(s) independently selected from the group consisting of F, Cl, Br, I, -CN, -CF₃, -OCF₃, -SCF₃, -OH, -SH, -NH₂, -O-C₁₋₅-alkyl, -S-C₁₋₅-alkyl, -NH(C₁₋₅-alkyl) and -N(C₁₋₅-alkyl)₂;
and R³⁷ represents a linear or branched, saturated or unsaturated C₁₋₁₀ aliphatic radical,
a saturated or unsaturated, unsubstituted or at least mono-substituted, optionally at least one heteroatom as a ring member containing cycloaliphatic radical, which may be bonded via a linear or branched alkylene, alkenylene or alkinylene group and which may be condensed with an unsubstituted or at least mono-substituted saturated, unsaturated or aromatic mono- or bicyclic ring system;
or an unsubstituted or at least mono-substituted aryl or heteroaryl radical, which may be bonded via a linear or branched alkylene, alkenylene or alkinylene group and which may be condensed with an unsubstituted or at least mono-substituted saturated or unsaturated, but not aromatic, mono- or bicyclic ring system;
optionally in form of one of its stereoisomers, preferably enantiomers or diasteromers, a racemate or in form of a mixture of at least two of its stereoisomers, preferably enantiomers and/or diastereomers, in any mixing ratio, or a salt thereof, or a corresponding solvate thereof;
and optionally at least one physiologically acceptable auxiliary agent.

Preferred is a medicament comprising at least one substituted tetrahydroisoquinoline compound of general formula I in form of its salt as defined in general formula II, 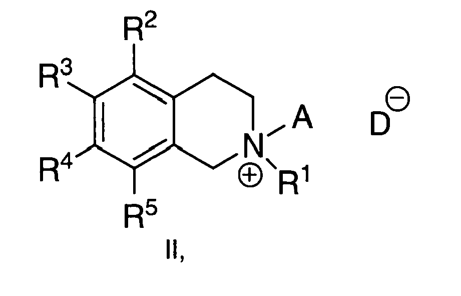 wherein
R¹, R², R³, R⁴ and R⁵ have the above defined meaning except for R¹ does not represent a -C(=O)-OR³⁷ moiety,
A represents a hydrogen atom or a radical selected from the group consisting of methyl, ethyl, n-propyl, isopropyl, n-butyl, isobutyl, 2-butyl, tert-butyl and n-pentyl;
D represents an anion selected from the group consisting of chloride, bromide, iodide, fluoride, hydrogensulfate, nitrate, dihydrogenphosphate, thiocyanate, cyanate, acrylate, fumarate, citrate, glutarate, succinate, maleate, tartrate, phosphate, 2-oxo-glutarate, formate, acetate, propionate, lactate, gluconate, benzoate or naphthoate whereby said benzoate or naphthoate may be substituted with 1, 2, 3, 4 or 5 substituents independently selected from the group consisting of F, Cl, Br, -OH, -O-CH₃ and -O-C₂H₅ , pyruvate, ascorbate, glycolate, nicotinate, phenylacetate, 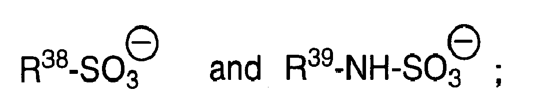 and R³⁸ and R³⁹, independently of one another, in each case represent a radical selected from the group consisting of -CF₃, -C₂F₅, methyl, ethyl, n-propyl, isopropyl, n-butyl, isobutyl, sec-butyl, tert-butyl, n-pentyl, 2-pentyl, 3-pentyl, neo-pentyl, n-hexyl, 2-hexyl, 3-hexyl, n-heptyl, 2-heptyl, 3-heptyl, 4-heptyl, n-octyl, 2-octyl, 3-octyl, 4-octyl, 2-(6-methyl)-heptyl, 2-(5-methyl)-heptyl, 2-(5-methyl)-hexyl, 2-(4-methyl)-hexyl, 2-(7-methyl)-octyl; 2-(6-methyl)-octyl, n-nonyl, n-decyl, n-undecyl, n-dodecyl, n-tridecyl and n-tetradecyl;
a radical selected from the group consisting of phenyl, pyridinyl, pyrazolyl, benzimidazolyl, isoquinolinyl and naphthyl, which may be substituted with 1, 2, 3, 4 or 5 substituent(s) independently selected from the group consisting of F, Cl, Br, -CF₃, methyl, ethyl, n-propyl, isopropyl, n-butyl, isobutyl, 2-butyl, tert-butyl, n-pentyl, 2-pentyl, n-hexyl, -O-CH₃, -O-C₂H₅, -O-CH₂-CH₂-CH₃, -O-CH(CH₃)₂, -O-CH₂-CH₂-CH₂-CH₃, -O-C(CH₃)₃, -OH, -NO₂, -NH₂, phenyl and -SO₃H;
or a radical selected from the group consisting of cyclopropyl, cyclobutyl, cyclopentyl, cyclohexyl, cycloheptyl, cyclooctyl, cyclononyl, cyclodecyl, cycloundecyl, cyclododecyl, cyclotridecyl, cyclotetradecyl, cyclopentenyl, cyclohexenyl, cycloheptenyl, cyclooctenyl and bicyclo[2.2.1]heptyl, which may be bonded via a -(CH₂)-, -(CH₂)-(CH₂)- or -(CH₂)-(CH₂)-(CH₂)-group.

Preferred is a medicament comprising at least one substituted tetrahydroisoquinoline compound in form of its salt as defined in general formula II, wherein
A represents a hydrogen atom or a radical selected from the group consisting of methyl, ethyl, n-propyl, n-butyl and n-pentyl;
and D represents an anion selected from the group consisting of chloride, bromide, iodide, fluoride, hydrogensulfate, nitrate, dihydrogenphosphate, thiocyanate, cyanate, acrylate, methanesulfonate, ethanesulfonate, toluenesulfonate, benzenesulfonate, (2,5)-dihydroxy-benzenesulfonate, naphthalene-2-sulfonate, 5-sulfonapthalene-1-sulfonate, cyclamate, dodecane-1-sulfonate and (7,7)-dimethyl-2-oxobicyclo[2.2.1]- -hept-1-yl-methanesulfonate;
and the other substituents have any of the above defined meanings, optionally in form of one of its stereoisomers, preferably enantiomers or diasteromers, a racemate or in form of a mixture of at least two of its stereoisomers, preferably enantiomers and/or diastereomers, in any mixing ratio, or a corresponding solvate thereof.

Also preferred is a medicament comprising at least one substituted tetrahydroisoquinoline compound of general formula I and/or II, wherein
R¹ represents a hydrogen atom;
a linear or branched C₁₋₁₀ alkyl radical, C₂₋₁₀ alkenyl radical or C₂₋₁₀ alkinyl radical;
a C₃₋₉ cycloalkyl radical or C₄₋₉ cycloalkenyl radical, which may be unsubstituted or optionally substituted with 1, 2, 3, 4 or 5 substituent(s) independently selected from the group consisting of oxo (=O), thioxo (=S), C₁₋₅-alkyl, -O-C₁₋₅-alkyl, -S-C₁₋₅-alkyl, -C(=O)-C₁₋₅-alkyl, -O-C(=O)-C₁₋₅-alkyl, F, Cl, Br, I, -CN, -CF₃, -OCF₃, -SCF₃, -OH, -SH, -NH₂, - NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl and which may be condensed with an unsubstituted or at least mono-substituted saturated, unsaturated or aromatic mono- or bicyclic ring system and which may be bonded via a linear or branched C₁₋₆ alkylene, C₂₋₆ alkenylene or C₂₋₆ alkinylene group;
or in general formula I R¹ additionally represents a -C(=O)-OR³⁷ moiety;
R², R³, R⁴ and R⁵, independently of one another, each represent a hydrogen atom; F, Cl, Br, I, -NO₂; -NH₂; -SH; -OH; -CN; -C(=O)-OH; -C(=O)-H; -S(=O)₂-OH; -C(=O)-NH₂; -S(=O)₂-NH₂; -C(=O)-R⁶; -S(=O)-R⁷; -S(=O)₂-R⁷; -OR⁸; -SR⁹; -C(=O)-OR¹⁰; -N(R¹¹)-S(=O)₂-R¹²; -NR¹³R¹⁴; -NH-R¹⁵; -C(=O)-NR¹⁶R¹⁷; C(=O)-NHR¹⁸;
a linear or branched C₁₋₁₀ alkyl radical, C₂₋₁₀ alkenyl radical or C₂₋₁₀ alkinyl radical;
a C₃₋₉ cycloalkyl radical or C₄₋₉ cycloalkenyl radical, which may be bonded via a linear or branched C₁₋₆ alkylene, C₂₋₆ alkenylene or C₂₋₆ alkinylene group and which may be condensed with an unsubstituted or at least mono-substituted saturated, unsaturated or aromatic mono- or bicyclic ring system;
or an unsubstituted or at least mono-substituted 6-, 10- or 14-membered aryl or 5-, 6-, 8-, 9-, 10-, 11-, 12-, 13- or 14-membered heteroaryl radical, which may be bonded via a linear or branched C₁₋₆ alkylene, C₂₋₆ alkenylene or C₂₋₆ alkinylene group and which may be condensed with an unsubstituted or at least mono-substituted saturated or unsaturated, but not aromatic, mono- or bicyclic ring system;
with the proviso that at least one of the substituents R², R³, R⁴ and R⁵ represents a - N(R¹¹)-S(=O)₂-R¹² moiety;
R⁶, R⁷, R⁸, R⁹, R¹⁰, R¹³, R¹⁴, R¹⁵, R¹⁶, R¹⁷ and R¹⁸, independently of one another, each represent a linear or branched C₁₋₁₀ alkyl radical, C₂₋₁₀ alkenyl radical or C₂₋₁₀ alkinyl radical;
a C₃₋₉ cycloalkyl radical or C₄₋₉ cycloalkenyl radical, which may be bonded via a linear or branched C₁₋₆ alkylene, C₂₋₆ alkenylene or C₂₋₆ alkinylene group and which may be condensed with an unsubstituted or at least mono-substituted saturated, unsaturated or aromatic mono- or bicyclic ring system;
or an unsubstituted or at least mono-substituted 6-, 10- or 14-membered aryl or 5-, 6-, 8-, 9-, 10-, 11-, 12-, 13- or 14-membered heteroaryl radical, which may be bonded via a linear or branched C₁₋₆ alkylene, C₂₋₆ alkenylene or C₂₋₆ alkinylene group and which may be condensed with an unsubstituted or at least mono-substituted saturated or unsaturated, but not aromatic, mono- or bicyclic ring system;
R¹¹ represents a hydrogen atom, -S(=O)₂-R¹² or a linear or branched, saturated or unsaturated, unsubstituted or at least mono-substituted C₁₋₆ alkylene, C₂₋₆ alkenylene or C₂₋₆ alkinylene group;
R¹² represents a phenyl radical of general formula (A), 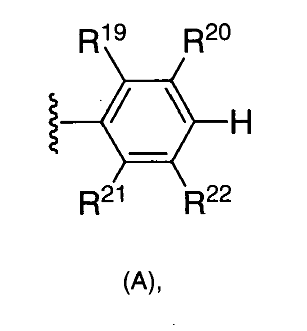 wherein
R¹⁹, R²⁰, R²¹ and R²², independently of one another, each represent a hydrogen atom; F, Cl, Br, l, -NO₂; -NH₂; -SH; -OH; -CN; -C(=O)-OH; -C(=O)-H; -S(=O)₂-OH; -C(=O)-NH₂; - S(=O)₂-NH₂; -C(=O)-R²³; -S(=O)-R²⁴; -S(=O)₂-R²⁴; -OR²⁵; -SR²⁶; -C(=O)-OR²⁷; -N(R²⁸)-S(=O)₂-R²⁹; -NH-S(=O)₂-R³⁰; -NR³¹R³²; -NH-R³³; -C(=O)-NHR³⁴; -C(=O)-NR³⁵R³⁶; or a linear or branched C₁₋₁₀ alkyl radical, C₂₋₁₀ alkenyl radical or C₂₋₁₀ alkinyl radical; or a C₃₋₉ cycloalkyl radical;
with the proviso that at least one of the substituents R¹⁹, R²⁰, R²¹ and R²² is unlike hydrogen;
an unsubstituted or at least mono-substituted 10-membered aryl radical;
a monocyclic 5- or 6-membered heteroaryl radical, which may be unsubstituted or optionally substituted with 1, 2, 3, 4 or 5 substituent(s) independently selected from the group consisting of F, Cl, Br, I, -NO₂; -NH₂; -SH; -OH; -CN; -C(=O)-OH; -C(=O)-H; - S(=O)₂-OH; -C(=O)-NH₂; -S(=O)₂-NH₂; -C(=O)-R²³; -S(=O)-R²⁴; -S(=O)₂-R²⁴; -OR²⁵; - SR²⁶; -C(=O)-OR²⁷; -N(R²⁸)-S(=O)₂-R²⁹; -NH-S(=O)₂-R³⁰; -NR³¹R³²; -NH-R³³; -C(=O)-NHR^{34;} -C(=O)-NR³⁵R³⁶; a linear or branched C₁₋₁₀ alkyl radical, C₂₋₁₀ alkenyl radical or C₂₋₁₀ alkinyl radical; and a C₃₋₉ cycloalkyl radical;
an unsubstituted or at least mono-substituted monocyclic 5- or 6-membered heteroaryl radical, which is condensed with an unsubstituted or at least mono-substituted saturated or unsaturated, but not aromatic, mono- or bicyclic ring system;
an unsubstituted or at least mono-substituted bi- or tricyclic 8-, 9-, 10-, 11-, 12-, 13- or 14-membered heteroaryl radical, which may be condensed with an unsubstituted or at least mono-substituted saturated or unsaturated, but not aromatic, mono- or bicyclic ring system;
or a C₃₋₉ cycloalkyl radical or C₄₋₉ cycloalkenyl radical, which may be condensed with an unsubstituted or at least mono-substituted saturated, unsaturated or aromatic mono- or bicyclic ring system;
R²³, R²⁷, R²⁸, R²⁹ and R³⁰, independently of one another, each represent a linear or branched C₁₋₁₀ alkyl radical, C₂₋₁₀ alkenyl radical or C₂₋₁₀ alkinyl radical;
a C₃₋₉ cycloalkyl radical or C₄₋₉ cycloalkenyl radical, which may be bonded via a linear or branched C₁₋₆ alkylene, C₂₋₆ alkenylene or C₂₋₆ alkinylene group and which may be condensed with an unsubstituted or at least mono-substituted saturated, unsaturated or aromatic mono- or bicyclic ring system;
or an unsubstituted or at least mono-substituted 6-, 10- or 14-membered aryl or 5-, 6-, 8-, 9-, 10-, 11-, 12-, 13- or 14-membered heteroaryl radical, which may be bonded via a linear or branched C₁₋₆ alkylene, C₂₋₆ alkenylene or C₂₋₆ alkinylene group and which may be condensed with an unsubstituted or at least mono-substituted saturated or unsaturated, but not aromatic, mono- or bicyclic ring system;
R²⁴, R²⁶, R³¹, R³² and R³³, each represent a linear or branched C₁₋₁₀ alkyl radical, C₂₋₁₀ alkenyl radical or C₂₋₁₀ alkinyl radical;
a C₃₋₉ cycloalkyl radical or C₄₋₉ cycloalkenyl radical, which may be bonded via a linear or branched C₁₋₆ alkylene, C₂₋₆ alkenylene or C₂₋₆ alkinylene group and which may be condensed with an unsubstituted or at least mono-substituted saturated, unsaturated or aromatic mono- or bicyclic ring system;
or an unsubstituted or at least mono-substituted 6-, 10- or 14-membered aryl or 5-, 6-, 8-, 9-, 10-, 11-, 12-, 13- or 14-membered heteroaryl radical, which may be condensed with an unsubstituted or at least mono-substituted saturated or unsaturated, but not aromatic, mono- or bicyclic ring system;
R²⁵, R³⁴, R³⁵ and R³⁶, represents a linear or branched C₁₋₁₀ alkyl radical, C₂₋₁₀ alkenyl radical or C₂₋₁₀ alkinyl radical;
and R³⁷ represents a linear or branched C₁₋₁₀ alkyl radical, C₂₋₁₀ alkenyl radical or C₂₋₁₀ alkinyl radical;
a C₃₋₉ cycloalkyl radical or C₄₋₉ cycloalkenyl radical, which may be bonded via a linear or branched C₁₋₆alkylene, C₂₋₆ alkenylene or C₂₋₆ alkinylene group and which may be condensed with an unsubstituted or at least mono-substituted saturated, unsaturated or aromatic mono- or bicyclic ring system;
or an unsubstituted or at least mono-substituted 6-, 10- or 14-membered aryl or 5-, 6-, 8-, 9-, 10-, 11-, 12-, 13- or 14-membered heteroaryl radical, which may be bonded via a linear or branched C₁₋₆ alkylene, C₂₋₆ alkenylene or C₂₋₆ alkinylene group and which may be condensed with an unsubstituted or at least mono-substituted saturated or unsaturated, but not aromatic, mono- or bicyclic ring system;
whereby
the aforementioned C₁₋₁₀ alkyl radical, C₂₋₁₀ alkenyl radical or C₂₋₁₀ alkinyl radicals may in each case be unsubstituted or optionally substituted with 1, 2, 3, 4 or 5 substituent(s) independently selected from the group consisting of F, CI, Br, I, -CN, -CF₃, -OCF₃, - SCF₃, -OH, -SH, -NH₂, -O-C₁₋₅-alkyl, -S-C₁₋₅-alkyl, -NH(C₁₋₅-alkyl) and -N(C₁₋₅-alkyl)₂;
if not defined otherwise, the aforementioned C₃₋₉ cycloalkyl radicals and C₄₋₉ cycloalkenyl radicals may in each case be unsubstituted or optionally substituted with 1, 2, 3, 4 or 5 substituent(s) independently selected from the group consisting of oxo (=O), thioxo (=S), C₁₋₅-alkyl, -O-C₁₋₅-alkyl, -S-C₁₋₅-alkyl, -C(=O)-OH, -C(=O)-C₁₋₅-alkyl, -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), - C(=O)-N(C₁₋₅-alkyl)₂, -S(=O)₂-C₁₋₅-alkyl, -S(=O)₂-phenyl, phenyl, phenoxy and benzyl;
the aforementioned C₃₋₉ cycloalkyl radicals and C₄₋₉ cycloalkenyl radicals in each case may optionally contain 1, 2 or 3 heteroatom(s) independently selected from the group consisting of nitrogen, oxygen and sulfur as ring member(s);
the aforementioned C₁₋₆ alkylene, C₂₋₆ alkenylene or C₂₋₆ alkinylene groups may in each case be unsubstituted or substituted with 1, 2 or 3 substituent(s) independently selected from the group consisting of F, Cl, Br, I, -CN, -CF₃, -OCF₃, -SCF₃, -OH, -SH, -NH₂, -O-C₁₋₅-alkyl, -S-C₁₋₅-alkyl, -NH(C₁₋₅-alkyl) and -N(C₁₋₅-alkyl)₂;
the rings of the aforementioned ring system are in each case independently of one another 5-, 6- or 7-membered and may in each case independently of one another optionally contain 1, 2 or 3 heteroatom(s) independently selected from the group consisting of nitrogen, oxygen and sulfur;
and the rings of the ring system may in each case be unsubstituted or substituted with 1, 2 or 3 substituent(s) independently selected from the group consisting of oxo (=O), thioxo (=S), C₁₋₅-alkyl, -O-C₁₋₅-alkyl, -S-C₁₋₅-alkyl, -C(=O)-OH, -C(=O)-C₁₋₅-alkyl, -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH₂, - NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl;
if not defined otherwise, the 6-, 10- or 14-membered aryl or 5-, 6-, 8-, 9-, 10-, 11-, 12-, 13- or 14-membered heteroaryl radicals may be unsubstituted or optionally substituted with 1, 2, 3, 4 or 5 substituent(s) independently selected from the group consisting of C₁-₅-alkyl, -O-C₁₋₅-alkyl, -S-C₁₋₅-alkyl, -C(=O)-OH, -C(=O)-C₁₋₅-alkyl, -C(=O)-O-C₁₋₅-alkyl, - O-C(=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), - C(=O)-N(C₁₋₅-alkyl)₂, -S(=O)₂-C₁₋₅-alkyl, -S(=O)₂-phenyl, phenyl, phenoxy and benzyl;
and the aforementioned 5-, 6-, 8-, 9-, 10-, 11-, 12-, 13- or 14-membered heteroaryl radicals in each case optionally contain 1, 2, 3 or 4 heteroatom(s) independently selected from the group consisting of nitrogen, oxygen and sulfur as ring member(s);
optionally in form of one of its stereoisomers, preferably enantiomers or diasteromers, a racemate or in form of a mixture of at least two of its stereoisomers, preferably enantiomers and/or diastereomers, in any mixing ratio, or a salt thereof, or a corresponding solvate thereof.

Also preferred is a medicament comprising at least one substituted tetrahydroisoquinoline compound of general formula I and/or II, wherein
R¹ represents a hydrogen atom; a radical selected from the group consisting of methyl, ethyl, n-propyl, isopropyl, n-butyl, sec-butyl, isobutyl, tert-butyl, n-pentyl, n-hexyl, -CF₃, -CFH₂, -CF₂H, -CH₂-CF₃, -CF₂-CF₃, -CH₂-CN, -CH₂-CH₂-CN, -CH₂-O-CF₃, - CH₂-S-CF₃, -CH₂-OH, -CH₂-CH₂-OH, -CH₂-SH, -CH₂-CH₂-SH, -CH₂-NH₂, -CH₂-NH-CH₃, -CH₂-N(CH₃)₂, -CH₂-N(C₂H₅)₂, -CH₂-NH-C₂H₅, -CH₂-CH₂-NH₂, -CH₂-CH₂-NH-CH₃, -CH₂-CH₂-N(CH₃)₂, -CH₂-CH₂-N(C₂H₅)₂, -CH₂-CH₂-NH-C₂H₅, -CH₂-CH₂-CH₂-NH-CH₃, -CH₂-CH₂-CH₂-N(CH₃)₂, -CH₂-CH₂-CH₂-N(C₂H₅)₂ and -CH₂-CH₂-CH₂-NH-C₂H₅; a (hetero)cycloaliphatic radical selected from the group consisting of cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl and azepanyl, which may be bonded via a -(CH₂)₁, _{2 or 3}- group and which may be unsubstituted or optionally substituted with 1, 2, 3, 4 or 5 substituent(s) independently selected from the group consisting of oxo (=O), thioxo (=S), methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, - NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃ or in general formula I R¹ additionally represents a -C(=O)-OR³⁷ moiety;
and the other substituents have any of the above defined meanings, optionally in form of one of its stereoisomers, preferably enantiomers or diasteromers, a racemate or in form of a mixture of at least two of its stereoisomers, preferably enantiomers and/or diastereomers, in any mixing ratio, or a corresponding solvate thereof.

A medicament comprising at least one substituted tetrahydroisoquinoline compound of general formula I and/or II is preferred as well, wherein
R², R³, R⁴ and R⁵, independently of one another, each represent a hydrogen atom; F, Cl, Br, I, -NO₂; -NH₂; -SH; -OH; -CN; -C(=O)-OH; -C(=O)-H; -S(=O)₂-OH; -C(=O)-NH₂; -S(=O)₂-NH₂; -C(=O)-R⁶; -S(=O)-R⁷; -S(=O)₂-R⁷; -OR⁸; -SR⁹; -C(=O)-OR¹⁰; -N(R¹¹)-S(=O)₂-R¹²; -NR¹³R¹⁴; -NH-R¹⁵; -C(=O)-NR¹⁶R¹⁷; C(=O)-NHR¹⁸; a radical selected from the group consisting of methyl, ethyl, n-propyl, isopropyl, n-butyl, sec-butyl, isobutyl, tert-butyl, n-pentyl, n-hexyl, vinyl, allyl, ethinyl, -CF₃, -CFH₂, -CF₂H, -CH₂-CF₃, -CF₂-CF₃, - CH₂-CN, -CH₂-CH₂-CN, -CH₂-O-CF₃, -CH₂-S-CF₃, -CH₂-OH, -CH₂-CH₂-OH, -CH₂-SH, - CH₂-CH₂-SH, -CH₂-NH₂, -CH₂-NH-CH₃, -CH₂-N(CH₃)₂, -CH₂-N(C₂H₅)₂ and -CH₂-NH-C₂H₅; or a radical selected from the group consisting of cyclopropyl, cyclobutyl, cyclopentyl, cyclohexyl, phenyl, benzyl, phenethyl, pyridinyl, pyrrolyl, furanyl and thiophenyl, which may be unsubstituted or optionally substituted with 1, 2, 3, 4 or 5 substituent(s) independently selected from the group consisting of oxo (=O), thioxo (=S), methyl, ethyl, n-propyl, isopropyl, n-butyl, tert-butyl, sec-butyl, isobutyl, n-pentyl, -O-CH₃, -O-C₂H₅, -S-CH₃, -S-C₂H₅, F, Cl, Br, -CN, -OH, -SH, -NO₂,-CHO, -CF₂H, -CFH₂ and - C(=O)-NH₂;
and the other substituents have any of the above defined meanings, optionally in form of one of its stereoisomers, preferably enantiomers or diasteromers, a racemate or in form of a mixture of at least two of its stereoisomers, preferably enantiomers and/or diastereomers, in any mixing ratio, or a corresponding solvate thereof.

Also preferred is a medicament comprising at least one substituted tetrahydroisoquinoline compound of general formula I and/or II, wherein R² represents - N(R¹¹)-S(=O)₂-R¹²;
and the other substituents have any of the above defined meanings, optionally in form of one of its stereoisomers, preferably enantiomers or diasteromers, a racemate or in form of a mixture of at least two of its stereoisomers, preferably enantiomers and/or diastereomers, in any mixing ratio, or a corresponding solvate thereof.

Also preferred is a medicament comprising at least one substituted tetrahydroisoquinoline compound of general formula I and/or II, wherein R³ represents - N(R¹¹)-S(=O)₂-R¹²;
and the other substituents have any of the above defined meanings, optionally in form of one of its stereoisomers, preferably enantiomers or diasteromers, a racemate or in form of a mixture of at least two of its stereoisomers, preferably enantiomers and/or diastereomers, in any mixing ratio, or a corresponding solvate thereof.

Also preferred is a medicament comprising at least one substituted tetrahydroisoquinoline compound of general formula I and/or II, wherein R⁴ represents - N(R¹¹)-S(=O)₂₋R¹²;
and the other substituents have any of the above defined meanings, optionally in form of one of its stereoisomers, preferably enantiomers or diasteromers, a racemate or in form of a mixture of at least two of its stereoisomers, preferably enantiomers and/or diastereomers, in any mixing ratio, or a corresponding solvate thereof.

Also preferred is a medicament comprising at least one substituted tetrahydroisoquinoline compound of general formula I and/or II, wherein R⁵ represents - N(R¹¹)-S(=O)₂-R¹²;
and the other substituents have any of the above defined meanings, optionally in form of one of its stereoisomers, preferably enantiomers or diasteromers, a racemate or in form of a mixture of at least two of its stereoisomers, preferably enantiomers and/or diastereomers, in any mixing ratio, or a corresponding solvate thereof.

A medicament comprising at least one substituted tetrahydroisoquinoline compound of general formula I and/or II is preferred as well, wherein
R⁶, R⁷, R⁸, R⁹, R¹⁰, R¹³, R¹⁴, R¹⁵, R¹⁶, R¹⁷ and R¹⁸, independently of one another, each represent a radical selected from the group consisting of methyl, ethyl, n-propyl, isopropyl, n-butyl, sec-butyl, isobutyl, tert-butyl, n-pentyl, n-hexyl, vinyl, allyl, ethinyl, - CF₃, -CFH₂, -CF₂H, -CH₂-CF₃, -CF₂-CF₃, -CH₂-CN, -CH₂-CH₂-CN, -CH₂-O-CF₃, -CH₂-S-CF₃, -CH₂-OH, -CH₂-CH₂-OH, -CH₂-SH, -CH₂-CH₂-SH, -CH₂-NH₂, -CH₂-NH-CH₃, -CH₂-N(CH₃)₂, -CH₂-N(C₂H₅)₂, -CH₂-NH-C₂H₅, -CH₂-CH₂-NH₂, -CH₂-CH₂-NH-CH₃, -CH₂-CH₂-N(CH₃)₂, -CH₂-CH₂-N(C₂H₅)₂, -CH₂-CH₂-NH-C₂H₅, -CH₂-CH₂-CH₂-NH-CH₃, -CH₂-CH₂-CH₂-N(CH₃)₂, -CH₂-CH₂-CH₂-N(C₂H₅)₂ and -CH₂-CH₂-CH₂-NH-C₂H₅; a (hetero)cycloaliphatic radical selected from the group consisting of cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl and azepanyl, which may be bonded via a -(CH₂)₁, _{2 or 3}- group and which may be unsubstituted or optionally substituted with 1, 2, 3, 4 or 5 substituent(s) independently selected from the group consisting of oxo (=O), thioxo (=S), methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, - NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃; or an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, which may be bonded via a -(CH₂)_{1,2 or 3}-group and which may be unsubstituted or optionally substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, - OCF₃, -SCF₃, -OH, -SH, -NH₂, -NH-CH₃, -NH-C₂H₅, -NH-CH₂-CH₂-CH₃, -NH-CH(CH₃)₂, - NH-C(CH₃)₃, -N(CH₃)₂, -N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃;
and the other substituents have any of the above defined meanings, optionally in form of one of its stereoisomers, preferably enantiomers or diasteromers, a racemate or in form of a mixture of at least two of its stereoisomers, preferably enantiomers and/or diastereomers, in any mixing ratio, or a corresponding solvate thereof.

Also preferred is a medicament comprising at least one substituted tetrahydroisoquinoline compound of general formula I and/or II, wherein
R¹¹ represents a hydrogen atom, -S(=O)₂-R¹² or an alkyl radical selected from the group consisting of methyl, ethyl, n-propyl, isopropyl, n-butyl, sec-butyl, isobutyl and tert-butyl;
and the other substituents have any of the above defined meanings, optionally in form of one of its stereoisomers, preferably enantiomers or diasteromers, a racemate or in form of a mixture of at least two of its stereoisomers, preferably enantiomers and/or diastereomers, in any mixing ratio, or a corresponding solvate thereof.

A medicament comprising at least one substituted tetrahydroisoquinoline compound of general formula I and/or II is preferred, wherein
R¹² represents a phenyl radical of general formula (A), 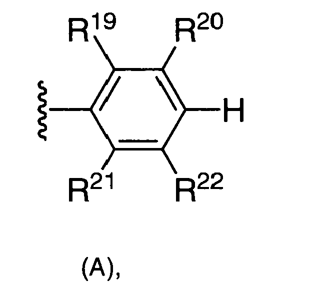 wherein
R¹⁹, R²⁰, R²¹ and R²², independently of one another, each represent a hydrogen atom; F, Cl, Br, l, -NO₂; -NH₂; -SH; -OH; -CN; -C(=O)-OH; -C(=O)-H; -S(=O)₂-OH; -C(=O)-NH₂; - S(=O)₂-NH₂; -C(=O)-R²³; -S(=O)-R²⁴; -S(=O)₂-R²⁴; -OR²⁵; -SR²⁶; -C(=O)-OR²⁷; -N(R²⁸)-S(-O)₂-R²⁹; -NH-S(=O)₂-R³⁰; -NR³¹R³²; -NH-R³³; -C(=O)-NHR³⁴; -C(=O)-NR³⁵R³⁶; methyl, ethyl, n-propyl, isopropyl, n-butyl, isobutyl, sec-butyl, tert-butyl, -CF₃, -CF₂H, - CFH₂, -CH₂-CF₃, -CF₂-CF₃, cyclopropyl, cyclobutyl, cyclopentyl and cyclohexyl;
with the proviso that at least one of the substituents R¹⁹, R²⁰, R²¹ and R²² is unlike hydrogen;
a radical selected from the group consisting of naphthyl, [1,3]-benzodioxolyl, [1,4]-benzodioxanyl, [1,2,3,4]-tetrahydronaphthyl, (2,3)-dihydro-1 H-cyclopenta[b]indolyl, [1,2,3,4]-tetrahydroquinolinyl, [1,2,3,4]-tetrahydroisoquinolinyl, [1,2,3,4]-tetrahydroquinazolinyl, [3,4]-dihydro-2H-benzo[1,4]oxazinyl, indolyl, isoindolyl, quinolinyl, isoquinolinyl, benzo[b]furanyl, benzo[b]thiophenyl, benzo[2,1,3]thiadiazolyl, [1,2,3]-benzothiadiazolyl, [2,1,3]-benzoxadiazolyl, [1,2,3]-benzoxadiazolyl, benzoxazolyl, benzothiazolyl, benzisoxazolyl, benzisothiazolyl, imidazo[2,1-b]thiazolyl, 2H-chromenyl, indazolyl, quinazolinyl, cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl and azepanyl, which may be unsubstituted or optionally substituted with 1, 2, 3, 4 or 5 substituent(s) independently selected from the group consisting of oxo (=O), thioxo (=S), methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, -SH, -NH₂, -NH-CH₃, -NH-C₂H₅, - NH-CH₂-CH₂-CH₃, -NH-CH(CH₃)₂, -NH-C(CH₃)₃, -N(CH₃)₂, -N(C₂H₅)₂, -NO₂, -CHO, - CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃;
or a radical selected from the group consisting of pyridinyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, pyrimidinyl, pyrazinyl and pyranyl, which may be unsubstituted or optionally substituted with 1, 2, 3, 4 or 5 substituent(s) independently selected from the group consisting of F, Cl, Br, 1, -NO₂; -NH₂; -SH; -OH; - CN; -C(=O)-OH; -C(=O)-H; -S(=O)₂-OH; -C(=O)-NH₂; -S(=O)₂-NH₂; -C(=O)-R²³; -S(=O)-R²⁴; -S(=O)₂-R²⁴; -OR²⁵; -SR²⁶; -C(=O)-OR²⁷; -N(R²⁸)-S(=O)₂-R²⁹; -NH-S(=O)₂-R³⁰; - NR³¹R³²; -NH-R³³; -C(=O)-NHR³⁴; -C(=O)-NR³⁵R³⁶; methyl, ethyl, n-propyl, isopropyl, n-butyl, isobutyl, sec-butyl, tert-butyl, -CF₃, -CF₂H, -CFH₂, -CH₂-CF₃, -CF₂-CF₃, cyclopropyl, cyclobutyl, cyclopentyl and cyclohexyl;
and the other substituents have any of the above defined meanings, optionally in form of one of its stereoisomers, preferably enantiomers or diasteromers, a racemate or in form of a mixture of at least two of its stereoisomers, preferably enantiomers and/or diastereomers, in any mixing ratio, or a corresponding solvate thereof.

Also preferred is a medicament comprising at least one substituted tetrahydroisoquinoline compound of general formula I and/or II, wherein
R²³, R²⁷, R²⁸, R²⁹ and R³⁰, independently of one another, each represent a radical selected from the group consisting of methyl, ethyl, n-propyl, isopropyl, n-butyl, sec-butyl, isobutyl, tert-butyl, n-pentyl, n-hexyl, vinyl, allyl, ethinyl, -CF₃, -CFH₂, -CF₂H, -CH₂-CF₃, -CF₂-CF₃, -CH₂-CN, -CH₂-CH₂-CN, -CH₂-O-CF₃, -CH₂-S-CF₃, -CH₂-OH, -CH₂-CH₂-OH, -CH₂-SH, -CH₂-CH₂-SH, -CH₂-NH₂, -CH₂-NH-CH₃, -CH₂-N(CH₃)₂, -CH₂-N(C₂H₅)₂, - CH₂-NH-C₂H₅, -CH₂-CH₂-NH₂, -CH₂-CH₂-NH-CH₃, -CH₂-CH₂-N(CH₃)₂, -CH₂-CH₂-N(C₂H₅)₂, -CH₂-CH₂-NH-C₂H₅, -CH₂-CH₂-CH₂-NH-CH₃, -CH₂-CH₂-CH₂-N(CH₃)₂, -CH₂-CH₂-CH₂-N(C₂H₅)₂ and -CH₂-CH₂-CH₂-NH-C₂H₅; a (hetero)cycloaliphatic radical selected from the group consisting of cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl and azepanyl, which may be bonded via a -(CH₂)_{1, 2} or ₃- group and which may be unsubstituted or optionally substituted with 1, 2, 3, 4 or 5 substituent(s) independently selected from the group consisting of oxo (=O), thioxo (=S), methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-CH₃, - C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂, -CHO, -CF₂H, -CFH₂, -C(=O)-NH₂, - C(=O)-NH-CH₃, -C(=O)-NH-C₂H₅, -C(=O)-N(CH₃)₂, -C(=O)-N(C₂H₅)₂ and -S(=O)₂-CH₃; or an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, which may be bonded via a -(CH₂)₁, _{2 or 3}-group and which may be unsubstituted or optionally substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, - N(CH₃)₂, -N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃;
and the other substituents have any of the above defined meanings, optionally in form of one of its stereoisomers, preferably enantiomers or diasteromers, a racemate or in form of a mixture of at least two of its stereoisomers, preferably enantiomers and/or diastereomers, in any mixing ratio, or a corresponding solvate thereof.

Also preferred is a medicament comprising at least one substituted tetrahydroisoquinoline compound of general formula I and/or II, wherein
R²⁴, R²⁶, R³¹, R³² and R³³, independently of one another, each represent a radical selected from the group consisting of methyl, ethyl, n-propyl, isopropyl, n-butyl, sec-butyl, isobutyl, tert-butyl, n-pentyl, n-hexyl, vinyl, allyl, ethinyl, -CF₃, -CFH₂, -CF₂H, -CH₂-CF₃, -CF₂-CF₃, -CH₂-CN, -CH₂-CH₂-CN, -CH₂-O-CF₃, -CH₂-S-CF₃, -CH₂-OH, -CH₂-CH₂-OH, -CH₂-SH, -CH₂-CH₂-SH, -CH₂-NH₂, -CH₂-NH-CH₃, -CH₂-N(CH₃)₂, -CH₂-N(C₂H₅)₂, - CH₂-NH-C₂H₅, -CH₂-CH₂-NH₂, -CH₂-CH₂-NH-CH₃, -CH₂-CH₂-N(CH₃)₂, -CH₂-CH₂-N(C₂H₅)₂, -CH₂-CH₂-NH-C₂H₅, -CH₂-CH₂-CH₂-NH-CH₃, -CH₂-CH₂-CH₂-N(CH₃)₂, -CH₂-CH₂-CH₂-N(C₂H₅)₂ and -CH₂-CH₂-CH₂-NH-C₂H₅; a (hetero)cycloaliphatic radical selected from the group consisting of cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl and azepanyl, which may be unsubstituted or optionally substituted with 1, 2, 3, 4 or 5 substituent(s) independently selected from the group consisting of oxo (=O), thioxo (=S), methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, - NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃; or an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, which may be unsubstituted or optionally substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, - C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, - NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃;
and the other substituents have any of the above defined meanings, optionally in form of one of its stereoisomers, preferably enantiomers or diasteromers, a racemate or in form of a mixture of at least two of its stereoisomers, preferably enantiomers and/or diastereomers, in any mixing ratio, or a corresponding solvate thereof.

Also preferred is a medicament comprising at least one substituted tetrahydroisoquinoline compound of general formula I and/or II, wherein
R²⁵, R³⁴, R³⁵ and R³⁶, independently of one another, each represent a radical selected from the group consisting of methyl, ethyl, n-propyl, isopropyl, n-butyl, sec-butyl, isobutyl, tert-butyl, n-pentyl, n-hexyl, vinyl, allyl, ethinyl, -CF₃, -CFH₂, -CF₂H, -CH₂-CF₃, - CF₂-CF₃, -CH₂-CN, -CH₂-CH₂-CN, -CH₂-O-CF₃, -CH₂-S-CF₃, -CH₂-OH, -CH₂-CH₂-OH, - CH₂-SH, -CH₂-CH₂-SH, -CH₂-NH₂, -CH₂-NH-CH₃, -CH₂-N(CH₃)₂, -CH₂-N(C₂H₅)₂, -CH₂-NH-C₂H₅, -CH₂-CH₂-NH₂, -CH₂-CH₂-NH-CH₃, -CH₂-CH₂-N(CH₃)₂, -CH₂-CH₂-N(C₂H₅)₂, - CH₂-CH₂-NH-C₂H₅, -CH₂-CH₂-CH₂-NH-CH₃, -CH₂-CH₂-CH₂-N(CH₃)₂, -CH₂-CH₂-CH₂-N(C₂H₅)₂ and -CH₂-CH₂-CH₂-NH-C₂H₅;
and the other substituents have any of the above defined meanings, optionally in form of one of its stereoisomers, preferably enantiomers or diasteromers, a racemate or in form of a mixture of at least two of its stereoisomers, preferably enantiomers and/or diastereomers, in any mixing ratio, or a corresponding solvate thereof.

Also preferred is a medicament comprising at least one substituted tetrahydroisoquinoline compound of general formula I and/or II, wherein
R³⁷ represents a radical selected from the group consisting of methyl, ethyl, n-propyl, isopropyl, n-butyl, sec-butyl, isobutyl, tert-butyl, n-pentyl, n-hexyl, vinyl, allyl, ethinyl, -CF₃, -CFH₂, -CF₂H, -CH₂-CF₃, -CF₂-CF₃, -CH₂-CN, -CH₂-CH₂-CN, -CH₂-O-CF₃, -CH₂-S-CF₃, -CH₂-OH, -CH₂-CH₂-OH, -CH₂-SH, -CH₂-CH₂-SH, -CH₂-NH₂, -CH₂-NH-CH₃, -CH₂-N(CH₃)₂, -CH₂-N(C₂H₅)₂, -CH₂-NH-C₂H₅, -CH₂-CH₂-NH₂, -CH₂-CH₂-NH-CH₃, -CH₂-CH₂-N(CH₃)₂, -CH₂-CH₂-N(C₂H₅)₂, -CH₂-CH₂-NH-C₂H₅, -CH₂-CH₂-CH₂-NH-CH₃, -CH₂-CH₂-CH₂-N(CH₃)₂, -CH₂-CH₂-CH₂-N(C₂H₅)₂ and -CH₂-CH₂-CH₂-NH-C₂H₅; a (hetero)cycloaliphatic radical selected from the group consisting of cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl, azepanyl and fluorenyl, which may be bonded via a -(CH₂)_{1, 2 or 3}- group and which may be unsubstituted or optionally substituted with 1, 2, 3, 4 or 5 substituent(s) independently selected from the group consisting of oxo (=O), thioxo (=S), methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃ and -OH; or an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, which may be bonded via a -(CH₂)_{1, 2} or ₃-group and which may be unsubstituted or optionally substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)3, F, Cl, Br, I, -CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂, -CF₂H and -CFH₂;
and the other substituents have any of the above defined meanings, optionally in form of one of its stereoisomers, preferably enantiomers or diasteromers, a racemate or in form of a mixture of at least two of its stereoisomers, preferably enantiomers and/or diastereomers, in any mixing ratio, or a corresponding solvate thereof.

Particularly preferred is a medicament comprising at least one substituted tetrahydroisoquinoline compound of general formula I and/or II, wherein
R¹ represents a hydrogen atom; a radical selected from the group consisting of methyl, ethyl, n-propyl, isopropyl, n-butyl, sec-butyl, isobutyl, tert-butyl, n-pentyl, n-hexyl, -CF₃, -CFH₂, -CF₂H, -CH₂-CF₃, -CF₂-CF₃, -CH₂-CN, -CH₂-CH₂-CN, -CH₂-O-CF₃, -CH₂-S-CF₃, -CH₂-OH, -CH₂-CH₂-OH, -CH₂-SH, -CH₂-CH₂-SH, -CH₂-NH₂, -CH₂-NH-CH₃, -CH₂-N(CH₃)₂, -CH₂-N(C₂H₅)₂, -CH₂-NH-C₂H₅, -CH₂-CH₂-NH₂, -CH₂-CH₂-NH-CH₃, -CH₂-CH₂-N(CH₃)₂, -CH₂-CH₂-N(C₂H₅)₂, -CH₂-CH₂-NH-C₂H₅, -CH₂-CH₂-CH₂-NH-CH₃, -CH₂-CH₂-CH₂-N(CH₃)₂, -CH₂-CH₂-CH₂-N(C₂H₅)₂ and -CH₂-CH₂-CH₂-NH-C₂H₅; or a (hetero)cycloaliphatic radical selected from the group consisting of cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl and azepanyl, which may be bonded via a -(CH₂)_{1,2 or 3}-group and which may be unsubstituted or optionally substituted with 1, 2, 3, 4 or 5 substituent(s) independently selected from the group consisting of oxo (=O), thioxo (=S), methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, - NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃ or in general formula I R¹ additionally represents a -C(=O)-OR³⁷ moiety;
R², R³, R⁴ and R⁵, independently of one another, each represent a hydrogen atom; F, Cl, Br, I, -NO₂; -NH₂; -SH; -OH; -CN; -C(=O)-OH; -C(=O)-H; -S(=O)₂-OH; -C(=O)-NH₂; -S(=O)₂-NH₂; -C(=O)-R⁶; -S(=O)-R⁷; -S(=O)₂-R⁷; -OR⁸; -SR⁹; -C(=O)-OR¹⁰; -N(R¹¹)-S(=O)₂-R¹²; -NR¹³R¹⁴ ; -NH-R¹⁵; -C(=O)-NR¹⁶R¹⁷; C(=O)-NHR¹⁸; a radical selected from the group consisting of methyl, ethyl, n-propyl, isopropyl, n-butyl, sec-butyl, isobutyl, tert-butyl, n-pentyl, n-hexyl, vinyl, allyl, ethinyl, -CF₃, -CFH₂, -CF₂H, -CH₂-CF₃, -CF₂-CF₃, - CH₂-CN, -CH₂-CH₂-CN, -CH₂-O-CF₃, -CH₂-S-CF₃, -CH₂-OH, -CH₂-CH₂-OH, -CH₂-SH, - CH₂-CH₂-SH, -CH₂-NH₂, -CH₂-NH-CH₃, -CH₂-N(CH₃)₂, -CH₂-N(C₂H₅)₂ and -CH₂-NH-C₂H₅; or a radical selected from the group consisting of cyclopropyl, cyclobutyl, cyclopentyl, cyclohexyl, phenyl, benzyl, phenethyl, pyridinyl, pyrrolyl, furanyl and thiophenyl, which may be unsubstituted or optionally substituted with 1, 2, 3, 4 or 5 substituent(s) independently selected from the group consisting of oxo (=O), thioxo (=S), methyl, ethyl, n-propyl, isopropyl, n-butyl, tert-butyl, sec-butyl, isobutyl, n-pentyl, -O-CH₃, -O-C₂H₅, -S-CH₃, -S-C₂H₅, F, Cl, Br, -CN, -OH, -SH, -NO₂, -CHO, -CF₂H, -CFH₂ and - C(=O)-NH₂;
with the proviso that at least one of the substituents R², R³, R⁴ and R⁵ represents a - N(R¹¹)-S(=O)₂-R¹² moiety;
R⁶, R⁷, R⁸, R⁹, R¹⁰, R¹³, R¹⁴, R¹⁵, R¹⁶, R¹⁷ and R¹⁸, independently of one another, each represent a radical selected from the group consisting of methyl, ethyl, n-propyl, isopropyl, n-butyl, sec-butyl, isobutyl, tert-butyl, n-pentyl, n-hexyl, vinyl, allyl, ethinyl, -CF₃, -CFH₂, -CF₂H, -CH₂-CF₃, -CF₂-CF₃, -CH₂-CN, -CH₂-CH₂-CN, -CH₂-O-CF₃, -CH₂-S-CF₃, -CH₂-OH, -CH₂-CH₂-OH, -CH₂-SH, -CH₂-CH₂-SH, -CH₂-NH₂, -CH₂-NH-CH₃, -CH₂-N(CH₃)₂, -CH₂-N(C₂H₅)₂, -CH₂-NH-C₂H₅, -CH₂-CH₂-NH₂, -CH₂-CH₂-NH-CH₃, -CH₂-CH₂-N(CH₃)₂, -CH₂-CH₂-N(C₂H₅)₂, -CH₂-CH₂-NH-C₂H₅, -CH₂-CH₂-CH₂-NH-CH₃, -CH₂-CH₂-CH₂-N(CH₃)₂, -CH₂-CH₂-CH₂-N(C₂H₅)₂ and -CH₂-CH₂-CH₂-NH-C₂H₅; a (hetero)cycloaliphatic radical selected from the group consisting of cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl and azepanyl, which may be bonded via a -(CH₂)₁, _{or 3}- group and which may be unsubstituted or optionally substituted with 1, 2, 3, 4 or 5 substituent(s) independently selected from the group consisting of oxo (=O), thioxo (=S), methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, - NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃; or an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, which may be bonded via a -(CH₂)_{1,2 or 3}-group and which may be unsubstituted or optionally substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, - NH-C(CH₃)₃, -N(CH₃)₂, -N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃;
R¹¹ represents a hydrogen atom, -S(=O)₂-R¹² or an alkyl radical selected from the group consisting of methyl, ethyl, n-propyl, isopropyl, n-butyl, sec-butyl, isobutyl and tert-butyl;
R¹² represents a phenyl radical of general formula (A), 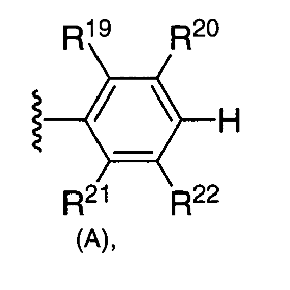 wherein
R¹⁹, R²⁰, R²¹ and R²², independently of one another, each represent a hydrogen atom; F, Cl, Br, I, -NO₂; -NH₂; -SH; -OH; -CN; -C(=O)-OH; -C(=O)-H; -S(=O)₂-OH; -C(=O)-NH₂; - S(=O)₂-NH₂; -C(=O)-R²³; -S(=O)-R²⁴; -S(=O)₂-R²⁴; -OR²⁵; -SR²⁶; -C(=O)-OR²⁷; -N(R²⁸)-S(=O)₂-R^{29;} -NH-S(=O)₂-R³⁰; -NR³¹R³²; -NH-R³³; -C(=O)-NHR³⁴; -C(=O)-NR³⁵R³⁶; methyl, ethyl, n-propyl, isopropyl, n-butyl, isobutyl, sec-butyl, tert-butyl, -CF₃, -CF₂H, - CFH₂, -CH₂-CF₃, -CF₂-CF₃, cyclopropyl, cyclobutyl, cyclopentyl and cyclohexyl;
with the proviso that at least one of the substituents R¹⁹, R²⁰, R²¹ and R²² is unlike hydrogen;
a radical selected from the group consisting of naphthyl, [1,3]-benzodioxolyl, [1,4]-benzodioxanyl, [1,2,3,4]-tetrahydronaphthyl, (2,3)-dihydro-1 H-cyclopenta[b]indolyl, [1,2,3,4]-tetrahydroquinolinyl, [1,2,3,4]-tetrahydroisoquinolinyl, [1,2,3,4]-tetrahydroquinazolinyl, [3,4]-dihydro-2H-benzo[1,4]oxazinyl, indolyl, isoindolyl, quinolinyl, isoquinolinyl, benzo[b]furanyl, benzo[b]thiophenyl, benzo[2,1,3]thiadiazolyl, [1,2,3]-benzothiadiazolyl, [2,1,3]-benzoxadiazolyl, [1,2,3]-benzoxadiazolyl, benzoxazolyl, benzothiazolyl, benzisoxazolyl, benzisothiazolyl, imidazo[2,1-b]thiazolyl, 2H-chromenyl, indazolyl, quinazolinyl, cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl and azepanyl, which may be unsubstituted or optionally substituted with 1, 2, 3, 4 or 5 substituent(s) independently selected from the group consisting of oxo (=O), thioxo (=S), methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, -SH, -NH₂, -NH-CH₃, -NH-C₂H₅, - NH-CH₂-CH₂-CH₃, -NH-CH(CH₃)₂, -NH-C(CH₃)₃, -N(CH₃)₂, -N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃;
or a radical selected from the group consisting of pyridinyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, pyrimidinyl, pyrazinyl and pyranyl, which may be unsubstituted or optionally substituted with 1, 2, 3, 4 or 5 substituent(s) independently selected from the group consisting of F, Cl, Br, I, -NO₂; -NH₂; -SH; -OH; - CN; -C(=O)-OH; -C(=O)-H; -S(=O)₂-OH; -C(=O)-NH₂; -S(=O)₂-NH₂; -C(=O)-R²³; -S(=O)-R²⁴; -S(=O)₂-R²⁴; -OR²⁵; -SR²⁶; -C(=O)-OR²⁷; -N(R²⁸)-S(=O)₂-R²⁹; -NH-S(=O)₂-R³⁰; - NR³¹R³²; -NH-R³³; -C(=O)-NHR³⁴; -C(=O)-NR³⁵R³⁶; methyl, ethyl, n-propyl, isopropyl, n-butyl, isobutyl, sec-butyl, tert-butyl, -CF₃, -CF₂H, -CFH₂, -CH₂-CF₃, -CF₂-CF₃, cyclopropyl, cyclobutyl, cyclopentyl and cyclohexyl;
R²³, R²⁷, R²⁸, R²⁹ and R³⁰, independently of one another, each represent a radical selected from the group consisting of methyl, ethyl, n-propyl, isopropyl, n-butyl, sec-butyl, isobutyl, tert-butyl, n-pentyl, n-hexyl, vinyl, allyl, ethinyl, - CF₃, -CFH₂, -CF₂H, -CH₂-CF₃, -CF₂-CF₃, -CH₂-CN, -CH₂-CH₂-CN, -CH₂-O-CF₃, -CH₂-S-CF₃, -CH₂-OH, -CH₂-CH₂-OH, -CH₂-SH, -CH₂-CH₂-SH, -CH₂-NH₂, -CH₂-NH-CH₃, -CH₂-N(CH₃)₂, -CH₂-N(C₂H₅)₂, -CH₂-NH-C₂H₅, -CH₂-CH₂-NH₂, -CH₂-CH₂-NH-CH₃, -CH₂-CH₂-N(CH₃)₂, -CH₂-CH₂-N(C₂H₅)₂, -CH₂-CH₂-NH-C₂H₅, -CH₂-CH₂-CH₂-NH-CH₃, -CH₂-CH₂-CH₂-N(CH₃)₂, -CH₂-CH₂-CH₂-N(C₂H₅)₂ and -CH₂-CH₂-CH₂-NH-C₂H₅; a (hetero)cycloaliphatic radical selected from the group consisting of cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl and azepanyl, which may be bonded via a -(CH₂)_{1,2 or 3}- group and which may be unsubstituted or optionally substituted with 1, 2, 3, 4 or 5 substituent(s) independently selected from the group consisting of oxo (=O), thioxo (=S), methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃; or an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, which may be bonded via a -(CH₂)_{1,2 or 3}-group and which may be unsubstituted or optionally substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, - OCF₃, -SCF₃, -OH, -SH, -NH₂, -NH-CH₃, -NH-C₂H₅, -NH-CH₂-CH₂-CH₃, -NH-CH(CH₃)₂, - NH-C(CH₃)₃, -N(CH₃)₂, -N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃;
R²⁴, R²⁶, R³¹, R³² and R³³, independently of one another, each represent a radical selected from the group consisting of methyl, ethyl, n-propyl, isopropyl, n-butyl, sec-butyl, isobutyl, tert-butyl, n-pentyl, n-hexyl, vinyl, allyl, ethinyl, -CF₃, -CFH₂, -CF₂H, -CH₂-CF₃, -CF₂-CF₃, -CH₂-CN, -CH₂-CH₂-CN, -CH₂-O-CF₃, -CH₂-S-CF₃, -CH₂-OH, -CH₂-CH₂-OH, -CH₂-SH, -CH₂-CH₂-SH, -CH₂-NH₂, -CH₂-NH-CH₃, -CH₂-N(CH₃)₂, -CH₂-N(C₂H₅)₂, - CH₂-NH-C₂H₅, -CH₂-CH₂-NH₂, -CH₂-CH₂-NH-CH₃, -CH₂-CH₂-N(CH₃)₂, -CH₂-CH₂-N(C₂H₅)₂, -CH₂-CH₂-NH-C₂H₅, -CH₂-CH₂-CH₂-NH-CH₃, -CH₂-CH₂-CH₂-N(CH₃)₂, -CH₂-CH₂-CH₂-N(C₂H₅)₂ and -CH₂-CH₂-CH₂-NH-C₂H₅; a (hetero)cycloaliphatic radical selected from the group consisting of cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl and azepanyl, which may be unsubstituted or optionally substituted with 1, 2, 3, 4 or 5 substituent(s) independently selected from the group consisting of oxo (=O), thioxo (=S), methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, - NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃; or an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, which may be unsubstituted or optionally substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, - C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, l, -CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, - NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃;
R²⁵, R³⁴, R³⁵ and R³⁶, independently of one another, each represent a radical selected from the group consisting of methyl, ethyl, n-propyl, isopropyl, n-butyl, sec-butyl, isobutyl, tert-butyl, n-pentyl, n-hexyl, vinyl, allyl, ethinyl, -CF₃, -CFH₂, -CF₂H, -CH₂-CF₃,-CF₂-CF₃, -CH₂-CN, -CH₂-CH₂-CN, -CH₂-O-CF₃, -CH₂-S-CF₃, -CH₂-OH, -CH₂-CH₂-OH, - CH₂-SH, -CH₂-CH₂-SH, -CH₂-NH₂, -CH₂-NH-CH₃, -CH₂-N(CH₃)₂, -CH₂-N(C₂H₅)₂, -CH₂-NH-C₂H₅, -CH₂-CH₂-NH₂, -CH₂-CH₂-NH-CH₃, -CH₂-CH₂-N(CH₃)₂, -CH₂-CH₂-N(C₂H₅)₂, -CH₂-CH₂-NH-C₂H₅, -CH₂-CH₂-CH₂-NH-CH₃, -CH₂-CH₂-CH₂-N(CH₃)₂, -CH₂-CH₂-CH₂-N(C₂H₅)₂ and -CH₂-CH₂-CH₂-NH-C₂H₅;
and R³⁷ represents a radical selected from the group consisting of methyl, ethyl, n-propyl, isopropyl, n-butyl, sec-butyl, isobutyl, tert-butyl, n-pentyl, n-hexyl, vinyl, allyl, ethinyl, -CF₃, -CFH₂, -CF₂H, -CH₂-CF₃, -CF₂-CF₃, -CH₂-CN, -CH₂-CH₂-CN, -CH₂-O-CF₃, -CH₂-S-CF₃, -CH₂-OH, -CH₂-CH₂-OH, -CH₂-SH, -CH₂-CH₂-SH, -CH₂-NH₂, -CH₂-NH-CH₃, -CH₂-N(CH₃)₂, -CH₂-N(C₂H₅)₂, -CH₂-NH-C₂H₅, -CH₂-CH₂-NH₂, -CH₂-CH₂-NH-CH₃, -CH₂-CH₂-N(CH₃)₂, -CH₂-CH₂-N(C₂H₅)₂, -CH₂-CH₂-NH-C₂H₅, -CH₂-CH₂-CH₂-NH-CH₃, -CH₂-CH₂-CH₂-N(CH₃)₂, -CH₂-CH₂-CH₂-N(C₂H₅)₂ and -CH₂-CH₂-CH₂-NH-C₂H₅; a (hetero)cycloaliphatic radical selected from the group consisting of cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl, azepanyl and fluorenyl, which may be bonded via a -(CH₂)_{1, 2 or 3}- group and which may be unsubstituted or optionally substituted with 1, 2, 3, 4 or 5 substituent(s) independently selected from the group consisting of oxo (=O), thioxo (=S), methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, F, Cl, Br, I, -CN, -CF₃, -OCF₃, -SCF₃ and -OH; or an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, which may be bonded via a -(CH₂)₁, _{2 or 3}-group and which may be unsubstituted or optionally substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, -SH, - NO₂, -CF₂H and -CFH₂;
optionally in form of one of its stereoisomers, preferably enantiomers or diasteromers, a racemate or in form of a mixture of at least two of its stereoisomers, preferably enantiomers and/or diastereomers, in any mixing ratio, or a salt thereof, or a corresponding solvate thereof.

Also particularly preferred is a medicament comprising at least one substituted tetrahydroisoquinoline compound of general formula I and/or II, wherein
R¹ represents a hydrogen atom; a radical selected from the group consisting of methyl, ethyl, n-propyl, isopropyl, n-butyl, sec-butyl, isobutyl, tert-butyl, n-pentyl, n-hexyl, -CH₂-NH₂, -CH₂-NH-CH₃, -CH₂-N(CH₃)₂, -CH₂-N(C₂H₅)₂, -CH₂-NH-C₂H₅, -CH₂-CH₂-NH₂, -CH₂-CH₂-NH-CH₃, -CH₂-CH₂-N(CH₃)₂, -CH₂-CH₂-N(C₂H₅)₂, -CH₂-CH₂-NH-C₂H₅, -CH₂-CH₂-CH₂-NH-CH₃, -CH₂-CH₂-CH₂-N(CH₃)₂, -CH₂-CH₂-CH₂-N(C₂H₅)₂ and -CH₂-CH₂-CH₂-NH-C₂H₅; or a (hetero)cycloaliphatic radical selected from the group consisting of imidazolidinyl, aziridinyl, azetidinyl, pyrrolidinyl, piperidinyl, morpholinyl, thiomorpholinyl, piperazinyl, pyrazolidinyl and azepanyl, which may be bonded via a -(CH₂)_{1, 2 or 3}- group and which may be unsubstituted or optionally substituted with 1, 2, 3, 4 or 5 substituent(s) independently selected from the group consisting of oxo (=O), thioxo (=S), methyl, ethyl, n-propyl, isopropyl, n-butyl, tert-butyl, sec-butyl, isobutyl, -C(=O)-CH₃, -C(=O)-C₂H₅, -C(=O)-CH₂-CH₂-CH₃, -C(=O)-CH(CH₃)₂, -C(=O)-C(CH₃)₃, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃ or in general formula I R¹ additionally represents a -C(=O)-OR³⁷ moiety;
R², R³, R⁴ and R⁵, independently of one another, each represent a hydrogen atom; F, Cl, Br, I, -NO₂; -NH₂; -SH; -OH; -CN; -C(=O)-OH; -C(=O)-H; -S(=O)₂-OH; -C(=O)-NH₂; -S(=O)₂-NH₂; -OR⁸; -SR⁹; -N(R¹¹)-S(=O)₂-R¹²; a radical selected from the group consisting of methyl, ethyl, n-propyl, isopropyl, n-butyl, sec-butyl, isobutyl, tert-butyl, vinyl, allyl, ethinyl, -CF₃, -CFH₂, -CF₂H, -CH₂-CF₃ and -CF₂-CF₃; or a radical selected from the group consisting of cyclopropyl, cyclobutyl, cyclopentyl and cyclohexyl;
with the proviso that at least one of the substituents R², R³, R⁴ and R⁵ represents a - N(R¹¹)-S(=O)₂-R¹² moiety;
R⁸ and R⁹, independently of one another, each represent a radical selected from the group consisting of methyl, ethyl, n-propyl, isopropyl, n-butyl, sec-butyl, isobutyl, tert-butyl, -CF₃, -CFH₂, -CF₂H, -CH₂-CF₃ and -CF₂-CF₃; a (hetero)cycloaliphatic radical selected from the group consisting of cyclopropyl, cyclobutyl, cyclopentyl and cyclohexyl; or an aryl or heteroaryl radical selected from the group consisting of phenyl, naphthyl, furyl (furanyl), thienyl (thiophenyl), pyrrolyl, and pyridinyl, which may be bonded via a -(CH₂)₁, _{2 or 3}-group and which may be unsubstituted or optionally substituted with 1, 2 or 3 substituent(s) independently selected from the group consisting of methyl, ethyl, n-propyl, isopropyl, n-butyl, tert-butyl, sec-butyl, isobutyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, F, Cl, Br, I, -CN, -CF₃, -OCF₃, -SCF₃, -OH, -SH, -NH₂, -NO₂, -CHO, -CF₂H and -CFH₂;
R¹¹ represents a hydrogen atom, -S(=O)₂-R¹² or an alkyl radical selected from the group consisting of methyl, ethyl, n-propyl, isopropyl, n-butyl, sec-butyl, isobutyl and tert-butyl;
R¹² represents a phenyl radical of general formula (A), 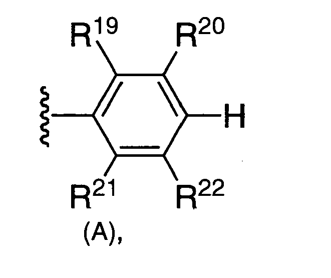 wherein
R¹⁹, R²⁰, R²¹ and R²², independently of one another, each represent a hydrogen atom; F, Cl, Br, I, -NO₂; -NH₂; -SH; -OH; -CN; -C(=O)-OH; -C(=O)-H; -S(=O)₂-OH; -C(=O)-NH₂; - S(=O)₂-NH₂; -C(=O)-R²³; -S(=O)-R²⁴; -S(=O)₂-R²⁴; -OR²⁵; -SR²⁶; -C(=O)-OR²⁷; methyl, ethyl, n-propyl, isopropyl, n-butyl, isobutyl, sec-butyl, tert-butyl, -CF₃, -CF₂H, -CFH₂, - CH₂-CF₃, -CF₂-CF₃, cyclopropyl, cyclobutyl, cyclopentyl and cyclohexyl;
with the proviso that at least one of the substituents R¹⁹, R²⁰, R²¹ and R²² is unlike hydrogen;
a radical selected from the group consisting of naphthyl, [1,3]-benzodioxolyl, [1,4]-benzodioxanyl, [1,2,3,4]-tetrahydronaphthyl, (2,3)-dihydro-1 H-cyclopenta[b]indolyl, [1,2,3,4]-tetrahydroquinolinyl, [1,2,3,4]-tetrahydroisoquinolinyl, [1,2,3,4]-tetrahydroquinazolinyl, [3,4]-dihydro-2H-benzo[1,4]oxazinyl, indolyl, isoindolyl, quinolinyl, isoquinolinyl, benzo[b]furanyl, benzo[b]thiophenyl, benzo[2,1,3]thiadiazolyl, [1,2,3]-benzothiadiazolyl, [2,1,3]-benzoxadiazolyl, [1,2,3]-benzoxadiazolyl, benzoxazolyl, benzothiazolyl, benzisoxazolyl, benzisothiazolyl, imidazo[2,1-b]thiazolyl, 2H-chromenyl, indazolyl and quinazolinyl, which may be unsubstituted or optionally substituted with 1, 2, 3, 4 or 5 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, - S-CH(CH₃)₂, -S-C(CH₃)₃, F, Cl, Br, I, -CN, -CF₃, -OCF₃, -SCF₃, -OH, -SH, -NH₂, -NO₂,-CHO, -CF₂H and -CFH₂;
or a radical selected from the group consisting of pyridinyl, furyl (furanyl), thienyl (thiophenyl), pyrrolyl, imidazolyl, pyrazolyl, triazolyl, pyridazinyl, pyrimidinyl and pyrazinyl, which may be unsubstituted or optionally substituted with 1, 2, 3, 4 or 5 substituent(s) independently selected from the group consisting of F, Cl, Br, I, -NO₂; - NH₂; -SH; -OH; -CN; -C(=O)-OH; -C(=O)-H; -S(=O)₂-OH; -C(=O)-NH₂; -S(=O)₂-NH₂; - C(=O)-R²³; -S(=O)-R²⁴; -S(=O)₂-R²⁴; -OR²⁵; -SR²⁶; -C(=O)-OR²⁷; methyl, ethyl, n-propyl, isopropyl, n-butyl, isobutyl, sec-butyl, tert-butyl, -CF₃, -CF₂H, -CFH₂, -CH₂-CF₃, -CF₂-CF₃, cyclopropyl, cyclobutyl, cyclopentyl and cyclohexyl;
R²³ and R²⁷, independently of one another, each represent a radical selected from the group consisting of methyl, ethyl, n-propyl, isopropyl, n-butyl, sec-butyl, isobutyl, tert-butyl, n-pentyl, n-hexyl, -CF₃, -CFH₂, -CF₂H, -CH₂-CF₃ and -CF₂-CF₃; or an aryl or heteroaryl radical selected from the group consisting of phenyl, naphthyl, furyl (furanyl), thienyl (thiophenyl), pyrrolyl, and pyridinyl, which may be bonded via a -(CH₂)_{1, 2 or 3}- group and which may be unsubstituted or optionally substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, F, Cl, Br, I, -CN, -CF₃, -OCF₃, -SCF₃, -OH, -SH, -NH₂-NO₂, -CHO, -CF₂H and -CFH₂;
R²⁴ and R²⁶, independently of one another, each represent a radical selected from the group consisting of methyl, ethyl, n-propyl, isopropyl, n-butyl, sec-butyl, isobutyl, tert-butyl, n-pentyl, n-hexyl, vinyl, allyl, ethinyl, -CF₃, -CFH₂, -CF₂H, -CH₂-CF₃ and -CF₂-CF₃; or an aryl or heteroaryl radical selected from the group consisting of phenyl, naphthyl, furyl (furanyl), thienyl (thiophenyl), pyrrolyl, and pyridinyl, which may be unsubstituted or optionally substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, F, Cl, Br, I, -CN, - CF₃, -OCF₃, -SCF₃, -OH, -SH, -NH₂, -NO₂, -CHO, -CF₂H and -CFH₂;
R²⁵ represents a radical selected from the group consisting of methyl, ethyl, n-propyl, isopropyl, n-butyl, sec-butyl, isobutyl, tert-butyl, -CF₃, -CFH₂, -CF₂H, - CH₂-CF₃ and -CF₂-CF₃;
and R³⁷ represents a radical selected from the group consisting of methyl, ethyl, n-propyl, isopropyl, n-butyl, sec-butyl, isobutyl, tert-butyl, n-pentyl, n-hexyl, vinyl, allyl, ethinyl, -CF₃, -CFH₂, -CF₂H, -CH₂-CF₃ and -CF₂-CF₃; a (hetero)cycloaliphatic radical selected from the group consisting of cyclopropyl, cyclobutyl, cyclopentyl, cyclohexyl, cycloheptyl, cyclooctyl, cyclononyl and fluorenyl, which may be bonded via a -(CH₂)₁, _{2 or 3}- group and which may be unsubstituted or optionally substituted with 1, 2, 3, 4 or 5 substituent(s) independently selected from the group consisting of methyl, ethyl, n-propyl, isopropyl, n-butyl, tert-butyl, sec-butyl, isobutyl and n-pentyl; or an aryl or heteroaryl radical selected from the group consisting of phenyl and naphthyl, which may be bonded via a -(CH₂)_{1, 2 or 3}-group and which may be unsubstituted or optionally substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, F, Cl, Br, I, -CN, -CF₃, - OCF₃, -SCF₃, -OH, -SH, -NO₂, -CF₂H and -CFH₂;
optionally in form of one of its stereoisomers, preferably enantiomers or diasteromers, a racemate or in form of a mixture of at least two of its stereoisomers, preferably enantiomers and/or diastereomers, in any mixing ratio, or a salt thereof, or a corresponding solvate thereof.

More particularly preferred is a medicament comprising at least one substituted tetrahydroisoquinoline compound of general formula I and/or II, wherein
R¹ represents a hydrogen atom; a radical selected from the group consisting of methyl, ethyl, n-propyl, isopropyl, n-butyl, sec-butyl, isobutyl, tert-butyl, n-pentyl, n-hexyl, -CH₂-NH₂, -CH₂-NH-CH₃, -CH₂-N(CH₃)₂, -CH₂-N(C₂H₅)₂, -CH₂-NH-C₂H₅, -CH₂-CH₂-NH₂, -CH₂-CH₂-NH-CH₃, -CH₂-CH₂-N(CH₃)₂, -CH₂-CH₂-N(C₂H₅)₂, -CH₂-CH₂-NH-C₂H₅, -CH₂-CH₂-CH₂-NH-CH₃, -CH₂-CH₂-CH₂-N(CH₃)₂, -CH₂-CH₂-CH₂-N(C₂H₅)₂ and -CH₂-CH₂-CH₂-NH-C₂H₅; a (hetero)cycloaliphatic radical selected from the group consisting of imidazolidinyl, aziridinyl, azetidinyl, pyrrolidinyl, piperidinyl, morpholinyl, thiomorpholinyl, piperazinyl, pyrazolidinyl and azepanyl, which may be bonded via a -(CH₂)_{1,2 or 3}- group and which may be unsubstituted or optionally substituted with 1, 2, 3, 4 or 5 substituent(s) independently selected from the group consisting of methyl, ethyl, n-propyl, isopropyl, n-butyl, tert-butyl, sec-butyl and isobutyl or in general formula I R¹ additionally represents a -C(=O)-OR³⁷ moiety;
R², R³, R⁴ and R⁵, independently of one another, each represent a hydrogen atom; F, Cl, Br, I, -NO₂; -O-CH₃; -O-C₂H₅; -O-CF₃; -O-CFH₂; -O-CF₂H; -O-CH₂-CF₃; -O-CF₂-CF₃; -S-CH₃; -S-C₂H₅; -S-CF₃; -S-CFH₂; -S-CF₂H; -S-CH₂-CF₃; -S-CF₂-CF₃; -N(R¹¹)-S(=O)₂-R¹²; or a radical selected from the group consisting of methyl, ethyl, n-propyl, isopropyl, tert-butyl, -CF₃, -CFH₂, -CF₂H, -CH₂-CF₃ and -CF₂-CF₃;
with the proviso that at least one of the substituents R², R³, R⁴ and R⁵ represents a -N(R¹¹)-S(=O)₂-R¹² moiety;
R¹¹ represents a hydrogen atom, -S(=O)₂-R¹² or an alkyl radical selected from the group consisting of methyl, ethyl, n-propyl, isopropyl, n-butyl, sec-butyl, isobutyl and tert-butyl;
R¹² represents a phenyl radical of general formula (A), 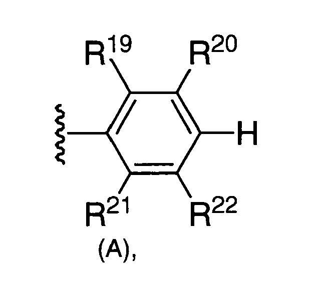 wherein
R¹⁹, R²⁰, R²¹ and R²², independently of one another, each represent a hydrogen atom; F, Cl, Br, I, -NO₂; -NH₂; -SH; -OH; -CN; -C(=O)-OH; -C(=O)-H; -C(=O)-CH₃; -C(=O)-C₂H₅; -O-CH₃; -O-C₂H₅; -O-CF₃; -O-CFH₂; -O-CF₂H; -O-CH₂-CF₃; -O-CF₂-CF₃; -S-CH₃; -S-C₂H₅; -S-CF₃; -S-CFH₂; -S-CF₂H; -S-CH₂-CF₃; -S-CF₂-CF₃; -C(=O)-OCH₃; -C(=O)-OC₂H₅; methyl, ethyl, n-propyl, isopropyl, n-butyl, isobutyl, sec-butyl, tert-butyl, -CF₃, -CF₂H, -CFH₂, -CH₂-CF₃ and -CF₂-CF₃;
with the proviso that at least one of the substituents R¹⁹, R²⁰, R²¹ and R²² is unlike hydrogen;
a radical selected from the group consisting of naphthyl, [1,3]-benzodioxolyl, [1,4]-benzodioxanyl, benzo[b]furanyl, benzo[b]thiophenyl, benzo[2,1,3]thiadiazolyl, [1,2,3]-benzothiadiazolyl, [2,1,3]-benzoxadiazolyl, [1,2,3]-benzoxadiazolyl, benzoxazolyl, benzothiazolyl, benzisoxazolyl, benzisothiazolyl and imidazo[2,1-b]thiazolyl, which may be unsubstituted or optionally substituted with 1, 2, 3, 4 or 5 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, F, Cl, Br, I, -CN, -CF₃, -OCF₃, - SCF₃, -CF₂H and -CFH₂;
or a radical selected from the group consisting of pyridinyl, pyrrolyl, imidazolyl, pyrazolyl, triazolyl, pyridazinyl, pyrimidinyl and pyrazinyl, which may be unsubstituted or optionally substituted with 1, 2, 3, 4 or 5 substituent(s) independently selected from the group consisting of F, Cl, Br, 1, -NO₂; methyl, ethyl, n-propyl, isopropyl, n-butyl, isobutyl, sec-butyl, tert-butyl, -CF₃, -CF₂H, -CFH₂, -CH₂-CF₃ and -CF₂-CF₃;
and R³⁷ represents a radical selected from the group consisting of methyl, ethyl, n-propyl, isopropyl, n-butyl, sec-butyl, isobutyl, tert-butyl, n-pentyl, n-hexyl, fluorenyl, fluorenylmethyl, phenyl, benzyl and naphthyl;
optionally in form of one of its stereoisomers, preferably enantiomers or diasteromers, a racemate or in form of a mixture of at least two of its stereoisomers, preferably enantiomers and/or diastereomers, in any mixing ratio, or a salt thereof, or a corresponding solvate thereof.

Also more particularly preferred is a medicament comprising at least one substituted tetrahydroisoquinoline compound of general formula I and/or II, wherein
R¹ represents a hydrogen atom; a radical selected from the group consisting of methyl, ethyl, n-propyl, n-butyl, n-pentyl and n-hexyl; or in general formula I R¹ additionally represents a -C(=O)-OR³⁷ moiety;
R², R³, R⁴ and R⁵, independently of one another, each represent a hydrogen atom or -N(R¹¹)-S(=O)₂-R¹²;
with the proviso that at least one of the substituents R², R³, R⁴ and R⁵ represents a - N(R¹¹)-S(=O)₂-R¹² moiety;
R¹¹ represents a hydrogen atom, -S(=O)₂-R¹² or an alkyl radical selected from the group consisting of methyl, ethyl and n-propyl;
R¹² represents a phenyl radical of general formula (A), 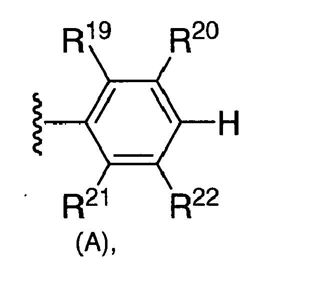 wherein
R¹⁹, R²⁰, R²¹ and R²², independently of one another, each represent a hydrogen atom; F, Cl, Br, I, -O-CH₃; -O-C₂H₅; -O-CF₃; -O-CFH₂; -O-CF₂H; -O-CH₂-CF₃; -O-CF₂-CF₃; methyl, ethyl, n-propyl, isopropyl, n-butyl, isobutyl, sec-butyl and tert-butyl;
with the proviso that at least one of the substituents R¹⁹, R²⁰, R²¹ and R²² is unlike hydrogen;
a radical selected from the group consisting of naphthyl, benzo[b]thiophenyl and imidazo[2,1-b]thiazolyl, which may be unsubstituted or optionally substituted with 1, 2, 3, 4 or 5 substituent(s) independently selected from the group consisting of methyl, ethyl, n-propyl, isopropyl, F, Cl and Br;
or a pyridinyl radical, which may be unsubstituted or optionally substituted with 1, 2, 3, 4 or 5 substituent(s) independently selected from the group consisting of F, Cl, Br, I, methyl, ethyl, n-propyl, isopropyl, n-butyl, isobutyl, sec-butyl and tert-butyl;
and R³⁷ represents a radical selected from the group consisting of methyl, ethyl, n-propyl, isopropyl, n-butyl, sec-butyl, isobutyl, tert-butyl, n-pentyl, n-hexyl, fluorenyl, fluorenylmethyl, phenyl, benzyl and naphthyl;
optionally in form of one of its stereoisomers, preferably enantiomers or diasteromers, a racemate or in form of a mixture of at least two of its stereoisomers, preferably enantiomers and/or diastereomers, in any mixing ratio, or a salt thereof, or a corresponding solvate thereof.

Also preferred is a medicament comprising at least one substituted tetrahydroisoquinoline compound of general formula la, 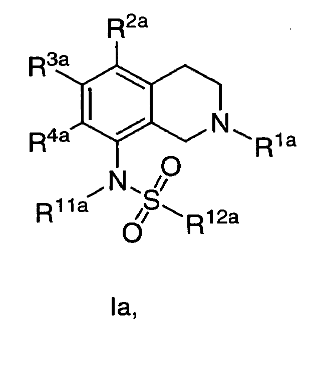 wherein
R^{1a} represents a hydrogen atom; a -C(=O)-OR^{37a} moiety; a radical selected from the group consisting of methyl, ethyl, n-propyl, isopropyl, n-butyl, sec-butyl, isobutyl, tert-butyl, n-pentyl, n-hexyl, -CH₂-NH₂, -CH₂-NH-CH₃, -CH₂-N(CH₃)₂, -CH₂-N(C₂H₅)₂, -CH₂-NH-C₂H₅, -CH₂-CH₂-NH₂, -CH₂-CH₂-NH-CH₃, -CH₂-CH₂-N(CH₃)₂, -CH₂-CH₂-N(C₂H₅)₂, -CH₂-CH₂-NH-C₂H₅, -CH₂-CH₂-CH₂-NH-CH₃, -CH₂-CH₂-CH₂-N(CH₃)₂, -CH₂-CH₂-CH₂-N(C₂H₅)₂ and -CH₂-CH₂-CH₂-NH-C₂H₅; or a (hetero)cycloaliphatic radical selected from the group consisting of imidazolidinyl, aziridinyl, azetidinyl, pyrrolidinyl, piperidinyl, morpholinyl, thiomorpholinyl, piperazinyl, pyrazolidinyl and azepanyl, which may be bonded via a -(CH₂)₁, _{2 or 3}- group and which may be unsubstituted or optionally substituted with 1, 2, 3, 4 or 5 substituent(s) independently selected from the group consisting of methyl, ethyl, n-propyl, isopropyl, n-butyl, tert-butyl, sec-butyl and isobutyl;
R^{2a}, R^{3a} and R^{4a}, independently of one another, each represent a hydrogen atom; F, Cl, Br, I, -NO₂; -O-CH₃; -O-C₂H₅; -O-CF₃; -O-CFH₂; -O-CF₂H; -O-CH₂-CF₃; -O-CF₂-CF₃; -S-CH₃; -S-C₂H₅; -S-CF₃; -S-CFH₂; -S-CF₂H; -S-CH₂-CF₃; -S-CF₂-CF₃; -N(R11^{a})-S(=O)₂-R^{12a}; or a radical selected from the group consisting of methyl, ethyl, n-propyl, isopropyl, tert-butyl, -CF₃, -CFH₂, -CF₂H, -CH₂-CF₃ and -CF₂-CF₃;
R^{11a} represents a hydrogen atom, -S(=O)₂-R^{12a} or an alkyl radical selected from the group consisting of methyl, ethyl, n-propyl, isopropyl, n-butyl, sec-butyl, isobutyl and tert-butyl;
R^{12a} represents a phenyl radical of general formula (Aa), 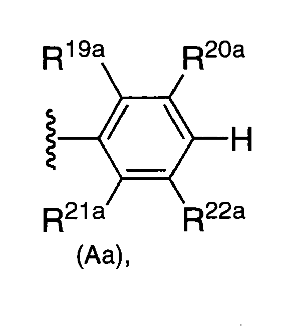 wherein
R^{19a}, R^{20a}, R^{21a} and R^{22a}, independently of one another, each represent a hydrogen atom; F, Cl, Br, l, -NO₂; -NH₂; -SH; -OH; -CN; -C(=O)-OH; -C(=O)-H; -C(=O)-CH₃; -C(=O)-C₂H₅; -O-CH₃; -O-C₂H₅; -O-CF₃; -O-CFH₂; -O-CF₂H; -O-CH₂-CF₃; -O-CF₂-CF₃; -S-CH₃; -S-C₂H₅; -S-CF₃; -S-CFH₂; -S-CF₂H; -S-CH₂-CF₃; -S-CF₂-CF₃; -C(=O)-OCH₃; - C(=O)-OC₂H₅; methyl, ethyl, n-propyl, isopropyl, n-butyl, isobutyl, sec-butyl, tert-butyl, -CF₃, -CF₂H, -CFH₂, -CH₂-CF₃ and -CF₂-CF₃;
with the proviso that at least one of the substituents R^{19a}, R^{20a}, R^{21a} and R^{22a} is unlike hydrogen;
a radical selected from the group consisting of naphthyl, [1,3]-benzodioxolyl, [1,4]-benzodioxanyl, benzo[b]furanyl, benzo[b]thiophenyl, benzo[2,1,3]thiadiazolyl, [1,2,3]-benzothiadiazolyl, [2,1,3]-benzoxadiazolyl, [1,2,3]-benzoxadiazolyl, benzoxazolyl, benzothiazolyl, benzisoxazolyl, benzisothiazolyl and imidazo[2,1-b]thiazolyl, which may be unsubstituted or optionally substituted with 1, 2, 3, 4 or 5 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, F, Cl, Br, I, -CN, -CF₃, -OCF₃, - SCF₃, -CF₂H and -CFH₂;
or a radical selected from the group consisting of pyridinyl, pyrrolyl, imidazolyl, pyrazolyl, triazolyl, pyridazinyl, pyrimidinyl and pyrazinyl, which may be unsubstituted or optionally substituted with 1, 2, 3, 4 or 5 substituent(s) independently selected from the group consisting of F, Cl, Br, I, -NO₂; methyl, ethyl, n-propyl, isopropyl, n-butyl, isobutyl, sec-butyl, tert-butyl, -CF₃, -CF₂H, -CFH₂, -CH₂-CF₃ and -CF₂-CF₃;
and R^{37a} represents a radical selected from the group consisting of methyl, ethyl, n-propyl, isopropyl, n-butyl, sec-butyl, isobutyl, tert-butyl, n-pentyl, n-hexyl, fluorenyl, fluorenylmethyl, phenyl, benzyl and naphthyl;
optionally in form of one of its stereoisomers, preferably enantiomers or diasteromers, a racemate or in form of a mixture of at least two of its stereoisomers, preferably enantiomers and/or diastereomers, in any mixing ratio, or a salt thereof, or a corresponding solvate thereof.

Also preferred is a medicament comprising at least one substituted tetrahydroisoquinoline compound of general formula lb, 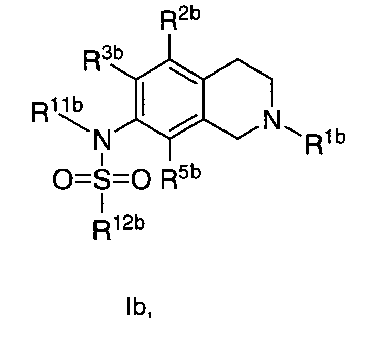 wherein
R^{1b} represents a hydrogen atom; a -C(=O)-OR^{37b} moiety; a radical selected from the group consisting of methyl, ethyl, n-propyl, isopropyl, n-butyl, sec-butyl, isobutyl, tert-butyl, n-pentyl, n-hexyl, -CH₂-NH₂, -CH₂-NH-CH₃, -CH₂-N(CH₃)₂, -CH₂-N(C₂H₅)₂, -CH₂-NH-C₂H₅, -CH₂-CH₂-NH₂, -CH₂-CH₂-NH-CH₃, -CH₂-CH₂-N(CH₃)₂, -CH₂-CH₂-N(C₂H₅)₂, -CH₂-CH₂-NH-C₂H₅, -CH₂-CH₂-CH₂-NH-CH₃, -CH₂-CH₂-CH₂-N(CH₃)₂, -CH₂-CH₂-CH₂-N(C₂H₅)₂ and -CH₂-CH₂-CH₂-NH-C₂H₅; or a (hetero)cycloaliphatic radical selected from the group consisting of imidazolidinyl, aziridinyl, azetidinyl, pyrrolidinyl, piperidinyl, morpholinyl, thiomorpholinyl, piperazinyl, pyrazolidinyl and azepanyl, which may be bonded via a -(CH₂)₁, _{2 or 3}- group and which may be unsubstituted or optionally substituted with 1, 2, 3, 4 or 5 substituent(s) independently selected from the group consisting of methyl, ethyl, n-propyl, isopropyl, n-butyl, tert-butyl, sec-butyl and isobutyl;
R^{2b}, R^{3b} and R^{5b}, independently of one another, each represent a hydrogen atom; F, Cl, Br, I, -NO₂; -O-CH₃; -O-C₂H₅; -O-CF₃; -O-CFH₂; -O-CF₂H; -O-CH₂-CF₃; -O-CF₂-CF₃; -S-CH₃; -S-C₂H₅; -S-CF₃; -S-CFH₂; -S-CF₂H; -S-CH₂-CF₃; -S-CF₂-CF₃; -N(R^{11b})-S(=O)₂-R^{12b}; or a radical selected from the group consisting of methyl, ethyl, n-propyl, isopropyl, tert-butyl, -CF₃, -CFH₂, -CF₂H, -CH₂-CF₃ and -CF₂-CF₃;
R^{11b} represents a hydrogen atom, -S(=O)₂-R^{12b} or an alkyl radical selected from the group consisting of methyl, ethyl, n-propyl, isopropyl, n-butyl, sec-butyl, isobutyl and tert-butyl;
R^{12b} represents a phenyl radical of general formula (Ab), 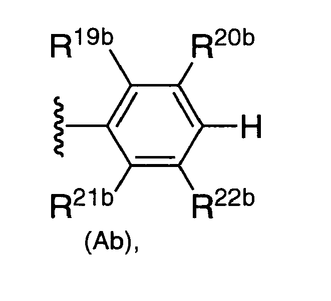 wherein
R^{19b}, R^{20b}, R^{21b} and R^{22b}, independently of one another, each represent a hydrogen atom; F, Cl, Br, l, -NO₂; -NH₂; -SH; -OH; -CN; -C(=O)-OH; -C(=O)-H; -C(=O)-CH₃; -C(=O)-C₂H₅; -O-CH₃; -O-C₂H₅; -O-CF₃; -O-CFH₂; -O-CF₂H; -O-CH₂-CF₃; -O-CF₂-CF₃; -S-CH₃; -S-C₂H₅; -S-CF₃; -S-CFH₂; -S-CF₂H; -S-CH₂-CF₃; -S-CF₂-CF₃; -C(=O)-OCH₃; -C(=O)-OC₂H₅; methyl, ethyl, n-propyl, isopropyl, n-butyl, isobutyl, sec-butyl, tert-butyl, -CF₃, -CF₂H, -CFH₂, -CH₂-CF₃ and -CF₂-CF₃;
with the proviso that at least one of the substituents R^{19b}, R^{20b}, R^{21b} and R^{22b} is unlike hydrogen;
a radical selected from the group consisting of naphthyl, [1,3]-benzodioxolyl, [1,4]-benzodioxanyl, benzo[b]furanyl, benzo[b]thiophenyl, benzo[2,1,3]thiadiazolyl, [1,2,3]-benzothiadiazolyl, [2,1,3]-benzoxadiazolyl, [1,2,3]-benzoxadiazolyl, benzoxazolyl, benzothiazolyl, benzisoxazolyl, benzisothiazolyl and imidazo[2,1-b]thiazolyl, which may be unsubstituted or optionally substituted with 1, 2, 3, 4 or 5 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, F, Cl, Br, I, -CN, -CF₃, -OCF₃, - SCF₃, -CF₂H and -CFH₂;
or a radical selected from the group consisting of pyridinyl, pyrrolyl, imidazolyl, pyrazolyl, triazolyl, pyridazinyl, pyrimidinyl and pyrazinyl, which may be unsubstituted or optionally substituted with 1, 2, 3, 4 or 5 substituent(s) independently selected from the group consisting of F, Cl, Br, 1, -NO₂; methyl, ethyl, n-propyl, isopropyl, n-butyl, isobutyl, sec-butyl, tert-butyl, -CF₃, -CF₂H, -CFH₂, -CH₂-CF₃ and -CF₂-CF₃;
and R^{37b} represents a radical selected from the group consisting of methyl, ethyl, n-propyl, isopropyl, n-butyl, sec-butyl, isobutyl, tert-butyl, n-pentyl, n-hexyl, fluorenyl, fluorenylmethyl, phenyl, benzyl and naphthyl;
optionally in form of one of its stereoisomers, preferably enantiomers or diasteromers, a racemate or in form of a mixture of at least two of its stereoisomers, preferably enantiomers and/or diastereomers, in any mixing ratio, or a salt thereof, or a corresponding solvate thereof.

Also preferred is a medicament comprising at least one substituted tetrahydroisoquinoline compound of general formula lc, 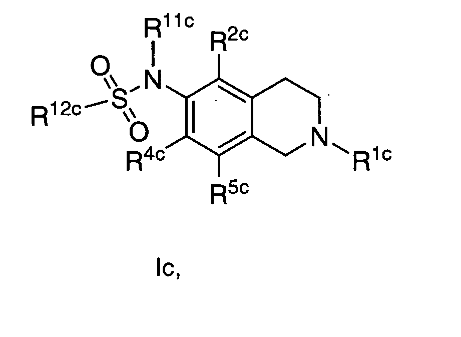 wherein
R^{1c} represents a hydrogen atom; a -C(=O)-OR^{37c} moiety; a radical selected from the group consisting of methyl, ethyl, n-propyl, isopropyl, n-butyl, sec-butyl, isobutyl, tert-butyl, n-pentyl, n-hexyl, -CH₂-NH₂, -CH₂-NH-CH₃, -CH₂-N(CH₃)₂, -CH₂-N(C₂H₅)₂, -CH₂-NH-C₂H₅, -CH₂-CH₂-NH₂, -CH₂-CH₂-NH-CH₃, -CH₂-CH₂-N(CH₃)₂, -CH₂-CH₂-N(C₂H₅)₂, -CH₂-CH₂-NH-C₂H₅, -CH₂-CH₂-CH₂-NH-CH₃, -CH₂-CH₂-CH₂-N(CH₃)₂, -CH₂-CH₂-CH₂-N(C₂H₅)₂ and -CH₂-CH₂-CH₂-NH-C₂H₅; or a (hetero)cycloaliphatic radical selected from the group consisting of imidazolidinyl, aziridinyl, azetidinyl, pyrrolidinyl, piperidinyl, morpholinyl, thiomorpholinyl, piperazinyl, pyrazolidinyl and azepanyl, which may be bonded via a -(CH₂)_{1,2 or 3}- group and which may be unsubstituted or optionally substituted with 1, 2, 3, 4 or 5 substituent(s) independently selected from the group consisting of methyl, ethyl, n-propyl, isopropyl, n-butyl, tert-butyl, sec-butyl and isobutyl;
R^{2c}, R^{4c} and R^{5c}, independently of one another, each represent a hydrogen atom; F, Cl, Br, I, -NO₂; -O-CH₃; -O-C₂H₅; -O-CF₃; -O-CFH₂; -O-CF₂H; -O-CH₂-CF₃; -O-CF₂-CF₃; -S-CH₃; -S-C₂H₅; -S-CF₃; -S-CFH₂; -S-CF₂H; -S-CH₂-CF₃; -S-CF₂-CF₃; -N(R^{11c})-S(=O)₂-R^{12c}; or a radical selected from the group consisting of methyl, ethyl, n-propyl, isopropyl, tert-butyl, -CF₃, -CFH₂, -CF₂H, -CH₂-CF₃ and -CF₂-CF₃;
R^{11c} represents a hydrogen atom, -S(=O)₂-R^{12c} or an alkyl radical selected from the group consisting of methyl, ethyl, n-propyl, isopropyl, n-butyl, sec-butyl, isobutyl and tert-butyl;
R^{12c} represents a phenyl radical of general formula (Ac), 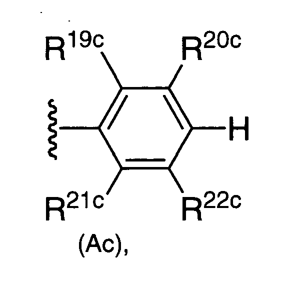 wherein
R^{19c}, R^{20c}, R^{21c} and R^{22c}, independently of one another, each represent a hydrogen atom; F, Cl, Br, 1, -NO₂; -NH₂; -SH; -OH; -CN; -C(=O)-OH; -C(=O)-H; -C(=O)-CH₃; -C(=O)-C₂H₅; -O-CH₃; -O-C₂H₅; -O-CF₃; -O-CFH₂; -O-CF₂H; -O-CH₂-CF₃; -O-CF₂-CF₃; -S-CH₃; -S-C₂H₅; -S-CF₃; -S-CFH₂; -S-CF₂H; -S-CH₂-CF₃; -S-CF₂-CF₃; -C(=O)-OCH₃; -C(=O)-OC₂H₅; methyl, ethyl, n-propyl, isopropyl, n-butyl, isobutyl, sec-butyl, tert-butyl, -CF₃, -CF₂H, -CFH₂, -CH₂-CF₃ and -CF₂-CF₃;
with the proviso that at least one of the substituents R^{19c}, R^{20c}, R^{21c} and R^{22c} is unlike hydrogen;
a radical selected from the group consisting of naphthyl, [1,3]-benzodioxolyl, [1,4]-benzodioxanyl, benzo[b]furanyl, benzo[b]thiophenyl, benzo[2,1,3]thiadiazolyl, [1,2,3]-benzothiadiazolyl, [2,1,3]-benzoxadiazolyl, [1,2,3]-benzoxadiazolyl, benzoxazolyl, benzothiazolyl, benzisoxazolyl, benzisothiazolyl and imidazo[2,1-b]thiazolyl, which may be unsubstituted or optionally substituted with 1, 2, 3, 4 or 5 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, F, Cl, Br, I, -CN, -CF₃, -OCF₃, - SCF₃, -CF₂H and -CFH₂;
or a radical selected from the group consisting of pyridinyl, pyrrolyl, imidazolyl, pyrazolyl, triazolyl, pyridazinyl, pyrimidinyl and pyrazinyl, which may be unsubstituted or optionally substituted with 1, 2, 3, 4 or 5 substituent(s) independently selected from the group consisting of F, Cl, Br**, l**, -NO₂; methyl, ethyl, n-propyl, isopropyl, n-butyl, isobutyl, sec-butyl, tert-butyl, -CF₃, -CF₂H, -CFH₂, -CH₂-CF₃ and -CF₂-CF₃;
and R^{37c} represents a radical selected from the group consisting of methyl, ethyl, n-propyl, isopropyl, n-butyl, sec-butyl, isobutyl, tert-butyl, n-pentyl, n-hexyl, fluorenyl, fluorenylmethyl, phenyl, benzyl and naphthyl;
optionally in form of one of its stereoisomers, preferably enantiomers or diasteromers, a racemate or in form of a mixture of at least two of its stereoisomers, preferably enantiomers and/or diastereomers, in any mixing ratio, or a salt thereof, or a corresponding solvate thereof.

Also preferred is a medicament comprising at least one substituted tetrahydroisoquinoline compound of general formula Id, 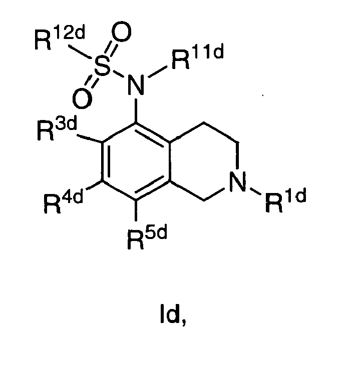 wherein
R^{1d} represents a hydrogen atom; a -C(=O)-OR^{37d} moiety; a radical selected from the group consisting of methyl, ethyl, n-propyl, isopropyl, n-butyl, sec-butyl, isobutyl, tert-butyl, n-pentyl, n-hexyl, -CH₂-NH₂, -CH₂-NH-CH₃, -CH₂-N(CH₃)₂, -CH₂-N(C₂H₅)₂, -CH₂-NH-C₂H₅, -CH₂-CH₂-NH₂, -CH₂-CH₂-NH-CH₃, -CH₂-CH₂-N(CH₃)₂, -CH₂-CH₂-N(C₂H₅)₂, -CH₂-CH₂-NH-C₂H₅, -CH₂-CH₂-CH₂-NH-CH₃, -CH₂-CH₂-CH₂-N(CH₃)₂, -CH₂-CH₂-CH₂-N(C₂H₅)₂ and -CH₂-CH₂-CH₂-NH-C₂H₅; or a (hetero)cycloaliphatic radical selected from the group consisting of imidazolidinyl, aziridinyl, azetidinyl, pyrrolidinyl, piperidinyl, morpholinyl, thiomorpholinyl, piperazinyl, pyrazolidinyl and azepanyl, which may be bonded via a -(CH₂)₁, _{2 or 3}- group and which may be unsubstituted or optionally substituted with 1, 2, 3, 4 or 5 substituent(s) independently selected from the group consisting of methyl, ethyl, n-propyl, isopropyl, n-butyl, tert-butyl, sec-butyl and isobutyl;
R^{3d}, R^{4d} and R^{5d}, independently of one another, each represent a hydrogen atom; F, Cl, Br, I, -NO₂; -O-CH₃; -O-C₂H₅; -O-CF₃; -O-CFH₂; -O-CF₂H; -O-CH₂-CF₃; -O-CF₂-CF₃; -S-CH₃; -S-C₂H₅; -S-CF₃; -S-CFH₂; -S-CF₂H; -S-CH₂-CF₃; -S-CF₂-CF₃; -N(R^{11d})-S(=O)₂-R^{12d}; or a radical selected from the group consisting of methyl, ethyl, n-propyl, isopropyl, tert-butyl, -CF₃, -CFH₂, -CF₂H, -CH₂-CF₃ and -CF₂-CF₃;
R^{11d} represents a hydrogen atom, -S(=O)₂-R^{12d} or an alkyl radical selected from the group consisting of methyl, ethyl, n-propyl, isopropyl, n-butyl, sec-butyl, isobutyl and tert-butyl;
R^{12d} represents a phenyl radical of general formula (Ad), 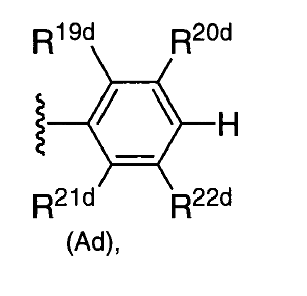 wherein
R^{19d}, R^{20d}, R^{21d} and R^{22d}, independently of one another, each represent a hydrogen atom; F, Cl, Br, 1, -NO₂; -NH₂; -SH; -OH; -CN; -C(=O)-OH; -C(=O)-H; -C(=O)-CH₃; -C(=O)-C₂H₅; -O-CH₃; -O-C₂H₅; -O-CF₃; -O-CFH₂; -O-CF₂H; -O-CH₂-CF₃; -O-CF₂-CF₃; -S-CH₃; -S-C₂H₅; -S-CF₃; -S-CFH₂; -S-CF₂H; -S-CH₂-CF₃; -S-CF₂-CF₃; -C(=O)-OCH₃; -C(=O)-OC₂H₅; methyl, ethyl, n-propyl, isopropyl, n-butyl, isobutyl, sec-butyl, tert-butyl, -CF₃, -CF₂H, -CFH₂, -CH₂-CF₃ and -CF₂-CF₃;
with the proviso that at least one of the substituents R^{19d}, R^{20d}, R^{21d} and R^{22d} is unlike hydrogen;
a radical selected from the group consisting of naphthyl, [1,3]-benzodioxolyl, [1,4]-benzodioxanyl, benzo[b]furanyl, benzo[b]thiophenyl, benzo[2,1,3]thiadiazolyl, [1,2,3]-benzothiadiazolyl, [2,1,3]-benzoxadiazolyl, [1,2,3]-benzoxadiazolyl, benzoxazolyl, benzothiazolyl, benzisoxazolyl, benzisothiazolyl and imidazo[2,1-b]thiazolyl, which may be unsubstituted or optionally substituted with 1, 2, 3, 4 or 5 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, F, Cl, Br, I, -CN, -CF₃, -OCF₃, - SCF₃, -CF₂H and -CFH₂;
or a radical selected from the group consisting of pyridinyl, pyrrolyl, imidazolyl, pyrazolyl, triazolyl, pyridazinyl, pyrimidinyl and pyrazinyl, which may be unsubstituted or optionally substituted with 1, 2, 3, 4 or 5 substituent(s) independently selected from the group consisting of F, Cl, Br, I, -NO₂; methyl, ethyl, n-propyl, isopropyl, n-butyl, isobutyl, sec-butyl, tert-butyl, -CF₃, -CF₂H, -CFH₂, -CH₂-CF₃ and -CF₂-CF₃;
and R^{37d} represents a radical selected from the group consisting of methyl, ethyl, n-propyl, isopropyl, n-butyl, sec-butyl, isobutyl, tert-butyl, n-pentyl, n-hexyl, fluorenyl, fluorenylmethyl, phenyl, benzyl and naphthyl;
optionally in form of one of its stereoisomers, preferably enantiomers or diasteromers, a racemate or in form of a mixture of at least two of its stereoisomers, preferably enantiomers and/or diastereomers, in any mixing ratio, or a salt thereof, or a corresponding solvate thereof.

Even more particularly preferred is a medicament comprising at least one substituted tetrahydroisoquinoline compound as defined above selected from the group consisting of
[1] N-(1,2,3,4-tetrahydroisoquinoiin-6-yt)naphthatene-1 -sulfonamide hydrochloride,
[2] 2,2-dimethyl-6-(N-methylnaphthalene-1-sulfonamido)-1,2,3,4-tetrahydroisoquinolinium iodide,
[3] N-(2-methyl-1,2,3,4-tetrahydroisoquinolin-6-yl)naphthalene-1-sulfonamide hydrochloride,
[4] 5-chloro-3-methyl-N-(1,2,3,4-tetrahydroisoquinolin-6-yl)benzo[b]thiophene-2-sulfonamide hydrochloride,
[5] 5-chloro-3-methyl-N-(2-methyl-1,2,3,4-tetrahydroisoquinolin-6-yl)benzo[b]thiophene-2-sulfonamide hydrochloride,
[6] 4-methyl-N-(1,2,3,4-tetrahydroisoquinolin-6-yl)naphthalene-1-sulfonamide hydrochloride,
[7] 4-methyl-N-(2-methyl-1,2,3,4-tetrahydroisoquinolin-6-yl)naphthalene-1-sulfonamide hydrochloride,
[8] N-(1,2,3,4-tetrahydroisoquinolin-6-yl)naphthalene-2-sulfonamide hydrochloride,
[9] N-(2-methyl-1,2,3,4-tetrahydroisoquinolin-6-yl)naphthalene-2-sulfonamide hydrochloride,
[10] 6-chloro-N-(1,2,3,4-tetrahydroisoquinolin-6-yl)imidazo[2,1-b]thiazole-5-sulfonamide hydrochloride,
[11] 2-methoxy-5-methyl-N-(1,2,3,4-tetrahydroisoquinolin-6-yl)benzenesulfonamide hydrochloride,
[12] N-(1,2,3,4-tetrahydroisoquinolin-6-yl)pyridine-3-sulfonamide dihydrochloride,
[13] 6-(naphthalene-1-sulfonylamino)-3,4-dihydro-1H-isoquinoline-2-carboxylic acid tert-butyl ester,
[14] 6-(5-chloro-3-methyl-benzo[b]thiophene-2-sulfonylamino)-3,4-dihydro-1H-isoquinoline-2-carboxylic acid tert-butyl ester,
[15] 6-(4-methyl-naphthalene-1-sulfonylamino)-3,4-dihydro-1 H-isoquinoline-2-carboxylic acid tert-butyl ester,
[16] 6-(naphthalene-2-sulfonylamino)-3,4-dihydro-1H-isoquinoline-2-carboxylic acid tert-butyl ester,
[17] 6-(2-methoxy-5-methyl-benzenesulfonylamino)-3,4-dihydro-1H-isoquinoline-2-carboxylic acid tert-butyl ester and
[18] 6-(pyridine-3-sulfonylamino)-3,4-dihydro-1H-isoquinoline-2carboxylic acid tert-butyl ester;
   or a corresponding solvate thereof.

A further aspect of the present invention relates to a medicament comprising at least one substituted tetrahydroisoquinoline compound of general formula I, II, III, Ia, Ib, Ic, Id, le, If or Ig given above, in the following only referred to as tetrahydroisoquinoline compound of general formula I, optionally in form of one of its stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof, and optionally at least one physiologically acceptable auxiliary agent.

Said medicament is particularly suitable for 5-HT₆-receptor regulation and therefore for the prophylaxis and/or treatment of a disorder or a disease that is at least partially mediated via 5-HT₆-receptors.

Preferably said medicament is suitable for the prophylaxis and/or treatment of the prophylaxis and/or treatment of a disorder or disease related to food intake, preferably for the regulation of appetite, for the maintenance, increase or reduction of body weight, for the prophylaxis and/or treatment of obesity, bulimia, anorexia, cachexia, type II diabetes (non insulin dependent diabetes mellitus), preferably type II diabetes that is caused by obesity; for the prophylaxis and/or treatment of stroke; migraine; head trauma; epilepsy; irritable colon syndrome; irritable bowl syndrome; emesis; vertigo; disorders of the central nervous system; anxiety; panic attacks; depression; bipolar disorders; obsessive compulsory disorder; cognitive disorders; cognitive dysfunction associated with psychiatric diseases; memory disorders; senile dementia; mood disorders; sleep disorders; psychosis; neurodegenerative disorders, preferably selected from the group consisting of Morbus Alzheimer, Morbus Parkinson, Morbus Huntington and Multiple Sclerosis; schizophrenia; amnesia; autism; sexual dysfunction; gastric motility disorders; circadian rhythm disorders; chronic intermittent hypoxia; convulsions; or hyperactivity disorder (ADHD, attention deficit/hyperactivity disorder); for improvement of cognition (cognitive enhancement) or cognitive memory (cognitive memory enhancement); for the prophylaxis and/or treatment of drug addiction and/or withdrawal; for the prophylaxis and/or treatment of alcohol addiction and/or withdrawal, for the prophylaxis and/or treatment of nicotine addiction and/or withdrawal.

More preferably said medicament is suitable for the prophylaxis and/or treatment of a disorder or disease related to food intake, preferably for the regulation of appetite, for the maintenance, increase or reduction of body weight, for the prophylaxis and/or treatment of obesity, bulimia, anorexia, cachexia or type II diabetes (non insulin dependent diabetes mellitus), preferably type II diabetes that is caused by obesity.

More preferably said medicament is suitable for improvement of cognition (cognitive enhancement) or cognitive memory (cognitive memory enhancement).

Most preferably, said medicament is suitable for the prophylaxis and/or treatment of obesity and/or disorders or diseases related thereto.

In another aspect the present invention relates to the use of at least one substituted tetrahydroisoquinoline compound of general formula I given above, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof, for the preparation of a medicament suitable for 5-HT₆-receptor regulation, preferably for the prophylaxis and/or treatment of a disorder or a disease that is least partially mediated via 5-HT₆-receptors.

In another aspect the present invention relates to the use of at least one substituted tetrahydroisoquinoline compound of general formula I given above, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof, for the prophylaxis and/or treatment of a disorder or disease related to food intake, preferably for the regulation of appetite; for the maintenance, increase or reduction of body weight; or for the prophylaxis and/or treatment of obesity, bulimia, anorexia, cachexia or type II diabetes (non insulin dependent diabetes mellitus), more preferably for the prophylaxis and/or treatment of obesity.

The present invention also relates to the use of at least one substituted tetrahydroisoquinoline compound of general formula I given above, optionally in form of one of its stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof, for the prophylaxis and/or treatment of stroke; migraine; head trauma; epilepsy; irritable colon syndrome; irritable bowl syndrome; emesis; vertigo; disorders of the central nervous system; anxiety; panic attacks; depression; bipolar disorders; obsessive compulsory disorder; cognitive disorders; cognitive dysfunction associated with psychiatric diseases; memory disorders; senile dementia; mood disorders; sleep disorders; psychosis; neurodegenerative disorders, preferably selected from the group consisting of Morbus Alzheimer, Morbus Parkinson, Morbus Huntington and Multiple Sclerosis; schizophrenia; amnesia; autism; sexual dysfunction; gastric motility disorders; circadian rhythm disorders; chronic intermittent hypoxia; convulsions; or hyperactivity disorder (ADHD, attention deficit/hyperactivity disorder).

The present invention also relates to the use of at least one substituted tetrahydroisoquinoline compound of general formula I given above, optionally in form of one of its stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof, for the manufacture of a medicament for the improvement of cognition (cognitive enhancement) and/or for the improvement of cognitive memory (cognitive memory enhancement).

The present invention also relates to the use of at least one substituted tetrahydroisoquinoline compound of general formula I given above, optionally in form of one of its stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof, for the manufacture of a medicament for the prophylaxis and/or treatment of drug addiction and/or withdrawal, preferably for the prophylaxis and/or treatment of addiction and/or withdrawal related to one or more of drugs selected from the group consisting of benzodiazepines, natural, semisynthetic or synthetic opioids like cocaine, ethanol and/or nicotine.

More preferred is the use of at least one substituted tetrahydroisoquinoline compound of general formula I as defined above, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof, for the preparation of a medicament for the maintenance, increase or reduction of body weight, for the prophylaxis and/or treatment of obesity, bulimia, anorexia, cachexia or type II diabetes (non insulin dependent diabetes mellitus), preferably type II diabetes that is caused by obesity.

More preferred is also the use of at least one substituted tetrahydroisoquinoline compound of general formula I as defined above, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof, for the preparation of a medicament for the improvement of cognition (cognitive enhancement) and/or for the improvement of cognitive memory (cognitive memory enhancement).

Any medicament according to the present invention may be in any form suitable for the application to humans and/or animals, preferably humans including infants, children and adults. The medicament can be produced by standard procedures known to those skilled in the art, e.g. from the table of contents of "Pharmaceutics: The Science of Dosage Forms", Second Edition, Aulton, M.E. (ED. Churchill Livingstone, Edinburgh (2002); "Encyclopedia of Pharmaceutical Technology", Second Edition, Swarbrick, J. and Boylan J.C. (Eds.), Marcel Dekker, Inc. New York (2002); "Modern Pharmaceutics", Fourth Edition, Banker G.S. and Rhodes C.T. (Eds.) Marcel Dekker, Inc. New York 2002 y "The Theory and Practice of Industrial Pharmacy", Lachman L., Lieberman H. And Kanig J. (Eds.), Lea & Febiger, Philadelphia (1986). The respective descriptions are hereby incorporated by reference and form part of the disclosure. The composition of the medicament may vary depending on the route of administration.

The medicament of the present invention may, for example, be administered parentally in combination with conventional injectable liquid carriers, such as water or suitable alcohols. Conventional pharmaceutical excipients for injection, such as stabilizing agents, solubilizing agents, and buffers, may be included in such injectable compositions. These medicaments may for example be injected intramuscularly, intraperitoneally, or intravenously.

Medicaments according to the present invention may also be formulated into orally administrable compositions containing one or more physiologically compatible carriers or excipients, in solid or liquid form. These compositions may contain conventional ingredients such as binding agents, fillers, lubricants, and acceptable wetting agents. The compositions may take any convenient form, such as tablets, pellets, granules, capsules, lozenges, aqueous or oily solutions, suspensions, emulsions, or dry powdered forms suitable for reconstitution with water or other suitable liquid medium before use, for immediate or retarded release. The multiparticulate forms, such as pellets or granules, may e.g. be filled into a capsule, compressed into tablets or suspended in a suitable liquid.

Suitable controlled release formulations, materials and methods for their preparation are are known from the prior art, e.g. from the table of contents of "Modified-Release Drug Delivery Technology", Rathbone, M.J. Hadgraft, J. and Roberts, M.S. (Eds.), Marcel Dekker, Inc., New York (2002); "Handbook of Pharmaceutical Controlled Release Technology", Wise, D.L. (Ed.), Marcel Dekker, Inc. New York, (2000); "Controlled Drug Delivery", Vol, I, Basic Concepts, Bruck, S.D. (Ed.), CRD Press Inc., Boca Raton (1983) y de Takada, K. and Yoshikawa, H., "Oral Drug Delivery", Encyclopedia of Controlled Drug Delivery, Mathiowitz, E. (Ed.), John Wiley & Sons, Inc., New York (1999), Vol. 2, 728-742; Fix, J., "Oral drug delivery, small intestine and colon", Encyclopedia of Controlled Drug Delivery, Mathiowitz, E. (Ed.), John Wiley & Sons, Inc., New York (1999), Vol. 2, 698-728. The respective descriptions are hereby incorporated by reference and form part of the disclosure.

Medicaments according to the present invention may also comprise an enteric coating, so that their dissolution is dependent on pH-value. Due to said coating the medicament can pass the stomach undissolved and the respective tetrahydroisoquinoline compound is liberated in the intestinal tract. Preferably the enteric coating is soluble at a pH value of 5 to 7.5. Suitable materials and methods for the preparation are are known from the prior art.

Typically, the medicaments according to the present invention may contain 1-60 % by weight of one or more substituted tetrahydroisoquinoline compounds as defined herein and 40-99 % by weight of one or more auxiliary substances (additives).

The liquid oral forms for administration may also contain certain additives such as sweeteners, flavoring, preservatives, and emulsifying agents. Non-aqueous liquid compositions for oral administration may also be formulated, containing edible oils. Such liquid compositions may be conveniently encapsulated in e.g., gelatin capsules in a unit dosage amount.

The compositions of the present invention may also be administered topically or via a suppository.

The daily dosage for humans and animals may vary depending on factors that have their basis in the respective species or other factors, such as age, sex, weight or degree of illness and so forth. The daily dosage for humans may preferably be in the range from 1 to 2000 mg, preferably 1 to 1000 mg, more preferably 1 to 500 mg, even more preferably 1 to 200 mg, of active substance to be administered during one or several intakes per day.

In yet another aspect the present invention relates to a substituted tetrahydroisoquinoline compound of general formula le, 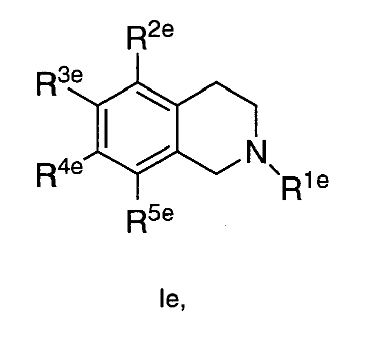 wherein
R^{1e} represents a hydrogen atom; a -C(=O)-OR^{37e} moiety;
a linear or branched, saturated or unsaturated C₁₋₁₀ aliphatic radical which may be unsubstituted or substituted with 1, 2 or 3 substituent(s) independently selected from the group consisting of F, Cl, Br, I, -CN, -CF₃, -OCF₃, -SCF₃, -OH, -SH, -NH₂, -O-C₁₋₅-alkyl, - S-C₁₋₅-alkyl, -NH(C₁₋₅-alkyl) and -N(C₁₋₅-alkyl)₂;
or a saturated or unsaturated 3- to 9-membered cycloaliphatic radical, which may contain 1, 2 or 3 heteroatom(s) independently selected from the group consisting of nitrogen, oxygen and sulfur as ring member(s) and which may be unsubstituted or optionally substituted with 1, 2, 3, 4 or 5 substituent(s) independently selected from the group consisting of oxo (=O), thioxo (=S), C₁₋₅-alkyl, -O-C₁₋₅-alkyl, -S-C₁₋₅-alkyl, -C(=O)-C₁₋₅-alkyl, -O-C(=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl and which may be condensed with an unsubstituted or at least mono-substituted saturated, unsaturated or aromatic mono- or bicyclic ring system and which may be bonded via a linear or branched C₁₋₆ alkylene, C₂₋₆ alkenylene or C₂₋₆ alkinylene group which may be unsubstituted or substituted with 1, 2 or 3 substituent(s) independently selected from the group consisting of F, Cl, Br, I, -CN, -CF₃, -OCF₃, -SCF₃, -OH, -SH, -NH₂, -O-C₁₋₅-alkyl, -S-C₁₋₅-alkyl, -NH(C₁₋₅-alkyl) and -N(C₁₋₅-alkyl)₂;
R^{2e}, R^{3e}, R^{4e} and R^{5e}, independently of one another, each represent a hydrogen atom; F, Cl, Br, I, -NO₂; -NH₂; -SH; -OH; -CN; -C(=O)-OH; -C(=O)-H; -S(=O)₂-OH; -C(=O)-NH₂; -S(=O)₂-NH₂; -C(=O)-R^{6e}; -S(=O)-R^{7e}; -S(=O)₂-R^{7e}; -OR^{8e} ; -SR^{9e}; -C(=O)-OR^{10e}; -N(R^{11e})-S(=O)₂-R^{12e}; -NR^{13e}R^{14e}; -NH-R^{15e}; -C(=O)-NR^{16e}R^{17e}; C(=O)-NHR^{18e};
a linear or branched, saturated or unsaturated, unsubstituted or at least mono-substituted aliphatic radical;
a saturated or unsaturated, unsubstituted or at least mono-substituted, optionally at least one heteroatom as a ring member containing cycloaliphatic radical, which may be bonded via a linear or branched alkylene, alkenylene or alkinylene group and which may be condensed with an unsubstituted or at least mono-substituted saturated, unsaturated or aromatic mono- or bicyclic ring system;
or an unsubstituted or at least mono-substituted aryl or heteroaryl radical, which may be bonded via a linear or branched alkylene, alkenylene or alkinylene group and which may be condensed with an unsubstituted or at least mono-substituted saturated or unsaturated, but not aromatic, mono- or bicyclic ring system;
with the proviso that at least one of the substituents R^{2e}, R^{3e}, R^{4e} and R^{5e} represents a -N(R^{11e})-S(=O)₂-R^{12e} moiety;
R^{6e}, R^{7e}, R^{8e}, R^{9e}, R^{10e}, R^{13e}, R^{14e}, R^{15e}, R^{16e}, R^{17e} and R^{18e}, independently of one another, each represent a linear or branched, saturated or unsaturated, unsubstituted or at least mono-substituted aliphatic radical;
a saturated or unsaturated, unsubstituted or at least mono-substituted, optionally at least one heteroatom as a ring member containing cycloaliphatic radical, which may be bonded via a linear or branched alkylene, alkenylene or alkinylene group and which may be condensed with an unsubstituted or at least mono-substituted saturated, unsaturated or aromatic mono- or bicyclic ring system;
or an unsubstituted or at least mono-substituted aryl or heteroaryl radical, which may be bonded via a linear or branched alkylene, alkenylene or alkinylene group and which may be condensed with an unsubstituted or at least mono-substituted saturated or unsaturated, but not aromatic, mono- or bicyclic ring system;
R^{11e} represents a hydrogen atom, -S(=O)₂-R^{12e} or a linear or branched, saturated or unsaturated, unsubstituted or at least mono-substituted aliphatic radical;
R^{12e} represents a monocyclic heteroaryl radical, which may be unsubstituted or optionally substituted with 1, 2, 3, 4 or 5 substituent(s) independently selected from the group consisting of F, Cl, -NO₂; -NH₂; -SH; -OH; -CN; -C(=O)-OH; -C(=O)-H; -S(=O)₂-OH; -C(=O)-NH₂; -S(=O)₂-NH₂; -C(=O)-R^{23e}; -OR^{25e}; -SR^{26e}; -C(=O)-OR^{27e}; -N(R^{28e})-S(=O)₂-R^{29e}; -NH-S(=O)₂-R^{30e}; -NR^{31e}R^{32e}; -NH-R^{33e}; -C(=O)-NHR^{34e}; -C(=O)-NR^{35e}R^{36e}; a linear or branched, saturated or unsaturated C₁₋₁₀ aliphatic radical which may be unsubstituted or substituted with 1, 2 or 3 substituent(s) independently selected from the group consisting of F, Cl, Br, I, -CN, -CF₃, -OCF₃, -SCF₃, -OH, -SH, -NH₂, -O-C₁₋₅-alkyl, -S-C₁₋₅-alkyl, -NH(C₁₋₅-alkyl) and -N(C₁₋₅-alkyl)₂; and a saturated or unsaturated, unsubstituted or at least mono-substituted, optionally at least one heteroatom as a ring member containing cycloaliphatic radical;
an unsubstituted or at least mono-substituted monocyclic heteroaryl radical, which is condensed with an unsubstituted or at least mono-substituted saturated or unsaturated, but not aromatic, mono- or bicyclic ring system;
an unsubstituted or at least mono-substituted bi- or tricyclic heteroaryl radical, which may be condensed with an unsubstituted or at least mono-substituted saturated or unsaturated, but not aromatic, mono- or bicyclic ring system;
or a saturated or unsaturated, unsubstituted or at least mono-substituted, optionally at least one heteroatom as a ring member containing cycloaliphatic radical, which may be condensed with an unsubstituted or at least mono-substituted saturated, unsaturated or aromatic mono- or bicyclic ring system;
R^{23e}, R^{27e}, R^{28e}, R^{29e} and R^{30e}, independently of one another, each represent a linear or branched, saturated or unsaturated C₁₋₁₀ aliphatic radical which may be unsubstituted or substituted with 1, 2 or 3 substituent(s) independently selected from the group consisting of F, Cl, Br, I, -CN, -CF₃, -OCF₃, -SCF₃, -OH, -SH, -NH₂, -O-C₁₋₅-alkyl, - S-C₁₋₅-alkyl, -NH(C₁₋₅-alkyl) and -N(C₁₋₅-alkyl)₂;
a saturated or unsaturated, unsubstituted or at least mono-substituted, optionally at least one heteroatom as a ring member containing cycloaliphatic radical, which may be bonded via a linear or branched alkylene, alkenylene or alkinylene group and which may be condensed with an unsubstituted or at least mono-substituted saturated, unsaturated or aromatic mono- or bicyclic ring system;
or an unsubstituted or at least mono-substituted aryl or heteroaryl radical, which may be bonded via a linear or branched alkylene, alkenylene or alkinylene group and which may be condensed with an unsubstituted or at least mono-substituted saturated or unsaturated, but not aromatic, mono- or bicyclic ring system;
R^{25e}, R^{34e} R^{35e} and R^{36e}, represents a linear or branched, saturated or unsaturated C₁₋₁₀ aliphatic radical which may be unsubstituted or substituted with 1, 2 or 3 substituent(s) independently selected from the group consisting of F, Cl, Br, I, -CN, -CF₃, -OCF₃, -SCF₃, -OH, -SH, -NH₂, -O-C₁₋₅-alkyl, -S-C₁₋₅-alkyl, -NH(C₁₋₅-alkyl) and - N(C₁₋₅alkyl)₂;
R^{26e}, R^{31e} R^{32e} and R^{33e}, each represent a linear or branched, saturated or unsaturated C₁₋₁₀ aliphatic radical which may be unsubstituted or substituted with 1, 2 or 3 substituent(s) independently selected from the group consisting of F, Cl, Br, I, -CN, -CF₃, -OCF₃, -SCF₃, -OH, -SH, -NH₂, -O-C₁₋₅-alkyl, -S-C₁₋₅-alkyl, -NH(C₁₋₅-alkyl) and -N(C₁₋₅-alkyl)₂;
a saturated or unsaturated, unsubstituted or at least mono-substituted, optionally at least one heteroatom as a ring member containing cycloaliphatic radical, which may be bonded via a linear or branched alkylene, alkenylene or alkinylene group and which may be condensed with an unsubstituted or at least mono-substituted saturated, unsaturated or aromatic mono- or bicyclic ring system;
or an unsubstituted or at least mono-substituted aryl or heteroaryl radical, which may be condensed with an unsubstituted or at least mono-substituted saturated or unsaturated, but not aromatic, mono- or bicyclic ring system;
and R^{37e} represents a linear or branched, saturated or unsaturated C₁₋₁₀ aliphatic radical
a saturated or unsaturated, unsubstituted or at least mono-substituted, optionally at least one heteroatom as a ring member containing cycloaliphatic radical, which may be bonded via a linear or branched alkylene, alkenylene or alkinylene group and which may be condensed with an unsubstituted or at least mono-substituted saturated, unsaturated or aromatic mono- or bicyclic ring system;
or an unsubstituted or at least mono-substituted aryl or heteroaryl radical, which may be bonded via a linear or branched alkylene, alkenylene or alkinylene group and which may be condensed with an unsubstituted or at least mono-substituted saturated or unsaturated, but not aromatic, mono- or bicyclic ring system;
optionally in form of one of its stereoisomers, preferably enantiomers or diasteromers, a racemate or in form of a mixture of at least two of its stereoisomers, preferably enantiomers and/or diastereomers, in any mixing ratio, or a salt thereof, or a corresponding solvate thereof.

Preferred is a substituted tetrahydroisoquinoline compound of general formula le in form of its salt, 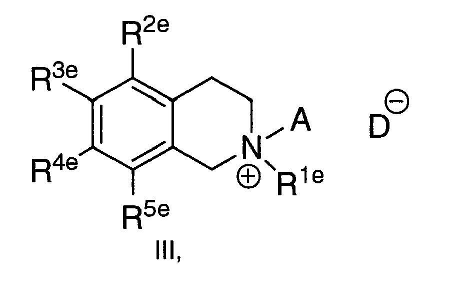 wherein
R^{1e}, R^{2e}, R^{3e}, R^{4e} and R^{5e} are defined as given above except for a -C(=O)-OR^{37e} moiety;
A represents a hydrogen atom or a radical selected from the group consisting of methyl, ethyl, isopropyl, n-butyl, isobutyl, 2-butyl, tert-butyl and n-pentyl;
D represents an anion selected from the group consisting of chloride, bromide, iodide, fluoride, hydrogensulfate, nitrate, dihydrogenphosphate, thiocyanate, cyanate, acrylate, fumarate, citrate, glutarate, succinate, maleate, tartrate, phosphate, 2-oxo-glutarate, formate, acetate, propionate, lactate, gluconate, benzoate or naphthoate whereby said benzoate or naphthoate may be substituted with 1, 2, 3, 4 or 5 substituents independently selected from the group consisting of F, Cl, Br, -OH, -O-CH₃ and -O-C₂H₅, pyruvate, ascorbate, glycolate, nicotinate, phenylacetate, 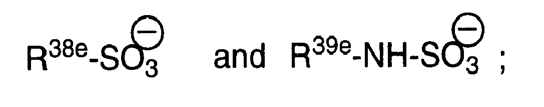 and R^{38e} and R^{39e}, independently of one another, in each case represent a radical selected from the group consisting of -CF₃, -C₂F₅, methyl, ethyl, n-propyl, isopropyl, n-butyl, isobutyl, sec-butyl, tert-butyl, n-pentyl, 2-pentyl, 3-pentyl, neo-pentyl, n-hexyl, 2-hexyl, 3-hexyl, n-heptyl, 2-heptyl, 3-heptyl, 4-heptyl, n-octyl, 2-octyl, 3-octyl, 4-octyl, 2-(6-methyl)-heptyl, 2-(5-methyl)-heptyl, 2-(5-methyl)-hexyl, 2-(4-methyl)-hexyl, 2-(7-methyl)-octyl; 2-(6-methyl)-octyl, n-nonyl, n-decyl, n-undecyl, n-dodecyl, n-tridecyl and n-tetradecyl;
a radical selected from the group consisting of phenyl, pyridinyl, pyrazolyl, benzimidazolyl, isoquinolinyl and naphthyl, which may be substituted with 1, 2, 3, 4 or 5 substituent(s) independently selected from the group consisting of F, Cl, Br, -CF₃, methyl, ethyl, n-propyl, isopropyl, n-butyl, isobutyl, 2-butyl, tert-butyl, n-pentyl, 2-pentyl, n-hexyl, -O-CH₃, -O-C₂H₅, -O-CH₂-CH₂-CH₃, -O-CH(CH₃)₂, -O-CH₂-CH₂-CH₂-CH₃, -O-C(CH₃)₃, -OH, -NO₂, -NH₂, phenyl and -SO₃H;
or a radical selected from the group consisting of cyclopropyl, cyclobutyl, cyclopentyl, cyclohexyl, cycloheptyl, cyclooctyl, cyclononyl, cyclodecyl, cycloundecyl, cyclododecyl, cyclotridecyl, cyclotetradecyl, cyclopentenyl, cyclohexenyl, cycloheptenyl, cyclooctenyl and bicyclo[2.2.1]heptyl, which may be bonded via a -(CH₂)-, -(CH₂)-(CH₂)- or -(CH₂)-(CH₂)-(CH₂)-group.

Also preferred is a substituted tetrahydroisoquinoline compound of general formula le and/or III, wherein
A represents a hydrogen atom or a radical selected from the group consisting of methyl, ethyl, n-propyl, isopropyl, n-butyl, isobutyl, 2-butyl, tert-butyl and n-pentyl;
and D represents an anion selected from the group consisting of chloride, bromide, iodide, fluoride, hydrogensulfate, nitrate, dihydrogenphosphate, thiocyanate, cyanate, acrylate, methanesulfonate, ethanesulfonate, toluenesulfonate, benzenesulfonate, (2,5)-dihydroxy-benzenesulfonate, naphthalene-2-sulfonate, 5-sulfonapthalene-1-sulfonate, cyclamate, dodecane-1-sulfonate and (7,7)-dimethyl-2-oxobicyclo[2.2.1]-hept-1-yl-methanesulfonate.

Also preferred is a substituted tetrahydroisoquinoline compound of general formula le and/or III, wherein
R^{1e} represents a hydrogen atom;
a linear or branched C₁₋₁₀ alkyl radical, C₂₋₁₀ alkenyl radical or C₂₋₁₀ alkinyl radical;
a C₃₋₉ cycloalkyl radical or C₄₋₉ cycloalkenyl radical, which may be unsubstituted or optionally substituted with 1, 2, 3, 4 or 5 substituent(s) independently selected from the group consisting of oxo (=O), thioxo (=S), C₁₋₅-alkyl, -O-C₁₋₅-alkyl, -S-C₁₋₅-alkyl, -C(=O)-C₁₋₅-alkyl, -O-C(=O)-C₁₋₅-alkyl, F, Cl, Br, I, -CN, -CF₃, -OCF₃, -SCF₃, -OH, -SH, -NH₂,-NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl and which may be condensed with an unsubstituted or at least mono-substituted saturated, unsaturated or aromatic mono- or bicyclic ring system and which may be bonded via a linear or branched C₁₋₆ alkylene, C₂₋₆ alkenylene or C₂₋₆ alkinylene group or in general formula le additionally a -C(=O)-OR^{37e} moiety;
R^{2e}, R^{3e}, R^{4e} and R^{5e}, independently of one another, each represent a hydrogen atom; F, Cl, Br, I, -NO₂; -NH₂; -SH; -OH; -CN; -C(=O)-OH; -C(=O)-H; -S(=O)₂-OH; -C(=O)-NH₂; -S(=O)₂-NH₂; -C(=O)-R^{6e}; -S(=O)-R^{7e}; -S(=O)₂-R^{7e}; -OR^{8e}; -SR^{9e}; -C(=O)-OR^{10e}; -N(R^{11e})-S(=O)₂-R^{12e}; -NR^{13e}R^{14e}; -NH-R^{15e}; -C(=O)-NR^{16e}R^{17e}; C(=O)-NHR^{18e};
a linear or branched C₁₋₁₀ alkyl radical, C₂₋₁₀ alkenyl radical or C₂₋₁₀ alkinyl radical;
a C₃₋₉ cycloalkyl radical or C₄₋₉ cycloalkenyl radical, which may be bonded via a linear or branched C₁₋₆ alkylene, C₂₋₆ alkenylene or C₂₋₆ alkinylene group and which may be condensed with an unsubstituted or at least mono-substituted saturated, unsaturated or aromatic mono- or bicyclic ring system;
or an unsubstituted or at least mono-substituted 6-, 10- or 14-membered aryl or 5-, 6-, 8-, 9-, 10-, 11-, 12-, 13- or 14-membered heteroaryl radical, which may be bonded via a linear or branched C₁₋₆ alkylene, C₂₋₆ alkenylene or C₂₋₆ alkinylene group and which may be condensed with an unsubstituted or at least mono-substituted saturated or unsaturated, but not aromatic, mono- or bicyclic ring system;
with the proviso that at least one of the substituents R^{2e}, R^{3e}, R^{4e} and R^{5e} represents a -N(R^{11e})-S(=O)₂-R^{12e} moiety;
R^{6e}, R^{7e}, R^{8e}, R^{9e}, R^{10e}, R^{13e}, R^{14e}, R^{15e}, R^{16e}, R^{17e} and R^{18e}, independently of one another, each represent a linear or branched C₁₋₁₀ alkyl radical, C₂₋₁₀ alkenyl radical or C₂₋₁₀ alkinyl radical;
a C₃₋₉ cycloalkyl radical or C₄₋₉ cycloalkenyl radical, which may be bonded via a linear or branched C₁₋₆ alkylene, C₂₋₆ alkenylene or C₂₋₆ alkinylene group and which may be condensed with an unsubstituted or at least mono-substituted saturated, unsaturated or aromatic mono- or bicyclic ring system;
or an unsubstituted or at least mono-substituted 6-, 10- or 14-membered aryl or 5-, 6-, 8-, 9-, 10-, 11-, 12-, 13- or 14-membered heteroaryl radical, which may be bonded via a linear or branched C₁₋₆ alkylene, C₂₋₆ alkenylene or C₂₋₆ alkinylene group and which may be condensed with an unsubstituted or at least mono-substituted saturated or unsaturated, but not aromatic, mono- or bicyclic ring system;
R^{11e} represents a hydrogen atom, -S(=O)₂-R^{12e} or a linear or branched, saturated or unsaturated, unsubstituted or at least mono-substituted C₁₋₆alkylene, C₂₋₆alkenylene or C₂₋₆ alkinylene group;
R^{12e} represents a monocyclic 5- or 6-membered heteroaryl radical, which may be unsubstituted or optionally substituted with 1, 2, 3, 4 or 5 substituent(s) independently selected from the group consisting of F, Cl, -NO₂; -NH₂; -SH; -OH; -CN; -C(=O)-OH; - C(=O)-H; -S(=O)₂-OH; -C(=O)-NH₂; -S(=O)₂-NH₂; -C(=O)-R^{23e}; -OR^{25e}; -SR^{26e}; -C(=O)-OR^{27e}; -N(R^{28e})-S(=O)₂-R^{29e}; -NH-S(=O)₂-R^{30e}; -NR^{31e}R^{32e}, -NH-R^{33e}; -C(=O)-NHR^{34e}; -C(=O)-NR^{35e}R^{36e}; a linear or branched C₁₋₁₀ alkyl radical, C₂₋₁₀ alkenyl radical or C₂₋₁₀ alkinyl radical; and a C₃₋₉ cycloalkyl radical;
an unsubstituted or at least mono-substituted monocyclic 5- or 6-membered heteroaryl radical, which is condensed with an unsubstituted or at least mono-substituted saturated or unsaturated, but not aromatic, mono- or bicyclic ring system;
an unsubstituted or at least mono-substituted bi- or tricyclic 8-, 9-, 10-, 11-, 12-, 13- or 14-membered heteroaryl radical, which may be condensed with an unsubstituted or at least mono-substituted saturated or unsaturated, but not aromatic, mono- or bicyclic ring system;
or a C₃₋₉ cycloalkyl radical or C₄₋₉ cycloalkenyl radical, which may be condensed with an unsubstituted or at least mono-substituted saturated, unsaturated or aromatic mono- or bicyclic ring system;
R^{23e}, R^{27e}, R^{28e}, R^{29e} and R^{30e}, independently of one another, each represent a linear or branched C₁₋₁₀alkyl radical, C₂₋₁₀ alkenyl radical or C₂₋₁₀ alkinyl radical;
a C₃₋₉ cycloalkyl radical or C₄₋₉ cycloalkenyl radical, which may be bonded via a linear or branched C₁₋₆ alkylene, C₂₋₆ alkenylene or C₂₋₆ alkinylene group and which may be condensed with an unsubstituted or at least mono-substituted saturated, unsaturated or aromatic mono- or bicyclic ring system;
or an unsubstituted or at least mono-substituted 6-, 10- or 14-membered aryl or 5-, 6-, 8-, 9-, 10-, 11-, 12-, 13- or 14-membered heteroaryl radical, which may be bonded via a linear or branched C₁₋₆ alkylene, C₂₋₆ alkenylene or C₂₋₆ alkinylene group and which may be condensed with an unsubstituted or at least mono-substituted saturated or unsaturated, but not aromatic, mono- or bicyclic ring system;
R^{25e}, R^{34e}, R^{35e} and R^{36e}, represents a linear or branched C₁₋₁₀ alkyl radical, C₂₋₁₀ alkenyl radical or C₂₋₁₀ alkinyl radical;
R^{26e}, R^{31e}, R^{32e} and R^{33e}, each represent a linear or branched C₁₋₁₀alkyl radical, C₂₋₁₀ alkenyl radical or C₂₋₁₀ alkinyl radical;
a C₃₋₉ cycloalkyl radical or C₄₋₉ cycloalkenyl radical, which may be bonded via a linear or branched C₁₋₆alkylene, C₂₋₆ alkenylene or C₂₋₆ alkinylene group and which may be condensed with an unsubstituted or at least mono-substituted saturated, unsaturated or aromatic mono- or bicyclic ring system;
or an unsubstituted or at least mono-substituted 6-, 10- or 14-membered aryl or 5-, 6-, 8-, 9-, 10-, 11-, 12-, 13- or 14-membered heteroaryl radical, which may be condensed with an unsubstituted or at least mono-substituted saturated or unsaturated, but not aromatic, mono- or bicyclic ring system;
and R^{37e} represents a linear or branched C₁₋₁₀ alkyl radical, C₂₋₁₀ alkenyl radical or C₂₋₁₀ alkinyl radical;
a C₃₋₉ cycloalkyl radical or C₄₋₉ cycloalkenyl radical, which may be bonded via a linear or branched C₁₋₆ alkylene, C₂₋₆ alkenylene or C₂₋₆ alkinylene group and which may be condensed with an unsubstituted or at least mono-substituted saturated, unsaturated or aromatic mono- or bicyclic ring system;
or an unsubstituted or at least mono-substituted 6-, 10- or 14-membered aryl or 5-, 6-, 8-, 9-, 10-, 11-, 12-, 13- or 14-membered heteroaryl radical, which may be bonded via a linear or branched C₁₋₆ alkylene, C₂₋₆ alkenylene or C₂₋₆ alkinylene group and which may be condensed with an unsubstituted or at least mono-substituted saturated or unsaturated, but not aromatic, mono- or bicyclic ring system;
whereby
the aforementioned C₁₋₁₀ alkyl radical, C₂₋₁₀ alkenyl radical or C₂₋₁₀ alkinyl radicals may in each case be unsubstituted or optionally substituted with 1, 2, 3, 4 or 5 substituent(s) independently selected from the group consisting of F, Cl, Br, I, -CN, -CF₃, -OCF₃, -SCF₃, -OH, -SH, -NH₂, -O-C₁₋₅-alkyl, -S-C₁₋₅-alkyl, -NH(C₁₋₅-alkyl) and -N(C₁₋₅-alkyl)₂;
if not defined otherwise, the aforementioned C₃₋₉ cycloalkyl radicals and C₄₋₉ cycloalkenyl radicals may in each case be unsubstituted or optionally substituted with 1, 2, 3, 4 or 5 substituent(s) independently selected from the group consisting of oxo (=O), thioxo (=S), C₁₋₅-alkyl, -O-C₁₋₅-alkyl, -S-C₁₋₅-alkyl, -C(=O)-OH, -C(=O)-C₁₋₅-alkyl, -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), - C(=O)-N(C₁₋₅-alkyl)₂, -S(=O)₂-C₁₋₅-alkyl, -S(=O)₂-phenyl, phenyl, phenoxy and benzyl;
the aforementioned C₃₋₉ cycloalkyl radicals and C₄₋₉ cycloalkenyl radicals in each case may optionally contain 1, 2 or 3 heteroatom(s) independently selected from the group consisting of nitrogen, oxygen and sulfur as ring member(s);
the aforementioned C₁₋₆ alkylene, C₂₋₆ alkenylene or C₂₋₆ alkinylene groups may in each case be unsubstituted or substituted with 1, 2 or 3 substituent(s) independently selected from the group consisting of F, Cl, Br, I, -CN, -CF₃, -OCF₃, -SCF₃, -OH, -SH, -NH₂, -O-C₁₋₅-alkyl, -S-C₁₋₅-alkyl, -NH(C₁₋₅-alkyl) and -N(C₁₋₅-alkyl)₂;
the rings of the aforementioned ring system are in each case independently of one another 5-, 6- or 7-membered and may in each case independently of one another optionally contain 1, 2 or 3 heteroatom(s) independently selected from the group consisting of nitrogen, oxygen and sulfur;
and the rings of the ring system may in each case be unsubstituted or substituted with 1, 2 or 3 substituent(s) independently selected from the group consisting of oxo (=O), thioxo (=S), C₁₋₅-alkyl, -O-C₁₋₅-alkyl, -S-C₁₋₅-alkyl, -C(=O)-OH, -C(=O)-C₁₋₅-alkyl, -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH₂, NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl;
if not defined otherwise, the 6-, 10- or 14-membered aryl or 5-, 6-, 8-, 9-, 10-, 11-, 12-, 13- or 14-membered heteroaryl radicals may be unsubstituted or optionally substituted with 1, 2, 3, 4 or 5 substituent(s) independently selected from the group consisting of C₁-₅-alkyl, -O-C₁₋₅-alkyl, -S-C₁₋₅-alkyl, -C(=O)-OH, -C(=O)-C₁₋₅-alkyl, -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl;
and the aforementioned 5-, 6-, 8-, 9-, 10-, 11-, 12-, 13- or 14-membered heteroaryl radicals in each case optionally contain 1, 2, 3 or 4 heteroatom(s) independently selected from the group consisting of nitrogen, oxygen and sulfur as ring member(s);
optionally in form of one of its stereoisomers, preferably enantiomers or diasteromers, a racemate or in form of a mixture of at least two of its stereoisomers, preferably enantiomers and/or diastereomers, in any mixing ratio, or a salt thereof, or a corresponding solvate thereof.

A substituted tetrahydroisoquinoline compound of general formula le and/or III is particularly preferred, wherein
R^{1e} represents a hydrogen atom; a radical selected from the group consisting of methyl, ethyl, n-propyl, isopropyl, n-butyl, sec-butyl, isobutyl, tert-butyl, n-pentyl, n-hexyl, -CH₂-NH₂, -CH₂-NH-CH₃, -CH₂-N(CH₃)₂, -CH₂-N(C₂H₅)₂, -CH₂-NH-C₂H₅, -CH₂-CH₂-NH₂, -CH₂-CH₂-NH-CH₃, -CH₂-CH₂-N(CH₃)₂, -CH₂-CH₂-N(C₂H₅)₂, -CH₂-CH₂-NH-C₂H₅, -CH₂-CH₂-CH₂-NH-CH₃, -CH₂-CH₂-CH₂-N(CH₃)₂, -CH₂-CH₂-CH₂-N(C₂H₅)₂ and -CH₂-CH₂-CH₂-NH-C₂H₅; a (hetero)cycloaliphatic radical selected from the group consisting of imidazolidinyl, aziridinyl, azetidinyl, pyrrolidinyl, piperidinyl, morpholinyl, thiomorpholinyl, piperazinyl, pyrazolidinyl and azepanyl, which may be bonded via a -(CH₂)_{1, 2 or 3}- group and which may be unsubstituted or optionally substituted with 1, 2, 3, 4 or 5 substituent(s) independently selected from the group consisting of oxo (=O), thioxo (=S), methyl, ethyl, n-propyl, isopropyl, n-butyl, tert-butyl, sec-butyl, isobutyl, -C(=O)-CH₃, -C(=O)-C₂H₅, -C(=O)-CH₂-CH₂-CH₃, -C(=O)-CH(CH₃)₂, -C(=O)-C(CH₃)₃, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃ or in general formula le R^{1e} additionally represents a -C(=O)-OR^{37e} moiety;
R^{2e}, R^{3e}, R^{4e} and R^{5e}, independently of one another, each represent a hydrogen atom; F, Cl, Br, I, -NO₂; -NH₂; -SH; -OH; -CN; -C(=O)-OH; -C(=O)-H; -S(=O)₂-OH; -C(=O)-NH₂; -S(=O)₂-NH₂; -OR^{8e}; -SR^{9e}; -N(R^{11e})-S(=O)₂-R^{12e}; a radical selected from the group consisting of methyl, ethyl, n-propyl, isopropyl, n-butyl, sec-butyl, isobutyl, tert-butyl, vinyl, allyl, ethinyl, -CF₃, -CFH₂, -CF₂H, -CH₂-CF₃ and -CF₂-CF₃; or a radical selected from the group consisting of cyclopropyl, cyclobutyl, cyclopentyl and cyclohexyl;
with the proviso that at least one of the substituents R^{2e}, R^{3e}, R^{4e} and R^{5e} represents a -N(R^{11e})-S(=O)₂-R^{12e} moiety;
R^{8e} and R^{9e}, independently of one another, each represent a radical selected from the group consisting of methyl, ethyl, n-propyl, isopropyl, n-butyl, sec-butyl, isobutyl, tert-butyl, -CF₃, -CFH₂, -CF₂H, -CH₂-CF₃ and -CF₂-CF₃; a (hetero)cycloaliphatic radical selected from the group consisting of cyclopropyl, cyclobutyl, cyclopentyl and cyclohexyl; or an aryl or heteroaryl radical selected from the group consisting of phenyl, naphthyl, furyl (furanyl), thienyl (thiophenyl), pyrrolyl, and pyridinyl, which may be bonded via a - (CH₂)_{1, 2 or 3}-group and which may be unsubstituted or optionally substituted with 1, 2 or 3 substituent(s) independently selected from the group consisting of methyl, ethyl, n-propyl, isopropyl, n-butyl, tert-butyl, sec-butyl, isobutyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, F, Cl, Br, I, -CN, -CF₃, -OCF₃, -SCF₃, -OH, -SH, -NH₂, -NO₂, -CHO, -CF₂H and -CFH₂;
R^{11e} represents a hydrogen atom, -S(=O)₂-R^{12e} or an alkyl radical selected from the group consisting of methyl, ethyl, n-propyl, isopropyl, n-butyl, sec-butyl, isobutyl and tert-butyl;
R^{12e} represents a radical selected from the group consisting of [1,3]-benzodioxolyl, [1,4]-benzodioxanyl, [1,2,3,4]-tetrahydronaphthyl, (2,3)-dihydro-1 H-cyclopenta[b]indolyl, [1,2,3,4]-tetrahydroquinolinyl, [1,2,3,4]-tetrahydroisoquinolinyl, [1,2,3,4]-tetrahydroquinazolinyl, [3,4]-dihydro-2H-benzo[1,4]oxazinyl, indolyl, isoindolyl, quinolinyl, isoquinolinyl, benzo[b]furanyl, benzo[b]thiophenyl, benzo[2,1,3]thiadiazolyl, [1,2,3]-benzothiadiazolyl, [2,1,3]-benzoxadiazolyl, [1,2,3]-benzoxadiazolyl, benzoxazolyl, benzothiazolyl, benzisoxazolyl, benzisothiazolyl, imidazo[2,1-b]thiazolyl, 2H-chromenyl, indazolyl and quinazolinyl, which may be unsubstituted or optionally substituted with 1, 2, 3, 4 or 5 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, - S-CH(CH₃)₂, -S-C(CH₃)₃, F, Cl, Br, I, -CN, -CF₃, -OCF₃, -SCF₃, -OH, -SH, -NH₂, -NO₂, -CHO, -CF₂H and -CFH₂;
or a radical selected from the group consisting of pyridinyl, furyl (furanyl), thienyl (thiophenyl), pyrrolyl, imidazolyl, pyrazolyl, triazolyl, pyridazinyl, pyrimidinyl and pyrazinyl, which may be unsubstituted or optionally substituted with 1, 2, 3, 4 or 5 substituent(s) independently selected from the group consisting of F, Cl, -NO₂; -NH₂; - SH; -OH; -CN; -C(=O)-OH; -C(=O)-H; -S(=O)₂-OH; -C(=O)-NH₂; -S(=O)₂-NH₂; -C(=O)-CH₃; -C(=O)-C₂H₅; -C(=O)-CH(CH₃)₂; -C(=O)-C(CH₃)₃; -O-CH₃; -O-C₂H₅; -O-C(CH₃)₃;-S-CH₃; -S-C₂H₅; -S-C(CH₃)₃; -C(=O)-O-CH₃; -C(=O)-O-C₂H₅; -C(=O)-O-CH(CH₃)₂; -C(=O)-O-C(CH₃)₃;methyl, ethyl, n-propyl, isopropyl, n-butyl, isobutyl, sec-butyl, tert-butyl, -CF₃, -CF₂H, -CFH₂, -CH₂-CF₃, -CF₂-CF₃, cyclopropyl, cyclobutyl, cyclopentyl and cyclohexyl;
and R^{37e} represents a radical selected from the group consisting of methyl, ethyl, n-propyl, isopropyl, n-butyl, sec-butyl, isobutyl, tert-butyl, n-pentyl, n-hexyl, fluorenyl, fluorenylmethyl, phenyl, benzyl and naphthyl;
optionally in form of one of its stereoisomers, preferably enantiomers or diasteromers, a racemate or in form of a mixture of at least two of its stereoisomers, preferably enantiomers and/or diastereomers, in any mixing ratio, or a salt thereof, or a corresponding solvate thereof.

More particularly preferred is a substituted tetrahydroisoquinoline compound of general formula le and/or III, wherein
R^{1e} represents a hydrogen atom; a radical selected from the group consisting of methyl, ethyl, n-propyl, isopropyl, n-butyl, sec-butyl, isobutyl, tert-butyl, n-pentyl, n-hexyl, -CH₂-NH₂, -CH₂-NH-CH₃, -CH₂-N(CH₃)₂, -CH₂-N(C₂H₅)₂, -CH₂-NH-C₂H₅, -CH₂-CH₂-NH₂, -CH₂-CH₂-NH-CH₃, -CH₂-CH₂-N(CH₃)₂, -CH₂-CH₂-N(C₂H₅)₂, -CH₂-CH₂-NH-C₂H₅, -CH₂-CH₂-CH₂-NH-CH₃, -CH₂-CH₂-CH₂-N(CH₃)₂, -CH₂-CH₂-CH₂-N(C₂H₅)₂ and -CH₂-CH₂-CH₂-NH-C₂H₅; a (hetero)cycloaliphatic radical selected from the group consisting of imidazolidinyl, aziridinyl, azetidinyl, pyrrolidinyl, piperidinyl, morpholinyl, thiomorpholinyl, piperazinyl, pyrazolidinyl and azepanyl, which may be bonded via a -(CH₂)_{1, 2 or 3}-group and which may be unsubstituted or optionally substituted with 1, 2, 3, 4 or 5 substituent(s) independently selected from the group consisting of methyl, ethyl, n-propyl, isopropyl, n-butyl, tert-butyl, sec-butyl and isobutyl or in general formula le R^{1e} additionally represents a -C(=O)-OR^{37e} moiety;
R^{2e}, R^{3e}, R^{4e} and R^{5e}, independently of one another, each represent a hydrogen atom; F, Cl, Br, 1, -NO₂; -O-CH₃; -O-C₂H₅; -O-CF₃; -O-CFH₂; -O-CF₂H; -O-CH₂-CF₃; -O-CF₂-CF₃; -S-CH₃; -S-C₂H₅; -S-CF₃; -S-CFH₂; -S-CF₂H; -S-CH₂-CF₃; -S-CF₂-CF₃; - N(R^{11e})-S(=O)₂-R^{12e}; or a radical selected from the group consisting of methyl, ethyl, n-propyl, isopropyl, tert-butyl, -CF₃, -CFH₂, -CF₂H, -CH₂-CF₃ and -CF₂-CF₃;
with the proviso that at least one of the substituents R^{2e}, R^{3e}, R^{4e} and R^{5e} represents a - N(R^{11e})-S(=O)₂-R^{12e} moiety;
R^{11e} represents a hydrogen atom, -S(=O)₂-R^{12e} or an alkyl radical selected from the group consisting of methyl, ethyl, n-propyl, isopropyl, n-butyl, sec-butyl, isobutyl and tert-butyl;
R^{12e} represents a radical selected from the group consisting of [1,3]-benzodioxolyl, [1,4]-benzodioxanyl, benzo[b]furanyl, benzo[b]thiophenyl, benzo[2,1,3]thiadiazolyl, [1,2,3]-benzothiadiazolyl, [2,1,3]-benzoxadiazolyl, [1,2,3]-benzoxadiazolyl, benzoxazolyl, benzothiazolyl, benzisoxazolyl, benzisothiazolyl and imidazo[2,1-b]thiazolyl, which may be unsubstituted or optionally substituted with 1, 2, 3, 4 or 5 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, F, Cl, Br, I, -CN, -CF₃, -OCF₃, -SCF₃, -CF₂H and -CFH₂;
or a radical selected from the group consisting of pyridinyl, pyrrolyl, imidazolyl, pyrazolyl, triazolyl, pyridazinyl, pyrimidinyl and pyrazinyl, which may be unsubstituted or optionally substituted with 1, 2, 3, 4 or 5 substituent(s) independently selected from the group consisting of F, Cl, -NO₂; methyl, ethyl, n-propyl, isopropyl, n-butyl, isobutyl, sec-butyl, tert-butyl, -CF₃, -CF₂H, -CFH₂, -CH₂-CF₃ and -CF₂-CF₃;
and R^{37e} represents a radical selected from the group consisting of methyl, ethyl, n-propyl, isopropyl, n-butyl, sec-butyl, isobutyl, tert-butyl, n-pentyl, n-hexyl, fluorenyl, fluorenylmethyl, phenyl, benzyl and naphthyl;
optionally in form of one of its stereoisomers, preferably enantiomers or diasteromers, a racemate or in form of a mixture of at least two of its stereoisomers, preferably enantiomers and/or diastereomers, in any mixing ratio, or a salt thereof, or a corresponding solvate thereof.

Also more particularly preferred is a substituted tetrahydroisoquinoline compound of general formula le and/or III, wherein
R^{1e} represents a hydrogen atom; or a radical selected from the group consisting of methyl, ethyl, n-propyl, n-butyl, n-pentyl and n-hexyl or in general formula le R^{1e} additionally represents a -C(=O)-OR^{37e} moiety;
R^{2e}, R^{3e}, R^{4e} and R^{5e}, independently of one another, each represent a hydrogen atom or -N(R^{11e})-S(=O)₂-R^{12e};
with the proviso that at least one of the substituents R^{2e}, R^{3e}, R^{4e} and R^{5e} represents a -N(R^{11e})-S(=O)₂-R^{12e} moiety;
R^{11e} represents a hydrogen atom, -S(=O)₂-R^{12e} or an alkyl radical selected from the group consisting of methyl, ethyl and n-propyl;
R^{12e} represents a radical selected from the group consisting of benzo[b]thiophenyl and imidazo[2,1-b]thiazolyl, which may be unsubstituted or optionally substituted with 1, 2, 3, 4 or 5 substituent(s) independently selected from the group consisting of methyl, ethyl, n-propyl, isopropyl, F, Cl and Br;
or a pyridinyl radical, which may be unsubstituted or optionally substituted with 1, 2, 3, 4 or 5 substituent(s) independently selected from the group consisting of F, Cl, methyl, ethyl, n-propyl, isopropyl, n-butyl, isobutyl, sec-butyl and tert-butyl;
and R^{37e} represents a radical selected from the group consisting of methyl, ethyl, n-propyl, isopropyl, n-butyl, sec-butyl, isobutyl, tert-butyl, n-pentyl, n-hexyl, fluorenyl, fluorenylmethyl, phenyl, benzyl and naphthyl;
optionally in form of one of its stereoisomers, preferably enantiomers or diasteromers, a racemate or in form of a mixture of at least two of its stereoisomers, preferably enantiomers and/or diastereomers, in any mixing ratio, or a salt thereof, or a corresponding solvate thereof.

Even more particularly preferred is a substituted tetrahydroisoquinoline compound of general formula le and/or III selected from the group consisting of
[4] 5-chloro-3-methyl-N-(1,2,3,4-tetrahydroisoquinolin-6-yl)benzo[b]thiophene-2-sulfonamide hydrochloride,
[5] 5-chloro-3-methyl-N-(2-methyl-1,2,3,4-tetrahydroisoquinolin-6-yl)benzo[b]thiophene-2-sulfonamide hydrochloride,
[10] 6-chloro-N-(1,2,3,4-tetrahydroisoquinolin-6-yl)imidazo[2,1-b]thiazole-5-sulfonamide hydrochloride,
[12] N-(1,2,3,4-tetrahydroisoquinolin-6-yl)pyridine-3-sulfonamide dihydrochloride,
[14] 6-(5-chloro-3-methyl-benzo[b]thiophene-2- sulfonylamino)-3,4-dihydro-1H-isoquinoline-2-carboxylic acid tert-butyl ester and
[18] 6-(pyridine-3-sulfonylamino)-3,4-dihydro-1H-isoquinoline-2carboxylic acid tert-butyl ester;
   optionally in form of a corresponding solvate thereof.

In yet another aspect the present invention relates to a salt of a substituted tetrahydroisoquinoline compound of general formula If, 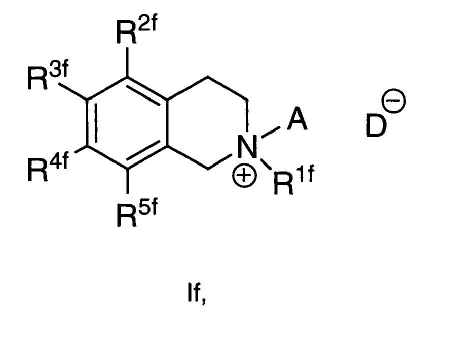 wherein
A represents a hydrogen atom or a radical selected from the group consisting of methyl, ethyl, n-propyl, isopropyl, n-butyl, isobutyl, 2-butyl, tert-butyl and n-pentyl;
D represents an anion selected from the group consisting of chloride, bromide, iodide, fluoride, hydrogensulfate, nitrate, dihydrogenphosphate, thiocyanate, cyanate, acrylate, methanesulfonate, ethanesulfonate, toluenesulfonate, benzenesulfonate, (2,5)-dihydroxy-benzenesulfonate, naphthalene-2-sulfonate, 5-sulfonapthalene-1-sulfonate, cyclamate, dodecane-1-sulfonate and (7,7)-dimethyl-2-oxobicyclo[2.2.1]-hept-1-yl-methanesulfonate;
R^{1f} represents a hydrogen atom; a radical selected from the group consisting of methyl, ethyl, n-propyl, isopropyl, n-butyl, sec-butyl, isobutyl, tert-butyl, n-pentyl, n-hexyl, -CH₂-NH₂, -CH₂-NH-CH₃, -CH₂-N(CH₃)₂, -CH₂-N(C₂H₅)₂, -CH₂-NH-C₂H₅, -CH₂-CH₂-NH₂, -CH₂-CH₂-NH-CH₃, -CH₂-CH₂-N(CH₃)₂, -CH₂-CH₂-N(C₂H₅)₂, -CH₂-CH₂-NH-C₂H₅, -CH₂-CH₂-CH₂-NH-CH₃, -CH₂-CH₂-CH₂-N(CH₃)₂, -CH₂-CH₂-CH₂-N(C₂H₅)₂ and -CH₂-CH₂-CH₂-NH-C₂H₅; or a (hetero)cycloaliphatic radical selected from the group consisting of imidazolidinyl, aziridinyl, azetidinyl, pyrrolidinyl, piperidinyl, morpholinyl, thiomorpholinyl, piperazinyl, pyrazolidinyl and azepanyl, which may be bonded via a - (CH₂)_{1, 2 or 3}- group and which may be unsubstituted or optionally substituted with 1, 2, 3, 4 or 5 substituent(s) independently selected from the group consisting of oxo (=O), thioxo (=S), methyl, ethyl, n-propyl, isopropyl, n-butyl, tert-butyl, sec-butyl, isobutyl, -C(=O)-CH₃, -C(=O)-C₂H₅, -C(=O)-CH₂-CH₂-CH₃, -C(=O)-CH(CH₃)₂, -C(=O)-C(CH₃)₃, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃;
R^{2f}, R^{3f}, R^{4f} and R^{5f}, independently of one another, each represent a hydrogen atom; F, Cl, Br, I, -NO₂; -NH₂; -SH; -OH; -CN; -C(=O)-OH; -C(=O)-H; -S(=O)₂-OH; -C(=O)-NH₂; -S(=O)₂-NH₂; -OR^{8f}; -SR^{9f}; -N(R^{11f})-S(=O)₂-R^{12f}; a radical selected from the group consisting of methyl, ethyl, n-propyl, isopropyl, n-butyl, sec-butyl, isobutyl, tert-butyl, vinyl, allyl, ethinyl, -CF₃, -CFH₂, -CF₂H, -CH₂-CF₃ and -CF₂-CF₃; or a radical selected from the group consisting of cyclopropyl, cyclobutyl, cyclopentyl and cyclohexyl;
with the proviso that at least one of the substituents R^{2f}, R^{3f}, R^{4f} and R^{5f} represents a -N(R^{11f})-S(=O)₂-R^{12f} moiety;
R^{8f} and R^{9f}, independently of one another, each represent a radical selected from the group consisting of methyl, ethyl, n-propyl, isopropyl, n-butyl, sec-butyl, isobutyl, tert-butyl, -CF₃, -CFH₂, -CF₂H, -CH₂-CF₃ and -CF₂-CF₃; a (hetero)cycloaliphatic radical selected from the group consisting of cyclopropyl, cyclobutyl, cyclopentyl and cyclohexyl; or an aryl or heteroaryl radical selected from the group consisting of phenyl, naphthyl, furyl (furanyl), thienyl (thiophenyl), pyrrolyl, and pyridinyl, which may be bonded via a -(CH₂)_{1, 2 or 3}-group and which may be unsubstituted or optionally substituted with 1, 2 or 3 substituent(s) independently selected from the group consisting of methyl, ethyl, n-propyl, isopropyl, n-butyl, tert-butyl, sec-butyl, isobutyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, F, Cl, Br, I, -CN, -CF₃, -OCF₃, -SCF₃, -OH, -SH, -NH₂, -NO₂, -CHO, -CF₂H and -CFH₂;
R^{11f} represents a hydrogen atom, -S(=O)₂-R^{12f} or an alkyl radical selected from the group consisting of methyl, ethyl, n-propyl, isopropyl, n-butyl, sec-butyl, isobutyl and tert-butyl;
R^{12f} represents a phenyl radical of general formula (Af), 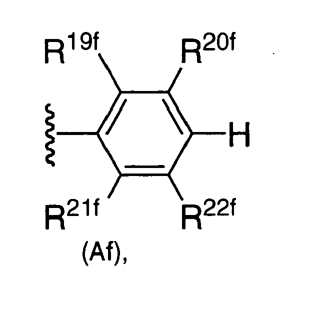 wherein
R^{19f}, R^{20f}, R^{21f} and R^{22f}, independently of one another, each represent a hydrogen atom; F, Cl, Br, I, -NO₂; -NH₂; -SH; -OH; -CN; -C(=O)-OH; -C(=O)-H; -S(=O)₂-OH; -C(=O)-NH₂; -S(=O)₂-NH₂; -C(=O)-R^{23f}; -S(=O)-R^{24f}; -S(=O)₂-R^{24f}; -OR^{25f}; -SR^{26f}; -C(=O)-OR^{27f}; methyl, ethyl, n-propyl, isopropyl, n-butyl, isobutyl, sec-butyl, tert-butyl, -CF₃, -CF₂H, - CFH₂, -CH₂-CF₃, -CF₂-CF₃, cyclopropyl, cyclobutyl, cyclopentyl and cyclohexyl;
with the proviso that at least one of the substituents R^{19f}, R^{20f}, R^{21f} and R^{22f} is unlike hydrogen;
or a naphthyl radical, which may be unsubstituted or optionally substituted with 1, 2, 3, 4 or 5 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, F, Cl, Br, I, -CN, -CF₃, -OCF₃, -SCF₃, -OH, -SH, -NH₂, -NO₂, - CHO, -CF₂H and -CFH₂;
R^{23f} and R^{27f}, independently of one another, each represent a radical selected from the group consisting of methyl, ethyl, n-propyl, isopropyl, n-butyl, sec-butyl, isobutyl, tert-butyl, n-pentyl, n-hexyl, -CF₃, -CFH₂, -CF₂H, -CH₂-CF₃ and -CF₂-CF₃; or an aryl or heteroaryl radical selected from the group consisting of phenyl, naphthyl, furyl (furanyl), thienyl (thiophenyl), pyrrolyl, and pyridinyl, which may be bonded via a -(CH₂)₁, _{2 or 3}-group and which may be unsubstituted or optionally substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, F, Cl, Br, I, -CN, -CF₃, -OCF₃, -SCF₃, -OH, -SH, -NH₂-NO₂, -CHO, -CF₂H and -CFH₂;
R^{24f} and R^{26f}, independently of one another, each represent a radical selected from the group consisting of methyl, ethyl, n-propyl, isopropyl, n-butyl, sec-butyl, isobutyl, tert-butyl, n-pentyl, n-hexyl, vinyl, allyl, ethinyl, -CF₃, -CFH₂, -CF₂H, -CH₂-CF₃ and -CF₂-CF₃; or an aryl or heteroaryl radical selected from the group consisting of phenyl, naphthyl, furyl (furanyl), thienyl (thiophenyl), pyrrolyl, and pyridinyl, which may be unsubstituted or optionally substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, F, Cl, Br, I, -CN, -CF₃, -OCF₃, -SCF₃, -OH, -SH, -NH₂, -NO₂, -CHO, -CF₂H and -CFH₂;
and R^{25f} represents a radical selected from the group consisting of methyl, ethyl, n-propyl, isopropyl, n-butyl, sec-butyl, isobutyl, tert-butyl, -CF₃, -CFH₂, -CF₂H, -CH₂-CF₃ and -CF₂-CF₃.

Preferred is a salt of a substituted tetrahydroisoquinoline compound of general formula If, wherein
A represents a hydrogen atom or a radical selected from the group consisting of methyl, ethyl, n-propyl, isopropyl, n-butyl, isobutyl, 2-butyl, tert-butyl and n-pentyl;
D represents an anion selected from the group consisting of chloride, bromide, iodide, fluoride, hydrogensulfate, nitrate, dihydrogenphosphate, thiocyanate, cyanate, acrylate, methanesulfonate, ethanesulfonate, toluenesulfonate, benzenesulfonate, (2,5)-dihydroxy-benzenesulfonate, naphthalene-2-sulfonate, 5-sulfonapthalene-1-sulfonate, cyclamate, dodecane-1-sulfonate and (7,7)-dimethyl-2-oxobicyclo[2.2.1]-hept-1-yl-methanesulfonate;
R^{1f} represents a hydrogen atom; a radical selected from the group consisting of methyl, ethyl, n-propyl, isopropyl, n-butyl, sec-butyl, isobutyl, tert-butyl, n-pentyl, n-hexyl, -CH₂-NH₂, -CH₂-NH-CH₃, -CH₂-N(CH₃)₂, -CH₂-N(C₂H₅)₂, -CH₂-NH-C₂H₅, -CH₂-CH₂-NH₂, -CH₂-CH₂-NH-CH₃, -CH₂-CH₂-N(CH₃)₂, -CH₂-CH₂-N(C₂H₅)₂, -CH₂-CH₂-NH-C₂H₅, -CH₂-CH₂-CH₂-NH-CH₃, -CH₂-CH₂-CH₂-N(CH₃)₂, -CH₂-CH₂-CH₂-N(C₂H₅)₂ and - CH₂-CH₂-CH₂-NH-C₂H₅; or a (hetero)cycloaliphatic radical selected from the group consisting of imidazolidinyl, aziridinyl, azetidinyl, pyrrolidinyl, piperidinyl, morpholinyl, thiomorpholinyl, piperazinyl, pyrazolidinyl and azepanyl, which may be bonded via a - (CH₂)₁, _{2 or 3}- group and which may be unsubstituted or optionally substituted with 1, 2, 3, 4 or 5 substituent(s) independently selected from the group consisting of methyl, ethyl, n-propyl, isopropyl, n-butyl, tert-butyl, sec-butyl and isobutyl;
R^{2f}, R^{3f}, R^{4f} and R^{5f}, independently of one another, each represent a hydrogen atom; F, Cl, Br, l, -NO₂; -O-CH₃; -O-C₂H₅; -O-CF₃; -O-CFH₂; -O-CF₂H; -O-CH₂-CF₃; -O-CF₂-CF₃; -S-CH₃; -S-C₂H₅; -S-CF₃; -S-CFH₂; -S-CF₂H; -S-CH₂-CF₃; -S-CF₂-CF₃; -N(R^{11f})-S(=O)₂-R^{12f}; or a radical selected from the group consisting of methyl, ethyl, n-propyl, isopropyl, tert-butyl, -CF₃, -CFH₂, -CF₂H, -CH₂-CF₃ and -CF₂-CF₃;
with the proviso that at least one of the substituents R^{2f}, R^{3f}, R^{4f} and R^{5f} represents a -N(R^{11f})-S(=O)₂-R^{12f} moiety;
R^{11f} represents a hydrogen atom, -S(=O)₂-R^{12f} or an alkyl radical selected from the group consisting of methyl, ethyl, n-propyl, isopropyl, n-butyl, sec-butyl, isobutyl and tert-butyl;
R^{12f} represents a phenyl radical of general formula (Af), 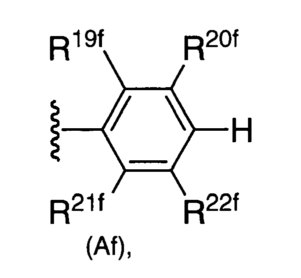 wherein
R^{19f}, R^{20f}, R^{21f} and R^{22f}, independently of one another, each represent a hydrogen atom; F, Cl, Br, l, -NO₂; -NH₂; -SH; -OH; -CN; -C(=O)-OH; -C(=O)-H; -C(=O)-CH₃; -C(=O)-C₂H₅; -O-CH₃; -O-C₂H₅; -O-CF₃; -O-CFH₂; -O-CF₂H; -O-CH₂-CF₃; -O-CF₂-CF₃; -S-CH₃; -S-C₂H₅; -S-CF₃; -S-CFH₂; -S-CF₂H; -S-CH₂-CF₃; -S-CF₂-CF₃; -C(=O)-OCH₃; -C(=O)-OC₂H₅; methyl, ethyl, n-propyl, isopropyl, n-butyl, isobutyl, sec-butyl, tert-butyl, -CF₃, - CF₂H, -CFH₂, -CH₂-CF₃ and -CF₂-CF₃;
with the proviso that at least one of the substituents R^{19f}, R^{20f}, R^{21f} and R^{22f} is unlike hydrogen;
or a naphthyl radical, which may be unsubstituted or optionally substituted with 1, 2, 3, 4 or 5 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, F, Cl, Br, I, -CN, -CF₃, -OCF₃, -SCF₃, -CF₂H and -CFH₂.

More preferred is a salt of a substituted tetrahydroisoquinoline compound of general formula If, wherein
R^{1f} represents a hydrogen atom; or a radical selected from the group consisting of methyl, ethyl, n-propyl, n-butyl, n-pentyl and n-hexyl;
R^{2f}, R^{3f}, R^{4f} and R^{5f}, independently of one another, each represent a hydrogen atom or -N(R^{11f})-S(=O)₂-R^{12f};
with the proviso that at least one of the substituents R^{2f}, R^{3f}, R^{4f} and R^{5f} represents a -N(R^{11f})-S(=O)₂-R^{12f} moiety;
R^{11f} represents a hydrogen atom, -S(=O)₂-R^{12f} or an alkyl radical selected from the group consisting of methyl, ethyl and n-propyl;
R^{12f} represents a phenyl radical of general formula (Af), 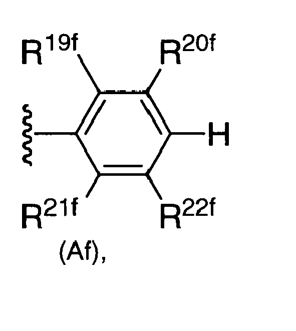 wherein
R^{19f}, R^{20f}, R^{21f} and R^{22f}, independently of one another, each represent a hydrogen atom; F, Cl, Br, I, -O-CH₃; -O-C₂H₅; -O-CF₃; -O-CFH₂; -O-CF₂H; -O-CH₂-CF₃; -O-CF₂-CF₃; methyl, ethyl, n-propyl, isopropyl, n-butyl, isobutyl, sec-butyl and tert-butyl;
with the proviso that at least one of the substituents R^{19f}, R^{20f}, R^{21f} and R^{22f} is unlike hydrogen;
or a naphthyl radical, which may be unsubstituted or optionally substituted with 1, 2, 3, 4 or 5 substituent(s) independently selected from the group consisting of methyl, ethyl, n-propyl, isopropyl, F, Cl and Br.

Even more preferred is a salt of a substituted tetrahydroisoquinoline compound of general formula If, wherein
[1] N-(1,2,3,4-tetrahydroisoquinolin-6-yl)naphthalene-1-sulfonamide hydrochloride,
[2] 2,2-dimethyl-6-(N-methylnaphthalene-1-sulfonamido)-1,2,3,4-tetrahydroisoquinolinium iodide,
[3] N-(2-methyl-1,2,3,4-tetrahydroisoquinolin-6-yl)naphthalene-1-sulfonamide hydrochloride,
[6] 4-methyl-N-(1,2,3,4-tetrahydroisoquinolin-6-yl)naphthalene-1-sulfonamide hydrochloride,
[7] 4-methyl-N-(2-methyl-1,2,3,4-tetrahydroisoquinolin-6-yl)naphthalene-1-sulfonamide hydrochloride,
[8] N-(1,2,3,4-tetrahydroisoquinolin-6-yl)naphthalene-2-sulfonamide hydrochloride,
[9] N-(2-methyl-1,2,3,4-tetrahydroisoquinolin-6-yl)naphthalene-2-sulfonamide hydrochloride and
[11] 2-methoxy-5-methyl-N-(1,2,3,4-tetrahydroisoquinolin-6-yl)benzenesulfonamide hydrochloride,
   or a corresponding solvate thereof.

In yet another aspect the present invention relates to a substituted tetrahydroisoquinoline compound of general formula Ig, 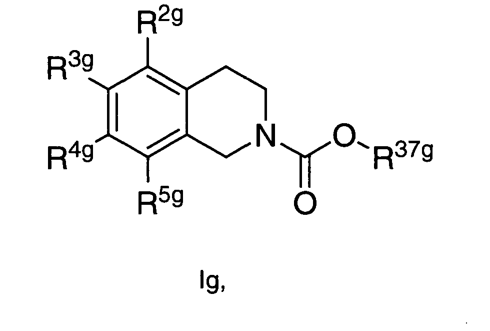 wherein
R^{1g} represents a hydrogen atom; a radical selected from the group consisting of methyl, ethyl, n-propyl, isopropyl, n-butyl, sec-butyl, isobutyl, tert-butyl, n-pentyl, n-hexyl, -CH₂-NH₂, -CH₂-NH-CH₃, -CH₂-N(CH₃)₂, -CH₂-N(C₂H₅)₂, -CH₂-NH-C₂H₅, -CH₂-CH₂-NH₂, -CH₂-CH₂-NH-CH₃, -CH₂-CH₂-N(CH₃)₂, -CH₂-CH₂-N(C₂H₅)₂, -CH₂-CH₂-NH-C₂H₅, -CH₂-CH₂-CH₂-NH-CH₃, -CH₂-CH₂-CH₂-N(CH₃)₂, -CH₂-CH₂-CH₂-N(C₂H₅)₂ and - CH₂-CH₂-CH₂-NH-C₂H₅; or a (hetero)cycloaliphatic radical selected from the group consisting of imidazolidinyl, aziridinyl, azetidinyl, pyrrolidinyl, piperidinyl, morpholinyl, thiomorpholinyl, piperazinyl, pyrazolidinyl and azepanyl, which may be bonded via a - (CH₂)_{1, 2 or 3}- group and which may be unsubstituted or optionally substituted with 1, 2, 3, 4 or 5 substituent(s) independently selected from the group consisting of oxo (=O), thioxo (=S), methyl, ethyl, n-propyl, isopropyl, n-butyl, tert-butyl, sec-butyl, isobutyl, - C(=O)-CH₃, -C(=O)-C₂H₅, -C(=O)-CH₂-CH₂-CH₃, -C(=O)-CH(CH₃)₂, -C(=O)-C(CH₃)₃, - C(=O)-NH₂, -C(=O)-NH-CH₃, -C(=O)-NH-C₂H₅, -C(=O)-N(CH₃)₂, -C(=O)-N(C₂H₅)₂ and - S(=O)₂-CH₃;
R^{2g}, R^{3g}, R^{4g} and R^{5g}, independently of one another, each represent a hydrogen atom; F, Cl, Br, I, -NO₂; -NH₂; -SH; -OH; -CN; -C(=O)-OH; -C(=O)-H; -S(=O)₂-OH; - C(=O)-NH₂; -S(=O)₂-NH₂; -OR^{8g}; -SR^{9g}; -N(R^{11g})-S(=O)₂-R^{12g}; a radical selected from the group consisting of methyl, ethyl, n-propyl, isopropyl, n-butyl, sec-butyl, isobutyl, tert-butyl, vinyl, allyl, ethinyl, -CF₃, -CFH₂, -CF₂H, -CH₂-CF₃ and -CF₂-CF₃; or a radical selected from the group consisting of cyclopropyl, cyclobutyl, cyclopentyl and cyclohexyl;
with the proviso that at least one of the substituents R^{2g}, R^{3g}, R^{4g} and R^{5g} represents a - N(R^{11g})-S(=O)₂-R^{12g} moiety;
R^{8g} and R^{9g}, independently of one another, each represent a radical selected from the group consisting of methyl, ethyl, n-propyl, isopropyl, n-butyl, sec-butyl, isobutyl, tert-butyl, -CF₃, -CFH₂, -CF₂H, -CH₂-CF₃ and -CF₂-CF₃; a (hetero)cycloaliphatic radical selected from the group consisting of cyclopropyl, cyclobutyl, cyclopentyl and cyclohexyl; or an aryl or heteroaryl radical selected from the group consisting of phenyl, naphthyl, furyl (furanyl), thienyl (thiophenyl), pyrrolyl, and pyridinyl, which may be bonded via a - (CH₂)₁, _{2 or 3}-group and which may be unsubstituted or optionally substituted with 1, 2 or 3 substituent(s) independently selected from the group consisting of methyl, ethyl, n-propyl, isopropyl, n-butyl, tert-butyl, sec-butyl, isobutyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, F, Cl, Br, I, -CN, - CF₃, -OCF₃, -SCF₃, -OH, -SH, -NH₂, -NO₂, -CHO, -CF₂H and -CFH₂;
R^{11g} represents a hydrogen atom, -S(=O)₂-R^{12g} or an alkyl radical selected from the group consisting of methyl, ethyl, n-propyl, isopropyl, n-butyl, sec-butyl, isobutyl and tert-butyl;
R^{12g} represents a phenyl radical of general formula (Ag), 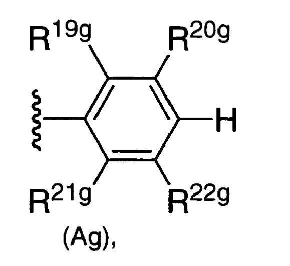 wherein
R^{19g}, R^{20g}, R^{21g} and R^{22g}, independently of one another, each represent a hydrogen atom; F, Cl, Br, I, -NO₂; -NH₂; -SH; -OH; -CN; -C(=O)-OH; -C(=O)-H; -S(=O)₂-OH; - C(=O)-NH₂; -S(=O)₂-NH₂; -C(=O)-R^{23g}; -S(=O)-R^{24g}; -S(=O)₂-R^{24g}; -OR^{25g} ; -SR^{26g}; - C(=O)-OR^{27g}; methyl, ethyl, n-propyl, isopropyl, n-butyl, isobutyl, sec-butyl, tert-butyl, - CF₃, -CF₂H, -CFH₂, -CH₂-CF₃, -CF₂-CF₃, cyclopropyl, cyclobutyl, cyclopentyl and cyclohexyl;
with the proviso that at least one of the substituents R^{19g}, R^{20g}, R^{21g} and R^{22g} is unlike hydrogen;
or a naphthyl radical, which may be unsubstituted or optionally substituted with 1, 2, 3, 4 or 5 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, F, Cl, Br, 1, -CN, -CF₃, -OCF₃, -SCF₃, -OH, -SH, -NH₂, -NO₂, - CHO, -CF₂H and -CFH₂;
R^{23g} and R^{27g}, independently of one another, each represent a radical selected from the group consisting of methyl, ethyl, n-propyl, isopropyl, n-butyl, sec-butyl, isobutyl, tert-butyl, n-pentyl, n-hexyl, -CF₃, -CFH₂, -CF₂H, -CH₂-CF₃ and -CF₂-CF₃; or an aryl or heteroaryl radical selected from the group consisting of phenyl, naphthyl, furyl (furanyl), thienyl (thiophenyl), pyrrolyl, and pyridinyl, which may be bonded via a -(CH₂)₁, _{2 or 3}-group and which may be unsubstituted or optionally substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, F, Cl, Br, I, -CN, -CF₃, -OCF₃, -SCF₃, -OH, -SH, -NH₂-NO₂, -CHO, -CF₂H and -CFH₂;
R^{24g} and R^{26g}, independently of one another, each represent a radical selected from the group consisting of methyl, ethyl, n-propyl, isopropyl, n-butyl, sec-butyl, isobutyl, tert-butyl, n-pentyl, n-hexyl, vinyl, allyl, ethinyl, -CF₃, -CFH₂, -CF₂H, -CH₂-CF₃ and -CF₂-CF₃; or an aryl or heteroaryl radical selected from the group consisting of phenyl, naphthyl, furyl (furanyl), thienyl (thiophenyl), pyrrolyl, and pyridinyl, which may be unsubstituted or optionally substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, F, Cl, Br, I, -CN, -CF₃, -OCF₃, -SCF₃, -OH, -SH, -NH₂, -NO₂, -CHO, -CF₂H and -CFH₂;
R^{25g} represents a radical selected from the group consisting of methyl, ethyl, n-propyl, isopropyl, n-butyl, sec-butyl, isobutyl, tert-butyl, -CF₃, -CFH₂, -CF₂H, -CH₂-CF₃ and -CF₂-CF₃
and R^{37g} represents a radical selected from the group consisting of methyl, ethyl, n-propyl, isopropyl, n-butyl, sec-butyl, isobutyl, tert-butyl, n-pentyl, n-hexyl, fluorenyl, fluorenylmethyl, phenyl, benzyl and naphthyl.

Preferred is a substituted tetrahydroisoquinoline compound of general formula Ig, wherein
R^{1g} represents a hydrogen atom; or a radical selected from the group consisting of methyl, ethyl, n-propyl, n-butyl, n-pentyl and n-hexyl;
R^{2g}, R^{3g}, R^{4g} and R^{5g}, independently of one another, each represent a hydrogen atom or -N(R^{11g})-S(=O)₂-R^{12g};
with the proviso that at least one of the substituents R^{2g}, R^{3g}, R^{4g} and R^{5g} represents a - N(R^{11g})-S(=O)₂-R^{12g} moiety;
R^{11g} represents a hydrogen atom, -S(=O)₂-R^{12g} or an alkyl radical selected from the group consisting of methyl, ethyl and n-propyl;
R^{12g} represents a phenyl radical of general formula (Ag), 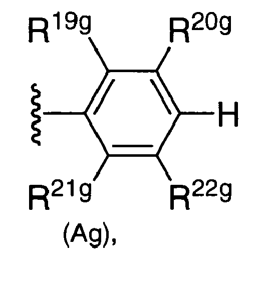 wherein
R^{19g}, R^{20g}, R^{21g} and R^{22g}, independently of one another, each represent a hydrogen atom; F, Cl, Br, I, -O-CH₃; -O-C₂H₅; -O-CF₃; -O-CFH₂; -O-CF₂H; -O-CH₂-CF₃; -O-CF₂-CF₃; methyl, ethyl, n-propyl, isopropyl, n-butyl, isobutyl, sec-butyl and tert-butyl;
with the proviso that at least one of the substituents R^{19g}, R^{20g} , R^{21g} and R^{22g} is unlike hydrogen;
or a naphthyl radical, which may be unsubstituted or optionally substituted with 1, 2, 3, 4 or 5 substituent(s) independently selected from the group consisting of methyl, ethyl, n-propyl, isopropyl, F, Cl and Br;
and R^{37g} represents a radical selected from the group consisting of methyl, ethyl, n-propyl, isopropyl, n-butyl, sec-butyl, isobutyl, tert-butyl, n-pentyl, n-hexyl, fluorenyl, fluorenylmethyl, phenyl, benzyl and naphthyl.

More preferred is a substituted tetrahydroisoquinoline compound of general formula lg, wherein
[13] 6-(naphthalene-1-sulfonylamino)-3,4-dihydro-1H-isoquinoline-2-carboxylic acid tert-butyl ester,
[15] 6-(4-methyl-naphthalene-1-sulfonylamino)-3,4 -dihydro-1 H-isoquinoline-2-carboxylic acid tert-butyl ester,
[16] 6-(naphthalene-2-sulfonylamino)-3,4-dihydro-1 H-isoquinoline-2-carboxylic acid tert-butyl ester and
[17] 6-(2-methoxy-5-methyl-benzenesulfonylamino)-3,4-dihydro-1 H-isoquinoline-2-carboxylic acid tert-butyl ester.

If any of the substituents in any of the above defined formulae represents or comprises a 3- to 9-membered (hetero)cycloaliphatic radical, C₃₋₉ cycloalkyl radical or C₄₋₉ cycloalkenyl radical, said (hetero)cycloaliphatic radical, C₃₋₉ cycloalkyl radical or C₄₋₉ cycloalkenyl radical may - if not defined otherwise - be unsubstituted or substituted by one or more substituents, preferably unsubstituted or optionally substituted with 1, 2, 3, 4 or 5 substituent(s). Said substituent(s) may preferably be selected independently from the group consisting of oxo (=O), thioxo (=S), C₁₋₅-alkyl, -O-C₁₋₅-alkyl, -S-C₁₋₅-alkyl, - C(=O)-OH, -C(=O)-C₁₋₅-alkyl, -C(=O)-O-C₁₋₅-alkyl, -O-C(=O)-C₁₋₅-alkyl, F, Cl, Br, I, -CN, - CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, - S(=O)₂-phenyl, phenyl, phenoxy and benzyl; whereby in each occurrence C₁₋₅-alkyl may be linear or branched and whereby said cyclic substituents may be unsubstituted or substituted by 1, 2 or 3 substituent(s) independently selected from the group consisting of methyl, ethyl, n-propyl, isopropyl, methoxy, ethoxy, F, Cl, Br, -CN, -CF₃, -OCF₃, - SCF₃, -OH, -SH, -NH₂ and -NO₂.

More preferably said substituents may be selected independently from the group consisting of oxo (=O), thioxo (=S), methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, - C(=O)-O-CH₃, -C(=O)-O-C₂H₅, -C(=O)-O-CH₃-CH₃-CH₃, -C(=O)-O-CH(CH₃)₂, -C(=O)-O-C(CH₃)₃, -C(=O)-CH₃, -C(=O)-C₂H₅, -C(=O)-CH₃-CH₃-CH₃, -C(=O)-CH(CH₃)₂, -C(=O)-C(CH₃)₃, F, Cl, Br, I, -CN, -CF₃, -OCF₃, -SCF₃, -OH, -SH, -NH₂, -NH-CH₃, -NH-C₂H₅, - NH-CH₂-CH₂-CH₃, -NH-CH(CH₃)₂, -NH-C(CH₃)₃, -N(CH₃)₂, -N(C₂H₅)₂, -NO₂, -CHO, - CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂, -S(=O)₂-CH₃, -S(=O)₂-phenyl, phenyl, phenoxy and benzyl; whereby in each occurrence said cyclic substituents may be unsubstituted or substituted by 1, 2 or 3 substituent(s) independently selected from the group consisting of methyl, ethyl, n-propyl, isopropyl, methoxy, ethoxy, F, Cl, Br,-CN, -CF₃, -OCF₃, -SCF₃, -OH, -SH, -NH₂ and -NO₂.

If any of the substituents in any of the above defined formulae represents or comprises a cycloaliphatic radical, C₃₋₉ cycloalkyl radical or C₄₋₉ cycloalkenyl radical which contains one or more, preferably 1, 2 or 3 heteroatom(s) as ring member(s), unless defined otherwise, each of these heteroatom(s) may preferably be selected independently from the group consisting of N, O and S.

Suitable saturated or unsaturated, optionally at least one heteroatom as ring member containing cycloaliphatic radicals, C₃₋₉ cycloalkyl radicals or C₄₋₉ cycloalkenyl radicals may preferably be selected from the group consisting of cyclopropyl, cyclobutyl, cyclopentyl, cyclohexyl, cycloheptyl, cyclooctyl, cyclononyl, cyclopentenyl, cyclohexenyl, cycloheptenyl, cyclooctenyl, pyrrolidinyl, piperidinyl, piperazinyl, homopiperazinyl, morpholinyl, aziridinyl, azetidinyl, imidazolidinyl, thiomorpholinyl, pyrazolidinyl, tetrahydrofuranyl, tetrahydrothiophenyl, azepanyl, diazepanyl, azocanyl, (2,5)-dihydrofuranyl, (2,5)-dihydrothiophenyl, (2,3)-dihydrofuranyl, (2,3)-dihydrofuranyl, (2,5)-dihydro-1 H-pyrrolyl, (2,3)-dihydro-1 H-pyrrolyl, tetrahydrothiopyranyl, tetrahydropyranyl, (3,4)-dihydro-2H-pyranyl, (3,4)-dihydro-2H-thiopyranyl, (1,2,3,6)-tetrahydropyridinyl, (1,2,3,4)-tetrahydropyridinyl, (1,2,5,6)-tetrahydropyridinyl, [1,3]-oxazinanyl, hexahydropyrimidinyl, (5,6)-dihydro-4H-pyrimidinyl, oxazolidinyl, (1,3)-dioxanyl, (1,4)-dioxanyl and (1,3)-dioxolanyl.

Suitable saturated or unsaturated, optionally at least one heteroatom as ring member containing cycloaliphatic radicals, C₃₋₉ cycloalkyl radicals or C₄₋₉ cycloalkenyl radicals which are condensed with an unsubstituted or at least mono-substituted mono- or bicyclic ring system may preferably be selected from the group consisting of indolinyl, isoindolinyl, decahydronaphthyl, (1,2,3,4)-tetrahydroquinolinyl, (1,2,3,4)-tetrahydroisoquinolinyl, (1,2,3,4)-tetrahydronaphthyl, octahydro-cyclopenta[c]pyrrolyl, (1,3,4,7,9a)-hexahydro-2H-quinolizinyl, (1,2,3,5,6,8a)-hexahydro-indolizinyl, decahydroquinolinyl, dodecahydrocarbazolyl, 9H-carbazolyl, decahydroisoquinolinyl, (6,7)-dihydro-4H-thieno[3,2-c]pyridinyl, (2,3)-dihydro-1 H-benzo[de]isoquinolinyl, fluorenyl and (1,2,3,4)-tetrahydroquinoxazlinyl.

If any of the substituents in any of the above defined formulae represents an alkylene group, preferably an C₁₋₆ alkylene group, an alkenylene group, preferably an C₂₋₆ alkenylene group or an alkinylene group, preferably an C₂₋₆ alkinylene group, which may be substituted, said alkylene group, C₂₋₆ alkylene group, alkenylene group, C₂₋₆ alkenylene group, alkinylene group or C₂₋₆ alkinylene group may be unsubstituted or substituted by one or more substituents, preferably unsubstituted or optionally substituted with 1, 2 or 3 substituent(s). Said substituent(s) may preferably be selected independently from the group consisting of -O-C₁₋₅-alkyl, -S-C₁₋₅-alkyl, -F, Cl, Br, I, -CN, - CF₃, -OCF₃, -SCF₃, -OH, -SH, -NH₂, -NH(C₁₋₅-alkyl) and -N(C₁₋₅-alkyl)₂, whereby in each occurrence C₁₋₅-alkyl may be linear or branched. An alkenylene group comprises at least one carbon-carbon double bond, an alkinylene group comprises at least one carbon-carbon triple bond.

Suitable alkylene groups include -(CH₂)-, -CH(CH₃)-, -CH(phenyl), -(CH₂)₂-, -(CH₂)₃-,-(CH₂)₄-,-(CH₂)₅ and -(CH₂)₆-, suitable alkenylene groups include -CH=CH-, -CH₂-CH=CH- and -CH=CH-CH₂- and suitable alkinylene groups include -C≡C- , -CH₂-C≡C- and -C≡C-CH₂-.

If any of the substituents in any of the above defined formulae represents or comprises an aryl radical, including a 6-membered aryl radical such as phenyl or a 10-membered aryl radical such as naphthyl or a 14-membered aryl radical such as anthracenyl, said aryl radical may - if not defined otherwise - be unsubstituted or substituted by one or more substituents, preferably unsubstituted or substituted with 1, 2, 3, 4 or 5 substituent(s). Said substituent(s) may preferably be selected independently from the group consisting of C₁₋₅-alkyl, -O-C₁₋₅-alkyl, -S-C₁₋₅-alkyl, -C(=O)-OH, -C(=O)-C₁₋₅-alkyl, - C(=O)-O-C₁₋₅-alkyl, -O-C(=O)-C₁₋₅-alkyl, F, Cl, Br, I, -CN, -CF₃, -OCF₃, -SCF₃, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl; whereby in each occurrence C₁₋₅-alkyl may be linear or branched and whereby said cyclic substituent(s) may be unsubstituted or substituted by 1, 2 or 3 substituent(s) independently selected from the group consisting of methyl, ethyl, n-propyl, isopropyl, methoxy, ethoxy, F, Cl, Br, -CN, -CF₃, -OCF₃, -SCF₃, -OH, -SH, -NH₂ and -NO₂.

More preferably said substituents may be selected independently from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₃-CH₃-CH₃, -C(=O)-O-CH(CH₃)₂, -C(=O)-O-C(CH₃)₃, -C(=O)-CH₃, -C(=O)-C₂H₅, -C(=O)-CH₃-CH₃-CH₃, -C(=O)-CH(CH₃)₂, -C(=O)-C(CH₃)₃, F, Cl, Br, I, -CN, -CF₃, - OCF₃, -SCF₃, -OH, -SH, -NH₂, -NH-CH₃, -NH-C₂H₅, -NH-CH₂-CH₂-CH₃, -NH-CH(CH₃)₂, - NH-C(CH₃)₃, -N(CH₃)₂, -N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃.

Preferred aryl radicals, which may optionally be at least mono-substituted, are phenyl and naphthyl.

If any of the substituents in any of the above defined formulae represents or comprises a heteroaryl radical, including a monocyclic 5- or 6-membered heteroaryl radical or a bi- or tricyclic 8-, 9-, 10-, 11-, 12-, 13- or 14 membered heteroaryl radical, said heteroaryl radical may - if not defined otherwise - be unsubstituted or substituted by one or more substituents, preferably unsubstituted or substituted with 1, 2, 3, 4 or 5 substituent(s). Said substituent(s) may preferably be selected independently from the group consisting of C₁₋₅-alkyl, -O-C₁₋₅-alkyl, -S-C₁₋₅-alkyl, -C(=O)-OH, -C(=O)-C₁₋₅-alkyl, -C(=O)-O-C₁₋₅-alkyl, -O-C(=O)-C₁₋₅-alkyl, F, Cl, Br, I, -CN, -CF₃, -OCF₃, -SCF₃, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), - C(=O)-N(C₁₋₅-alkyl)₂, -S(=O)₂-C₁₋₅-alkyl, -S(=O)₂-phenyl, phenyl, phenoxy and benzyl; whereby in each occurrence C₁₋₅-alkyl may be linear or branched and whereby said cyclic substituent(s) may be unsubstituted or substituted by 1, 2 or 3 substituent(s) independently selected from the group consisting of methyl, ethyl, n-propyl, isopropyl, methoxy, ethoxy, F, Cl, Br, -CN, -CF₃, -OCF₃, -SCF₃, -OH, -SH, -NH₂ and -NO₂.

More preferably said substituents may be selected independently from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₃-CH₃-CH₃, -C(=O)-O-CH(CH₃)₂, -C(=O)-O-C(CH₃)₃, -C(=O)-CH₃, -C(=O)-C₂H₅, -C(=O)-CH₃-CH₃-CH₃, -C(=O)-CH(CH₃)₂, -C(=O)-C(CH₃)₃, F, Cl, Br, I, -CN, -CF₃, - OCF₃, -SCF₃, -OH, -SH, -NH₂, -NH-CH₃, -NH-C₂H₅, -NH-CH₂-CH₂-CH₃, -NH-CH(CH₃)₂, - NH-C(CH₃)₃, -N(CH₃)₂, -N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃.

The heteroatom(s), which are present as ring member(s) in the heteroaryl radical, may, unless defined otherwise, independently be selected from the group consisting of nitrogen, oxygen and sulphur. Preferably the heteroaryl radical comprises 1, 2, 3 or 4 heteroatom(s).

Suitable bi- or tricyclic heteroaryl radicals, which may optionally be at least mono-substituted, may preferably be selected from the group consisting of indolyl, isoindolyl, quinolinyl, isoquinolinyl, benzo[b]furanyl, benzo[b]thiophenyl, benzo[2,1,3]thiadiazolyl, [1,2,3]-benzothiadiazolyl, [2,1,3]-benzoxadiazolyl, [1,2,3]-benzoxadiazolyl, benzoxazolyl, benzothiazolyl, benzisoxazolyl, benzisothiazolyl, imidazo[2,1-b]thiazolyl, 2H-chromenyl, indazolyl and quinazolinyl.

Suitable mono-, bi- or tricyclic heteroaryl radicals, which are condensed with an unsubstituted or at least mono-substituted saturated or unsaturated mono- or bicyclic ring system, may preferably be selected from the group consisting of [1,3]-benzodioxolyl, [1,4]-benzodioxanyl, [1,2,3,4]-tetrahydronaphthyl, (2,3)-dihydro-1 H-cyclopenta[b]indolyl, [1,2,3,4]-tetrahydroquinolinyl, [1,2,3,4]-tetrahydroisoquinolinyl, [1,2,3,4]-tetrahydroquinazolinyl and [3,4]-dihydro-2H-benzo[1,4]oxazinyl.

Suitable monocyclic heteroaryl radicals, which may optionally be at least mono-substituted, may preferably be selected from the group consisting of pyridinyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, pyrimidinyl, pyrazinyl and pyranyl.

A mono- or bicyclic ring system according to the present invention - if not defined otherwise - means a mono- or bicyclic hydrocarbon ring system that may be saturated, unsaturated or aromatic. Each of its different rings may show a different degree of saturation, i.e. it may be saturated, unsaturated or aromatic. Optionally each of the rings of the mono- or bicyclic ring system may contain one or more, preferably 1, 2 or 3, heteroatom(s) as ring member(s), which may be identical or different and which can preferably be selected from the group consisting of N, O and S. The rings of the mono-or bicyclic ring system are preferably 5-, 6- or 7-membered.

Preferably a mono-or bicyclic ring system according to the present invention is a phenyl or naphthyl ring system.

The term "condensed" according to the present invention means that a ring or ring system is attached to another ring or ring system, whereby the terms "annulated" or "annelated" are also used by those skilled in the art to designate this kind of attachment.

Such a mono- or bicyclic ring system may - if not defined otherwise - be unsubstituted or substituted by one or more substituents, preferably unsubstituted or substituted with 1, 2, 3, 4 or 5 substituent(s). Said substituents may preferably be selected independently from the group consisting of C₁₋₅-alkyl, -O-C₁₋₅-alkyl, -S-C₁₋₅-alkyl, -C(=O)-OH, oxo (=O), thioxo (=S), -C(=O)-O-C₁₋₅-alkyl, -O-C(=O)-C₁₋₅-alkyl, F, Cl, Br, I, -CN, -CF₃, -OCF₃, - SCF₃, -OH, -SH, -NH₂, -NH(C₁₋₅-alkyl), -N(C₁₋₅-alkyl)₂, -NO₂, -CHO, -CF₂H, -CFH₂, - C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl; whereby in each occurrence C₁₋₅-alkyl may be linear or branched and whereby said cyclic substituents may be unsubstituted or substituted by 1, 2 or 3 substituent(s) independently selected from the group consisting of methyl, ethyl, n-propyl, isopropyl, methoxy, ethoxy, F, Cl, Br, -CN, -CF₃, -OCF₃, - SCF₃, -OH, -SH, -NH₂ and -NO₂.

More preferably said substituents may be selected from the group consisting of oxo (=O), thioxo (=S), methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₃-CH₃-CH₃, -C(=O)-O-CH(CH₃)₂, -C(=O)-O-C(CH₃)₃, -C(=O)-CH₃, -C(=O)-C₂H₅, -C(=O)-CH₃-CH₃-CH₃, -C(=O)-CH(CH₃)₂, -C(=O)-C(CH₃)₃, F, Cl, Br, l, -CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂, -CHO, -CF₂H, -CFH₂, -C(=O)-NH₂, - C(=O)-NH-CH₃, -C(=O)-NH-C₂H₅, -C(=O)-N(CH₃)₂, -C(=O)-N(C₂H₅)₂, -S(=O)₂-CH₃, - S(=O)₂-phenyl, phenyl, phenoxy and benzyl; whereby in each occurrence said cyclic substituents may be unsubstituted or substituted by 1, 2 or 3 substituent(s) independently selected from the group consisting of methyl, ethyl, n-propyl, isopropyl, methoxy, ethoxy, F, Cl, Br, -CN, -CF₃, -OCF₃, -SCF₃, -OH, -SH, -NH₂ and -NO₂.

If any of the substituents in any of the above defined formulae represents a saturated or unsaturated aliphatic radical, i.e. an alkyl radical, preferably an C₁₋₁₀alkyl radical; an alkenyl radical, preferably an C₂₋₁₀ alkenyl radical or an alkinyl radical, preferably an C₂₋₁₀ alkinyl radical; said aliphatic radical may - if not defined otherwise - be unsubstituted or substituted by one or more substituents, preferably unsubstituted or substituted with 1, 2, 3, 4 or 5 substituent(s). Said substituent(s) may preferably be selected independently from the group consisting of -O-C₁₋₅-alkyl, -S-C₁₋₅-alkyl, F, Cl, Br, I, -CN, -CF₃, -OCF₃,-SCF₃, -OH, -SH, -NH₂, -NH(C₁₋₅-alkyl) and -N(C₁₋₅-alkyl)₂ , whereby in each occurrence C₁₋₅-alkyl may be linear or branched. More preferably said substituent(s) may preferably be selected independently from the group consisting of -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, F, Cl, Br, I, -CN, -CF₃, -OCF₃, -SCF₃, -OH, -SH, -NH₂, NH-CH₃, -NH-C₂H₅, - NH-CH₂-CH₂-CH₃, -NH-CH(CH₃)₂, -NH-C(CH₃)₃, -N(CH₃)₂, -N(C₂H₅)_{2.}

An alkenyl radical comprises at least one carbon-carbon double bond, an alkinyl radical comprises at least one carbon-carbon triple bond.

Suitable alkyl radicals, which may be substituted by one or more substituents, may preferably be selected from the group consisting of methyl, ethyl, n-propyl, isopropyl, n-butyl, isobutyl, sec-butyl, tert-butyl, n-pentyl, n-hexyl, n-heptyl, n-octyl, n-nonyl and n-decyl.

Suitable alkenyl radicals, which may be substituted by one or more substituents, may preferably be selected from the group consisting of vinyl, 1-propenyl, 2-propenyl, 1-butenyl, 2-butenyl and 3-butenyl.

Suitable alkinyl radicals, which may be substituted by one or more substituents, may preferably be selected from the group consisting of ethinyl, 1-propinyl, 2-propinyl, 1-butinyl, 2-butinyl and 3-butinyl.

In still another aspect the present invention relates to a process for the preparation of a substituted tetrahydroisoquinoline compound of general formula le, wherein at least one compound of general formula IV, 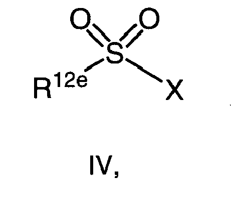 wherein R^{12e} has the meaning given above and X represents a leaving group, preferably a halogen atom, particularly preferably a chlorine atom, is reacted with at least one compound of general formula V, 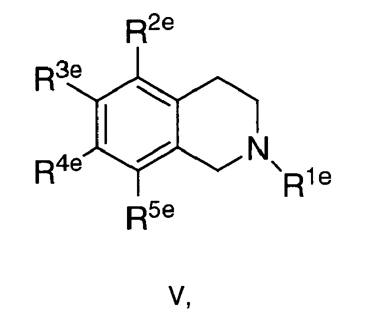 wherein R^{1e} to R^{5e} have the meaning given above, with the proviso that at least one substituent of the group consisting of R^{2e}, R^{3e}, R^{4e} and R^{5e} represents a -N(H)(R11^{e}) moiety, wherein R^{11e} has the meaning given above, or a protected derivative thereof, in a reaction medium, preferably in a reaction medium selected from the group consisting of pyridine, chloroform, dichloromethane, tetrahydrofurane and mixtures thereof, preferably in the presence of at least one base, more preferably in the presence of at least one base selected from the group consisting of triethylamine, diisopropylethylamine and diethylisopropylamine, preferably at a temperature between 0 °C and 30 °C.

If the substituted tetrahydroisoquinoline compounds of general formula I are obtained in form of a mixture of stereoisomers, particularly enantiomers or diastereomers, said mixtures may be separated by standard procedures known to those skilled in the art, e.g. chromatographic methods or crystallization with chiral reagents.

Compounds of general formula IV are in most cases commercially available or may be prepared by processes known to those skilled in the art.

Compounds of general formula V are in most cases commercially available or may be prepared by processes known to those skilled in the art.

In particular, 1,2,3,4-tetrahydroisoquinoline compounds with an amino group in position 5 can be prepared starting from 5-nitro-1,2,3,4-tetrahydroisoquinoline compounds. A process for the preparation of the latter compounds is described in K. V. Rao et al., Journal of Heterocyclic Chemistry, 1973, 10, 213 to 215.

In particular, 1,2,3,4-tetrahydroisoquinoline compounds with an amino group in position 6 are commercially available or can be prepared starting from 6-nitro-1,2,3,4-tetrahydroisoquinoline compounds. A process for the preparation of the latter compounds is described in G. J. Quallich, Journal of Organic Chemistry, 1998, 63, 4116 to 4119.

1,2,3,4-tetrahydroisoquinoline compounds with a nitro group in position 6 or 8 may be prepared by established procedures described in M. Tercel, Journal of Medicinal Chemistry, 1996, 39, 1084 to 1094.

In particular, 1,2,3,4-tetrahydroisoquinoline compounds with an amino group in position 7 are commercially available or can be prepared starting from 7-nitro-1,2,3,4-tetrahydroisoquinoline compounds. A process for the preparation of the latter compounds is described in J. F. Ajao et al., Journal of Heterocyclic Chemistry, 1985, 22, 329 to 331.

The N-methyl-8-amino-substituted 1,2,3,4-tetrahydroisoquinoline compounds were prepared by bromination and nitration of the corresponding 1,2,3,4-tetrahydroisoquinolines followed by two-step standard reduction conditions as described in M. Rey, Helvetica Chimica Acta, 1985, 66, 1828 to 1834 .

In all the following schemes 1 to 7 protecting groups for the nitrogen atom may be used. Some examples include cyclic imide derivatives, such as maleimides or succinimides, a variety of carbamates, such as tert-butoxy-carbonyl (BOC) and fluorenylmethyloxycarbonyl (Fmoc)m a variety of amides, such as acetamides, and alkyl and aryl amine derivatives, such as *N*-benzyl or *N*-allyl amines. Additional examples of nitrogen protecting groups can be found in reference books such as Protective groups in Organic Chemistry, ed. J. F. W. McOmie, Plenum Press, 1973; and T.W. Greene & P.G.M. Wuts, Protective Groups in Organic Chemistry, John Wiley & sons, 1999.

### Preparation of substituted tetrahydroisoquinoline compounds of general formula la (scheme 1 and 2)

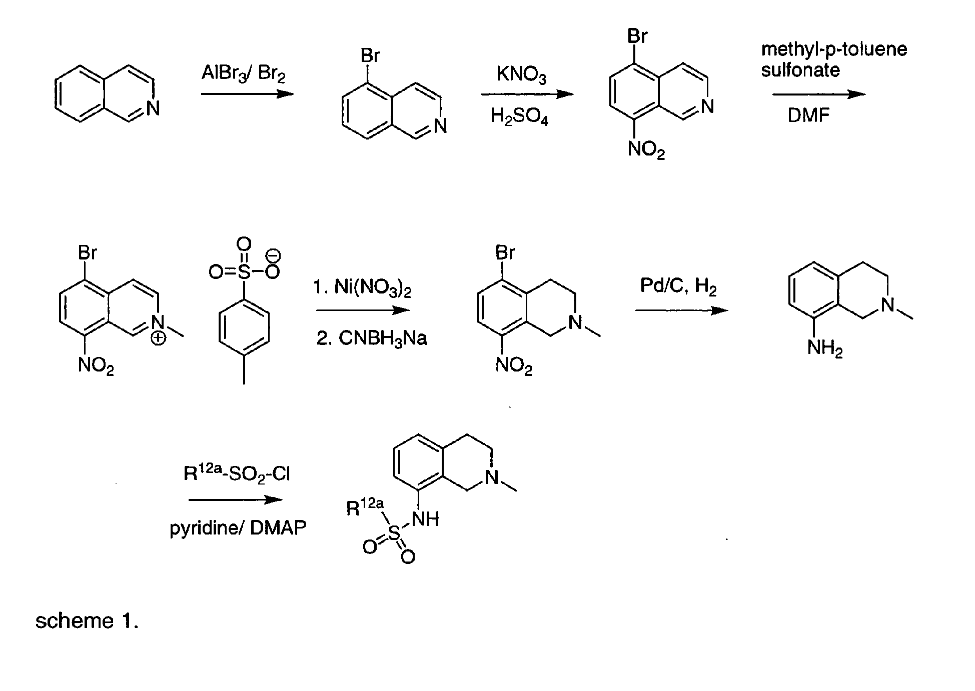

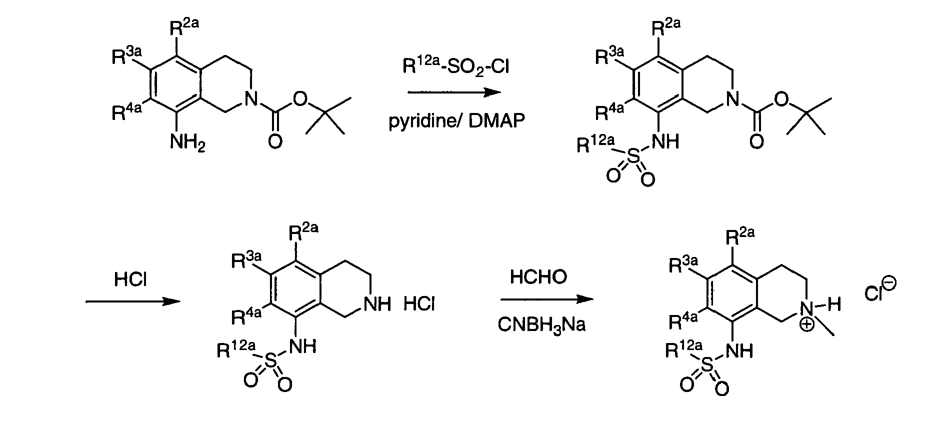

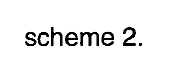

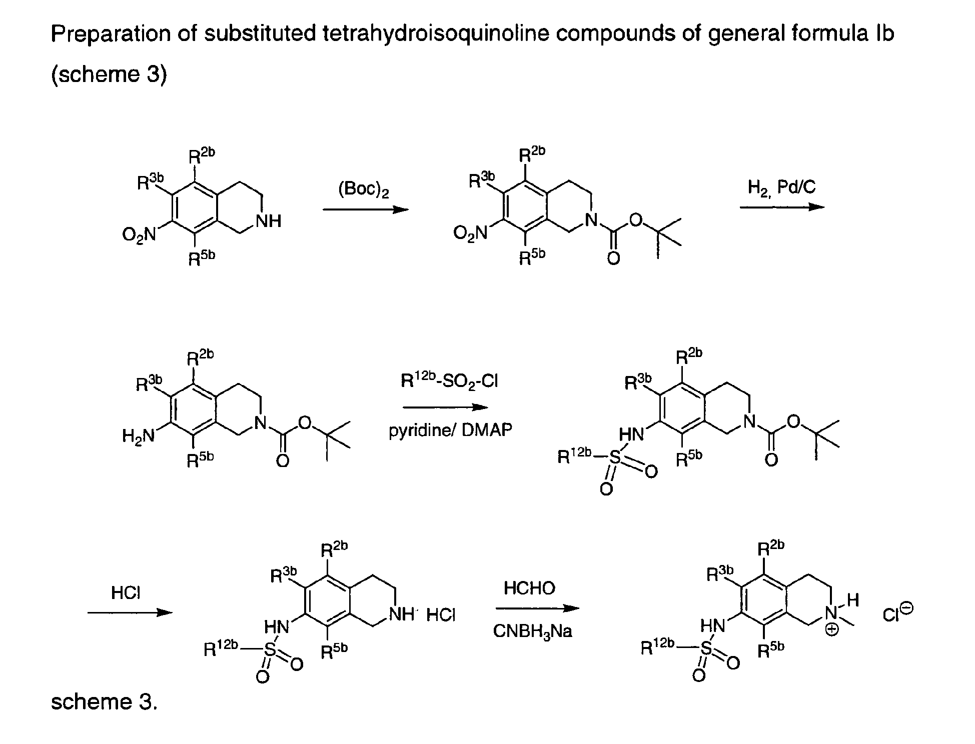

### Preparation of substituted tetrahydroisoquinoline compounds of general formula Ic (scheme 4, 5 and 6)

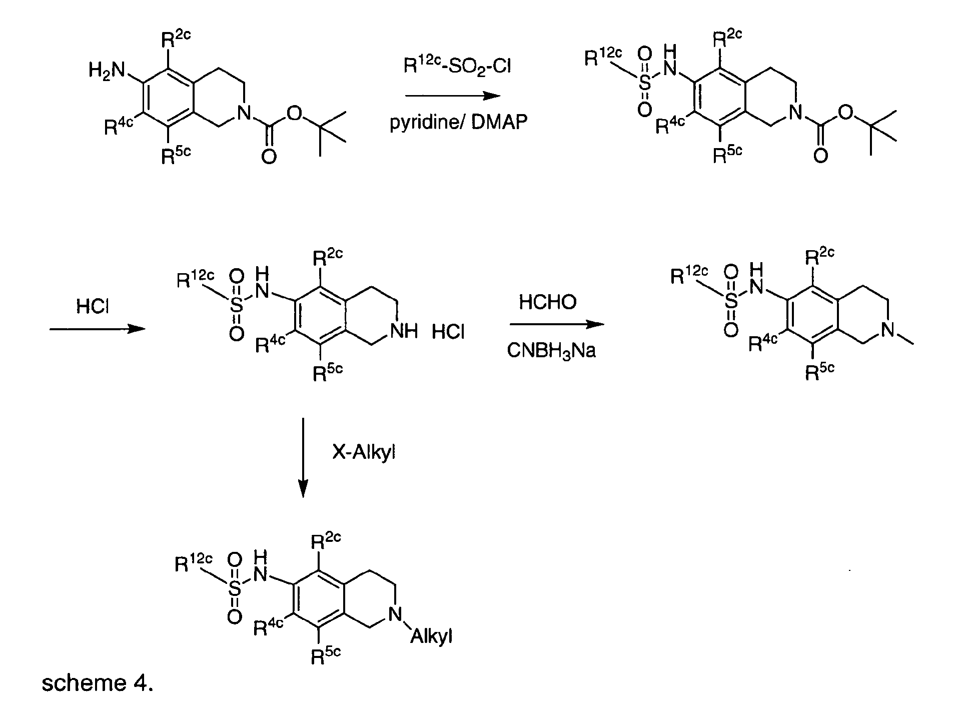

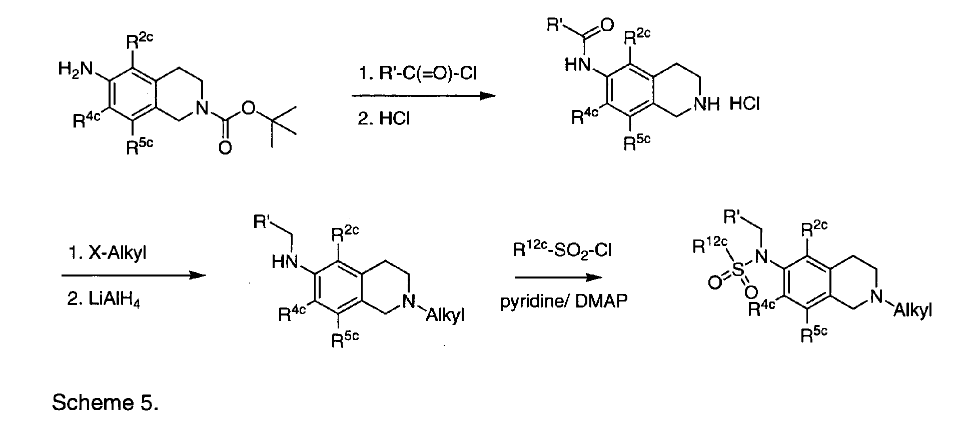

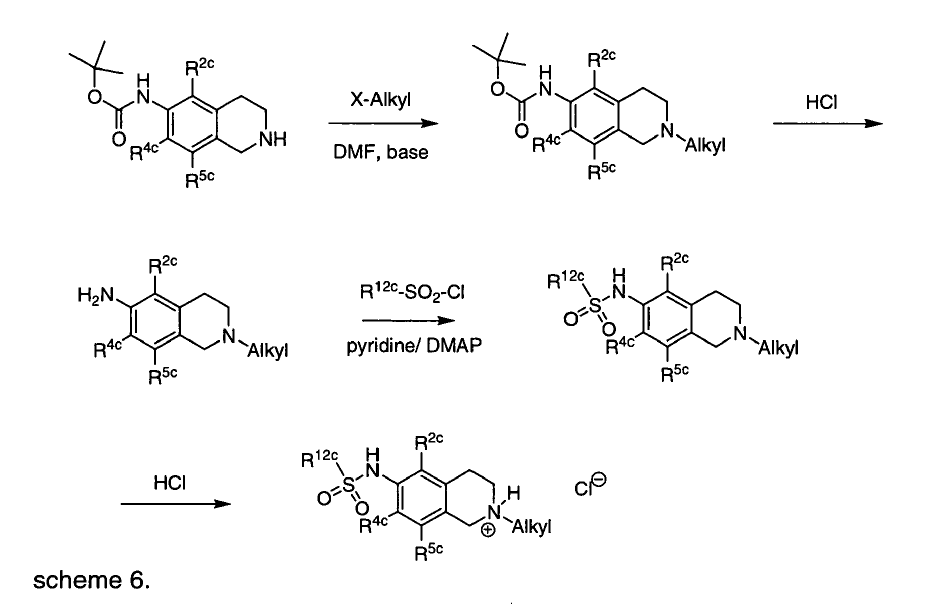

### Preparation of substituted tetrahydroisoquinoline compounds of general formula Id (scheme 7)

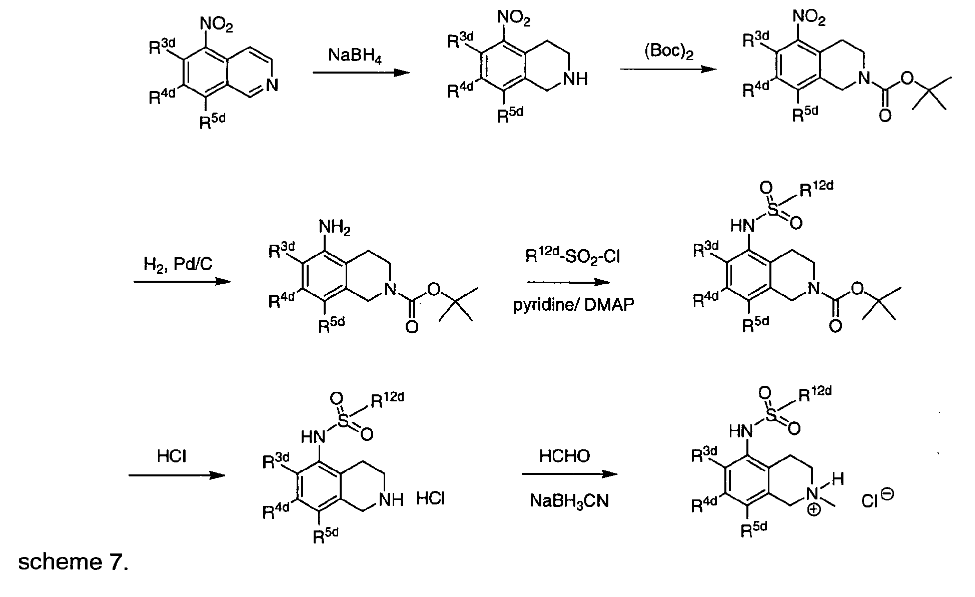

In still another aspect the present invention relates to a process for the preparation of a salt of a substituted tetrahydroisoquinoline compound of general formula If, wherein at least one compound of general formula VI, 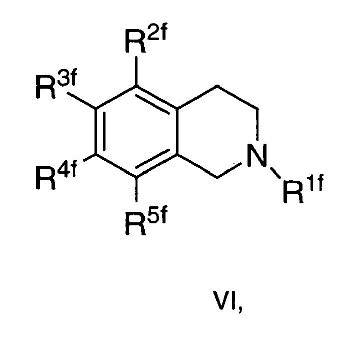 wherein R^{1f} to R^{5f} have the meaning given above, with the proviso that at least one substituent of the group consisting of R^{2f}, R^{3f}, R^{4f} and R^{5f} represents a -NR^{11f}-S(=O)₂-R^{12f} moiety, wherein R^{11f} and R^{12f} have the meaning given above, is reacted with at least one compound of general formula A-D, wherein A and D have the meaning given above, in a reaction medium, preferably in a reaction medium selected from the group consisting of acetone, tetrahydrofurane, water, ethyl acetate, chloroform, acetonitrile, dichloromethane and mixtures thereof, to yield at least one salt of general formula If, 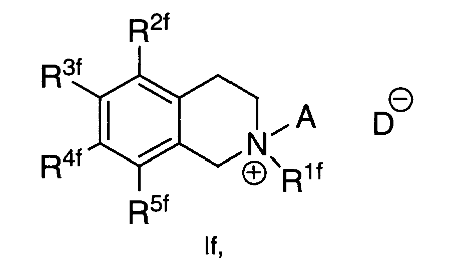 wherein A, D and R^{1f} to R^{5f} have the meaning given above with the proviso that at least one substituent of the group consisting of R^{2f}, R^{3f}, R^{4f} and R^{5f} represents a -NR^{11f}-S(=O)₂-R^{12f} moiety, wherein R^{11f} and R^{12f} have the meaning given above.

The term "salt" is to be understood as meaning any form of the substituted tetrahydroisoquinoline compounds of general formula I in which they assume an ionic form or are charged and are coupled with a counter-ion (a cation or anion) or are in solution. By this are also to be understood complexes of the active compound with other molecules and ions, in particular complexes which are complexed via ionic interactions.

The term "physiologically acceptable salt" is understood in particular, in the context of this invention, as salt (as defined above) formed either with a physiologically tolerated acid, that is to say salts of the particular active compound with inorganic or organic acids which are physiologically tolerated - especially if used on humans and/or mammals - or with at least one, preferably inorganic, cation which are physiologically tolerated - especially if used on humans and/or mammals. Examples of physiologically tolerated salts of particular acids are salts of: hydrochloric acid, hydrobromic acid, sulfuric acid, hydrobromide, monohydrobromide, monohydrochloride or hydrochloride, methiodide, methanesulfonic acid, formic acid, acetic acid, oxalic acid, succinic acid, malic acid, tartaric acid, mandelic acid, fumaric acid, lactic acid, citric acid, glutamic acid, hippuric acid picric acid and/or aspartic acid. Examples of physiologically tolerated salts of particular bases are salts of alkali metals and alkaline earth metals and with NH₄.

Solvates, preferably hydrates, of the substituted tetrahydroisoquinoline compounds of general formula I or in each case of corresponding stereoisomers may also be obtained by standard procedures known to those skilled in the art.

The term "solvate" according to this invention is to be understood as meaning any form of the substituted tetrahydroisoquinoline compounds of general formula I in which they have attached to it via non-covalent binding another molecule (most likely a polar solvent) especially including hydrates and alcoholates, e.g. methanolate.

In the following methods for determining the pharmacological activity of the substituted tetrahydroisoquinoline compounds are described.

### Pharmacological Methods:

### I) BINDING TO SEROTONIN RECEPTOR 5-HT₆

Cell membranes of HEK-293 cells expressing the 5HT₆-human recombinant receptor were supplied by Receptor Biology. In said membranes the receptor concentration is 2.18 pmol/mg protein and the protein concentration is 9.17 mg/ml. The experimental protocol follows the method of B. L. Roth et al. [B. L. Roth, S. C. Craigo, M. S. Choudhary, A. Uluer, F. J. Monsma, Y. Shen, H. Y. Meltzer, D. R. Sibley: Binding of Typical and Atypical Antipsychotic Agents to 5-Hydroxytryptamine-6 and Hydroxytryptamine-7 Receptors. The Journal of Pharmacology and Experimental Therapeutics, 1994, 268, 1403] with the following slight changes. The respective part of the literature description is hereby incorporated by reference and forms part of the disclosure.

The commercial membrane is diluted (1:40 dilution) with the binding buffer: 50 mM Tris-HCl, 10 mM MgCl₂, 0.5 mM EDTA (pH 7.4). The radioligand used is [³H]-LSD at a concentration of 2.7 nM with a final volume of 200 µl. Incubation is initiated by adding 100 µl of membrane suspension, (≈ 22.9 µg membrane protein), and is prolonged for 60 minutes at a temperature of 37 °C. The incubation is ended by fast filtration in a Brandel Cell Harvester through fiber glass filters made by Schleicher & Schuell GF 3362 pretreated with a solution of polyethylenimine at 0.5 %. The filters are washed three times with three milliliters of buffer Tris-HCl 50 mM pH 7.4. The filters are transferred to flasks and 5 ml of Ecoscint H liquid scintillation cocktail are added to each flask. The flasks are allowed to reach equilibrium for several hours before counting with a Wallac Winspectral 1414 scintillation counter. Non-specific binding is determined in the presence of 100 µM of serotonin. Tests were made in triplicate. The inhibition constants (Kᵢ, nM) were calculated by non-linear regression analysis using the program EBDA/LIGAND described in Munson and Rodbard, Analytical Biochemistry, 1980, 107, 220, the respective part of which is hereby incorporated by reference and forms part of the disclosure.

### II.) FOOD INTAKE MEASUREMENT (BEHAVIOURAL MODEL):

Male W rats (200-270 g) obtained from Harlan, S.A. are used. The animals are acclimatized to the animal facility for at least 5 days before they are subjected to any treatment. During this period the animals are housed (in groups of five) in translucid cages and provided with food and water ad libitum. At least 24 hours before the treatment starts, the animals are adapted to single-housing conditions.

The acute effect of the substituted tetrahydroisoquinoline compounds according to the present invention in fasted rats is then determined as follows:

The rats were fasted for 23 hours in their single homecages. After this period, the rats are orally or intraperitoneally dosed with a composition comprising a substituted tetrahydroisoquinoline compound or a corresponding composition (vehicle) without said substituted tetrahydroisoquinoline compound. Immediately afterwards, the rat is left with preweighed food and cumulative food intake is measured after 1, 2, 4 and 6 hours.

Said method of measuring food intake is also described in the literature publications of Kask et al., European Journal of Pharmacology 414 (2001), 215-224 and Turnbull et al., Diabetes, Vol. 51, August 2002. The respective parts of the descriptions are hereby incorporated by reference and form part of the disclosure.

The present invention is illustrated below with the aid of examples. These illustrations are given solely by way of example and do not limit the general spirit of the present invention.

### Preparation of example compound 15: 6-(4-Methyl-naphthalene-1-sulfonylamino)-3,4-dihydro-1H-isoquinoline-2 -carboxylic acid tert-butyl ester

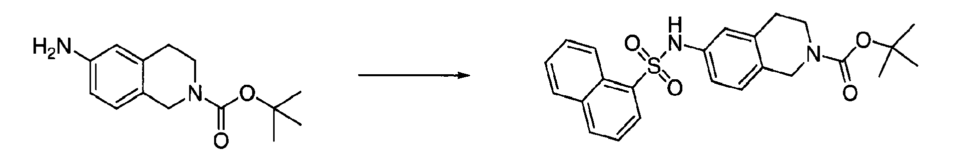

4-Methyl-naphthalene-1-sulfonyl chloride (310 mg, 1.288 mmol) was added to a solution of tert-butyl 6-amino-3,4-dihydroisoquinoline-2(1 H)-carboxylate (318 mg, 1.28 mmol), pyridine (102 mg, 1.28 mmol) and dimethylaminopyridine (15 mg) in dichloromethane (50 mL). After stirring overnight at room temperature the mixture was washed with water, dried over sodium sulfate and filtered. The organic extracts were diluted with ethanol and partial evaporation afforded the title compound (435 mg, 74 %).

The example compounds 13, 14, 16, 17, 18 were prepared analogously to the process described for the preparation of example compound 15.

### Preparation of example compound 6: 4-Methyl-naphthalene-1-sulfonic acid (1,2,3,4-tetrahydroisoquinolin-6-yl)amide hydrochloride

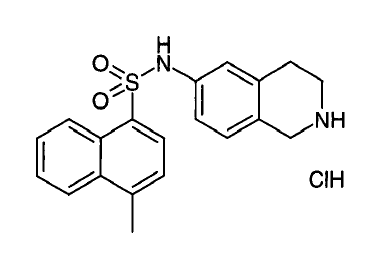

A solution of hydrogen chloride [2.0 M in diethylether] was added to a suspension of 6-(4-methyl-naphthalene- 1 -sulfonylamino)-3,4-dihydro-1 H-isoquinoline-2-carboxylic acid tert-butyl ester (390 mg, 0.86mmol) in ethyl acetate (15 mL). Stirring was continued overnight at room temperature to precipitate the hydrochloride of 4-methyl-naphthalene-1-sulfonic acid (1,2,3,4-tetrahydro-isoquinolin-6-yl)amide (320 mg, 95 %).

The example compounds 1, 4, 8, 10, 11 and 12 were prepared analogously to the process described for the preparation of example compound 6.

### Preparation of example compound 7: 4-Methyl-naphthalene-1-sulfonic acid (2-methyl- 1,2,3,4 -tetrahydroiso quinolin-6-yl)amide hydrochloride

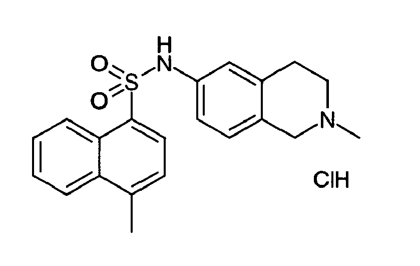

A solution of 4-methyl-naphthalene-1-sulfonic acid(1,2,3,4-tetrahydro-isoquinolin-6-yl)amide (254 mg, 0.72 mmol) in acetonitrile (15 mL) was treated with formaldehyde (37% aqueous solution, 1 mL) and stirred for 2 hours at room temperature. Sodium cyanoborohydride (210mg, 3.3 mmol) was added and the mixture was allowed to stir for 2 hours following addition of acetic acid (pH 7). After stirring overnight the mixture was evaporated and the residue partitioned between chloroform and water. The organic layer was separated and evaporated to give the crude free base which was purified by chromatography eluting with 2% methanol in chloroform. Treatment with a 2.0 M ethereal hydrogen chloride solution gave the title compound as the hydrochloride salt (104 mg, 41 %).

The example compounds 3, 4 and 9 were prepared analogously to the process described for the preparation of example compound 7.

### Preparation of example compound 2: 2,2-Dimethyl-6-[methyl-(naphthalene-1-sulfonyl)-amino]-1,2,3,4-tetrahydro isoquinolinium iodide

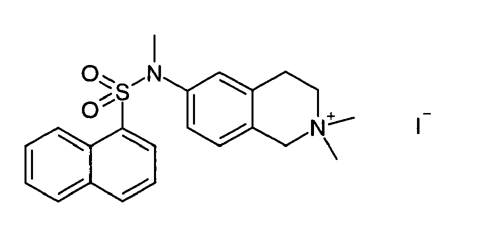

Naphthalene-1-sulfonic acid (1,2,3,4-tetrahydrisoquinolin-6-yl)-amide hydrochloride (100 mg, 0.26 mmol) and potassium carbonate(111 mg, 0.80 mmol) were dissolved in acetone (20 ml) and methyl iodide (150mg,1.04mmol) was added. The mixture was heated under reflux for 6 hours. The solution was evaporated and the crude quaternary ammonium salt was crystallised by addition of ethanol (5 ml) to afforded the title compound (120 mg, 91 %).

| **N°** | **STRUCTURE** | **Autonom** | **¹H-NMR** | **MS (APCI (M+H)** ⁺**)** |
|---|---|---|---|---|
| 1 | 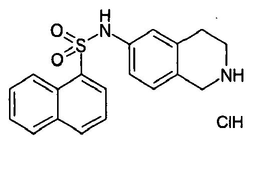 | Naphthalene-1-sulfonic acid (1,2,3,4-tetrahydroisoquinolin-6-yl)-amide hydrochloride | ¹H NMR (300 MHz, DMSO-*d*₆) δ ppm 2.73 - 2.84 (m, 2 H) 3.19 (s, 2 H) 4.02 (s, 2 H) 6.83 - 6.92 (m, 2 H) 6.92 - 6.99 (m, 1H) 7.56 - 7.69 (m, 2 H) 7.69 - 7.77 (m, 1H) 8.05 (d, *J*=8.06 Hz, 1 H) 8.16 - 8.24 (m, 2 H) 8.71 (d, *J*=8.49 Hz, 1 H) 9.23 (s, 2 H) 10.79 (s, 1 H) | 339 |
| 2 | 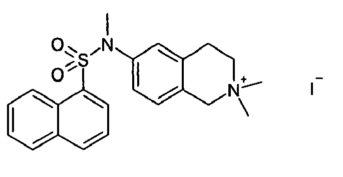 | 2,2-Dimethyl-6-[methyl-(naphthalene-1-sulfonyl) -amino]-1,2,3,4-tetrahydro isoquinolinium iodide | ¹H NMR (300 MHz, DMSO-*d*₆) δ ppm 2.95 - 3.03 (m, 2 H) 3.11 (s, 6 H) 3.15 (s, 3 H) 3.56 - 3.68 (m, 2 H) 4.56 (s, 2 H) 7.01 - 7.13 (m, 3 H) 7.42 - 7.51 (m, 1 H) 7.56 - 7.64 (m, 1 H) 7.64 - 7.71 (m, 1 H) 8.03 - 8.15 (m, 3 H) 8.29 (d, J=8.20 Hz, 1 H) | 381 |
| 3 | 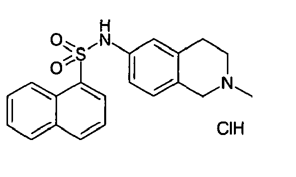 | Naphthalene-1-sulfonic acid (2-methyl-1,2,3,4-tetrahydro -isoquinolin-6-yl)-amide hydrochloride | ¹H NMR (300 MHz, DMSO-*d*₆) δ ppm 2.52 (s, 3 H) 2.73 - 2.86 (m, 2 H) 3.02 - 3.12 (m, 2 H) 3.79 - 3.95 (m, 2 H) 6.82 - 6.95 (m, 3 H) 7.57 - 7.76 (m, 3 H) 8.06 (d, J=7.76 Hz, 1 H) 8.17 - 8.25 (m, 2 H) 8.69 (d, J=8.50 Hz, 1 H) 10.72 (s, 1 H) | 353 |
| 4 | 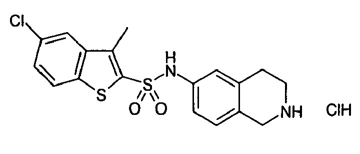 | 5-Chloro-3-methylbenzo[b]thiophene-2-sulfonic acid (1,2,3,4-tetrahydro-isoquinolin-6-yl)-amide hydrochloride | ¹H NMR (300 MHz, DMSO-*d*₆) δ ppm 2.53 (s, 3 H) 2.83 - 2.91 (m, 2 H) 3.20 - 3.29 (m, 2 H) 4.10 (s, 2 H) 6.95 - 7.05 (m, 2 H) 7.05 - 7.11 (m, 1 H) 7.52 - 7.59 (m, 1 H) 8.00 (d, J=1.90 Hz, 1 H) 8.06 (d, J=8.64 Hz, 1 H) 9.27 (s, 2 H) 10.89 (s, 1 H) | 393 |
| 5 | 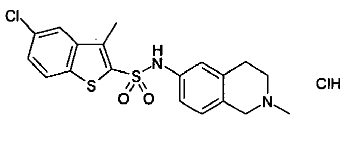 | 5-Chloro-3-methylbenzo[b]thiophene-2-sulfonic acid (2-methyl-1,2,3,4-tetrahydro - isoquinolin-6-yl)-amide hydrochloride | ¹H NMR (300 MHz, DMSO-d₆) δ ppm 2.52 (s, 3 H) 2.80 (s, 3 H) 2.96 (s, 2 H) 3.48 (m, 2 H) 4.21 (s, 2 H) 6.93 - 7.12 (m, 3 H) 7.48 - 7.63 (m, 1 H) 7.99 (d, J=1.90 Hz, 1 H) 8.05 (d, J=8.64 Hz, 1 H) 10.67 (s, 1 H) 10.90 (s, 1 H) | 407 |
| 6 | 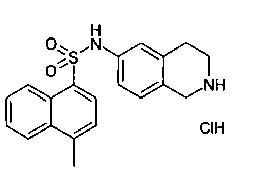 | 4-Methyl-naphthalene-1-sulfonic acid (1,2,3,4-tetrahydroisoquinolin-6 -yl)amide hydrochloride | ¹H NMR (300 MHz, DMSO-*d₆*) δ ppm 2.67 (s, 3 H) 2.72 - 2.84 (m, 2 H) 3.19 (s, 2 H) 4.02 (s, 2 H) 6.78 - 6.91 (m, 2 H) 6.91 - 7.00 (m, 1 H) 7.46 (d, J=7.91 Hz, 1 H) 7.60 - 7.77 (m, 2 H) 8.07 - 8.17 (m, 2 H) 8.67 - 8.78 (m, 1 H) 9.23 (s, 2 H) 10.74 (s, 1 H) | 353 |
| 7 | 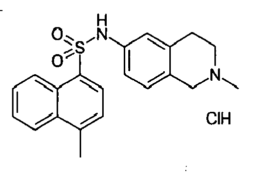 | 4-Methyl-naphthalene-1-sulfonic acid (2-methyl-1,2,3,4 - tetrahydro-isoquinolin-6-yl)amide hydrochloride | ¹H NMR (300 MHz, DMSO-*d₆*) δ ppm 2.67 (s, 3 H) 2.77 (s, 3 H) 2.97 (m, 2 H) 3.46 (m, 2 H) 4.13 (m, 2 H) 6.82 - 6.97 (m, 3 H) 7.47 (d, *J*=7.62 Hz, 1 H) 7.61 - 7.78 (m, 2 H) 8.12 (d, *J*=7.47 Hz, 2 H) 8.72 (d, J=7.91 Hz, 1 H) 10.61 (s, 1 H) 10.78 (s, 1 H) | 367 |
| 8 | 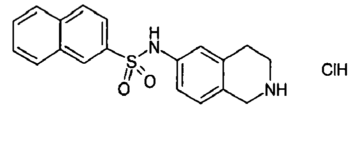 | Naphthalene-2-sulfonic acid (1,2,3,4-tetrahydro- isoquinolin-6-yl)-amide hydrochloride | ¹H NMR (300 MHz, DMSO-*d*₆) δ ppm 2.80 - 2.89 (m, 2 H) 3.22 (t, J=6.15 Hz, 2 H) 4.05 (s, 2 H) 6.89 - 7.07 (m, 3 H) 7.59 - 7.72 (m, 2 H) 7.73 - 7.82 (m, 1 H) 7.99 (d, J=7.62 Hz, 1 H) 8.03 - 8.16 (m, 2 H) 8.47 (d, J=0.59 Hz, 1 H) 9.22 (s, 2 H) 10.52 (s, 1 H) | 339 |
| 9 | 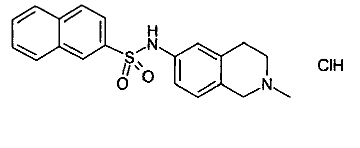 | Naphthalene-2-sulfonic acid (2-methyl-1,2,3,4 -tetrahydro-isoquinolin-6-yl)-amide hydrochloride | ¹H NMR (400 MHz, DMSO-*d*₆) δ ppm 2.79 (s, 3 H) 2.95 (m, 2 H) 3.23 (m, 2 H) 4.09 - 4.25 (m, 2 H) 6.97 - 7.03 (m, 3 H) 7.61 - 7.70 (m, 2 H) 7.78 (dd, J=8.60, 1.95 Hz, 1 H) 7.99 (d, J=7.82 Hz, 1 H) 8.08 (d, J=8.99 Hz, 1 H) 8.13 (d, J=8.21 Hz, 1 H) 8.47 (s, 1 H) 10.37 (s, 1 H) 10.52 (s, 1H) | 353 |
| 10 | 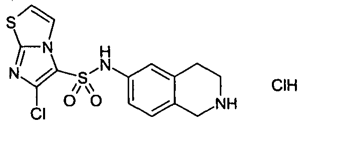 | 6-Chloro-imidazo[2,1-b] thiazole-5-sulfonic acid (1,2,3,4-tetrahydroisoquinolin-6-yl)-amide hydrochloride | ¹H NMR (300 MHz, DMSO-*d₆*) δ ppm 2.80 - 2.91 (m, 2 H) 3.25 (m, 2 H) 4.10 (s, 2 H) 6.93 - 6.99 (m, 2 H) 7.04 - 7.11 (m, 1 H) 7.65 (d, J=4.39 Hz, 1 H) 8.07 (d, J=4.39 Hz, 1 H) 9.31 (s, 2 H) 11.18 (s, 1 H) | 369 |
| 11 | 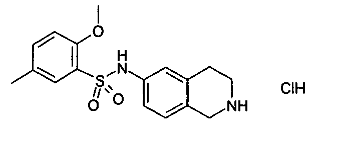 | 2-Methoxy-5-methyl-N-(1,2,3,4-tetrahydroisoquinolin-6-yl)-benzene sulfonamide hydrochloride | ¹H NMR (300 MHz, DMSO-*d₆*) δ ppm 2.22 (s, 3 H) 2.80 - 2.88 (m, 2 H) 3.24 (t, J=6.15 Hz, 2 H) 3.81 (s, 3 H) 4.07 (s, 2 H) 6.87 - 6.97 (m, 2 H) 6.98 - 7.05 (m, 2 H) 7.30 - 7.37 (m, 1 H) 7.55 (d, J=1.90 Hz, 1 H) 9.28 (s, 2 H) 9.99 (s, 1 H) | 333 |
| 12 | 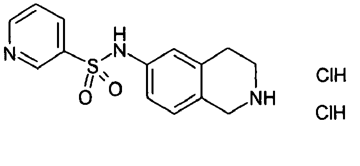 | Pyridine-3-sulfonic acid (1,2,3,4-tetrahydroisoquinolin-6-yl)-amide dihydrochloride | ¹H NMR (300 MHz, DMSO-*d*₆) δ ppm 2.90 (t, J=6.01 Hz, 2 H) 3.27 (s, 2 H) 4.13 (s, 2 H) 6.93 - 7.05 (m, 2 H) 7.05 - 7.14 (m, 1 H) 7.61 (dd, *J*=8.06, 4.83 Hz, 1 H) 8.10 - 8.22 (m, 1 H) 8.78 (dd, J=4.83, 1.46 Hz, 1 H) 8.89 (d, J=2.05 Hz, 1 H) 9.31 (s, 2 H) 10.66 (s, 1 H) | 290 |
| 13 | 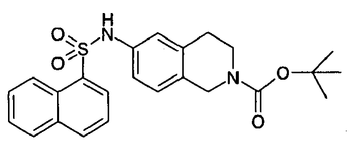 | 6-(Naphthalene-1-sulfonylamino)-3,4 -dihydro-1H-isoquinoline-2-carboxylic acid tert-butyl ester | ¹H NMR (300 MHz, DMSO-d₆) δ ppm 1.35 (s, 9 H) 2.55 (t, J=5.71 Hz, 2 H) 3.38 (t, J=5.86 Hz, 2 H) 4.27 (s, 2 H) 6.72 - 6.85 (m, 2 H) 6.85 - 6.96 (m, 1 H) 7.52 - 7.78 (m, 3 H) 8.05 (d, J=7.91 Hz, 1 H) 8.11 - 8.23 (m, 2 H) 8.69 (d, J=8.35 Hz, 1H) 10.59 (s, 1H) | 439 |
| 14 | 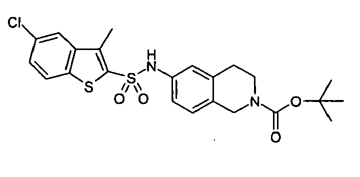 | 6-(5-Chloro-3-methyl-benzo[b]thiophene-2-sulfonylamino)-3,4-dihydro-1H-isoquinoline-2-carboxylic acid tert-butyl ester | ¹H NMR (300 MHz, DMSO-d₆) δ ppm 1.37 (s, 9 H) 2.50 (s, 3 H) 2.63 (t, J=5.71 Hz, 2 H) 3.44 (t, J=5.86 Hz, 2 H) 4.34 (s, 2 H) 6.91 - 6.96 (m, 2 H) 6.99 - 7.05 (m, 1 H) 7.51 - 7.57 (m, 1 H) 7.98 (d, J=2.05 Hz, 1 H) 8.04 (d, J=8.79 Hz, 1 H) 10.69 (s, 1 H) | 493 |
| 15 | 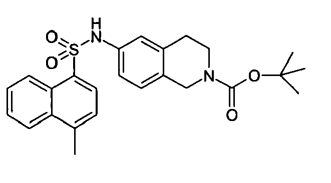 | 6-(4-Methyl-naphthalene-1-sulfonylamino)-3,4 -dihydro-1H-isoquinoline-2-carboxylic acid tert-butyl ester | ¹H NMR (300 MHz, DMSO-*d*₆) δ ppm 1.35 (s, 9 H) 2.55 (t, J=5.71 Hz, 2 H) 2.67 (s, 3 H) 3.39 - 3.48 (m, 2 H) 4.27 (s, 2 H) 6.75 - 6.86 (m, 2 H) 6.86 - 6.94 (m, 1 H) 7.45 (d, J=7.76 Hz, 1 H) 7.61 - 7.76 (m, 2 H) 8.04 - 8.17 (m, 2 H) 8.65 - 8.79 (m, 1 H) 10.57 (s, 1 H) | 453 |
| 16 | 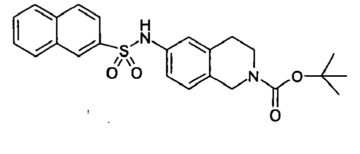 | 6-(Naphthalene-2-sulfonylamino)-3,4 -dihydro-1H-isoquinoline-2-carboxylic acid tert-butyl ester | ¹H NMR (300 MHz, DMSO-d₆) δ ppm 1.35 (s, 9 H) 2.60 (t, J=5.71 Hz, 2 H) 3.40 (t, J=5.86 Hz, 2 H) 4.30 (s, 2 H) 6.86 - 7.00 (m, 3 H) 7.59 - 7.71 (m, 2 H) 7.75 (dd, J=8.64, 1.61 Hz, 1 H) 7.98 (d, J=7.76 Hz, 1 H) 8.07 (d, J=8.64 Hz, 1 H) 8.11 (d, J=7.76 Hz, 1 H) 8.42 (s, 1 H) | 439 |
| 17 | 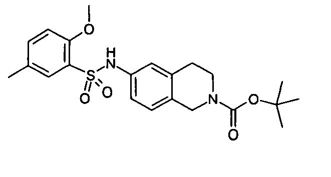 | 6-(2-Methoxy-5-methylbenzenesulfonylamino) -3,4-dihydro-1H-isoquinolin e-2-carboxylic acid tert-butyl ester | ¹H NMR (300 MHz, DMSO-*d*₆) δ ppm 1.37 (s, 9 H) 2.21 (s, 3 H) 2.55 - 2.65 (m, 2 H) 3.43 (t, J=5.86 Hz, 2 H) 3.82 (s, 3 H) 4.32 (s, 2 H) 6.82 - 6.91 (m, 2 H) 6.91 - 6.98 (m, 1 H) 7.03 (d, J=8.50 Hz, 1 H) 7.33 (dd, J=8.50, 2.05 Hz, 1 H) 7.52 (d, J=1.90 Hz, 1 H) 9.84 (s, 1 H) | 433 |
| 18 | 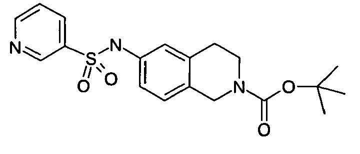 | 6-(Pyridine-3-sulfonylamino)-3,4-dihydro-1H-isoquinoline-2-carboxylic acid tert-butyl ester | ¹H NMR (300 MHz, DMSO-d₆) δ ppm 1.38 (s, 9 H) 2.62 (t, J=5.71 Hz, 2 H) 3.39 - 3.49 (m, 2 H) 4.35 (s, 2 H) 6.77 - 6.93 (m, 2 H) 7.03 (d, J=8.94 Hz, 1 H) 7.62 (d, J=4.39 Hz, 1 H) 7.95 (d, J=4.54 Hz, 1H) 10.86 (s, 1H) | 469 |

### Pharmacological data:

The binding of the substituted tetrahydroisoquinoline compounds to the 5-HT₆ receptor was determined as described above.

The binding results for some of these compounds are given in the following table :

| **Compound according to example:** | **Kᵢ (nM)** | **Binding % [100 nM]** | **Binding % [10 nM]** |
|---|---|---|---|
| 1 | 13.8 | 74.8 | 53.3 |
| 2 | | 19.6 | 17.0 |
| 3 | | 18.2 | 3.9 |
| 4 | | 71.1 | 27.3 |
| 5 | | 76.3 | 25.9 |
| 6 | 21.0 | 88.8 | 60.1 |
| 7 | 13.3 | 92.2 | 80.3 |
| 8 | | 74.6 | 37.4 |
| 9 | 10.4 | 86.5 | 60.1 |
| 10 | | 94.6 | 85.8 |
| 13 | 1.3 | 28.8 | 30.7 |
| 14 | | -3,0 | -7,3 |
| 15 | | 8,2 | -5,0 |
| 16 | | 10,6 | 5,6 |
| 17 | | -2,0 | -1,3 |
| 18 | | 6,8 | -0,8 |

## Claims

1. A medicament comprising at least one substituted tetrahydroisoquinoline compound of general formula I, 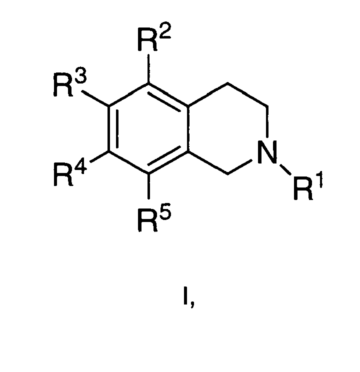 wherein
R¹ represents a hydrogen atom; a -C(=O)-OR³⁷ moiety;
a linear or branched, saturated or unsaturated C₁₋₁₀ aliphatic radical which may be unsubstituted or optionally substituted with 1, 2, 3, 4 or 5 substituent(s) independently selected from the group consisting of F, Cl, Br, I, -CN, -CF₃, -OCF₃, -SCF₃, -OH, -SH, -NH₂, -O-C₁₋₅-alkyl, -S-C₁₋₅-alkyl, -NH(C₁₋₅-alkyl) and -N(C₁₋₅-alkyl)₂;
or a saturated or unsaturated 3- to 9-membered cycloaliphatic radical, which may contain 1, 2 or 3 heteroatom(s) independently selected from the group consisting of nitrogen, oxygen and sulfur as ring member(s) and which may be unsubstituted or optionally substituted with 1, 2, 3, 4 or 5 substituent(s) independently selected from the group consisting of oxo (=O), thioxo (=S), C₁₋₅-alkyl, -O-C₁₋₅-alkyl, -S-C₁₋₅-alkyl, -C(=O)-C₁₋₅-alkyl, - O-C(=O)-C₁₋₅-alkyl, F, Cl, Br, I, -CN, -CF₃, -OCF₃, -SCF₃, -OH, -SH, -NH₂, - NH(C₁₋₅-alkyl), -N(C₁₋₅-alkyl)₂, -NO₂, -CHO, -CF₂H, -CFH₂, -C(=O)-NH₂, - C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl and which may be condensed with an unsubstituted or at least mono-substituted saturated, unsaturated or aromatic mono- or bicyclic ring system and which may be bonded via a linear or branched C₁₋₆ alkylene, C₂₋₆ alkenylene or C₂₋₆ alkinylene group which may be unsubstituted or substituted with 1, 2 or 3 substituent(s) independently selected from the group consisting of F, Cl, Br, I, -CN, -CF₃, -OCF₃, -SCF₃, -OH, -SH, -NH₂, -O-C₁₋₅-alkyl, -S-C₁₋₅-alkyl, -NH(C₁₋₅-alkyl) and -N(C₁₋₅-alkyl)₂;
R², R³, R⁴ and R⁵, independently of one another, each represent a hydrogen atom; F, Cl, Br, I, -NO₂; -NH₂; -SH; -OH; -CN; -C(=O)-OH; -C(=O)-H; - S(=O)₂-OH; -C(=O)-NH₂; -S(=O)₂-NH₂; -C(=O)-R⁶; -S(=O)-R⁷; -S(=O)₂-R⁷;-OR⁸; -SR⁹; -C(=O)-OR¹⁰; -N(R¹¹)-S(=O)₂-R¹²; -NR¹³R¹⁴; -NH-R¹⁵; -C(=O)-NR¹⁶R¹⁷; C(=O)-NHR¹⁸;
a linear or branched, saturated or unsaturated, unsubstituted or at least mono-substituted aliphatic radical;
a saturated or unsaturated, unsubstituted or at least mono-substituted, optionally at least one heteroatom as a ring member containing cycloaliphatic radical, which may be bonded via a linear or branched alkylene, alkenylene or alkinylene group and which may be condensed with an unsubstituted or at least mono-substituted saturated, unsaturated or aromatic mono- or bicyclic ring system;
or an unsubstituted or at least mono-substituted aryl or heteroaryl radical, which may be bonded via a linear or branched alkylene, alkenylene or alkinylene group and which may be condensed with an unsubstituted or at least mono-substituted saturated or unsaturated, but not aromatic, mono-or bicyclic ring system;
with the proviso that at least one of the substituents R², R³, R⁴ and R⁵ represents a -N(R¹¹)-S(=O)₂-R¹² moiety;
R⁶, R⁷, R⁸, R⁹, R¹⁰, R¹³, R¹⁴, R¹⁵, R¹⁶, R¹⁷ and R¹⁸, independently of one another, each represent a linear or branched, saturated or unsaturated, unsubstituted or at least mono-substituted aliphatic radical;
a saturated or unsaturated, unsubstituted or at least mono-substituted, optionally at least one heteroatom as a ring member containing cycloaliphatic radical, which may be bonded via a linear or branched alkylene, alkenylene or alkinylene group and which may be condensed with an unsubstituted or at least mono-substituted saturated, unsaturated or aromatic mono- or bicyclic ring system;
or an unsubstituted or at least mono-substituted aryl or heteroaryl radical, which may be bonded via a linear or branched alkylene, alkenylene or alkinylene group and which may be condensed with an unsubstituted or at least mono-substituted saturated or unsaturated, but not aromatic, mono-or bicyclic ring system;
R¹¹ represents a hydrogen atom, -S(=O)₂-R¹² or a linear or branched, saturated or unsaturated, unsubstituted or at least mono-substituted aliphatic radical;
R¹² represents a phenyl radical of general formula (A), 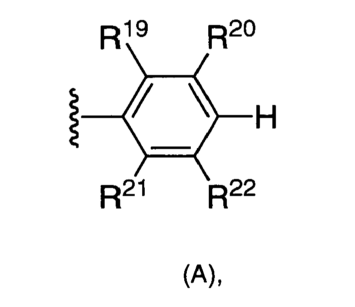 wherein
R¹⁹, R²⁰, R²¹ and R²², independently of one another, each represent a hydrogen atom; F, Cl, Br, I, -NO₂; -NH₂; -SH; -OH; -CN; -C(=O)-OH; -C(=O)-H; -S(=O)₂-OH; -C(=O)-NH₂; -S(=O)₂-NH₂; -C(=O)-R²³;-S(=O)-R²⁴; -S(=O)₂-R²⁴; -OR²⁵; -SR²⁶; -C(=O)-OR²⁷; -N(R²⁸)-S(=O)₂-R²⁹; -NH-S(=O)₂-R³⁰; -NR³¹R³²; -NH-R³³; -C(=O)-NHR³⁴; -C(=O)-NR³⁵R³⁶; a linear or branched, saturated or unsaturated C₁₋₁₀ aliphatic radical which may be unsubstituted or optionally substituted with 1, 2, 3, 4 or 5 substituent(s) independently selected from the group consisting of F, Cl, Br, I, -CN, -CF₃, -OCF₃, -SCF₃, -OH, -SH, - NH₂, -O-C₁₋₅-alkyl, -S-C₁₋₅-alkyl, -NH(C₁₋₅-alkyl) and -N(C₁₋₅-alkyl)₂; or a saturated or unsaturated, unsubstituted or at least mono-substituted, optionally at least one heteroatom as a ring member containing cycloaliphatic radical;
with the proviso that at least one of the substituents R¹⁹, R²⁰, R²¹ and R²² is unlike hydrogen;
an unsubstituted or at least mono-substituted 10-membered aryl radical;
a monocyclic heteroaryl radical, which may be unsubstituted or optionally substituted with 1, 2, 3, 4 or 5 substituent(s) independently selected from the group consisting of F, Cl, Br, I, -NO₂; -NH₂; -SH; -OH; -CN; -C(=O)-OH; -C(=O)-H; -S(=O)₂-OH; -C(=O)-NH₂; -S(=O)₂-NH₂; -C(=O)-R²³; -S(=O)-R²⁴; -S(=O)₂-R²⁴; -OR²⁵; -SR²⁶; -C(=O)-OR²⁷; -N(R²⁸)-S(=O)₂-R²⁹; -NH-S(=O)₂-R³⁰; -NR³¹R³²; -NH-R³³; -C(=O)-NHR³⁴; -C(=O)-NR³⁵R³⁶; a linear or branched, saturated or unsaturated C₁₋₁₀ aliphatic radical which may be unsubstituted or substituted with 1, 2 or 3 substituent(s) independently selected from the group consisting of F, Cl, Br, I, -CN, -CF₃, -OCF₃, -SCF₃, -OH, -SH, -NH₂, -O-C₁₋₅-alkyl, -S-C₁₋₅-alkyl, -NH(C₁₋₅-alkyl) and -N(C₁₋₅-alkyl)₂; and a saturated or unsaturated, unsubstituted or at least mono-substituted, optionally at least one heteroatom as a ring member containing cycloaliphatic radical;
an unsubstituted or at least mono-substituted monocyclic heteroaryl radical, which is condensed with an unsubstituted or at least mono-substituted saturated or unsaturated, but not aromatic, mono- or bicyclic ring system;
an unsubstituted or at least mono-substituted bi- or tricyclic heteroaryl radical, which may be condensed with an unsubstituted or at least mono-substituted saturated or unsaturated, but not aromatic, mono- or bicyclic ring system;
or a saturated or unsaturated, unsubstituted or at least mono-substituted, optionally at least one heteroatom as a ring member containing cycloaliphatic radical, which may be condensed with an unsubstituted or at least mono-substituted saturated, unsaturated or aromatic mono- or bicyclic ring system;
R²³, R²⁷, R²⁸, R²⁹ and R³⁰, independently of one another, each represent a linear or branched, saturated or unsaturated C₁₋₁₀ aliphatic radical which may be unsubstituted or optionally substituted with 1, 2, 3, 4 or 5 substituent(s) independently selected from the group consisting of F, Cl, Br, I, -CN, -CF₃, -OCF₃, -SCF₃, -OH, -SH, -NH₂, -O-C₁₋₅-alkyl, -S-C₁₋₅-alkyl, -NH(C₁₋₅-alkyl) and -N(C₁₋₅-alkyl)₂;
a saturated or unsaturated, unsubstituted or at least mono-substituted, optionally at least one heteroatom as a ring member containing cycloaliphatic radical, which may be bonded via a linear or branched alkylene, alkenylene or alkinylene group and which may be condensed with an unsubstituted or at least mono-substituted saturated, unsaturated or aromatic mono- or bicyclic ring system;
or an unsubstituted or at least mono-substituted aryl or heteroaryl radical, which may be bonded via a linear or branched alkylene, alkenylene or alkinylene group and which may be condensed with an unsubstituted or at least mono-substituted saturated or unsaturated, but not aromatic, mono-or bicyclic ring system;
R²⁴, R²⁶, R³¹, R³² and R³³, each represent a linear or branched, saturated or unsaturated C₁₋₁₀ aliphatic radical which may be unsubstituted or substituted with 1, 2, 3, 4 or 5 substituent(s) independently selected from the group consisting of F, Cl, Br, I, -CN, -CF₃, -OCF₃, -SCF₃, -OH, -SH, - NH₂, -O-C₁₋₅-alkyl, -S-C₁₋₅-alkyl, -NH(C₁₋₅-alkyl) and -N(C₁₋₅-alkyl)₂;
a saturated or unsaturated, unsubstituted or at least mono-substituted, optionally at least one heteroatom as a ring member containing cycloaliphatic radical, which may be bonded via a linear or branched alkylene, alkenylene or alkinylene group and which may be condensed with an unsubstituted or at least mono-substituted saturated, unsaturated or aromatic mono- or bicyclic ring system;
or an unsubstituted or at least mono-substituted aryl or heteroaryl radical, which may be condensed with an unsubstituted or at least mono-substituted saturated or unsaturated, but not aromatic, mono- or bicyclic ring system;
R²⁵, R³⁴, R³⁵ and R³⁶, represents a linear or branched, saturated or unsaturated C₁₋₁₀ aliphatic radical which may be unsubstituted or optionally substituted with 1, 2, 3, 4 or 5 substituent(s) independently selected from the group consisting of F, Cl, Br, I, -CN, -CF₃, -OCF₃, -SCF₃, -OH, -SH, - NH₂, -O-C₁₋₅-alkyl, -S-C₁₋₅-alkyl, -NH(C₁₋₅-alkyl) and -N(C₁₋₅-alkyl)₂;
and R³⁷ represents a linear or branched, saturated or unsaturated C₁₋₁₀ aliphatic radical,
a saturated or unsaturated, unsubstituted or at least mono-substituted, optionally at least one heteroatom as a ring member containing cycloaliphatic radical, which may be bonded via a linear or branched alkylene, alkenylene or alkinylene group and which may be condensed with an unsubstituted or at least mono-substituted saturated, unsaturated or aromatic mono- or bicyclic ring system;
or an unsubstituted or at least mono-substituted aryl or heteroaryl radical, which may be bonded via a linear or branched alkylene, alkenylene or alkinylene group and which may be condensed with an unsubstituted or at least mono-substituted saturated or unsaturated, but not aromatic, mono-or bicyclic ring system;
optionally in form of one of its stereoisomers, preferably enantiomers or diasteromers, a racemate or in form of a mixture of at least two of its stereoisomers, preferably enantiomers and/or diastereomers, in any mixing ratio, or a salt thereof, or a corresponding solvate thereof;
and optionally at least one physiologically acceptable auxiliary agent.

2. A medicament according to claim 1, **characterized in that** the salt has the general formula II, 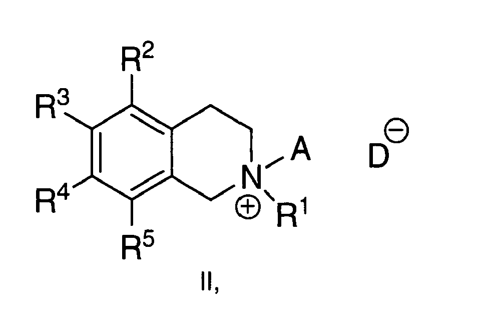 wherein
R¹, R², R³, R⁴ and R⁵ are defined as in claim 1 except for R¹ does not represent a -C(=O)-OR³⁷ moiety,
A represents a hydrogen atom or a radical selected from the group consisting of methyl, ethyl, n-propyl, isopropyl, n-butyl, isobutyl, 2-butyl, tert-butyl and n-pentyl;
D represents an anion selected from the group consisting of chloride, bromide, iodide, fluoride, hydrogensulfate, nitrate, dihydrogenphosphate, thiocyanate, cyanate, acrylate, fumarate, citrate, glutarate, succinate, maleate, tartrate, phosphate, 2-oxo-glutarate, formate, acetate, propionate, lactate, gluconate, benzoate or naphthoate whereby said benzoate or naphthoate may be substituted with 1, 2, 3, 4 or 5 substituents independently selected from the group consisting of F, Cl, Br, -OH, -O-CH₃ and -O-C₂H₅ , pyruvate, ascorbate, glycolate, nicotinate, phenylacetate, 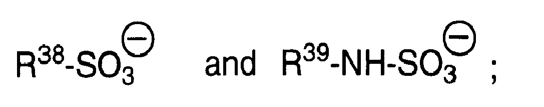 and R³⁸ and R³⁹, independently of one another, in each case represent a radical selected from the group consisting of -CF₃, -C₂F₅, methyl, ethyl, n-propyl, isopropyl, n-butyl, isobutyl, sec-butyl, tert-butyl, n-pentyl, 2-pentyl, 3-pentyl, neo-pentyl, n-hexyl, 2-hexyl, 3-hexyl, n-heptyl, 2-heptyl, 3-heptyl, 4-heptyl, n-octyl, 2-octyl, 3-octyl, 4-octyl, 2-(6-methyl)-heptyl, 2-(5-methyl)-heptyl, 2-(5-methyl)-hexyl, 2-(4-methyl)-hexyl, 2-(7-methyl)-octyl; 2-(6-methyl)-octyl, n-nonyl, n-decyl, n-undecyl, n-dodecyl, n-tridecyl and n-tetradecyl;
a radical selected from the group consisting of phenyl, pyridinyl, pyrazolyl, benzimidazolyl, isoquinolinyl and naphthyl, which may be substituted with 1, 2, 3, 4 or 5 substituent(s) independently selected from the group consisting of F, Cl, Br, -CF₃, methyl, ethyl, n-propyl, isopropyl, n-butyl, isobutyl, 2-butyl, tert-butyl, n-pentyl, 2-pentyl, n-hexyl, -O-CH₃, -O-C₂H₅, - O-CH₂-CH₂-CH₃, -O-CH(CH₃)₂, -O-CH₂-CH₂-CH₂-CH₃, -O-C(CH₃)₃, -OH, - NO₂, -NH₂, phenyl and -SO₃H;
or a radical selected from the group consisting of cyclopropyl, cyclobutyl, cyclopentyl, cyclohexyl, cycloheptyl, cyclooctyl, cyclononyl, cyclodecyl, cycloundecyl, cyclododecyl, cyclotridecyl, cyclotetradecyl, cyclopentenyl, cyclohexenyl, cycloheptenyl, cyclooctenyl and bicyclo[2.2.1]heptyl, which may be bonded via a -(CH₂)-, -(CH₂)-(CH₂)- or -(CH₂)-(CH₂)-(CH₂)-group.

3. A medicament according to claim 2, **characterized in that**
A represents a hydrogen atom or a radical selected from the group consisting of methyl, ethyl, n-propyl, isopropyl, n-butyl, isobutyl, 2-butyl, tert-butyl and n-pentyl;
and D represents an anion selected from the group consisting of chloride, bromide, iodide, fluoride, hydrogensulfate, nitrate, dihydrogenphosphate, thiocyanate, cyanate, acrylate, methanesulfonate, ethanesulfonate, toluenesulfonate, benzenesulfonate, (2,5)-dihydroxy-benzenesulfonate, naphthalene-2-sulfonate, 5-sulfo-napthalene-1-sulfonate, cyclamate, dodecane-1-sulfonate and (7,7)-dimethyl-2-oxo-bicyclo[2.2.1]-hept-1-yl-methanesulfonate.

4. A medicament according to one or more of claims 1 to 3, **characterized in that**
R¹ represents a hydrogen atom;
a linear or branched C₁₋₁₀ alkyl radical, C₂₋₁₀ alkenyl radical or C₂₋₁₀ alkinyl radical;
a C₃₋₉ cycloalkyl radical or C₄₋₉ cycloalkenyl radical, which may be unsubstituted or optionally substituted with 1, 2, 3, 4 or 5 substituent(s) independently selected from the group consisting of oxo (=O), thioxo (=S), C₁₋₅-alkyl, -O-C₁₋₅-alkyl, -S-C₁₋₅-alkyl, -C(=O)-C₁₋₅-alkyl, -O-C(=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl and which may be condensed with an unsubstituted or at least mono-substituted saturated, unsaturated or aromatic mono- or bicyclic ring system and which may be bonded via a linear or branched C₁₋₆ alkylene, C₂₋₆ alkenylene or C₂₋₆ alkinylene group;
or in general formula I R¹ additionally represents a -C(=O)-OR³⁷ moiety;
R², R³, R⁴ and R⁵, independently of one another, each represent a hydrogen atom; F, Cl, Br, I, -NO₂; -NH₂; -SH; -OH; -CN; -C(=O)-OH; -C(=O)-H; - S(=O)₂-OH; -C(=O)-NH₂; -S(=O)₂-NH₂; -C(=O)-R⁶; -S(=O)-R⁷; -S(=O)₂-R⁷; - OR⁸; -SR⁹; -C(=O)-OR¹⁰; -N(R¹¹)-S(=O)₂-R¹²; -NR¹³R¹⁴; -NH-R¹⁵; -C(=O)-NR¹⁶R¹⁷; C(=O)-NHR¹⁸;
a linear or branched C₁₋₁₀alkyl radical, C₂₋₁₀alkenyl radical or C₂₋₁₀ alkinyl radical;
a C₃₋₉ cycloalkyl radical or C₄₋₉ cycloalkenyl radical, which may be bonded via a linear or branched C₁₋₆ alkylene, C₂₋₆ alkenylene or C₂₋₆ alkinylene group and which may be condensed with an unsubstituted or at least mono-substituted saturated, unsaturated or aromatic mono- or bicyclic ring system;
or an unsubstituted or at least mono-substituted 6-, 10- or 14-membered aryl or 5-, 6-, 8-, 9-, 10-, 11-, 12-, 13- or 14-membered heteroaryl radical, which may be bonded via a linear or branched C₁₋₆ alkylene, C₂₋₆ alkenylene or C₂₋₆ alkinylene group and which may be condensed with an unsubstituted or at least mono-substituted saturated or unsaturated, but not aromatic, mono- or bicyclic ring system;
with the proviso that at least one of the substituents R², R³, R⁴ and R⁵ represents a -N(R¹¹)-S(=O)₂-R¹² moiety;
R⁶, R⁷, R⁸, R⁹, R¹⁰, R¹³, R¹⁴, R¹⁵, R¹⁶, R¹⁷ and R¹⁸, independently of one another, each represent a linear or branched C₁₋₁₀ alkyl radical, C₂₋₁₀ alkenyl radical or C₂₋₁₀ alkinyl radical;
a C₃₋₉ cycloalkyl radical or C₄₋₉ cycloalkenyl radical, which may be bonded via a linear or branched C₁₋₆ alkylene, C₂₋₆ alkenylene or C₂₋₆ alkinylene group and which may be condensed with an unsubstituted or at least mono-substituted saturated, unsaturated or aromatic mono- or bicyclic ring system;
or an unsubstituted or at least mono-substituted 6-, 10- or 14-membered aryl or 5-, 6-, 8-, 9-, 10-, 11-, 12-, 13- or 14-membered heteroaryl radical, which may be bonded via a linear or branched C₁₋₆ alkylene, C₂₋₆ alkenylene or C₂₋₆ alkinylene group and which may be condensed with an unsubstituted or at least mono-substituted saturated or unsaturated, but not aromatic, mono- or bicyclic ring system;
R¹¹ represents a hydrogen atom, -S(=O)₂-R¹² or a linear or branched, saturated or unsaturated, unsubstituted or at least mono-substituted C₁₋₆ alkylene, C₂₋₆ alkenylene or C₂₋₆ alkinylene group;
R¹² represents a phenyl radical of general formula (A), 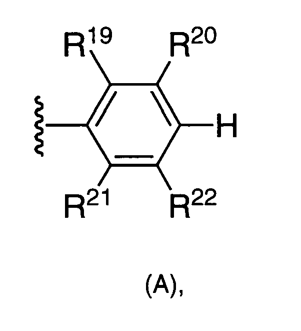 wherein
R¹⁹, R²⁰, R²¹ and R²², independently of one another, each represent a hydrogen atom; F, Cl, Br, I, -NO₂; -NH₂; -SH; -OH; -CN; -C(=O)-OH; -C(=O)-H; -S(=O)₂-OH; -C(=O)-NH₂; -S(=O)₂-NH₂; -C(=O)-R²³; - S(=O)-R²⁴; -S(=O)₂-R²⁴; -OR²⁵; -SR²⁶; -C(=O)-OR²⁷; -N(R²⁸)-S(=O)₂-R²⁹; -NH-S(=O)₂-R³⁰; -NR³¹R³²; -NH-R³³; -C(=O)-NHR³⁴; -C(=O)-NR³⁵R³⁶; or a linear or branched C₁₋₁₀ alkyl radical, C₂₋₁₀ alkenyl radical or C₂₋₁₀ alkinyl radical; or a C₃₋₉ cycloalkyl radical;
with the proviso that at least one of the substituents R¹⁹, R²⁰, R²¹ and R²² is unlike hydrogen;
an unsubstituted or at least mono-substituted 10-membered aryl radical;
a monocyclic 5- or 6-membered heteroaryl radical, which may be unsubstituted or optionally substituted with 1, 2, 3, 4 or 5 substituent(s) independently selected from the group consisting of F, Cl, Br, I, -NO₂;-NH₂; -SH; -OH; -CN; -C(=O)-OH; -C(=O)-H; -S(=O)₂-OH; -C(=O)-NH₂; - S(=O)₂-NH₂; -C(=O)-R²³; -S(=O)-R²⁴; -S(=O)₂-R²⁴; -OR²⁵; -SR²⁶; -C(=O)-OR²⁷; -N(R²⁸)S(=O)₂-R²⁹; -NH-S(=O)₂-R³⁰; -NR³¹R³²; -NH-R³³; -C(=O)-NHR³⁴; -C(=O)-NR³⁵R³⁶; a linear or branched C₁₋₁₀alkyl radical, C₂₋₁₀ alkenyl radical or C₂₋₁₀ alkinyl radical; and a C₃₋₉cycloalkyl radical;
an unsubstituted or at least mono-substituted monocyclic 5- or 6-membered heteroaryl radical, which is condensed with an unsubstituted or at least mono-substituted saturated or unsaturated, but not aromatic, mono- or bicyclic ring system;
an unsubstituted or at least mono-substituted bi- or tricyclic 8-, 9-, 10-, 11-, 12-, 13- or 14-membered heteroaryl radical, which may be condensed with an unsubstituted or at least mono-substituted saturated or unsaturated, but not aromatic, mono- or bicyclic ring system;
or a C₃₋₉cycloalkyl radical or C₄₋₉cycloalkenyl radical, which may be condensed with an unsubstituted or at least mono-substituted saturated, unsaturated or aromatic mono- or bicyclic ring system;
R²³, R²⁷, R²⁸, R²⁹ and R³⁰, independently of one another, each represent a linear or branched C₁₋₁₀ alkyl radical, C₂₋₁₀ alkenyl radical or C₂₋₁₀ alkinyl radical;
a C₃₋₉cycloalkyl radical or C₄₋₉cycloalkenyl radical, which may be bonded via a linear or branched C₁₋₆alkylene, C₂₋₆alkenylene or C₂₋₆alkinylene group and which may be condensed with an unsubstituted or at least mono-substituted saturated, unsaturated or aromatic mono- or bicyclic ring system;
or an unsubstituted or at least mono-substituted 6-, 10- or 14-membered aryl or 5-, 6-, 8-, 9-, 10-, 11-, 12-, 13- or 14-membered heteroaryl radical, which may be bonded via a linear or branched C₁₋₆alkylene, C₂₋₆ alkenylene or C₂₋₆alkinylene group and which may be condensed with an unsubstituted or at least mono-substituted saturated or unsaturated, but not aromatic, mono- or bicyclic ring system;
R²⁴ R²⁶, R³¹, R³² and R³³, each represent a linear or branched C₁₋₁₀ alkyl radical, C₂₋₁₀ alkenyl radical or C₂₋₁₀ alkinyl radical;
a C₃₋₉ cycloalkyl radical or C₄₋₉ cycloalkenyl radical, which may be bonded via a linear or branched C₁₋₆ alkylene, C₂₋₆alkenylene or C₂₋₆ alkinylene group and which may be condensed with an unsubstituted or at least mono-substituted saturated, unsaturated or aromatic mono- or bicyclic ring system;
or an unsubstituted or at least mono-substituted 6-, 10- or 14-membered aryl or 5-, 6-, 8-, 9-, 10-, 11-, 12-, 13- or 14-membered heteroaryl radical, which may be condensed with an unsubstituted or at least mono-substituted saturated or unsaturated, but not aromatic, mono- or bicyclic ring system;
R²⁵ R³⁴, R³⁵ and R³⁶, represents a linear or branched C₁₋₁₀ alkyl radical, C₂₋₁₀ alkenyl radical or C₂₋₁₀ alkinyl radical;
and R³⁷ represents a linear or branched C₁₋₁₀ alkyl radical, C₂₋₁₀ alkenyl radical or C₂₋₁₀ alkinyl radical;
a C₃₋₉ cycloalkyl radical or C₄₋₉ cycloalkenyl radical, which may be bonded via a linear or branched C₁₋₆ alkylene, C₂₋₆ alkenylene or C₂₋₆ alkinylene group and which may be condensed with an unsubstituted or at least mono-substituted saturated, unsaturated or aromatic mono- or bicyclic ring system;
or an unsubstituted or at least mono-substituted 6-, 10- or 14-membered aryl or 5-, 6-, 8-, 9-, 10-, 11-, 12-, 13- or 14-membered heteroaryl radical, which may be bonded via a linear or branched C₁₋₆alkylene, C₂₋₆ alkenylene or C₂₋₆ alkinylene group and which may be condensed with an unsubstituted or at least mono-substituted saturated or unsaturated, but not aromatic, mono- or bicyclic ring system;
whereby
the aforementioned C₁₋₁₀ alkyl radical, C₂₋₁₀ alkenyl radical or C₂₋₁₀ alkinyl radicals may in each case be unsubstituted or optionally substituted with 1, 2, 3, 4 or 5 substituent(s) independently selected from the group consisting of F, Cl, Br, I, - CN, -CF₃, -OCF₃, -SCF₃, -OH, -SH, -NH₂, -O-C₁₋₅-alkyl, -S-C₁₋₅-alkyl, -NH(C₁₋₅-alkyl) and -N(C₁₋₅-alkyl)₂;
if not defined otherwise, the aforementioned C₃₋₉ cycloalkyl radicals and C₄₋₉ cycloalkenyl radicals may in each case be unsubstituted or optionally substituted with 1, 2, 3, 4 or 5 substituent(s) independently selected from the group consisting of oxo (=O), thioxo (=S), C₁₋₅-alkyl, -O-C₁₋₅-alkyl, -S-C₁₋₅-alkyl, -C(=O)-OH, - C(=O)-C₁₋₅-alkyl, -C(=O)-O-C₁₋₅-alkyl, -O-C(=O)-C₁₋₅-alkyl, F, Cl, Br, I, -CN, -CF₃, - OCF₃, -SCF₃, -OH, -SH, -NH₂, -NH(C₁₋₅-alkyl), -N(C₁₋₅-alkyl)₂, -NO₂, -CHO, - CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl;
the aforementioned C₃₋₉ cycloalkyl radicals and C₄₋₉ cycloalkenyl radicals in each case may optionally contain 1, 2 or 3 heteroatom(s) independently selected from the group consisting of nitrogen, oxygen and sulfur as ring member(s);
the aforementioned C₁₋₆alkylene, C₂₋₆alkenylene or C₂₋₆alkinylene groups may in each case be unsubstituted or substituted with 1, 2 or 3 substituent(s) independently selected from the group consisting of F, Cl, Br, I, -CN, -CF₃, -OCF₃, -SCF₃, -OH, -SH, -NH₂, -O-C₁₋₅-alkyl, -S-C₁₋₅-alkyl, -NH(C₁₋₅-alkyl) and -N(C₁₋₅-alkyl)₂;
the rings of the aforementioned ring system are in each case independently of one another 5-, 6- or 7-membered and may in each case independently of one another optionally contain 1, 2 or 3 heteroatom(s) independently selected from the group consisting of nitrogen, oxygen and sulfur;
and the rings of the ring system may in each case be unsubstituted or substituted with 1, 2 or 3 substituent(s) independently selected from the group consisting of oxo (=O), thioxo (=S), C₁₋₅-alkyl, -O-C₁₋₅-alkyl, -S-C₁₋₅-alkyl, -C(=O)-OH, -C(=O)-C₁₋₅-alkyl, -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, - CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl;
if not defined otherwise, the 6-, 10- or 14-membered aryl or 5-, 6-, 8-, 9-, 10-, 11-, 12-, 13- or 14-membered heteroaryl radicals may be unsubstituted or optionally substituted with 1, 2, 3, 4 or 5 substituent(s) independently selected from the group consisting of C₁₋₅-alkyl, -O-C₁₋₅-alkyl, -S-C₁₋₅-alkyl, -C(=O)-OH, -C(=O)-C₁₋₅-alkyl, -C(=O)-O-C₁₋₅-alkyl, -O-C(=O)-C₁₋₅-alkyl, F, Cl, Br, I, -CN, -CF₃, -OCF₃, - SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, - S(=O)₂-phenyl, phenyl, phenoxy and benzyl;
and the aforementioned 5-, 6-, 8-, 9-, 10-, 11-, 12-, 13- or 14-membered heteroaryl radicals in each case optionally contain 1, 2, 3 or 4 heteroatom(s) independently selected from the group consisting of nitrogen, oxygen and sulfur as ring member(s);
optionally in form of one of its stereoisomers, preferably enantiomers or diasteromers, a racemate or in form of a mixture of at least two of its stereoisomers, preferably enantiomers and/or diastereomers, in any mixing ratio, or a salt thereof, or a corresponding solvate thereof.

5. A medicament according to one or more of claims 1 to 4, **characterized in that**
R¹ represents a hydrogen atom; a radical selected from the group consisting of methyl, ethyl, n-propyl, isopropyl, n-butyl, sec-butyl, isobutyl, tert-butyl, n-pentyl, n-hexyl, -CF₃, -CFH₂, -CF₂H, -CH₂-CF₃, -CF₂-CF₃, -CH₂-CN, - CH₂-CH₂-CN, -CH₂-O-CF₃, -CH₂-S-CF₃, -CH₂-OH, -CH₂-CH₂-OH, -CH₂-SH, -CH₂-CH₂-SH, -CH₂-NH₂, -CH₂-NH-CH₃, -CH₂-N(CH₃)₂, -CH₂-N(C₂H₅)₂, - CH₂-NH-C₂H₅, -CH₂-CH₂-NH₂, -CH₂-CH₂-NH-CH₃, -CH₂-CH₂-N(CH₃)₂, - CH₂-CH₂-N(C₂H₅)₂, -CH₂-CH₂-NH-C₂H₅, -CH₂-CH₂-CH₂-NH-CH₃, -CH₂-CH₂-CH₂-N(CH₃)₂, -CH₂-CH₂-CH₂-N(C₂H₅)₂ and -CH₂-CH₂-CH₂-NH-C₂H₅; a (hetero)cycloaliphatic radical selected from the group consisting of cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl and azepanyl, which may be bonded via a -(CH₂)_{1, 2 or 3}- group and which may be unsubstituted or optionally substituted with 1, 2, 3, 4 or 5 substituent(s) independently selected from the group consisting of oxo (=O), thioxo (=S), methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH3)₂, -S-C(CH₃)₃, -C(=O)-CH₃, -C(=O)-C₂H₅, - C(=O)-CH₂-CH₂-CH₃, -C(=O)-CH(CH₃)₂, -C(=O)-C(CH₃)₃, F, Cl, Br, I, -CN, - CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂, -CHO, - CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃ or in general formula I R¹ additionally represents a -C(=O)-OR³⁷ moiety.

6. A medicament according to one or more of claims 1 to 5, **characterized in that**
R², R³, R⁴ and R⁵, independently of one another, each represent a hydrogen atom; F, Cl, Br, l, -NO₂; -NH₂; -SH; -OH; -CN; -C(=O)-OH; -C(=O)-H; - S(=O)₂-OH; -C(=O)-NH₂; -S(=O)₂-NH₂; -C(=O)-R⁶; -S(=O)-R⁷; -S(=O)₂-R⁷; - OR⁸; -SR⁹; -C(=O)-OR¹⁰; -N(R¹¹)-S(=O)₂-R¹²; -NR¹³R¹⁴; -NH-R¹⁵; -C(=O)-NR¹⁶R¹⁷; C(=O)-NHR¹⁸; a radical selected from the group consisting of methyl, ethyl, n-propyl, isopropyl, n-butyl, sec-butyl, isobutyl, tert-butyl, n-pentyl, n-hexyl, vinyl, allyl, ethinyl, -CF₃, -CFH₂, -CF₂H, -CH₂-CF₃, -CF₂-CF₃, -CH₂-CN, -CH₂-CH₂-CN, -CH₂-O-CF₃, -CH₂-S-CF₃, -CH₂-OH, -CH₂-CH₂-OH, -CH₂-SH, -CH₂-CH₂-SH, -CH₂-NH₂, -CH₂-NH-CH₃, -CH₂-N(CH₃)₂, -CH₂-N(C₂H₅)₂ and -CH₂-NH-C₂H₅; or a radical selected from the group consisting of cyclopropyl, cyclobutyl, cyclopentyl, cyclohexyl, phenyl, benzyl, phenethyl, pyridinyl, pyrrolyl, furanyl and thiophenyl, which may be unsubstituted or optionally substituted with 1, 2, 3, 4 or 5 substituent(s) independently selected from the group consisting of oxo (=O), thioxo (=S), methyl, ethyl, n-propyl, isopropyl, n-butyl, tert-butyl, sec-butyl, isobutyl, n-pentyl, -O-CH₃, -O-C₂H₅, -S-CH₃, -S-C₂H₅, F, Cl, Br, -CN, -OH, -SH, -NO₂, -CHO, -CF₂H, -CFH₂ and -C(=O)-NH₂.

7. A medicament according to one or more of claims 1 to 6, **characterized in that** R² represents -N(R¹¹)-S(=O)₂-R¹².

8. A medicament according to one or more of claims 1 to 6, **characterized in that** R³ represents -N(R¹¹)-S(=O)₂-R¹².

9. A medicament according to one or more of claims 1 to 6, **characterized in that** R⁴ represents -N(R¹¹)-S(=O)₂-R¹².

10. A medicament according to one or more of claims 1 to 6, **characterized in that** R⁵ represents -N(R¹¹)-S(=O)₂-R¹².

11. A medicament according to one or more of claims 1 to 10, **characterized in that**
R⁶, R⁷, R⁸, R⁹, R¹⁰, R¹³, R¹⁴, R¹⁵, R¹⁶, R¹⁷ and R¹⁸, independently of one another, each represent a radical selected from the group consisting of methyl, ethyl, n-propyl, isopropyl, n-butyl, sec-butyl, isobutyl, tert-butyl, n-pentyl, n-hexyl, vinyl, allyl, ethinyl, -CF₃, -CFH₂, -CF₂H, -CH₂-CF₃, -CF₂-CF₃, -CH₂-CN, -CH₂-CH₂-CN, -CH₂-O-CF₃, -CH₂-S-CF₃, -CH₂-OH, -CH₂-CH₂-OH, - CH₂-SH, -CH₂-CH₂-SH, -CH₂-NH₂, -CH₂-NH-CH₃, -CH₂-N(CH₃)₂, -CH₂-N(C₂H₅)₂, -CH₂-NH-C₂H₅, -CH₂-CH₂-NH₂, -CH₂-CH₂-NH-CH₃, -CH₂-CH₂-N(CH₃)₂, -CH₂-CH₂-N(C₂H₅)₂, -CH₂-CH₂-NH-C₂H₅, -CH₂-CH₂-CH₂-NH-CH₃, -CH₂-CH₂-CH₂-N(CH₃)₂, -CH₂-CH₂-CH₂-N(C₂H₅)₂ and -CH₂-CH₂-CH₂-NH-C₂H₅; a (hetero)cycloaliphatic radical selected from the group consisting of cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl and azepanyl, which may be bonded via a -(CH₂)₁, _{2 or 3}- group and which may be unsubstituted or optionally substituted with 1, 2, 3, 4 or 5 substituent(s) independently selected from the group consisting of oxo (=O), thioxo (=S), methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-CH₃, -C(=O)-C₂H₅, - C(=O)-CH₂-CH₂-CH₃, -C(=O)-CH(CH₃)₂, -C(=O)-C(CH₃)₃, F, CI, Br, I, -CN, - CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂, -CHO, - CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃; or an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, which may be bonded via a -(CH₂)₁, _{2 or 3}-group and which may be unsubstituted or optionally substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, - S-CH(CH₃)₂, -S-C(CH₃)₃, -C(=O)-OH, -C(=O)-O-CH₃, -C(=O)-O-C₂H₅, - C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, -SH, -NH₂, -NH-CH₃, - NH-C₂H₅, -NH-CH₂-CH₂-CH₃, -NH-CH(CH₃)₂, -NH-C(CH₃)₃, -N(CH₃)₂, - N(C₂H₅)₂, -NO₂, -CHO, -CF₂H, -CFH₂, -C(=O)-NH₂, -C(=O)-NH-CH₃, - C(=O)-NH-C₂H₅, -C(=O)-N(CH₃)₂, -C(=O)-N(C₂H₅)₂ and -S(=O)₂-CH₃.

12. A medicament according to one or more of claims 1 to 11, **characterized in that**
R¹¹ represents a hydrogen atom, -S(=O)₂-R¹² or an alkyl radical selected from the group consisting of methyl, ethyl, n-propyl, isopropyl, n-butyl, sec-butyl, isobutyl and tert-butyl.

13. A medicament according to one or more of claims 1 to 12, **characterized in that**
R¹² represents a phenyl radical of general formula (A), 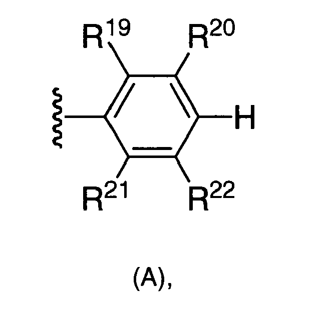 wherein
R¹⁹, R²⁰, R²¹ and R²², independently of one another, each represent a hydrogen atom; F, Cl, Br, I, -NO₂; -NH₂; -SH; -OH; -CN; -C(=O)-OH; -C(=O)-H; -S(=O)₂-OH; -C(=O)-NH₂; -S(=O)₂-NH₂; -C(=O)-R²³; - S(=O)-R²⁴; -S(=O)₂-R²⁴; -OR²⁵; -SR²⁶; -C(=O)-OR²⁷; -N(R²⁸)-S(=O)₂-R²⁹; -NH-S(=O)₂-R³⁰; -NR³¹R³²; -NH-R³³; -C(=O)-NHR³⁴; -C(=O)-NR³⁵R³⁶; methyl, ethyl, n-propyl, isopropyl, n-butyl, isobutyl, sec-butyl, tert-butyl, -CF₃, -CF₂H, -CFH₂, -CH₂-CF₃, -CF₂-CF₃, cyclopropyl, cyclobutyl, cyclopentyl and cyclohexyl;
with the proviso that at least one of the substituents R¹⁹, R²⁰, R²¹ and R²² is unlike hydrogen;
a radical selected from the group consisting of naphthyl, [1,3]-benzodioxolyl, [1,4]-benzodioxanyl, [1,2,3,4]-tetrahydronaphthyl, (2,3)-dihydro-1 H-cyclopenta[b]indolyl, [1,2,3,4]-tetrahydroquinolinyl, [1,2,3,4]-tetrahydroisoquinolinyl, [1,2,3,4]-tetrahydroquinazolinyl, [3,4]-dihydro-2H-benzo[1,4]oxazinyl, indolyl, isoindolyl, quinolinyl, isoquinolinyl, benzo[b]furanyl, benzo[b]thiophenyl, benzo[2,1,3]thiadiazolyl, [1,2,3]-benzothiadiazolyl, [2,1,3]-benzoxadiazolyl, [1,2,3]-benzoxadiazolyl, benzoxazolyl, benzothiazolyl, benzisoxazolyl, benzisothiazolyl, imidazo[2,1-b]thiazolyl, 2H-chromenyl, indazolyl, quinazolinyl, cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl and azepanyl, which may be unsubstituted or optionally substituted with 1, 2, 3, 4 or 5 substituent(s) independently selected from the group consisting of oxo (=O), thioxo (=S), methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-CH₃, - C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, -SH, -NH₂, -NH-CH₃, -NH-C₂H₅, - NH-CH₂-CH₂-CH₃, -NH-CH(CH₃)₂, -NH-C(CH₃)₃, -N(CH₃)₂, -N(C₂H₅)₂, - NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃;
or a radical selected from the group consisting of pyridinyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, pyrimidinyl, pyrazinyl and pyranyl, which may be unsubstituted or optionally substituted with 1, 2, 3, 4 or 5 substituent(s) independently selected from the group consisting of F, Cl, Br, l, -NO₂; -NH₂; -SH; -OH; -CN; -C(=O)-OH; -C(=O)-H; -S(=O)₂-OH; -C(=O)-NH₂; -S(=O)₂-NH₂; -C(=O)-R²³; -S(=O)-R²⁴; -S(=O)₂-R²⁴; -OR²⁵; -SR²⁶; -C(=O)-OR²⁷; -N(R²⁸)-S(=O)₂-R²⁹; -NH-S(=O)₂-R³⁰; -NR³¹R³²; -NH-R³³; -C(=O)-NHR³⁴; -C(=O)-NR³⁵R³⁶; methyl, ethyl, n-propyl, isopropyl, n-butyl, isobutyl, sec-butyl, tert-butyl, -CF₃, -CF₂H, -CFH₂, -CH₂-CF₃, -CF₂-CF₃, cyclopropyl, cyclobutyl, cyclopentyl and cyclohexyl.

14. A medicament according to one or more of claims 1 to 13, **characterized in that**
R²³, R²⁷, R²⁸, R²⁹ and R³⁰, independently of one another,
each represent a radical selected from the group consisting of methyl, ethyl, n-propyl, isopropyl, n-butyl, sec-butyl, isobutyl, tert-butyl, n-pentyl, n-hexyl, vinyl, allyl, ethinyl, -CF₃, -CFH₂, -CF₂H, -CH₂-CF₃, -CF₂-CF₃, -CH₂-CN, -CH₂-CH₂-CN, - CH₂-O-CF₃, -CH₂-S-CF₃, -CH₂-OH, -CH₂-CH₂-OH, -CH₂-SH, -CH₂-CH₂-SH, -CH₂-NH₂, -CH₂-NH-CH₃, -CH₂-N(CH₃)₂, -CH₂-N(C₂H₅)₂, -CH₂-NH-C₂H₅, -CH₂-CH₂-NH₂, -CH₂-CH₂-NH-CH₃, -CH₂-CH₂-N(CH₃)₂, -CH₂-CH₂-N(C₂H₅)₂, -CH₂-CH₂-NH-C₂H₅, -CH₂-CH₂-CH₂-NH-CH₃, -CH₂-CH₂-CH₂-N(CH₃)₂, -CH₂-CH₂-CH₂-N(C₂H₅)₂ and -CH₂-CH₂-CH₂-NH-C₂H₅; a (hetero)cycloaliphatic radical selected from the group consisting of cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl and azepanyl, which may be bonded via a -(CH₂)_{1, 2 or 3}- group and which may be unsubstituted or optionally substituted with 1, 2, 3, 4 or 5 substituent(s) independently selected from the group consisting of oxo (=O), thioxo (=S), methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, - O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-CH₃, -C(=O)-C₂H₅, -C(=O)-CH₂-CH₂-CH₃, -C(=O)-CH(CH₃)₂, - C(=O)-C(CH₃)₃, F, Cl, Br, I, -CN, -CF₃, -OCF₃, -SCF₃, -OH, -SH, -NH₂, -NH-CH₃, - NH-C₂H₅, -NH-CH₂-CH₂-CH₃, -NH-CH(CH₃)₂, -NH-C(CH₃)₃, -N(CH₃)₂, -N(C₂H₅)₂, - NO₂, -CHO, -CF₂H, -CFH₂, -C(=O)-NH₂, -C(=O)-NH-CH₃, -C(=O)-NH-C₂H₅, - C(=O)-N(CH₃)₂, -C(=O)-N(C₂H₅)₂ and -S(=O)₂-CH₃; or an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, which may be bonded via a -(CH₂)₁, _{2 or 3}-group and which may be unsubstituted or optionally substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, - C(=O)-O-CH₂-CH₂-CH₃, -C(=O)-O-CH(CH₃)₂, -C(=O)-O-C(CH₃)₃, -C(=O)-CH₃, - C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂, -CHO, -CF₂H, - CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃.

15. A medicament according to one or more of claims 1 to 14, **characterized in that**
R^{24,} R²⁶, R³¹, R³² and R³³, independently of one another,
each represent a radical selected from the group consisting of methyl, ethyl, n-propyl, isopropyl, n-butyl, sec-butyl, isobutyl, tert-butyl, n-pentyl, n-hexyl, vinyl, allyl, ethinyl, -CF₃, -CFH₂, -CF₂H, -CH₂-CF₃, -CF₂-CF₃, -CH₂-CN, -CH₂-CH₂-CN, - CH₂-O-CF₃, -CH₂-S-CF₃, -CH₂-OH, -CH₂-CH₂-OH, -CH₂-SH, -CH₂-CH₂-SH, -CH₂-NH₂, -CH₂-NH-CH₃, -CH₂-N(CH₃)₂, -CH₂-N(C₂H₅)₂, -CH₂-NH-C₂H₅, -CH₂-CH₂-NH₂, -CH₂-CH₂-NH-CH₃, -CH₂-CH₂-N(CH₃)₂, -CH₂-CH₂-N(C₂H₅)₂, -CH₂-CH₂-NH-C₂H₅, -CH₂-CH₂-CH₂-NH-CH₃, -CH₂-CH₂-CH₂-N(CH₃)₂, -CH₂-CH₂-CH₂-N(C₂H₅)₂ and -CH₂-CH₂-CH₂-NH-C₂H₅; a (hetero)cycloaliphatic radical selected from the group consisting of cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl and azepanyl, which may be unsubstituted or optionally substituted with 1, 2, 3, 4 or 5 substituent(s) independently selected from the group consisting of oxo (=O), thioxo (=S), methyl, ethyl, n-propyl, isopropyl, n-butyl, tert-butyl, sec-butyl, isobutyl, n-pentyl, -O-CH₃, - O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, - OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃; or an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, which may be unsubstituted or optionally substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, - C(=O)-O-CH₂-CH₂-CH₃, -C(=O)-O-CH(CH₃)₂, -C(=O)-O-C(CH₃)₃, -C(=O)-CH₃, - C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂, -CHO, -CF₂H, - CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃.

16. A medicament according to one or more of claims 1 to 15, **characterized in that**
R²⁵, R³⁴, R³⁵ and R³⁶, independently of one another, each represent a radical selected from the group consisting of methyl, ethyl, n-propyl, isopropyl, n-butyl, sec-butyl, isobutyl, tert-butyl, n-pentyl, n-hexyl, vinyl, allyl, ethinyl, -CF₃, -CFH₂, - CF₂H, -CH₂-CF₃, -CF₂-CF₃, -CH₂-CN, -CH₂-CH₂-CN, -CH₂-O-CF₃, -CH₂-S-CF₃, - CH₂-OH, -CH₂-CH₂-OH, -CH₂-SH, -CH₂-CH₂-SH, -CH₂-NH₂, -CH₂-NH-CH₃, -CH₂-N(CH₃)₂, -CH₂-N(C₂H₅)₂, -CH₂-NH-C₂H₅, -CH₂-CH₂-NH₂, -CH₂-CH₂-NH-CH₃, - CH₂-CH₂-N(CH₃)₂, -CH₂-CH₂-N(C₂H₅)₂, -CH₂-CH₂-NH-C₂H₅, -CH₂-CH₂-CH₂-NH-CH₃, -CH₂-CH₂-CH₂-N(CH₃)₂, -CH₂-CH₂-CH₂-N(C₂H₅)₂ and -CH₂-CH₂-CH₂-NH-C₂H₅.

17. A medicament according to one or more of claims 1 to 16, **characterized in that**
R³⁷ represents a radical selected from the group consisting of methyl, ethyl, n-propyl, isopropyl, n-butyl, sec-butyl, isobutyl, tert-butyl, n-pentyl, n-hexyl, vinyl, allyl, ethinyl, -CF₃, -CFH₂, -CF₂H, -CH₂-CF₃, -CF₂-CF₃, -CH₂-CN, -CH₂-CH₂-CN, - CH₂-O-CF₃, -CH₂-S-CF₃, -CH₂-OH, -CH₂-CH₂-OH, -CH₂-SH, -CH₂-CH₂-SH, -CH₂-NH₂, -CH₂-NH-CH₃, -CH₂-N(CH₃)₂, -CH₂-N(C₂H₅)₂, -CH₂-NH-C₂H₅, -CH₂-CH₂-NH₂, -CH₂-CH₂-NH-CH₃, -CH₂-CH₂-N(CH₃)₂, -CH₂-CH₂-N(C₂H₅)₂, -CH₂-CH₂-NH-C₂H₅, -CH₂-CH₂-CH₂-NH-CH₃, -CH₂-CH₂-CH₂-N(CH₃)₂, -CH₂-CH₂-CH₂-N(C₂H₅)₂ and -CH₂-CH₂-CH₂-NH-C₂H₅; a (hetero)cycloaliphatic radical selected from the group consisting of cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl, azepanyl and fluorenyl, which may be bonded via a -(CH₂)_{1, 2 or 3}-group and which may be unsubstituted or optionally substituted with 1, 2, 3, 4 or 5 substituent(s) independently selected from the group consisting of oxo (=O), thioxo (=S), methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, - SCF₃ and -OH; or an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, which may be bonded via a -(CH₂)₁, _{2 or 3}-group and which may be unsubstituted or optionally substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, -SH, -NH₂, -NH-CH₃, -NH-C₂H₅, -NH-CH₂-CH₂-CH₃, - NH-CH(CH₃)₂, -NH-C(CH₃)₃, -N(CH₃)₂, -N(C₂H₅)₂, -NO₂, -CF₂H and -CFH₂.

18. A medicament according to one or more of claims 1 to 17, **characterized in that**
R¹ represents a hydrogen atom; a radical selected from the group consisting of methyl, ethyl, n-propyl, isopropyl, n-butyl, sec-butyl, isobutyl, tert-butyl, n-pentyl, n-hexyl, -CF₃, -CFH₂, -CF₂H, -CH₂-CF₃, -CF₂-CF₃, -CH₂-CN, - CH₂-CH₂-CN, -CH₂-O-CF₃, -CH₂-S-CF₃, -CH₂-OH, -CH₂-CH₂-OH, -CH₂-SH, -CH₂-CH₂-SH, -CH₂-NH₂, -CH₂-NH-CH₃, -CH₂-N(CH₃)₂, -CH₂-N(C₂H₅)₂, - CH₂-NH-C₂H₅, -CH₂-CH₂-NH₂, -CH₂-CH₂-NH-CH₃, -CH₂-CH₂-N(CH₃)₂, - CH₂-CH₂-N(C₂H₅)₂, -CH₂-CH₂-NH-C₂H₅, -CH₂-CH₂-CH₂-NH-CH₃, -CH₂-CH₂-CH₂-N(CH₃)₂, -CH₂-CH₂-CH₂-N(C₂H₅)₂ and -CH₂-CH₂-CH₂-NH-C₂H₅; or a (hetero)cycloaliphatic radical selected from the group consisting of cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl and azepanyl, which may be bonded via a -(CH₂)₁, _{2 or 3}- group and which may be unsubstituted or optionally substituted with 1, 2, 3, 4 or 5 substituent(s) independently selected from the group consisting of oxo (=O), thioxo (=S), methyl, ethyl, n-propyl, isopropyl, n-butyl, tert-butyl, sec-butyl, isobutyl, n-pentyl, -O-CH₃, -O-C_{2H5}, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-CH₃, -C(=O)-C₂H₅, - C(=O)-CH₂-CH₂-CH₃, -C(=O)-CH(CH₃)₂, -C(=O)-C(CH₃)₃, F, Cl, Br, I, -CN, - CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂, -CHO, - CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃ or in general formula I R¹ additionally represents a -C(=0)-OR³⁷ moiety;
R², R³, R⁴ and R⁵, independently of one another, each represent a hydrogen atom;
F, Cl, Br, I, -NO₂; -NH₂; -SH; -OH; -CN; -C(=O)-OH; -C(=O)-H; -S(=O)₂-OH; -C(=O)-NH₂; -S(=O)₂-NH₂; -C(=O)-R⁶; -S(=O)-R⁷; -S(=O)₂-R⁷; -OR⁸; -SR⁹; - C(=O)-OR¹⁰; -N(R¹¹)-S(=O)₂-R¹²; -NR¹³R¹⁴; -NH-R¹⁵; -C(=O)-NR¹⁶R¹⁷; C(=O)-NHR¹⁸ ; a radical selected from the group consisting of methyl, ethyl, n-propyl, isopropyl, n-butyl, sec-butyl, isobutyl, tert-butyl, n-pentyl, n-hexyl, vinyl, allyl, ethinyl, -CF₃, -CFH₂, -CF₂H, -CH₂-CF₃, -CF₂-CF₃, -CH₂-CN, -CH₂-CH₂-CN, -CH₂-O-CF₃, -CH₂-S-CF₃, -CH₂-OH, -CH₂-CH₂-OH, -CH₂-SH, -CH₂-CH₂-SH, -CH₂-NH₂, -CH₂-NH-CH₃, -CH₂-N(CH₃)₂, -CH₂-N(C₂H₅)₂ and -CH₂-NH-C₂H₅; or a radical selected from the group consisting of cyclopropyl, cyclobutyl, cyclopentyl, cyclohexyl, phenyl, benzyl, phenethyl, pyridinyl, pyrrolyl, furanyl and thiophenyl, which may be unsubstituted or optionally substituted with 1, 2, 3, 4 or 5 substituent(s) independently selected from the group consisting of oxo (=O), thioxo (=S), methyl, ethyl, n-propyl, isopropyl, n-butyl, tert-butyl, sec-butyl, isobutyl, n-pentyl, -O-CH₃, -O-C₂H₅, -S-CH₃, -S-C₂H₅, F, Cl, Br, -CN, -OH, -SH, -NO₂, -CHO, -CF₂H, -CFH₂ and -C(=O)-NH₂;
with the proviso that at least one of the substituents R², R³, R⁴ and R⁵ represents a -N(R¹¹)-S(=O)₂-R¹² moiety;
R⁶, R⁷, R⁸, R⁹, R¹⁰, R¹³, R¹⁴, R¹⁵, R¹⁶, R¹⁷ and R¹⁸, independently of one another, each represent a radical selected from the group consisting of methyl, ethyl, n-propyl, isopropyl, n-butyl, sec-butyl, isobutyl, tert-butyl, n-pentyl, n-hexyl, vinyl, allyl, ethinyl, -CF₃, -CFH₂, -CF₂H, -CH₂-CF₃, -CF₂-CF₃, -CH₂-CN, -CH₂-CH₂-CN, -CH₂-O-CF₃, -CH₂-S-CF₃, -CH₂-OH, -CH₂-CH₂-OH, - CH₂-SH, -CH₂-CH₂-SH, -CH₂-NH₂, -CH₂-NH-CH₃, -CH₂-N(CH₃)₂, -CH₂-N(C₂H₅)₂, -CH₂-NH-C₂H₅, -CH₂-CH₂-NH₂, -CH₂-CH₂-NH-CH₃, -CH₂-CH₂-N(CH₃)₂, -CH₂-CH₂-N(C₂H₅)₂, -CH₂-CH₂-NH-C₂H₅, -CH₂-CH₂-CH₂-NH-CH₃, -CH₂-CH₂-CH₂-N(CH₃)₂, -CH₂-CH₂-CH₂-N(C₂H₅)₂ and -CH₂-CH₂-CH₂-NH-C₂H₅; a (hetero)cycloaliphatic radical selected from the group consisting of cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl and azepanyl, which may be bonded via a -(CH₂)_{1,2 or 3}- group and which may be unsubstituted or optionally substituted with 1, 2, 3, 4 or 5 substituent(s) independently selected from the group consisting of oxo (=O), thioxo (=S), methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-CH₃, -C(=O)-C₂H₅, - C(=O)-CH₂-CH₂-CH₃, -C(=O)-CH(CH₃)₂, -C(=O)-C(CH₃)₃, F, Cl, Br, I, -CN, - CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂, -CHO, - CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃; or an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, which may be bonded via a -(CH₂)₁, _{2 or 3}-group and which may be unsubstituted or optionally substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, - S-CH(CH₃)₂, -S-C(CH₃)₃, -C(=O)-OH, -C(=O)-O-CH₃, -C(=O)-O-C₂H₅, - C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, -SH, -NH₂, -NH-CH₃, - NH-C₂H₅, -NH-CH₂-CH₂-CH₃, -NH-CH(CH₃)₂, -NH-C(CH₃)₃, -N(CH₃)₂, - N(C₂H₅)₂, -NO₂, -CHO, -CF₂H, -CFH₂, -C(=O)-NH₂, -C(=O)-NH-CH₃, - C(=O)-NH-C₂H₅, -C(=O)-N(CH₃)₂, -C(=O)-N(C₂H₅)₂ and -S(=O)₂-CH₃;
R¹¹ represents a hydrogen atom, -S(=O)₂-R¹² or an alkyl radical selected from the group consisting of methyl, ethyl, n-propyl, isopropyl, n-butyl, sec-butyl, isobutyl and tert-butyl;
R¹² represents a phenyl radical of general formula (A), 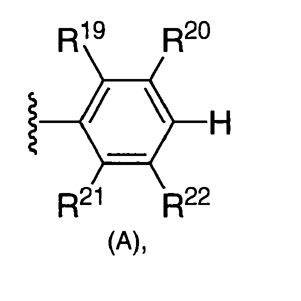 wherein
R¹⁹, R²⁰, R²¹ and R²², independently of one another, each represent a hydrogen atom; F, Cl, Br, I, -NO₂; -NH₂; -SH; -OH; -CN; -C(=O)-OH; -C(=O)-H; -S(=O)₂-OH; -C(=O)-NH₂; -S(=O)₂-NH₂; -C(=O)-R²³; - S(=O)-R²⁴; -S(=O)₂-R²⁴; -OR²⁵; -SR²⁶; -C(=O)-OR²⁷ -N(R²⁸)-S(=O)₂-R²⁹; -NH-S(=O)₂-R³⁰; -NR³¹R³²; -NH-R³³; -C(=O)-NHR³⁴; -C(=O)-NR³⁵R³⁶ ; methyl, ethyl, n-propyl, isopropyl, n-butyl, isobutyl, sec-butyl, tert-butyl, -CF₃, -CF₂H, -CFH₂, -CH₂-CF₃, -CF₂-CF₃, cyclopropyl, cyclobutyl, cyclopentyl and cyclohexyl;
with the proviso that at least one of the substituents R¹⁹, R²⁰, R²¹ and R²² is unlike hydrogen;
a radical selected from the group consisting of naphthyl, [1,3]-benzodioxolyl, [1,4]-benzodioxanyl, [1,2,3,4]-tetrahydronaphthyl, (2,3)-dihydro-1H-cyclopenta[b]indolyl, [1,2,3,4]-tetrahydroquinolinyl, [1,2,3,4]-tetrahydroisoquinolinyl, [1,2,3,4]-tetrahydroquinazolinyl, [3,4]-dihydro-2H-benzo[1,4]oxazinyl, indolyl, isoindolyl, quinolinyl, isoquinolinyl, benzo[b]furanyl, benzo[b]thiophenyl, benzo[2,1,3]thiadiazolyl, [1,2,3]-benzothiadiazolyl, [2,1,3]-benzoxadiazolyl, [1,2,3]-benzoxadiazolyl, benzoxazolyl, benzothiazolyl, benzisoxazolyl, benzisothiazolyl, imidazo[2,1-b]thiazolyl, 2H-chromenyl, indazolyl, quinazolinyl, cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl and azepanyl, which may be unsubstituted or optionally substituted with 1, 2, 3, 4 or 5 substituent(s) independently selected from the group consisting of oxo (=O), thioxo (=S), methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-CH₃, - C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, -SH, -NH₂, -NH-CH₃, -NH-C₂H₅, - NH-CH₂-CH₂-CH₃, -NH-CH(CH₃)₂, -NH-C(CH₃)₃, -N(CH₃)₂, -N(C₂H₅)₂, - NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃;
or a radical selected from the group consisting of pyridinyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, pyrimidinyl, pyrazinyl and pyranyl, which may be unsubstituted or optionally substituted with 1, 2, 3, 4 or 5 substituent(s) independently selected from the group consisting of F, Cl, Br, I, -NO₂; -NH₂; -SH; -OH; -CN; -C(=O)-OH; -C(=O)-H; -S(=O)₂-OH; -C(=O)-NH₂; -S(=O)₂-NH₂; -C(=O)-R²³; -S(=O)-R²⁴; -S(=O)₂-R²⁴; -OR²⁵; -SR²⁶; -C(=O)-OR²⁷; -N(R²⁸)-S(=O)₂-R²⁹; -NH-S(=O)₂-R³⁰; -NR³¹R³²; -NH-R³³; -C(=O)-NHR³⁴; -C(=O)-NR³⁵R³⁶; methyl, ethyl, n-propyl, isopropyl, n-butyl, isobutyl, sec-butyl, tert-butyl, -CF₃, -CF₂H, -CFH₂, -CH₂-CF₃, -CF₂-CF₃, cyclopropyl, cyclobutyl, cyclopentyl and cyclohexyl;
R²³, R²⁷, R²⁸, R²⁹ and R³⁰, independently of one another,
each represent a radical selected from the group consisting of methyl, ethyl, n-propyl, isopropyl, n-butyl, sec-butyl, isobutyl, tert-butyl, n-pentyl, n-hexyl, vinyl, allyl, ethinyl, -CF₃, -CFH₂, -CF₂H, -CH₂-CF₃, -CF₂-CF₃, -CH₂-CN, -CH₂-CH₂-CN, -CH₂-O-CF₃, -CH₂-S-CF₃, -CH₂-OH, -CH₂-CH₂-OH, - CH₂-SH, -CH₂-CH₂-SH, -CH₂-NH₂, -CH₂-NH-CH₃, -CH₂-N(CH₃)₂, -CH₂-N(C₂H₅)₂, -CH₂-NH-C₂H₅, -CH₂-CH₂-NH₂, -CH₂-CH₂-NH-CH₃, -CH₂-CH₂-N(CH₃)₂, -CH₂-CH₂-N(C₂H₅)₂, -CH₂-CH₂-NH-C₂H₅, -CH₂-CH₂-CH₂-NH-CH₃, -CH₂-CH₂-CH₂-N(CH₃)₂, -CH₂-CH₂-CH₂-N(C₂H₅)₂ and -CH₂-CH₂-CH₂-NH-C₂H₅; a (hetero)cycloaliphatic radical selected from the group consisting of cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl and azepanyl, which may be bonded via a -(CH₂)₁, _{2 or 3}- group and which may be unsubstituted or optionally substituted with 1, 2, 3, 4 or 5 substituent(s) independently selected from the group consisting of oxo (=O), thioxo (=S), methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-CH₃, -C(=O)-C₂H₅, - C(=O)-CH₂-CH₂-CH₃, -C(=O)-CH(CH₃)₂, -C(=O)-C(CH₃)₃, F, Cl, Br, I, -CN, - CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂, -CHO, - CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃; or an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, which may be bonded via a -(CH₂)₁, _{2 or 3}-group and which may be unsubstituted or optionally substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, - S-CH(CH₃)₂, -S-C(CH₃)₃, -C(=O)-OH, -C(=O)-O-CH₃, -C(=O)-O-C₂H₅, - C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, -SH, -NH₂, -NH-CH₃, - NH-C₂H₅, -NH-CH₂-CH₂-CH₃, -NH-CH(CH₃)₂, -NH-C(CH₃)₃, -N(CH₃)₂, - N(C₂H₅)₂, -NO₂, -CHO, -CF₂H, -CFH₂, -C(=O)-NH₂, -C(=O)-NH-CH₃, - C(=O)-NH-C₂H₅, -C(=O)-N(CH₃)₂, -C(=O)-N(C₂H₅)₂ and -S(=O)₂-CH₃;
R²⁴, R²⁶, R³¹, R³² and R³³, independently of one another,
each represent a radical selected from the group consisting of methyl, ethyl, n-propyl, isopropyl, n-butyl, sec-butyl, isobutyl, tert-butyl, n-pentyl, n-hexyl, vinyl, allyl, ethinyl, -CF₃, -CFH₂, -CF₂H, -CH₂-CF₃, -CF₂-CF₃, -CH₂-CN, -CH₂-CH₂-CN, -CH₂-O-CF₃, -CH₂-S-CF₃, -CH₂-OH, -CH₂-CH₂-OH, - CH₂-SH, -CH₂-CH₂-SH, -CH₂-NH₂, -CH₂-NH-CH₃, -CH₂-N(CH₃)₂, -CH₂-N(C₂H₅)₂, -CH₂-NH-C₂H₅, -CH₂-CH₂-NH₂, -CH₂-CH₂-NH-CH₃, -CH₂-CH₂-N(CH₃)₂, -CH₂-CH₂-N(C₂H₅)₂, -CH₂-CH₂-NH-C₂H₅, -CH₂-CH₂-CH₂-NH-CH₃, -CH₂-CH₂-CH₂-N(CH₃)₂, -CH₂-CH₂-CH₂-N(C₂H₅)₂ and -CH₂-CH₂-CH₂-NH-C₂H₅; a (hetero)cycloaliphatic radical selected from the group consisting of cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl and azepanyl, which may be unsubstituted or optionally substituted with 1, 2, 3, 4 or 5 substituent(s) independently selected from the group consisting of oxo (=O), thioxo (=S), methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-CH₃, -C(=O)-C₂H₅, -C(=O)-CH₂-CH₂-CH₃, -C(=O)-CH(CH₃)₂, - C(=O)-C(CH₃)₃, F, Cl, Br, I, -CN, -CF₃, -OCF₃, -SCF₃, -OH, -SH, -NH₂, - NH-CH₃, -NH-C₂H₅, -NH-CH₂-CH₂-CH₃, -NH-CH(CH₃)₂, -NH-C(CH₃)₃, - N(CH₃)₂, -N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃; or an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, which may be unsubstituted or optionally substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, - S-C(CH₃)₃, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂,-CHO, -CF₂H, -CFH₂, -C(=O)-NH₂, -C(=O)-NH-CH₃, -C(=O)-NH-C₂H₅, - C(=O)-N(CH₃)₂, -C(=O)-N(C₂H₅)₂ and -S(=O)₂-CH₃;
R²⁵, R³⁴, R³⁵ and R³⁶, independently of one another,
each represent a radical selected from the group consisting of methyl, ethyl, n-propyl, isopropyl, n-butyl, sec-butyl, isobutyl, tert-butyl, n-pentyl, n-hexyl, vinyl, allyl, ethinyl, -CF₃, -CFH₂, -CF₂H, -CH₂-CF₃, -CF₂-CF₃, -CH₂-CN, -CH₂-CH₂-CN, -CH₂-O-CF₃, -CH₂-S-CF₃, -CH₂-OH, -CH₂-CH₂-OH, - CH₂-SH, -CH₂-CH₂-SH, -CH₂-NH₂, -CH₂-NH-CH₃, -CH₂-N(CH₃)₂, -CH₂-N(C₂H₅)₂, -CH₂-NH-C₂H₅, -CH₂-CH₂-NH₂, -CH₂-CH₂-NH-CH₃, -CH₂-CH₂-N(CH₃)₂, -CH₂-CH₂-N(C₂H₅)₂, -CH₂-CH₂-NH-C₂H₅, -CH₂-CH₂-CH₂-NH-CH₃, -CH₂-CH₂-CH₂-N(CH₃)₂, -CH₂-CH₂-CH₂-N(C₂H₅)₂ and -CH₂-CH₂-CH₂-NH-C₂H₅;
and R³⁷ represents a radical selected from the group consisting of methyl, ethyl, n-propyl, isopropyl, n-butyl, sec-butyl, isobutyl, tert-butyl, n-pentyl, n-hexyl, vinyl, allyl, ethinyl, -CF₃, -CFH₂, -CF₂H, -CH₂-CF₃, -CF₂-CF₃, -CH₂-CN, -CH₂-CH₂-CN, -CH₂-O-CF₃, -CH₂-S-CF₃, -CH₂-OH, -CH₂-CH₂-OH, - CH₂-SH, -CH₂-CH₂-SH, -CH₂-NH₂, -CH₂-NH-CH₃, -CH₂-N(CH₃)₂, -CH₂-N(C₂H₅)₂, -CH₂-NH-C₂H₅, -CH₂-CH₂-NH₂, -CH₂-CH₂-NH-CH₃, -CH₂-CH₂-N(CH₃)₂, -CH₂-CH₂-N(C₂H₅)₂, -CH₂-CH₂-NH-C₂H₅, -CH₂-CH₂-CH₂-NH-CH₃, -CH₂-CH₂-CH₂-N(CH₃)₂, -CH₂-CH₂-CH₂-N(C₂H₅)₂ and -CH₂-CH₂-CH₂-NH-C₂H₅; a (hetero)cycloaliphatic radical selected from the group consisting of cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl, azepanyl and fluorenyl, which may be bonded via a -(CH₂)_{1, 2 or 3}- group and which may be unsubstituted or optionally substituted with 1, 2, 3, 4 or 5 substituent(s) independently selected from the group consisting of oxo (=O), thioxo (=S), methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, F, Cl, Br, I, -CN, -CF₃, -OCF₃, - SCF₃ and -OH; or an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, which may be bonded via a - (CH₂)₁, _{2 or 3}-group and which may be unsubstituted or optionally substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, - C(=O)-CH₂-CH₂-CH₃, -C(=O)-CH(CH₃)₂, -C(=O)-C(CH₃)₃, F, Cl, Br, I, -CN, - CF₃, -OCF₃, -SCF₃, -OH, -SH, -NO₂, -CF₂H and -CFH₂; optionally in form of one of its stereoisomers, preferably enantiomers or diasteromers, a racemate or in form of a mixture of at least two of its stereoisomers, preferably enantiomers and/or diastereomers, in any mixing ratio, or a salt thereof, or a corresponding solvate thereof.

19. A medicament according to one or more of claims 1 to 18, **characterized in that**
R¹ represents a hydrogen atom; a radical selected from the group consisting of methyl, ethyl, n-propyl, isopropyl, n-butyl, sec-butyl, isobutyl, tert-butyl, n-pentyl, n-hexyl, -CH₂-NH₂, -CH₂-NH-CH₃, -CH₂-N(CH₃)₂, -CH₂-N(C₂H₅)₂, -CH₂-NH-C₂H₅, -CH₂-CH₂-NH₂, -CH₂-CH₂-NH-CH₃, -CH₂-CH₂-N(CH₃)₂, -CH₂-CH₂-N(C₂H₅)₂, -CH₂-CH₂-NH-C₂H₅, -CH₂-CH₂-CH₂-NH-CH₃, -CH₂-CH₂-CH₂-N(CH₃)₂, - CH₂-CH₂-CH₂-N(C₂H₅)₂ and -CH₂-CH₂-CH₂-NH-C₂H₅; or a (hetero)cycloaliphatic radical selected from the group consisting of imidazolidinyl, aziridinyl, azetidinyl, pyrrolidinyl, piperidinyl, morpholinyl, thiomorpholinyl, piperazinyl, pyrazolidinyl and azepanyl, which may be bonded via a -(CH₂)_{1, 2 or 3}- group and which may be unsubstituted or optionally substituted with 1, 2, 3, 4 or 5 substituent(s) independently selected from the group consisting of oxo (=O), thioxo (=S), methyl, ethyl, n-propyl, isopropyl, n-butyl, tert-butyl, sec-butyl, isobutyl, -C(=O)-CH₃, - C(=O)-C₂H₅, -C(=O)-CH₂-CH₂-CH₃, -C(=O)-CH(CH₃)₂, -C(=O)-C(CH₃)₃, -C(=O)-NH₂, -C(=O)-NH-CH₃, -C(=O)-NH-C₂H₅, -C(=O)-N(CH₃)₂, -C(=O)-N(C₂H₅)₂ and - S(=O)₂-CH₃ or in general formula I R¹ additionally represents a -C(=O)-OR³⁷ moiety;
R², R³, R⁴ and R⁵, independently of one another, each represent a hydrogen atom; F, Cl, Br, I, -NO₂; -NH₂; -SH; -OH; -CN; -C(=O)-OH; -C(=O)-H; - S(=O)₂-OH; -C(=O)-NH₂; -S(=O)₂-NH₂; -OR⁸; -SR⁹; -N(R¹¹)-S(=O)₂-R¹²; a radical selected from the group consisting of methyl, ethyl, n-propyl, isopropyl, n-butyl, sec-butyl, isobutyl, tert-butyl, vinyl, allyl, ethinyl, -CF₃, - CFH₂, -CF₂H, -CH₂-CF₃ and -CF₂-CF₃; or a radical selected from the group consisting of cyclopropyl, cyclobutyl, cyclopentyl and cyclohexyl; with the proviso that at least one of the substituents R², R³, R⁴ and R⁵ represents a -N(R¹¹)-S(=O)₂-R¹² moiety;
R⁸ and R⁹, independently of one another, each represent a radical selected from the group consisting of methyl, ethyl, n-propyl, isopropyl, n-butyl, sec-butyl, isobutyl, tert-butyl, -CF₃, -CFH₂, -CF₂H, -CH₂-CF₃ and -CF₂-CF₃; a (hetero)cycloaliphatic radical selected from the group consisting of cyclopropyl, cyclobutyl, cyclopentyl and cyclohexyl; or an aryl or heteroaryl radical selected from the group consisting of phenyl, naphthyl, furyl (furanyl), thienyl (thiophenyl), pyrrolyl, and pyridinyl, which may be bonded via a -(CH₂)_{1, 2 or 3}-group and which may be unsubstituted or optionally substituted with 1, 2 or 3 substituent(s) independently selected from the group consisting of methyl, ethyl, n-propyl, isopropyl, n-butyl, tert-butyl, sec-butyl, isobutyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, F, Cl, Br, I, -CN, -CF₃, -OCF₃, -SCF₃, -OH, -SH, -NH₂, -NO₂, -CHO, -CF₂H and -CFH₂;
R¹¹ represents a hydrogen atom, -S(=O)₂-R¹² or an alkyl radical selected from the group consisting of methyl, ethyl, n-propyl, isopropyl, n-butyl, sec-butyl, isobutyl and tert-butyl;
R¹² represents a phenyl radical of general formula (A), 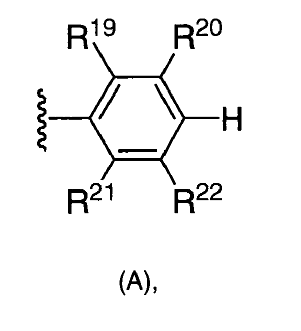 wherein
R¹⁹, R²⁰, R²¹ and R²², independently of one another, each represent a hydrogen atom; F, Cl, Br, I, -NO₂; -NH₂; -SH; -OH; -CN; -C(=O)-OH; -C(=O)-H; -S(=O)₂-OH; -C(=O)-NH₂; -S(=O)₂-NH₂; -C(=O)-R²³; - S(=O)-R²⁴; -S(=O)₂-R²⁴; -OR²⁵; -SR²⁶; -C(=O)-OR²⁷; methyl, ethyl, n-propyl, isopropyl, n-butyl, isobutyl, sec-butyl, tert-butyl, -CF₃, -CF₂H, -CFH₂, -CH₂-CF₃, -CF₂-CF₃, cyclopropyl, cyclobutyl, cyclopentyl and cyclohexyl;
with the proviso that at least one of the substituents R¹⁹, R²⁰, R²¹ and R²² is unlike hydrogen;
a radical selected from the group consisting of naphthyl, [1,3]-benzodioxolyl, [1,4]-benzodioxanyl, [1 ,2,3,4]-tetrahydronaphthyl, (2,3)-dihydro-1 H-cyclopenta[b]indolyl, [1,2,3,4]-tetrahydroquinolinyl, [1,2,3,4]-tetrahydroisoquinolinyl, [1,2,3,4]-tetrahydroquinazolinyl, [3,4]-dihydro-2H-benzo[1,4]oxazinyl, indolyl, isoindolyl, quinolinyl, isoquinolinyl, benzo[b]furanyl, benzo[b]thiophenyl, benzo[2,1,3]thiadiazolyl, [1,2,3]-benzothiadiazolyl, [2,1,3]-benzoxadiazolyl, [1,2,3]-benzoxadiazolyl, benzoxazolyl, benzothiazolyl, benzisoxazolyl, benzisothiazolyl, imidazo[2,1-b]thiazolyl, 2H-chromenyl, indazolyl and quinazolinyl, which may be unsubstituted or optionally substituted with 1, 2, 3, 4 or 5 substituent(s) independently selected from the group consisting of methyl, ethyl, n-propyl, isopropyl, n-butyl, tert-butyl, sec-butyl, isobutyl, n-pentyl, - O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, F, Cl, Br, I, -CN, -CF₃, - OCF₃, -SCF₃, -OH, -SH, -NH₂, -NO₂, -CHO, -CF₂H and -CFH₂;
or a radical selected from the group consisting of pyridinyl, furyl (furanyl), thienyl (thiophenyl), pyrrolyl, imidazolyl, pyrazolyl, triazolyl, pyridazinyl, pyrimidinyl and pyrazinyl, which may be unsubstituted or optionally substituted with 1, 2, 3, 4 or 5 substituent(s) independently selected from the group consisting of F, Cl, Br, I, -NO₂; -NH₂; -SH; -OH; -CN; -C(=O)-OH; -C(=O)-H; -S(=O)₂-OH; -C(=O)-NH₂; -S(=O)₂-NH₂; -C(=O)-R²³; -S(=O)-R²⁴; -S(=O)₂-R²⁴; -OR²⁵; -SR²⁶; -C(=O)-OR²⁷; methyl, ethyl, n-propyl, isopropyl, n-butyl, isobutyl, sec-butyl, tert-butyl, -CF₃, -CF₂H, -CFH₂, -CH₂-CF₃, -CF₂-CF₃, cyclopropyl, cyclobutyl, cyclopentyl and cyclohexyl;
R²³ and R²⁷, independently of one another, each represent a radical selected from the group consisting of methyl, ethyl, n-propyl, isopropyl, n-butyl, sec-butyl, isobutyl, tert-butyl, n-pentyl, n-hexyl, -CF₃, -CFH₂, -CF₂H, -CH₂-CF₃ and - CF₂-CF₃; or an aryl or heteroaryl radical selected from the group consisting of phenyl, naphthyl, furyl (furanyl), thienyl (thiophenyl), pyrrolyl, and pyridinyl, which may be bonded via a -(CH₂)₁, _{2 or 3}-group and which may be unsubstituted or optionally substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, F, Cl, Br, I, -CN, -CF₃, -OCF₃, -SCF₃, -OH, -SH, -NH₂-NO₂, -CHO, -CF₂H and -CFH₂;
R²⁴ and R²⁶, independently of one another, each represent a radical selected from the group consisting of methyl, ethyl, n-propyl, isopropyl, n-butyl, sec-butyl, isobutyl, tert-butyl, n-pentyl, n-hexyl, vinyl, allyl, ethinyl, -CF₃, -CFH₂, - CF₂H, -CH₂-CF₃ and -CF₂-CF₃; or an aryl or heteroaryl radical selected from the group consisting of phenyl, naphthyl, furyl (furanyl), thienyl (thiophenyl), pyrrolyl, and pyridinyl, which may be unsubstituted or optionally substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, F, Cl, Br, I, -CN, -CF₃, -OCF₃, -SCF₃, -OH, -SH, - NH₂, -NO₂, -CHO, -CF₂H and -CFH₂;
R²⁵ represents a radical selected from the group consisting of methyl, ethyl, n-propyl, isopropyl, n-butyl, sec-butyl, isobutyl, tert-butyl, -CF₃, - CFH₂, -CF₂H, -CH₂-CF₃ and -CF₂-CF₃;
and R³⁷ represents a radical selected from the group consisting of methyl, ethyl, n-propyl, isopropyl, n-butyl, sec-butyl, isobutyl, tert-butyl, n-pentyl, n-hexyl, vinyl, allyl, ethinyl, -CF₃, -CFH₂, -CF₂H, -CH₂-CF₃ and -CF₂-CF₃; a (hetero)cycloaliphatic radical selected from the group consisting of cyclopropyl, cyclobutyl, cyclopentyl, cyclohexyl, cycloheptyl, cyclooctyl, cyclononyl and fluorenyl, which may be bonded via a -(CH₂)₁, _{2 or 3}- group and which may be unsubstituted or optionally substituted with 1, 2, 3, 4 or 5 substituent(s) independently selected from the group consisting of methyl, ethyl, n-propyl, isopropyl, n-butyl, tert-butyl, sec-butyl, isobutyl and n-pentyl; or an aryl or heteroaryl radical selected from the group consisting of phenyl and naphthyl, which may be bonded via a -(CH₂)_{1,2 or 3}-group and which may be unsubstituted or optionally substituted with 1, 2 or 3 substituent(s) independently selected from the group consisting of methyl, ethyl, n-propyl, isopropyl, n-butyl, tert-butyl, sec-butyl, isobutyl, n-pentyl, - O-CH₃, -O-C₂H₅, -O-CH₂-CH₂-CH₃, -O-CH(CH₃)₂, -O-C(CH₃)₃, F, Cl, Br, I, - CN, -CF₃, -OCF₃, -SCF₃, -OH, -SH, -NO₂, -CF₂H and -CFH₂;
optionally in form of one of its stereoisomers, preferably enantiomers or diasteromers, a racemate or in form of a mixture of at least two of its stereoisomers, preferably enantiomers and/or diastereomers, in any mixing ratio, or a salt thereof, or a corresponding solvate thereof.

20. A medicament according to one or more of claims 1 to 19, **characterized in that**
R¹ represents a hydrogen atom; a radical selected from the group consisting of methyl, ethyl, n-propyl, isopropyl, n-butyl, sec-butyl, isobutyl, tert-butyl, n-pentyl, n-hexyl, -CH₂-NH₂, -CH₂-NH-CH₃, -CH₂-N(CH₃)₂, -CH₂-N(C₂H₅)₂, -CH₂-NH-C₂H₅, -CH₂-CH₂-NH₂, -CH₂-CH₂-NH-CH₃, -CH₂-CH₂-N(CH₃)₂, - CH₂-CH₂-N(C₂H₅)₂, -CH₂-CH₂-NH-C₂H₅, -CH₂-CH₂-CH₂-NH-CH₃, -CH₂-CH₂-CH₂-N(CH₃)₂, -CH₂-CH₂-CH₂-N(C₂H₅)₂ and -CH₂-CH₂-CH₂-NH-C₂H₅; a (hetero)cycloaliphatic radical selected from the group consisting of imidazolidinyl, aziridinyl, azetidinyl, pyrrolidinyl, piperidinyl, morpholinyl, thiomorpholinyl, piperazinyl, pyrazolidinyl and azepanyl, which may be bonded via a -(CH₂)₁, _{2 or 3}- group and which may be unsubstituted or optionally substituted with 1, 2, 3, 4 or 5 substituent(s) independently selected from the group consisting of methyl, ethyl, n-propyl, isopropyl, n-butyl, tert-butyl, sec-butyl and isobutyl or in general formula I R¹ additionally represents a -C(=O)-OR³⁷ moiety;
R² R³, R⁴ and R⁵, independently of one another, each represent a hydrogen atom; F, Cl, Br, 1, -NO₂; -O-CH₃; -O-C₂H₅; -O-CF₃; -O-CFH₂; -O-CF₂H; -O-CH₂-CF₃; -O-CF₂-CF₃; -S-CH₃; -S-C₂H₅; -S-CF₃; -S-CFH₂; -S-CF₂H; -S-CH₂-CF₃; -S-CF₂-CF₃; -N(R¹¹)-S(=O)₂-R¹²; or a radical selected from the group consisting of methyl, ethyl, n-propyl, isopropyl, tert-butyl, -CF₃, - CFH₂, -CF₂H, -CH₂-CF₃ and -CF₂-CF₃;
with the proviso that at least one of the substituents R², R³, R⁴ and R⁵ represents a -N(R¹¹)-S(=O)₂-R¹² moiety;
R¹¹ represents a hydrogen atom, -S(=O)₂-R¹² or an alkyl radical selected from the group consisting of methyl, ethyl, n-propyl, isopropyl, n-butyl, sec-butyl, isobutyl and tert-butyl;
R¹² represents a phenyl radical of general formula (A), 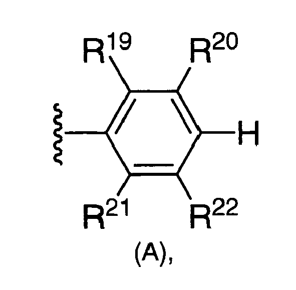 wherein
R¹⁹, R²⁰, R²¹ and R²², independently of one another, each represent a hydrogen atom; F, Cl, Br, I, -NO₂; -NH₂; -SH; -OH; -CN; -C(=O)-OH; -C(=O)-H; -C(=O)-CH₃; -C(=O)-C₂H₅; -O-CH₃; -O-C₂H₅; -O-CF₃; -O-CFH₂; -O-CF₂H; -O-CH₂-CF₃; -O-CF₂-CF₃; -S-CH₃; -S-C₂H₅; -S-CF₃; -S-CFH₂; -S-CF₂H; -S-CH₂-CF₃; -S-CF₂-CF₃; -C(=O)-OCH₃; - C(=O)-OC₂H₅; methyl, ethyl, n-propyl, isopropyl, n-butyl, isobutyl, sec-butyl, tert-butyl, -CF₃, -CF₂H, -CFH₂, -CH₂-CF₃ and -CF₂-CF₃;
with the proviso that at least one of the substituents R¹⁹, R²⁰, R²¹ and R²² is unlike hydrogen;
a radical selected from the group consisting of naphthyl, [1,3]-benzodioxolyl, [1,4]-benzodioxanyl, benzo[b]furanyl, benzo[b]thiophenyl, benzo[2,1,3]thiadiazolyl, [1,2,3]-benzothiadiazolyl, [2,1,3]-benzoxadiazolyl, [1,2,3]-benzoxadiazolyl, benzoxazolyl, benzothiazolyl, benzisoxazolyl, benzisothiazolyl and imidazo[2,1-b]thiazolyl, which may be unsubstituted or optionally substituted with 1, 2, 3, 4 or 5 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, F, Cl, Br, I, - CN, -CF₃, -OCF₃, -SCF₃, -CF₂H and -CFH₂;
or a radical selected from the group consisting of pyridinyl, pyrrolyl, imidazolyl, pyrazolyl, triazolyl, pyridazinyl, pyrimidinyl and pyrazinyl, which may be unsubstituted or optionally substituted with 1, 2, 3, 4 or 5 substituent(s) independently selected from the group consisting of F, Cl, Br, I, -NO₂; methyl, ethyl, n-propyl, isopropyl, n-butyl, isobutyl, sec-butyl, tert-butyl, -CF₃, -CF₂H, -CFH₂, -CH₂-CF₃ and -CF₂-CF₃;
and R³⁷ represents a radical selected from the group consisting of methyl, ethyl, n-propyl, isopropyl, n-butyl, sec-butyl, isobutyl, tert-butyl, n-pentyl, n-hexyl, fluorenyl, fluorenylmethyl, phenyl, benzyl and naphthyl;
optionally in form of one of its stereoisomers, preferably enantiomers or diasteromers, a racemate or in form of a mixture of at least two of its stereoisomers, preferably enantiomers and/or diastereomers, in any mixing ratio, or a salt thereof, or a corresponding solvate thereof.

21. A medicament according to one or more of claims 1 to 20, **characterized in that**
R¹ represents a hydrogen atom; a radical selected from the group consisting of methyl, ethyl, n-propyl, n-butyl, n-pentyl and n-hexyl; or in general formula I R¹ additionally represents a -C(=O)-OR³⁷ moiety;
R², R³, R⁴ and R⁵, independently of one another, each represent a hydrogen atom or -N(R¹¹)-S(=O)₂-R¹²;
with the proviso that at least one of the substituents R², R³, R⁴ and R⁵ represents a -N(R¹¹)-S(=O)₂-R¹² moiety;
R¹¹ represents a hydrogen atom, -S(=O)₂-R¹² or an alkyl radical selected from the group consisting of methyl, ethyl and n-propyl;
R¹² represents a phenyl radical of general formula (A), 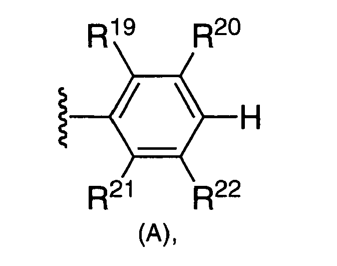 wherein
R¹⁹, R²⁰, R²¹ and R²², independently of one another, each represent a hydrogen atom; F, Cl, Br, I, -O-CH₃; -O-C₂H₅; -O-CF₃; -O-CFH₂; - O-CF₂H; -O-CH₂-CF₃; -O-CF₂-CF₃; methyl, ethyl, n-propyl, isopropyl, n-butyl, isobutyl, sec-butyl and tert-butyl;
with the proviso that at least one of the substituents R¹⁹, R²⁰, R²¹ and R²² is unlike hydrogen;
a radical selected from the group consisting of naphthyl, benzo[b]thiophenyl and imidazo[2,1-b]thiazolyl, which may be unsubstituted or optionally substituted with 1, 2, 3, 4 or 5 substituent(s) independently selected from the group consisting of methyl, ethyl, n-propyl, isopropyl, F, Cl and Br;
or a pyridinyl radical, which may be unsubstituted or optionally substituted with 1, 2, 3, 4 or 5 substituent(s) independently selected from the group consisting of F, Cl, Br, I, methyl, ethyl, n-propyl, isopropyl, n-butyl, isobutyl, sec-butyl and tert-butyl;
and R³⁷ represents a radical selected from the group consisting of methyl, ethyl, n-propyl, isopropyl, n-butyl, sec-butyl, isobutyl, tert-butyl, n-pentyl, n-hexyl, fluorenyl, fluorenylmethyl, phenyl, benzyl and naphthyl;
optionally in form of one of its stereoisomers, preferably enantiomers or diasteromers, a racemate or in form of a mixture of at least two of its stereoisomers, preferably enantiomers and/or diastereomers, in any mixing ratio, or a salt thereof, or a corresponding solvate thereof.

22. A medicament comprising at least one compound of general formula la according to claim 20, 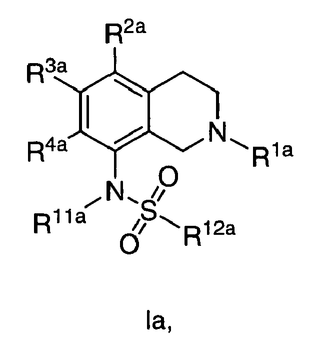 wherein
R^{1a} represents a hydrogen atom; a -C(=O)-OR^{37a} moiety; a radical selected from the group consisting of methyl, ethyl, n-propyl, isopropyl, n-butyl, sec-butyl, isobutyl, tert-butyl, n-pentyl, n-hexyl, -CH₂-NH₂, -CH₂-NH-CH₃, -CH₂-N(CH₃)₂, -CH₂-N(C₂H₅)₂, -CH₂-NH-C₂H₅, -CH₂-CH₂-NH₂, -CH₂-CH₂-NH-CH₃, -CH₂-CH₂-N(CH₃)₂, -CH₂-CH₂-N(C₂H₅)₂, -CH₂-CH₂-NH-C₂H₅, -CH₂-CH₂-CH₂-NH-CH₃, -CH₂-CH₂-CH₂-N(CH₃)₂, -CH₂-CH₂-CH₂-N(C₂H₅)₂ and - CH₂-CH₂-CH₂-NH-C₂H₅; or a (hetero)cycloaliphatic radical selected from the group consisting of imidazolidinyl, aziridinyl, azetidinyl, pyrrolidinyl, piperidinyl, morpholinyl, thiomorpholinyl, piperazinyl, pyrazolidinyl and azepanyl, which may be bonded via a -(CH₂)_{1,2 or 3}- group and which may be unsubstituted or optionally substituted with 1, 2, 3, 4 or 5 substituent(s) independently selected from the group consisting of methyl, ethyl, n-propyl, isopropyl, n-butyl, tert-butyl, sec-butyl and isobutyl;
R^{2a}, R^{3a} and R^{4a}, independently of one another, each represent a hydrogen atom; F, CI, Br, 1, -NO₂; -O-CH₃; -O-C₂H₅; -O-CF₃; -O-CFH₂; -O-CF₂H; -O-CH₂-CF₃; -O-CF₂-CF₃; -S-CH₃; -S-C₂H₅; -S-CF₃; -S-CFH₂; -S-CF₂H; -S-CH₂-CF₃; -S-CF₂-CF₃; -N(R^{11a})-S(=O)₂-R^{12a}; or a radical selected from the group consisting of methyl, ethyl, n-propyl, isopropyl, tert-butyl, -CF₃, - CFH₂, -CF₂H, -CH₂-CF₃ and -CF₂-CF₃;
R^{11a} represents a hydrogen atom, -S(=O)₂-R^{12a} or an alkyl radical selected from the group consisting of methyl, ethyl, n-propyl, isopropyl, n-butyl, sec-butyl, isobutyl and tert-butyl;
R^{12a} represents a phenyl radical of general formula (Aa), 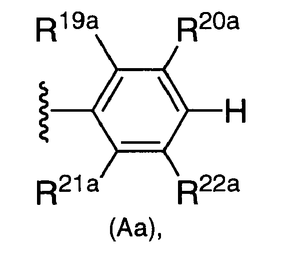 wherein
R^{19a}, R^{20a}, R^{21a} and R^{22a}, independently of one another, each represent a hydrogen atom; F, Cl, Br, I, -NO₂; -NH₂; -SH; -OH; -CN; -C(=O)-OH; -C(=O)-H; -C(=O)-CH₃; -C(=O)-C₂H₅; -O-CH₃; -O-C₂H₅; -O-CF₃; -O-CFH₂; -O-CF₂H; -O-CH₂-CF₃; -O-CF₂-CF₃; -S-CH₃; -S-C₂H₅; -S-CF₃; -S-CFH₂; -S-CF₂H; -S-CH₂-CF₃; -S-CF₂-CF₃; -C(=O)-OCH₃; -C(=O)-OC₂H₅; methyl, ethyl, n-propyl, isopropyl, n-butyl, isobutyl, sec-butyl, tert-butyl, -CF₃, -CF₂H, -CFH₂, -CH₂-CF₃ and - CF₂-CF₃;
with the proviso that at least one of the substituents R^{19a}, R^{20a}, R^{21a} and R^{22a} is unlike hydrogen;
a radical selected from the group consisting of naphthyl, [1,3]-benzodioxolyl, [1,4]-benzodioxanyl, benzo[b]furanyl, benzo[b]thiophenyl, benzo[2,1,3]thiadiazolyl, [1,2,3]-benzothiadiazolyl, [2,1,3]-benzoxadiazolyl, [1,2,3]-benzoxadiazolyl, benzoxazolyl, benzothiazolyl, benzisoxazolyl, benzisothiazolyl and imidazo[2,1-b]thiazolyl, which may be unsubstituted or optionally substituted with 1, 2, 3, 4 or 5 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, F, Cl, Br, I, - CN, -CF₃, -OCF₃, -SCF₃, -CF₂H and -CFH₂;
or a radical selected from the group consisting of pyridinyl, pyrrolyl, imidazolyl, pyrazolyl, triazolyl, pyridazinyl, pyrimidinyl and pyrazinyl, which may be unsubstituted or optionally substituted with 1, 2, 3, 4 or 5 substituent(s) independently selected from the group consisting of F, Cl, Br, 1, -NO₂; methyl, ethyl, n-propyl, isopropyl, n-butyl, isobutyl, sec-butyl, tert-butyl, -CF₃, -CF₂H, -CFH₂, -CH₂-CF₃ and -CF₂-CF₃;
and R^{37a} represents a radical selected from the group consisting of methyl, ethyl, n-propyl, isopropyl, n-butyl, sec-butyl, isobutyl, tert-butyl, n-pentyl, n-hexyl, fluorenyl, fluorenylmethyl, phenyl, benzyl and naphthyl;
optionally in form of one of its stereoisomers, preferably enantiomers or diasteromers, a racemate or in form of a mixture of at least two of its stereoisomers, preferably enantiomers and/or diastereomers, in any mixing ratio, or a salt thereof, or a corresponding solvate thereof.

23. A medicament comprising at least one compound of general formula lb according to claim 20, 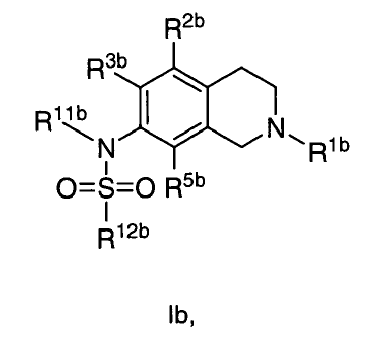 wherein
R^{1b} represents a hydrogen atom; a -C(=O)-OR^{37b} moiety; a radical selected from the group consisting of methyl, ethyl, n-propyl, isopropyl, n-butyl, sec-butyl, isobutyl, tert-butyl, n-pentyl, n-hexyl, -CH₂-NH₂, -CH₂-NH-CH₃, -CH₂-N(CH₃)₂, -CH₂-N(C₂H₅)₂, -CH₂-NH-C₂H₅, -CH₂-CH₂-NH₂, -CH₂-CH₂-NH-CH₃, -CH₂-CH₂-N(CH₃)₂, -CH₂-CH₂-N(C₂H₅)₂, -CH₂-CH₂-NH-C₂H₅, -CH₂-CH₂-CH₂-NH-CH₃, -CH₂-CH₂-CH₂-N(CH₃)₂, -CH₂-CH₂-CH₂-N(C₂H₅)₂ and - CH₂-CH₂-CH₂-NH-C₂H₅; or a (hetero)cycloaliphatic radical selected from the group consisting of imidazolidinyl, aziridinyl, azetidinyl, pyrrolidinyl, piperidinyl, morpholinyl, thiomorpholinyl, piperazinyl, pyrazolidinyl and azepanyl, which may be bonded via a -(CH₂)_{1,2 or 3}- group and which may be unsubstituted or optionally substituted with 1, 2, 3, 4 or 5 substituent(s) independently selected from the group consisting of methyl, ethyl, n-propyl, isopropyl, n-butyl, tert-butyl, sec-butyl and isobutyl;
R^{2b}, R^{3b} and R^{5b}, independently of one another, each represent a hydrogen atom; F, Cl, Br, 1, -NO₂; -O-CH₃; -O-C₂H₅; -O-CF₃; -O-CFH₂; -O-CF₂H; -O-CH₂-CF₃; -O-CF₂-CF₃; -S-CH₃; -S-C₂H₅; -S-CF₃; -S-CFH₂; -S-CF₂H; -S-CH₂-CF₃; -S-CF₂-CF₃; -N(R^{11b})-S(=O)₂-R^{12b}; or a radical selected from the group consisting of methyl, ethyl, n-propyl, isopropyl, tert-butyl, -CF₃, - CFH₂, -CF₂H, -CH₂-CF₃ and -CF₂-CF₃;
R^{11b} represents a hydrogen atom, -S(=O)₂-R^{12b} or an alkyl radical selected from the group consisting of methyl, ethyl, n-propyl, isopropyl, n-butyl, sec-butyl, isobutyl and tert-butyl;
R^{12b} represents a phenyl radical of general formula (Ab), 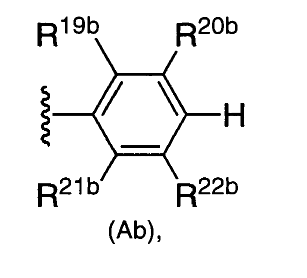 wherein
R^{19b}, R^{20b}, R^{21b} and R^{22b}, independently of one another, each represent a hydrogen atom; F, Cl, Br, I, -NO₂; -NH₂; -SH; -OH; -CN; -C(=O)-OH; -C(=O)-H; -C(=O)-CH₃; -C(=O)-C₂H₅; -O-CH₃; -O-C₂H₅; -O-CF₃; -O-CFH₂; -O-CF₂H; -O-CH₂-CF₃; -O-CF₂-CF₃; -S-CH₃; -S-C₂H₅; -S-CF₃; -S-CFH₂; -S-CF₂H; -S-CH₂-CF₃; -S-CF₂-CF₃; -C(=O)-OCH₃; -C(=O)-OC₂H₅; methyl, ethyl, n-propyl, isopropyl, n-butyl, isobutyl, sec-butyl, tert-butyl, -CF₃, -CF₂H, -CFH₂, -CH₂-CF₃ and - CF₂-CF₃;
with the proviso that at least one of the substituents R^{19b} , R^{20b}, R^{21b} and R^{22b} is unlike hydrogen;
a radical selected from the group consisting of naphthyl, [1,3]-benzodioxolyl, [1,4]-benzodioxanyl, benzo[b]furanyl, benzo[b]thiophenyl, benzo[2,1,3]thiadiazolyl, [1,2,3]-benzothiadiazolyl, [2,1,3]-benzoxadiazolyl, [1,2,3]-benzoxadiazolyl, benzoxazolyl, benzothiazolyl, benzisoxazolyl, benzisothiazolyl and imidazo[2,1-b]thiazolyl, which may be unsubstituted or optionally substituted with 1, 2, 3, 4 or 5 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, F, Cl, Br, I, - CN, -CF₃, -OCF₃, -SCF₃, -CF₂H and -CFH₂;
or a radical selected from the group consisting of pyridinyl, pyrrolyl, imidazolyl, pyrazolyl, triazolyl, pyridazinyl, pyrimidinyl and pyrazinyl, which may be unsubstituted or optionally substituted with 1, 2, 3, 4 or 5 substituent(s) independently selected from the group consisting of F, Cl, Br, I, -NO₂; methyl, ethyl, n-propyl, isopropyl, n-butyl, isobutyl, sec-butyl, tert-butyl, -CF₃, -CF₂H, -CFH₂, -CH₂-CF₃ and -CF₂-CF₃;
and R^{37b} represents a radical selected from the group consisting of methyl, ethyl, n-propyl, isopropyl, n-butyl, sec-butyl, isobutyl, tert-butyl, n-pentyl, n-hexyl, fluorenyl, fluorenylmethyl, phenyl, benzyl and naphthyl;
optionally in form of one of its stereoisomers, preferably enantiomers or diasteromers, a racemate or in form of a mixture of at least two of its stereoisomers, preferably enantiomers and/or diastereomers, in any mixing ratio, or a salt thereof, or a corresponding solvate thereof.

24. A medicament comprising at least one compound of general formula lc according to claim 20, 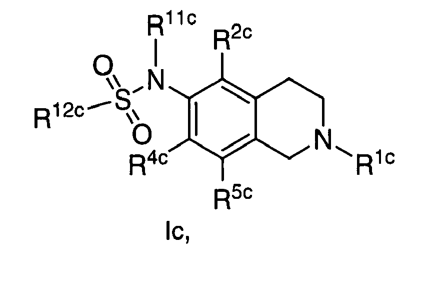 wherein
R^{1c} represents a hydrogen atom; a -C(=O)-OR^{37c} moiety; a radical selected from the group consisting of methyl, ethyl, n-propyl, isopropyl, n-butyl, sec-butyl, isobutyl, tert-butyl, n-pentyl, n-hexyl, -CH₂-NH₂, -CH₂-NH-CH₃, -CH₂-N(CH₃)₂, -CH₂-N(C₂H₅)₂, -CH₂-NH-C₂H₅, -CH₂-CH₂-NH₂, -CH₂-CH₂-NH-CH₃, -CH₂-CH₂-N(CH₃)₂, -CH₂-CH₂-N(C₂H₅)₂, -CH₂-CH₂-NH-C₂H₅, -CH₂-CH₂-CH₂-NH-CH₃, -CH₂-CH₂-CH₂-N(CH₃)₂, -CH₂-CH₂-CH₂-N(C₂H₅)₂ and - CH₂-CH₂-CH₂-NH-C₂H₅; or a (hetero)cycloaliphatic radical selected from the group consisting of imidazolidinyl, aziridinyl, azetidinyl, pyrrolidinyl, piperidinyl, morpholinyl, thiomorpholinyl, piperazinyl, pyrazolidinyl and azepanyl, which may be bonded via a -(CH₂)_{1,2 or 3}- group and which may be unsubstituted or optionally substituted with 1, 2, 3, 4 or 5 substituent(s) independently selected from the group consisting of methyl, ethyl, n-propyl, isopropyl, n-butyl, tert-butyl, sec-butyl and isobutyl;
R^{2c}, R^{4c} and R^{5c}, independently of one another, each represent a hydrogen atom; F, Cl, Br, 1, -NO₂; -O-CH₃; -O-C₂H₅; -O-CF₃; -O-CFH₂; -O-CF₂H; -O-CH₂-CF₃; -O-CF₂-CF₃; -S-CH₃; -S-C₂H₅; -S-CF₃; -S-CFH₂; -S-CF₂H; -S-CH₂-CF₃; -S-CF₂-CF₃; -N(R^{11c})-S(=O)₂-R^{12c}; or a radical selected from the group consisting of methyl, ethyl, n-propyl, isopropyl, tert-butyl, -CF₃, -CFH₂, - CF₂H, -CH₂-CF₃ and -CF₂-CF₃;
R^{11c} represents a hydrogen atom, -S(=O)₂-R^{12c} or an alkyl radical selected from the group consisting of methyl, ethyl, n-propyl, isopropyl, n-butyl, sec-butyl, isobutyl and tert-butyl;
R^{12c} represents a phenyl radical of general formula (Ac), 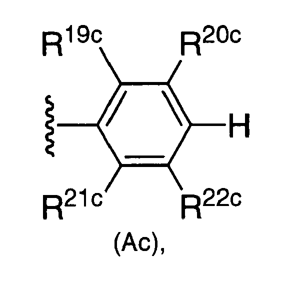 wherein
R^{19c}, R^{20c}, R^{21c} and R^{22c}, independently of one another, each represent a hydrogen atom; F, Cl, Br, I, -NO₂; -NH₂; -SH; -OH; -CN; -C(=O)-OH; -C(=O)-H; -C(=O)-CH₃; -C(=O)-C₂H₅; -O-CH₃; -O-C₂H₅; -O-CF₃; -O-CFH₂; -O-CF₂H; -O-CH₂-CF₃; -O-CF₂-CF₃; -S-CH₃; -S-C₂H₅; -S-CF₃; -S-CFH₂; -S-CF₂H; -S-CH₂-CF₃; -S-CF₂-CF₃; -C(=O)-OCH₃; -C(=O)-OC₂H₅; methyl, ethyl, n-propyl, isopropyl, n-butyl, isobutyl, sec-butyl, tert-butyl, -CF₃, -CF₂H, -CFH₂, -CH₂-CF₃ and - CF₂-CF₃;
with the proviso that at least one of the substituents R^{19c}, R^{20c}, R^{21c} and R^{22c} is unlike hydrogen;
a radical selected from the group consisting of naphthyl, [1,3]-benzodioxolyl, [1,4]-benzodioxanyl, benzo[b]furanyl, benzo[b]thiophenyl, benzo[2,1,3]thiadiazolyl, [1,2,3]-benzothiadiazolyl, [2,1,3]-benzoxadiazolyl, [1,2,3]-benzoxadiazolyl, benzoxazolyl, benzothiazolyl, benzisoxazolyl, benzisothiazolyl and imidazo[2,1-b]thiazolyl, which may be unsubstituted or optionally substituted with 1, 2, 3, 4 or 5 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, F, Cl, Br, I, - CN, -CF₃, -OCF₃, -SCF₃, -CF₂H and -CFH₂;
or a radical selected from the group consisting of pyridinyl, pyrrolyl, imidazolyl, pyrazolyl, triazolyl, pyridazinyl, pyrimidinyl and pyrazinyl, which may be unsubstituted or optionally substituted with 1, 2, 3, 4 or 5 substituent(s) independently selected from the group consisting of F, Cl, Br, 1, -NO₂; methyl, ethyl, n-propyl, isopropyl, n-butyl, isobutyl, sec-butyl, tert-butyl, -CF₃, -CF₂H, -CFH₂, -CH₂-CF₃ and -CF₂-CF₃;
and R^{37c} represents a radical selected from the group consisting of methyl, ethyl, n-propyl, isopropyl, n-butyl, sec-butyl, isobutyl, tert-butyl, n-pentyl, n-hexyl, fluorenyl, fluorenylmethyl, phenyl, benzyl and naphthyl;
optionally in form of one of its stereoisomers, preferably enantiomers or diasteromers, a racemate or in form of a mixture of at least two of its stereoisomers, preferably enantiomers and/or diastereomers, in any mixing ratio, or a salt thereof, or a corresponding solvate thereof.

25. A medicament comprising at least one compound of general formula ld according to claim 20, 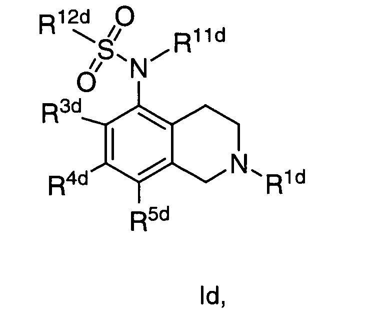 wherein
R^{1d} represents a hydrogen atom; a -C(=O)-OR^{37d} moiety; a radical selected from the group consisting of methyl, ethyl, n-propyl, isopropyl, n-butyl, sec-butyl, isobutyl, tert-butyl, n-pentyl, n-hexyl, -CH₂-NH₂, -CH₂-NH-CH₃, -CH₂-N(CH₃)₂, -CH₂-N(C₂H₅)₂, -CH₂-NH-C₂H₅, -CH₂-CH₂-NH₂, -CH₂-CH₂-NH-CH₃, -CH₂-CH₂-N(CH₃)₂, -CH₂-CH₂-N(C₂H₅)₂, -CH₂-CH₂-NH-C₂H₅, -CH₂-CH₂-CH₂-NH-CH₃, -CH₂-CH₂-CH₂-N(CH₃)₂, -CH₂-CH₂-CH₂-N(C₂H₅)₂ and - CH₂-CH₂-CH₂-NH-C₂H₅; or a (hetero)cycloaliphatic radical selected from the group consisting of imidazolidinyl, aziridinyl, azetidinyl, pyrrolidinyl, piperidinyl, morpholinyl, thiomorpholinyl, piperazinyl, pyrazolidinyl and azepanyl, which may be bonded via a -(CH₂)_{1, 2 or 3}- group and which may be unsubstituted or optionally substituted with 1, 2, 3, 4 or 5 substituent(s) independently selected from the group consisting of methyl, ethyl, n-propyl, isopropyl, n-butyl, tert-butyl, sec-butyl and isobutyl;
R^{3d}, R^{4d} and R^{5d}, independently of one another, each represent a hydrogen atom; F, Cl, Br, I, -NO₂; -O-CH₃; -O-C₂H₅; -O-CF₃; -O-CFH₂; -O-CF₂H; -O-CH₂-CF₃; -O-CF₂-CF₃; -S-CH₃; -S-C₂H₅; -S-CF₃; -S-CFH₂; -S-CF₂H; -S-CH₂-CF₃; -S-CF₂-CF₃; -N(R^{11d})-S(=O)₂-R^{12d}; or a radical selected from the group consisting of methyl, ethyl, n-propyl, isopropyl, tert-butyl, -CF₃, - CFH₂, -CF₂H, -CH₂-CF₃ and -CF₂-CF₃;
R^{11d} represents a hydrogen atom, -S(=O)₂-R^{12d} or an alkyl radical selected from the group consisting of methyl, ethyl, n-propyl, isopropyl, n-butyl, sec-butyl, isobutyl and tert-butyl;
R^{12d} represents a phenyl radical of general formula (Ad), 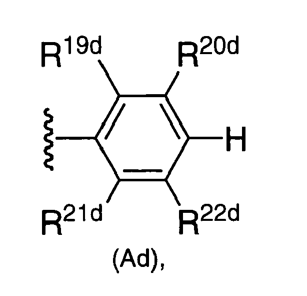 wherein
R^{19d}, R^{20d} R^{21d} and R^{22d}, independently of one another, each represent a hydrogen atom; F, Cl, Br, I, -NO₂; -NH₂; -SH; -OH; -CN; -C(=O)-OH; -C(=O)-H; -C(=O)-CH₃; -C(=O)-C₂H₅; -O-CH₃; -O-C₂H₅; -O-CF₃; -O-CFH₂; -O-CF₂H; -O-CH₂-CF₃; -O-CF₂-CF₃; -S-CH₃; -S-C₂H₅; -S-CF₃; -S-CFH₂; -S-CF₂H; -S-CH₂-CF₃; -S-CF₂-CF₃; -C(=O)-OCH₃; -C(=O)-OC₂H₅; methyl, ethyl, n-propyl, isopropyl, n-butyl, isobutyl, sec-butyl, tert-butyl, -CF₃, -CF₂H, -CFH₂, -CH₂-CF₃ and - CF₂-CF₃;
with the proviso that at least one of the substituents R^{19d}, R^{20d} , R^{21d} and R^{22d} is unlike hydrogen;
a radical selected from the group consisting of naphthyl, [1,3]-benzodioxolyl, [1,4]-benzodioxanyl, benzo[b]furanyl, benzo[b]thiophenyl, benzo[2,1,3]thiadiazolyl, [1,2,3]-benzothiadiazolyl, [2,1,3]-benzoxadiazolyl, [1,2,3]-benzoxadiazolyl, benzoxazolyl, benzothiazolyl, benzisoxazolyl, benzisothiazolyl and imidazo[2,1-b]thiazolyl, which may be unsubstituted or optionally substituted with 1, 2, 3, 4 or 5 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, F, Cl, Br, I, - CN, -CF₃, -OCF₃, -SCF₃, -CF₂H and -CFH₂;
or a radical selected from the group consisting of pyridinyl, pyrrolyl, imidazolyl, pyrazolyl, triazolyl, pyridazinyl, pyrimidinyl and pyrazinyl, which may be unsubstituted or optionally substituted with 1, 2, 3, 4 or 5 substituent(s) independently selected from the group consisting of F, Cl, Br, I, -NO₂; methyl, ethyl, n-propyl, isopropyl, n-butyl, isobutyl, sec-butyl, tert-butyl, -CF₃, -CF₂H, -CFH₂, -CH₂-CF₃ and -CF₂-CF₃;
and R^{37d} represents a radical selected from the group consisting of methyl, ethyl, n-propyl, isopropyl, n-butyl, sec-butyl, isobutyl, tert-butyl, n-pentyl, n-hexyl, fluorenyl, fluorenylmethyl, phenyl, benzyl and naphthyl;
optionally in form of one of its stereoisomers, preferably enantiomers or diasteromers, a racemate or in form of a mixture of at least two of its stereoisomers, preferably enantiomers and/or diastereomers, in any mixing ratio, or a salt thereof, or a corresponding solvate thereof.

26. A medicament according to one or more of claims 1 to 25 comprising at least one compound selected from the group consisting of
[1] N-(1,2,3,4-tetrahydroisoquinolin-6-yl)naphthalene-1-sulfonamide hydrochloride,
[2] 2,2-dimethyl-6-(N-methylnaphthalene-1-sulfonamido)-1,2,3,4-tetrahydroisoquinolinium iodide,
[3] N-(2-methyl-1,2,3,4-tetrahydroisoquinolin-6-yl)naphthalene-1-sulfonamide hydrochloride,
[4] 5-chloro-3-methyl-N-(1,2,3,4-tetrahydroisoquinolin-6-yl)benzo[b]thiophene-2-sulfonamide hydrochloride,
[5] 5-chloro-3-methyl-N-(2-methyl-1,2,3,4-tetrahydroisoquinolin-6-yl)benzo[b]thiophene-2-sulfonamide hydrochloride,
[6] 4-methyl-N-(1,2,3,4-tetrahydroisoquinolin-6-yl)naphthalene-1-sulfonamide hydrochloride,
[7] 4-methyl-N-(2-methyl-1,2,3,4-tetrahydroisoquinolin-6-yl)naphthalene-1-sulfonamide hydrochloride,
[8] N-(1,2,3,4-tetrahydroisoquinolin-6-yl)naphthalene-2-sulfonamide hydrochloride,
[9] N-(2-methyl-1,2,3,4-tetrahydroisoquinolin-6-yl)naphthalene-2-sulfonamide hydrochloride,
[10] 6-chloro-N-(1,2,3,4-tetrahydroisoquinolin-6-yl)imidazo[2,1-b]thiazole-5-sulfonamide hydrochloride,
[11] 2-methoxy-5-methyl-N-(1,2,3,4-tetrahydroisoquinolin-6-yl)benzenesulfonamide hydrochloride,
[12] N-(1,2,3,4-tetrahydroisoquinolin-6-yl)pyridine-3-sulfonamide dihydrochloride,
[13] 6-(naphthalene-1-sulfonylamino)-3,4-dihydro-1H-isoquinoline-2-carboxylic acid tert-butyl ester,
[14] 6-(5-chloro-3-methyl-benzo[b]thiophene-2- sulfonylamino)-3,4-dihydro-1 H-isoquinoline-2-carboxylic acid tert-butyl ester,
[15] 6-(4-methyl-naphthalene-1-sulfonylamino)-3,4 -dihydro-1 H-isoquinoline-2-carboxylic acid tert-butyl ester,
[16] 6-(naphthalene-2-sulfonylamino)-3,4-dihydro-1 H-isoquinoline-2-carboxylic acid tert-butyl ester,
[17] 6-(2-methoxy-5-methyl-benzenesulfonylamino) -3,4-dihydro-1H-isoquinoline-2-carboxylic acid tert-butyl ester and
[18] 6-(pyridine-3-sulfonylamino)-3,4-dihydro-1H-isoquinoline-2carboxylic acid tert-butyl ester;
or a corresponding solvate thereof.

27. A medicament according to one or more of claims 1 to 26 for the prophylaxis and/or treatment of a disorder or disease related to food intake, preferably for the regulation of appetite, for the maintenance, increase or reduction of body weight, for the prophylaxis and/or treatment of obesity, bulimia, anorexia, cachexia, type II diabetes (non insulin dependent diabetes mellitus), preferably type II diabetes that is caused by obesity; for the prophylaxis and/or treatment of stroke; migraine; head trauma; epilepsy; irritable colon syndrome; irritable bowl syndrome; emesis; vertigo; disorders of the central nervous system; anxiety; panic attacks; depression; bipolar disorders; obsessive compulsory disorder; cognitive disorders; cognitive dysfunction associated with psychiatric diseases; memory disorders; senile dementia; mood disorders; sleep disorders; psychosis; neurodegenerative disorders, preferably selected from the group consisting of Morbus Alzheimer, Morbus Parkinson, Morbus Huntington and Multiple Sclerosis; schizophrenia; amnesia; autism; sexual dysfunction; gastric motility disorders; circadian rhythm disorders; chronic intermittent hypoxia; convulsions; or hyperactivity disorder (ADHD, attention deficit/hyperactivity disorder); for improvement of cognition (cognitive enhancement) or cognitive memory (cognitive memory enhancement); for the prophylaxis and/or treatment of drug addiction and/or withdrawal; for the prophylaxis and/or treatment of alcohol addiction and/or withdrawal, for the prophylaxis and/or treatment of nicotine addiction and/or withdrawal.

28. Use of at least one substituted tetrahydroisoquinoline compound as defined in one or more of claims 1 to 26 for the manufacture of a medicament for the prophylaxis and/or treatment of a disorder or disease related to food intake, preferably for the regulation of appetite; for the maintenance, increase or reduction of body weight; or for the prophylaxis and/or treatment of obesity, bulimia, anorexia, cachexia or type II diabetes (non insulin dependent diabetes mellitus), more preferably for the prophylaxis and/or treatment of obesity.

29. Use of at least one substituted tetrahydroisoquinoline compound as defined in one or more of claims 1 to 26 for the manufacture of a medicament for the prophylaxis and/or treatment of stroke; migraine; head trauma; epilepsy; irritable colon syndrome; irritable bowl syndrome; emesis; vertigo; disorders of the central nervous system; anxiety; panic attacks; depression; bipolar disorders; obsessive compulsory disorder; cognitive disorders; cognitive dysfunction associated with psychiatric diseases; memory disorders; senile dementia; mood disorders; sleep disorders; psychosis; neurodegenerative disorders, preferably selected from the group consisting of Morbus Alzheimer, Morbus Parkinson, Morbus Huntington and Multiple Sclerosis; schizophrenia; amnesia; autism; sexual dysfunction; gastric motility disorders; circadian rhythm disorders; chronic intermittent hypoxia; convulsions; or hyperactivity disorder (ADHD, attention deficit/hyperactivity disorder).

30. Use of at least one substituted tetrahydroisoquinoline compound as defined in one or more of claims 1 to 26 for the manufacture of a medicament for the improvement of cognition (cognitive enhancement) and/or for the improvement of cognitive memory (cognitive memory enhancement).

31. Use of at least one substituted tetrahydroisoquinoline compound as defined in one or more of claims 1 to 26 for the manufacture of a medicament for the prophylaxis and/or treatment of drug addiction and/or withdrawal, preferably for the prophylaxis and/or treatment of addiction and/or withdrawal related to one or more of drugs selected from the group consisting of benzodiazepines, natural, semisynthetic or synthetic opioids like cocaine, ethanol and/or nicotine.

32. A substituted tetrahydroisoquinoline compound of general formula le, 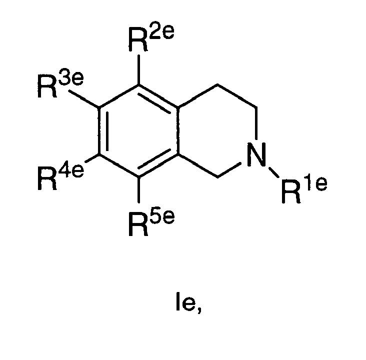 wherein
R^{1e} represents a hydrogen atom; a -C(=O)-OR^{37e} moiety;
a linear or branched, saturated or unsaturated C₁₋₁₀ aliphatic radical which may be unsubstituted or substituted with 1, 2 or 3 substituent(s) independently selected from the group consisting of F, Cl, Br, I, -CN, -CF₃, -OCF₃, -SCF₃, -OH, -SH, -NH₂, -O-C₁₋₅-alkyl, -S-C₁₋₅-alkyl, -NH(C₁₋₅-alkyl) and -N(C₁₋₅-alkyl)₂;
or a saturated or unsaturated 3- to 9-membered cycloaliphatic radical, which may contain 1, 2 or 3 heteroatom(s) independently selected from the group consisting of nitrogen, oxygen and sulfur as ring member(s) and which may be unsubstituted or optionally substituted with 1, 2, 3, 4 or 5 substituent(s) independently selected from the group consisting of oxo (=O), thioxo (=S), C₁₋₅-alkyl, -O-C₁₋₅-alkyl, -S-C₁₋₅-alkyl, -C(=O)-C₁₋₅-alkyl, - O-C(=O)-C₁₋₅-alkyl, F, Cl, Br, l, -CN, -CF₃, -OCF₃, -SCF₃, -OH, -SH, -NH₂, - NH(C₁₋₅-alkyl), -N(C₁₋₅-alkyl)₂, -NO₂, -CHO, -CF₂H, -CFH₂, -C(=O)-NH₂, - C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl and which may be condensed with an unsubstituted or at least mono-substituted saturated, unsaturated or aromatic mono- or bicyclic ring system and which may be bonded via a linear or branched C₁₋₆alkylene, C₂₋₆alkenylene or C₂₋₆alkinylene group which may be unsubstituted or substituted with 1, 2 or 3 substituent(s) independently selected from the group consisting of F, Cl, Br, I, -CN, -CF₃, -OCF₃, -SCF₃, -OH, -SH, -NH₂, -O-C₁₋₅-alkyl, -S-C₁₋₅-alkyl, -NH(C₁₋₅-alkyl) and -N(C₁₋₅-alkyl)₂;
R^{2e}, R^{3e}, R^{4e} and R^{5e}, independently of one another, each represent a hydrogen atom; F, Cl, Br, I, -NO₂; -NH₂; -SH; -OH; -CN; -C(=O)-OH; -C(=O)-H; - S(=O)₂-OH; -C(=O)-NH₂; -S(=O)₂-NH₂; -C(=O)-R^{6e}; -S(=O)-R^{7e}; -S(=O)₂-R^{7e}; -OR^{8e}; -SR^{9e}; -C(=O)-OR^{10e}; -N(R^{11e})-S(=O)₂-R^{12e}; -NR^{13e}R^{14e}; -NH-R^{15e}; -C(=O)-NR^{16e}R^{17e}; C(=O)-NHR^{18e};
a linear or branched, saturated or unsaturated, unsubstituted or at least mono-substituted aliphatic radical;
a saturated or unsaturated, unsubstituted or at least mono-substituted, optionally at least one heteroatom as a ring member containing cycloaliphatic radical, which may be bonded via a linear or branched alkylene, alkenylene or alkinylene group and which may be condensed with an unsubstituted or at least mono-substituted saturated, unsaturated or aromatic mono- or bicyclic ring system;
or an unsubstituted or at least mono-substituted aryl or heteroaryl radical, which may be bonded via a linear or branched alkylene, alkenylene or alkinylene group and which may be condensed with an unsubstituted or at least mono-substituted saturated or unsaturated, but not aromatic, mono-or bicyclic ring system;
with the proviso that at least one of the substituents R^{2e}, R^{3e}, R^{4e} and R^{5e} represents a -N(R^{11e})-S(=O)₂-R^{12e} moiety;
R^{6e}, R^{7e}, R^{8e}, R^{9e}, R^{10e}, R^{13e}, R^{14e}, R^{15e}, R^{16e}, R^{17e} and R^{18e}, independently of one another, each represent a linear or branched, saturated or unsaturated, unsubstituted or at least mono-substituted aliphatic radical;
a saturated or unsaturated, unsubstituted or at least mono-substituted, optionally at least one heteroatom as a ring member containing cycloaliphatic radical, which may be bonded via a linear or branched alkylene, alkenylene or alkinylene group and which may be condensed with an unsubstituted or at least mono-substituted saturated, unsaturated or aromatic mono- or bicyclic ring system;
or an unsubstituted or at least mono-substituted aryl or heteroaryl radical, which may be bonded via a linear or branched alkylene, alkenylene or alkinylene group and which may be condensed with an unsubstituted or at least mono-substituted saturated or unsaturated, but not aromatic, mono-or bicyclic ring system;
R^{11e} represents a hydrogen atom, -S(=O)₂-R^{12e} or a linear or branched, saturated or unsaturated, unsubstituted or at least mono-substituted aliphatic radical;
R^{12e} represents a monocyclic heteroaryl radical, which may be unsubstituted or optionally substituted with 1, 2, 3, 4 or 5 substituent(s) independently selected from the group consisting of F, Cl, -NO₂; -NH₂; -SH; -OH; -CN; - C(=O)-OH; -C(=O)-H; -S(=O)₂-OH; -C(=O)-NH₂; -S(=O)₂-NH₂; -C(=O)-R^{23e}; -OR^{25e}; -SR^{26e}; -C(=O)-OR^{27e}; -N(R^{28e}) -S(=O)₂-R^{29e}; -NH-S(=O)₂-R^{30e};-NR^{31e}R^{32e}; -NH-R^{33e}; -C(=O)-NHR^{34e}; -C(=O)-NR^{35e}R^{36e}; a linear or branched, saturated or unsaturated C₁₋₁₀ aliphatic radical which may be unsubstituted or substituted with 1, 2 or 3 substituent(s) independently selected from the group consisting of F, Cl, Br, I, -CN, -CF₃, -OCF₃, -SCF₃, -OH, -SH, -NH₂, -O-C₁₋₅-alkyl, -S-C₁₋₅-alkyl, -NH(C₁₋₅-alkyl) and -N(C₁₋₅-alkyl)₂; and a saturated or unsaturated, unsubstituted or at least mono-substituted, optionally at least one heteroatom as a ring member containing cycloaliphatic radical;
an unsubstituted or at least mono-substituted monocyclic heteroaryl radical, which is condensed with an unsubstituted or at least mono-substituted saturated or unsaturated, but not aromatic, mono- or bicyclic ring system;
an unsubstituted or at least mono-substituted bi- or tricyclic heteroaryl radical, which may be condensed with an unsubstituted or at least mono-substituted saturated or unsaturated, but not aromatic, mono- or bicyclic ring system;
or a saturated or unsaturated, unsubstituted or at least mono-substituted, optionally at least one heteroatom as a ring member containing cycloaliphatic radical, which may be condensed with an unsubstituted or at least mono-substituted saturated, unsaturated or aromatic mono- or bicyclic ring system;
R^{23e}, R^{27e}, R^{28e}, R^{29e} and R^{30e}, independently of one another, each represent a linear or branched, saturated or unsaturated C₁₋₁₀ aliphatic radical which may be unsubstituted or substituted with 1, 2 or 3 substituent(s) independently selected from the group consisting of F, Cl, Br, I, -CN, -CF₃, -OCF₃, -SCF₃, -OH, -SH, -NH₂, -O-C₁₋₅-alkyl, -S-C₁₋₅-alkyl, -NH(C₁₋₅-alkyl) and -N(C₁₋₅-alkyl)₂;
a saturated or unsaturated, unsubstituted or at least mono-substituted, optionally at least one heteroatom as a ring member containing cycloaliphatic radical, which may be bonded via a linear or branched alkylene, alkenylene or alkinylene group and which may be condensed with an unsubstituted or at least mono-substituted saturated, unsaturated or aromatic mono- or bicyclic ring system;
or an unsubstituted or at least mono-substituted aryl or heteroaryl radical, which may be bonded via a linear or branched alkylene, alkenylene or alkinylene group and which may be condensed with an unsubstituted or at least mono-substituted saturated or unsaturated, but not aromatic, mono-or bicyclic ring system;
R^{25e}, R^{34e}, R^{35e} and R^{36e}, represents a linear or branched, saturated or unsaturated C₁₋₁₀ aliphatic radical which may be unsubstituted or substituted with 1, 2 or 3 substituent(s) independently selected from the group consisting of F, Cl, Br, I, -CN, -CF₃, -OCF₃, -SCF₃, -OH, -SH, -NH₂, - O-C₁₋₅-alkyl, -S-C₁₋₅-alkyl, -NH(C₁₋₅-alkyl) and -N(C₁₋₅-alkyl)₂;
R^{26e}, R^{31e}, R^{32e} and R^{33e}, each represent a linear or branched, saturated or unsaturated C₁₋₁₀ aliphatic radical which may be unsubstituted or substituted with 1, 2 or 3 substituent(s) independently selected from the group consisting of F, Cl, Br, I, -CN, -CF₃, -OCF₃, -SCF₃, -OH, -SH, -NH₂, - O-C₁₋₅-alkyl, -S-C₁₋₅-alkyl, -NH(C₁₋₅-alkyl) and -N(C₁₋₅-alkyl)₂;
a saturated or unsaturated, unsubstituted or at least mono-substituted, optionally at least one heteroatom as a ring member containing cycloaliphatic radical, which may be bonded via a linear or branched alkylene, alkenylene or alkinylene group and which may be condensed with an unsubstituted or at least mono-substituted saturated, unsaturated or aromatic mono- or bicyclic ring system;
or an unsubstituted or at least mono-substituted aryl or heteroaryl radical, which may be condensed with an unsubstituted or at least mono-substituted saturated or unsaturated, but not aromatic, mono- or bicyclic ring system;
and R^{37e} represents a linear or branched, saturated or unsaturated C₁₋₁₀ aliphatic radical
a saturated or unsaturated, unsubstituted or at least mono-substituted, optionally at least one heteroatom as a ring member containing cycloaliphatic radical, which may be bonded via a linear or branched alkylene, alkenylene or alkinylene group and which may be condensed with an unsubstituted or at least mono-substituted saturated, unsaturated or aromatic mono- or bicyclic ring system;
or an unsubstituted or at least mono-substituted aryl or heteroaryl radical, which may be bonded via a linear or branched alkylene, alkenylene or alkinylene group and which may be condensed with an unsubstituted or at least mono-substituted saturated or unsaturated, but not aromatic, mono-or bicyclic ring system;
optionally in form of one of its stereoisomers, preferably enantiomers or diasteromers, a racemate or in form of a mixture of at least two of its stereoisomers, preferably enantiomers and/or diastereomers, in any mixing ratio, or a salt thereof, or a corresponding solvate thereof.

33. A compound according to claim 32, **characterized in that** the salt has the general formula III, 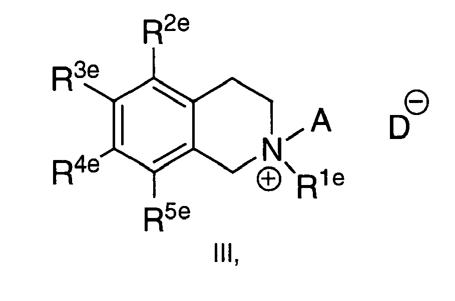 wherein
R^{1e}, R^{2e}, R^{3e} , R^{4e} and R^{5e} are defined as in claim 31 except for a -C(=O)-OR^{37e} moiety;
A represents a hydrogen atom or a radical selected from the group consisting of methyl, ethyl, n-propyl, isopropyl, n-butyl, isobutyl, 2-butyl, tert-butyl and n-pentyl;
D represents an anion selected from the group consisting of chloride, bromide, iodide, fluoride, hydrogensulfate, nitrate, dihydrogenphosphate, thiocyanate, cyanate, acrylate, fumarate, citrate, glutarate, succinate, maleate, tartrate, phosphate, 2-oxo-glutarate, formate, acetate, propionate, lactate, gluconate, benzoate or naphthoate whereby said naphthoate or benzoate may be substituted with 1, 2, 3, 4 or 5 substituents independently selected from the group consisting of F, Cl, Br, -OH, -O-CH₃ and -O-C₂H₅ , pyruvate, ascorbate, glycolate, nicotinate, phenylacetate, 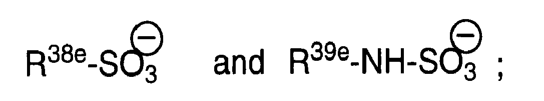 and R^{38e} and R^{39e}, independently of one another, in each case represent a radical selected from the group consisting of -CF₃, -C₂F₅, methyl, ethyl, n-propyl, isopropyl, n-butyl, isobutyl, sec-butyl, tert-butyl, n-pentyl, 2-pentyl, 3-pentyl, neo-pentyl, n-hexyl, 2-hexyl, 3-hexyl, n-heptyl, 2-heptyl, 3-heptyl, 4-heptyl, n-octyl, 2-octyl, 3-octyl, 4-octyl, 2-(6-methyl)-heptyl, 2-(5-methyl)-heptyl, 2-(5-methyl)-hexyl, 2-(4-methyl)-hexyl, 2-(7-methyl)-octyl; 2-(6-methyl)-octyl, n-nonyl, n-decyl, n-undecyl, n-dodecyl, n-tridecyl and n-tetradecyl;
a radical selected from the group consisting of phenyl, pyridinyl, pyrazolyl, benzimidazolyl, isoquinolinyl and naphthyl, which may be substituted with 1, 2, 3, 4 or 5 substituent(s) independently selected from the group consisting of F, Cl, Br, -CF₃, methyl, ethyl, n-propyl, isopropyl, n-butyl, isobutyl, 2-butyl, tert-butyl, n-pentyl, 2-pentyl, n-hexyl, -O-CH₃, -O-C₂H₅, - O-CH₂-CH₂-CH₃, -O-CH(CH₃)₂, -O-CH₂-CH₂-CH₂-CH₃, -O-C(CH₃)₃, -OH, - NO₂, -NH₂, phenyl and -SO₃H;
or a radical selected from the group consisting of cyclopropyl, cyclobutyl, cyclopentyl, cyclohexyl, cycloheptyl, cyclooctyl, cyclononyl, cyclodecyl, cycloundecyl, cyclododecyl, cyclotridecyl, cyclotetradecyl, cyclopentenyl, cyclohexenyl, cycloheptenyl, cyclooctenyl and bicyclo[2.2.1]heptyl, which may be bonded via a -(CH₂)-, -(CH₂)-(CH₂)- or -(CH₂)-(CH₂)-(CH₂)-group.

34. A compound according to claim 33, **characterized in that**
A represents a hydrogen atom or a radical selected from the group consisting of methyl, ethyl, n-propyl, isopropyl, n-butyl, isobutyl, 2-butyl, tert-butyl and n-pentyl;
and D represents an anion selected from the group consisting of chloride, bromide, iodide, fluoride, hydrogensulfate, nitrate, dihydrogenphosphate, thiocyanate, cyanate, acrylate, methanesulfonate, ethanesulfonate, toluenesulfonate, benzenesulfonate, (2,5)-dihydroxy-benzenesulfonate, naphthalene-2-sulfonate, 5-sulfo-napthalene-1-sulfonate, cyclamate, dodecane-1-sulfonate and (7,7)-dimethyl-2-oxo-bicyclo[2.2.1]-hept-1-yl-methanesulfonate.

35. A compound according to one or more of claims 32 to 34, **characterized in that**
R^{1e} represents a hydrogen atom;
a linear or branched C₁₋₁₀ alkyl radical, C₂₋₁₀ alkenyl radical or C₂₋₁₀ alkinyl radical;
a C₃₋₉ cycloalkyl radical or C₄₋₉ cycloalkenyl radical, which may be unsubstituted or optionally substituted with 1, 2, 3, 4 or 5 substituent(s) independently selected from the group consisting of oxo (=O), thioxo (=S), C₁₋₅-alkyl, -O-C₁₋₅-alkyl, -S-C₁₋₅-alkyl, -C(=O)-C₁₋₅-alkyl, -O-C(=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl and which may be condensed with an unsubstituted or at least mono-substituted saturated, unsaturated or aromatic mono- or bicyclic ring system and which may be bonded via a linear or branched C₁₋₆ alkylene, C₂₋₆ alkenylene or C₂₋₆ alkinylene group or in general formula le additionally a -C(=O)-OR^{37e} moiety;
R^{2e}, R^{3e}, R^{4e} and R^{5e}, independently of one another, each represent a hydrogen atom; F, Cl, Br, I, -NO₂; -NH₂; -SH; -OH; -CN; -C(=O)-OH; -C(=O)-H; - S(=O)₂-OH; -C(=O)-NH₂; -S(=O)₂-NH₂; -C(=O)-R^{6e}; -S(=O)-R^{7e}; -S(=O)₂-R^{7e}; -OR^{8e}; -SR^{9e}; -C(=O)-OR^{10e}; -N(R^{11e})-S(=O)₂-R^{12e}; -NR^{13e}R^{14e}; -NH-R^{15e}; -C(=O)-NR^{16e}R^{17e}; C(=O)-NHR^{18e};
a linear or branched C₁₋₁₀alkyl radical, C₂₋₁₀ alkenyl radical or C₂₋₁₀ alkinyl radical;
a C₃₋₉ cycloalkyl radical or C₄₋₉ cycloalkenyl radical, which may be bonded via a linear or branched C₁₋₆ alkylene, C₂₋₆ alkenylene or C₂₋₆ alkinylene group and which may be condensed with an unsubstituted or at least mono-substituted saturated, unsaturated or aromatic mono- or bicyclic ring system;
or an unsubstituted or at least mono-substituted 6-, 10- or 14-membered aryl or 5-, 6-, 8-, 9-, 10-, 11-, 12-, 13- or 14-membered heteroaryl radical, which may be bonded via a linear or branched C₁₋₆ alkylene, C₂₋₆ alkenylene or C₂₋₆ alkinylene group and which may be condensed with an unsubstituted or at least mono-substituted saturated or unsaturated, but not aromatic, mono- or bicyclic ring system;
with the proviso that at least one of the substituents R^{2e}, R^{3e}, R^{4e} and R^{5e} represents a -N(R^{11e})-S(=O)₂-R^{12e} moiety;
R^{6e} R^{7e}, R^{8e}, R^{9e}, R^{10e}, R^{13e}, R^{14e}, R^{15e}, R^{16e}, R^{17e} and R^{18e}, independently of one another, each represent a linear or branched C₁₋₁₀ alkyl radical, C₂₋₁₀ alkenyl radical or C₂₋₁₀ alkinyl radical;
a C₃₋₉ cycloalkyl radical or C₄₋₉ cycloalkenyl radical, which may be bonded via a linear or branched C₁₋₆ alkylene, C₂₋₆ alkenylene or C₂₋₆ alkinylene group and which may be condensed with an unsubstituted or at least mono-substituted saturated, unsaturated or aromatic mono- or bicyclic ring system;
or an unsubstituted or at least mono-substituted 6-, 10- or 14-membered aryl or 5-, 6-, 8-, 9-, 10-, 11-, 12-, 13- or 14-membered heteroaryl radical, which may be bonded via a linear or branched C₁₋₆ alkylene, C₂₋₆ alkenylene or C₂₋₆ alkinylene group and which may be condensed with an unsubstituted or at least mono-substituted saturated or unsaturated, but not aromatic, mono- or bicyclic ring system;
R^{11e} represents a hydrogen atom, -S(=O)₂-R^{12e} or a linear or branched, saturated or unsaturated, unsubstituted or at least mono-substituted C₁₋₆ alkylene, C₂₋₆ alkenylene or C₂₋₆ alkinylene group;
R^{12e} represents a monocyclic 5- or 6-membered heteroaryl radical, which may be unsubstituted or optionally substituted with 1, 2, 3, 4 or 5 substituent(s) independently selected from the group consisting of F, Cl, -NO₂; -NH₂; -SH; -OH; -CN; -C(=O)-OH; -C(=O)-H; -S(=O)₂-OH; -C(=O)-NH₂; -S(=O)₂-NH₂; - C(=O)-R^{23e}; -OR^{25e}; -SR^{26e}; -C(=O)-OR^{27e}; -N(R^{28e})-S(=O)₂-R^{29e}; -NH-S(=O)₂-R^{30e}; -NR^{31e}R^{32e}; -NH-R^{33e}; -C(=O)-NHR^{34e}; -C(=O)-NR^{35e}R^{36e}; a linear or branched C₁₋₁₀ alkyl radical, C₂₋₁₀ alkenyl radical or C₂₋₁₀ alkinyl radical; and a C₃₋₉ cycloalkyl radical;
an unsubstituted or at least mono-substituted monocyclic 5- or 6-membered heteroaryl radical, which is condensed with an unsubstituted or at least mono-substituted saturated or unsaturated, but not aromatic, mono- or bicyclic ring system;
an unsubstituted or at least mono-substituted bi- or tricyclic 8-, 9-, 10-, 11-, 12-, 13- or 14-membered heteroaryl radical, which may be condensed with an unsubstituted or at least mono-substituted saturated or unsaturated, but not aromatic, mono- or bicyclic ring system;
or a C₃₋₉ cycloalkyl radical or C₄₋₉ cycloalkenyl radical, which may be condensed with an unsubstituted or at least mono-substituted saturated, unsaturated or aromatic mono- or bicyclic ring system;
R^{23e}, R^{27e} R^{28e}, R^{29e} and R^{30e}, independently of one another, each represent a linear or branched C₁₋₁₀ alkyl radical, C₂₋₁₀ alkenyl radical or C₂₋₁₀ alkinyl radical;
a C₃₋₉ cycloalkyl radical or C₄₋₉ cycloalkenyl radical, which may be bonded via a linear or branched C₁₋₆ alkylene, C₂₋₆ alkenylene or C₂₋₆ alkinylene group and which may be condensed with an unsubstituted or at least mono-substituted saturated, unsaturated or aromatic mono- or bicyclic ring system;
or an unsubstituted or at least mono-substituted 6-, 10- or 14-membered aryl or 5-, 6-, 8-, 9-, 10-, 11-, 12-, 13- or 14-membered heteroaryl radical, which may be bonded via a linear or branched C₁₋₆ alkylene, C₂₋₆ alkenylene or C₂₋₆ alkinylene group and which may be condensed with an unsubstituted or at least mono-substituted saturated or unsaturated, but not aromatic, mono- or bicyclic ring system;
R^{25e}, R^{34e}, R^{35e} and R^{36e}, represents a linear or branched C₁₋₁₀alkyl radical, C₂₋₁₀ alkenyl radical or C₂₋₁₀ alkinyl radical;
R^{26e}, R^{31e}, R^{32e} and R^{33e}, each represent a linear or branched C₁₋₁₀alkyl radical, C₂₋₁₀ alkenyl radical or C₂₋₁₀ alkinyl radical;
a C₃₋₉cycloalkyl radical or C₄₋₉ cycloalkenyl radical, which may be bonded via a linear or branched C₁₋₆ alkylene, C₂₋₆ alkenylene or C₂₋₆ alkinylene group and which may be condensed with an unsubstituted or at least mono-substituted saturated, unsaturated or aromatic mono- or bicyclic ring system;
or an unsubstituted or at least mono-substituted 6-, 10- or 14-membered aryl or 5-, 6-, 8-, 9-, 10-, 11-, 12-, 13- or 14-membered heteroaryl radical, which may be condensed with an unsubstituted or at least mono-substituted saturated or unsaturated, but not aromatic, mono- or bicyclic ring system;
and R^{37e} represents a linear or branched C₁₋₁₀ alkyl radical, C₂₋₁₀ alkenyl radical or C₂₋₁₀ alkinyl radical;
a C₃₋₉cycloalkyl radical or C₄₋₉cycloalkenyl radical, which may be bonded via a linear or branched C₁₋₆alkylene, C₂₋₆alkenylene or C₂₋₆alkinylene group and which may be condensed with an unsubstituted or at least mono-substituted saturated, unsaturated or aromatic mono- or bicyclic ring system;
or an unsubstituted or at least mono-substituted 6-, 10- or 14-membered aryl or 5-, 6-, 8-, 9-, 10-, 11-, 12-, 13- or 14-membered heteroaryl radical, which may be bonded via a linear or branched C₁₋₆ alkylene, C₂₋₆ alkenylene or C₂₋₆ alkinylene group and which may be condensed with an unsubstituted or at least mono-substituted saturated or unsaturated, but not aromatic, mono- or bicyclic ring system;
whereby
the aforementioned C₁₋₁₀ alkyl radical, C₂₋₁₀ alkenyl radical or C₂₋₁₀ alkinyl radicals may in each case be unsubstituted or optionally substituted with 1, 2, 3, 4 or 5 substituent(s) independently selected from the group consisting of F, Cl, Br, I, - CN, -CF₃, -OCF₃, -SCF₃, -OH, -SH, -NH₂, -O-C₁₋₅-alkyl, -S-C₁₋₅-alkyl, -NH(C₁₋₅-alkyl) and -N(C₁₋₅-alkyl)₂;
if not defined otherwise, the aforementioned C₃₋₉cycloalkyl radicals and C₄₋₉ cycloalkenyl radicals may in each case be unsubstituted or optionally substituted with 1, 2, 3, 4 or 5 substituent(s) independently selected from the group consisting of oxo (=O), thioxo (=S), C₁₋₅-alkyl, -O-C₁₋₅-alkyl, -S-C₁₋₅-alkyl, -C(=O)-OH, - C(=O)-C₁₋₅-alkyl, -C(=O)-O-C₁₋₅-alkyl, -O-C(=O)-C₁₋₅-alkyl, F, Cl, Br, I, -CN, -CF₃,-OCF₃, -SCF₃, -OH, -SH, -NH₂, -NH(C₁₋₅-alkyl), -N(C₁₋₅-alkyl)₂, -NO₂, -CHO, - CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl;
the aforementioned C₃₋₉ cycloalkyl radicals and C₄₋₉ cycloalkenyl radicals in each case may optionally contain 1, 2 or 3 heteroatom(s) independently selected from the group consisting of nitrogen, oxygen and sulfur as ring member(s);
the aforementioned C₁₋₆ alkylene, C₂₋₆ alkenylene or C₂-₆ alkinylene groups may in each case be unsubstituted or substituted with 1, 2 or 3 substituent(s) independently selected from the group consisting of F, Cl, Br, I, -CN, -CF₃, -OCF₃, -SCF₃, -OH, -SH, -NH₂, -O-C₁₋₅-alkyl, -S-C₁₋₅-alkyl, -NH(C₁₋₅-alkyl) and -N(C₁₋₅-alkyl)₂;
the rings of the aforementioned ring system are in each case independently of one another 5-, 6- or 7-membered and may in each case independently of one another optionally contain 1, 2 or 3 heteroatom(s) independently selected from the group consisting of nitrogen, oxygen and sulfur;
and the rings of the ring system may in each case be unsubstituted or substituted with 1, 2 or 3 substituent(s) independently selected from the group consisting of oxo (=O), thioxo (=S), C₁₋₅-alkyl, -O-C₁₋₅-alkyl, -S-C₁₋₅-alkyl, -C(=O)-OH, -C(=O)-C₁₋₅-alkyl, -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H,-CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl;
if not defined otherwise, the 6-, 10- or 14-membered aryl or 5-, 6-, 8-, 9-, 10-, 11-, 12-, 13- or 14-membered heteroaryl radicals may be unsubstituted or optionally substituted with 1, 2, 3, 4 or 5 substituent(s) independently selected from the group consisting of C₁₋₅-alkyl, -O-C₁₋₅-alkyl, -S-C₁₋₅-alkyl, -C(=O)-OH, -C(=O)-C₁₋₅-alkyl, -C(=O)-O-C₁₋₅-alkyl, -O-C(=O)-C₁₋₅-alkyl, F, Cl, Br, I, -CN, -CF₃, -OCF₃, - SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, - S(=O)₂-phenyl, phenyl, phenoxy and benzyl;
and the aforementioned 5-, 6-, 8-, 9-, 10-, 11-, 12-, 13- or 14-membered heteroaryl radicals in each case optionally contain 1, 2, 3 or 4 heteroatom(s) independently selected from the group consisting of nitrogen, oxygen and sulfur as ring member(s);
optionally in form of one of its stereoisomers, preferably enantiomers or diasteromers, a racemate or in form of a mixture of at least two of its stereoisomers, preferably enantiomers and/or diastereomers, in any mixing ratio, or a salt thereof, or a corresponding solvate thereof.

36. A compound according to one or more of claims 32 to 35, **characterized in that**
R^{1e} represents a hydrogen atom; a radical selected from the group consisting of methyl, ethyl, n-propyl, isopropyl, n-butyl, sec-butyl, isobutyl, tert-butyl, n-pentyl, n-hexyl, -CH₂-NH₂, -CH₂-NH-CH₃, -CH₂-N(CH₃)₂, -CH₂-N(C₂H₅)₂, -CH₂-NH-C₂H₅, -CH₂-CH₂-NH₂, -CH₂-CH₂-NH-CH₃, -CH₂-CH₂-N(CH₃)₂, -CH₂-CH₂-N(C₂H₅)₂, -CH₂-CH₂-NH-C₂H₅, -CH₂-CH₂-CH₂-NH-CH₃, -CH₂-CH₂-CH₂-N(CH₃)₂, -CH₂-CH₂-CH₂-N(C₂H₅)₂ and - CH₂-CH₂-CH₂-NH-C₂H₅; a (hetero)cycloaliphatic radical selected from the group consisting of imidazolidinyl, aziridinyl, azetidinyl, pyrrolidinyl, piperidinyl, morpholinyl, thiomorpholinyl, piperazinyl, pyrazolidinyl and azepanyl, which may be bonded via a -(CH₂)_{1, 2 or 3}- group and which may be unsubstituted or optionally substituted with 1, 2, 3, 4 or 5 substituent(s) independently selected from the group consisting of oxo (=O), thioxo (=S), methyl, ethyl, n-propyl, isopropyl, n-butyl, tert-butyl, sec-butyl, isobutyl, - C(=O)-CH₃, -C(=O)-C₂H₅, -C(=O)-CH₂-CH₂-CH₃, -C(=O)-CH(CH₃)₂, - C(=O)-C(CH₃)₃, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃ or in general formula le R^{1e} additionally represents a -C(=O)-OR^{37e} moiety;
R^{2e}, R^{3e}, R^{4e} and R^{5e}, independently of one another, each represent a hydrogen atom; F, Cl, Br, I, -NO₂; -NH₂; -SH; -OH; -CN; -C(=O)-OH; -C(=O)-H; - S(=O)₂-OH; -C(=O)-NH₂; -S(=O)₂-NH₂; -OR^{8e}; -SR^{9e}; -N(R^{11e})-S(=O)₂-R^{12e};
a radical selected from the group consisting of methyl, ethyl, n-propyl, isopropyl, n-butyl, sec-butyl, isobutyl, tert-butyl, vinyl, allyl, ethinyl, -CF₃,-CFH₂, -CF₂H, -CH₂-CF₃ and -CF₂-CF₃; or a radical selected from the group consisting of cyclopropyl, cyclobutyl, cyclopentyl and cyclohexyl;
with the proviso that at least one of the substituents R^{2e}, R^{3e}, R^{4e} and R^{5e} represents a -N(R^{11e})-S(=O)₂-R^{12e} moiety;
R^{8e} and R^{9e}, independently of one another, each represent a radical selected from the group consisting of methyl, ethyl, n-propyl, isopropyl, n-butyl, sec-butyl, isobutyl, tert-butyl, -CF₃, -CFH₂, -CF₂H, -CH₂-CF₃ and -CF₂-CF₃; a (hetero)cycloaliphatic radical selected from the group consisting of cyclopropyl, cyclobutyl, cyclopentyl and cyclohexyl; or an aryl or heteroaryl radical selected from the group consisting of phenyl, naphthyl, furyl (furanyl), thienyl (thiophenyl), pyrrolyl, and pyridinyl, which may be bonded via a -(CH₂)₁, _{2 or 3}-group and which may be unsubstituted or optionally substituted with 1, 2 or 3 substituent(s) independently selected from the group consisting of methyl, ethyl, n-propyl, isopropyl, n-butyl, tert-butyl, sec-butyl, isobutyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, F, Cl, Br, I, -CN, -CF₃, -OCF₃, -SCF₃, -OH, -SH, -NH₂, -NO₂, -CHO, -CF₂H and -CFH₂;
R^{11e} represents a hydrogen atom, -S(=O)₂-R^{12e} or an alkyl radical selected from the group consisting of methyl, ethyl, n-propyl, isopropyl, n-butyl, sec-butyl, isobutyl and tert-butyl;
R^{12e} represents a radical selected from the group consisting of [1,3]-benzodioxolyl, [1,4]-benzodioxanyl, [1,2,3,4]-tetrahydronaphthyl, (2,3)-dihydro-1 H-cyclopenta[b]indolyl, [1,2,3,4]-tetrahydroquinolinyl, [1,2,3,4]-tetrahydroisoquinolinyl, [1,2,3,4]-tetrahydroquinazolinyl, [3,4]-dihydro-2H-benzo[1,4]oxazinyl, indolyl, isoindolyl, quinolinyl, isoquinolinyl, benzo[b]furanyl, benzo[b]thiophenyl, benzo[2,1,3]thiadiazolyl, [1,2,3]-benzothiadiazolyl, [2,1,3]-benzoxadiazolyl, [1,2,3]-benzoxadiazolyl, benzoxazolyl, benzothiazolyl, benzisoxazolyl, benzisothiazolyl, imidazo[2,1-b]thiazolyl, 2H-chromenyl, indazolyl and quinazolinyl, which may be unsubstituted or optionally substituted with 1, 2, 3, 4 or 5 substituent(s) independently selected from the group consisting of methyl, ethyl, n-propyl, isopropyl, n-butyl, tert-butyl, sec-butyl, isobutyl, n-pentyl, - O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, F, Cl, Br, I, -CN, -CF₃,-OCF₃, -SCF₃, -OH, -SH, -NH₂, -NO₂, -CHO, -CF₂H and -CFH₂;
or a radical selected from the group consisting of pyridinyl, furyl (furanyl), thienyl (thiophenyl), pyrrolyl, imidazolyl, pyrazolyl, triazolyl, pyridazinyl, pyrimidinyl and pyrazinyl, which may be unsubstituted or optionally substituted with 1, 2, 3, 4 or 5 substituent(s) independently selected from the group consisting of F, Cl, -NO₂; -NH₂; -SH; -OH; -CN; -C(=O)-OH; - C(=O)-H; -S(=O)₂-OH; -C(=O)-NH₂; -S(=O)₂-NH₂; -C(=O)-CH₃; -C(=O)-C₂H₅; -C(=O)-CH(CH₃)₂; -C(=O)-C(CH₃)₃; -O-CH₃; -O-C₂H₅; -O-C(CH₃)₃; - S-CH₃; -S-C₂H₅; -S-C(CH₃)₃; -C(=O)-O-CH₃; -C(=O)-O-C₂H₅; -C(=O)-O-CH(CH₃)₂; -C(=O)-O-C(CH₃)₃;methyl, ethyl, n-propyl, isopropyl, n-butyl, isobutyl, sec-butyl, tert-butyl, -CF₃, -CF₂H, -CFH₂, -CH₂-CF₃, -CF₂-CF₃, cyclopropyl, cyclobutyl, cyclopentyl and cyclohexyl;
and R^{37e} represents a radical selected from the group consisting of methyl, ethyl, n-propyl, isopropyl, n-butyl, sec-butyl, isobutyl, tert-butyl, n-pentyl, n-hexyl, fluorenyl, fluorenylmethyl, phenyl, benzyl and naphthyl;
optionally in form of one of its stereoisomers, preferably enantiomers or diasteromers, a racemate or in form of a mixture of at least two of its stereoisomers, preferably enantiomers and/or diastereomers, in any mixing ratio, or a salt thereof, or a corresponding solvate thereof.

37. A compound according to one or more of claims 32 to 36, **characterized in that**
R^{1e} represents a hydrogen atom; a radical selected from the group consisting of methyl, ethyl, n-propyl, isopropyl, n-butyl, sec-butyl, isobutyl, tert-butyl, n-pentyl, n-hexyl, -CH₂-NH₂, -CH₂-NH-CH₃, -CH₂-N(CH₃)₂, -CH₂-N(C₂H₅)₂, -CH₂-NH-C₂H₅, -CH₂-CH₂-NH₂, -CH₂-CH₂-NH-CH₃, -CH₂-CH₂-N(CH₃)₂, -CH₂-CH₂-N(C₂H₅)₂, -CH₂-CH₂-NH-C₂H₅, -CH₂-CH₂-CH₂-NH-CH₃, -CH₂-CH₂-CH₂-N(CH₃)₂, -CH₂-CH₂-CH₂-N(C₂H₅)₂ and - CH₂-CH₂-CH₂-NH-C₂H₅; a (hetero)cycloaliphatic radical selected from the group consisting of imidazolidinyl, aziridinyl, azetidinyl, pyrrolidinyl, piperidinyl, morpholinyl, thiomorpholinyl, piperazinyl, pyrazolidinyl and azepanyl, which may be bonded via a -(CH₂)₁, _{2 or 3}- group and which may be unsubstituted or optionally substituted with 1, 2, 3, 4 or 5 substituent(s) independently selected from the group consisting of methyl, ethyl, n-propyl, isopropyl, n-butyl, tert-butyl, sec-butyl and isobutyl or in general formula le R^{1e} additionally represents a -C(=O)-OR^{37e} moiety;
R^{2e}, R^{3e}, R^{4e} and R^{5e} independently of one another, each represent a hydrogen atom; F, Cl, Br, I, -NO₂; -O-CH₃; -O-C₂H₅; -O-CF₃; -O-CFH₂; -O-CF₂H; -O-CH₂-CF₃; -O-CF₂-CF₃; -S-CH₃; -S-C₂H₅; -S-CF₃; -S-CFH₂; -S-CF₂H; -S-CH₂-CF₃; -S-CF₂-CF₃; -N(R^{11e})-S(=O)₂-R^{12e}; or a radical selected from the group consisting of methyl, ethyl, n-propyl, isopropyl, tert-butyl, -CF₃, - CFH₂, -CF₂H, -CH₂-CF₃ and -CF₂-CF₃;
with the proviso that at least one of the substituents R^{2e}, R^{3e}, R^{4e} and R^{5e} represents a -N(R^{11e})-S(=O)₂-R^{12e} moiety;
R^{11e} represents a hydrogen atom, -S(=O)₂-R^{12e} or an alkyl radical selected from the group consisting of methyl, ethyl, n-propyl, isopropyl, n-butyl, sec-butyl, isobutyl and tert-butyl;
R^{12e} represents a radical selected from the group consisting of [1,3]-benzodioxolyl, [1,4]-benzodioxanyl, benzo[b]furanyl, benzo[b]thiophenyl, benzo[2,1,3]thiadiazolyl, [1,2,3]-benzothiadiazolyl, [2,1,3]-benzoxadiazolyl, [1,2,3]-benzoxadiazolyl, benzoxazolyl, benzothiazolyl, benzisoxazolyl, benzisothiazolyl and imidazo[2,1-b]thiazolyl, which may be unsubstituted or optionally substituted with 1, 2, 3, 4 or 5 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, F, Cl, Br, I, -CN, -CF₃, -OCF₃, -SCF₃, -CF₂H and - CFH₂;
or a radical selected from the group consisting of pyridinyl, pyrrolyl, imidazolyl, pyrazolyl, triazolyl, pyridazinyl, pyrimidinyl and pyrazinyl, which may be unsubstituted or optionally substituted with 1, 2, 3, 4 or 5 substituent(s) independently selected from the group consisting of F, Cl, - NO₂; methyl, ethyl, n-propyl, isopropyl, n-butyl, isobutyl, sec-butyl, tert-butyl, -CF₃, -CF₂H, -CFH₂, -CH₂-CF₃ and -CF₂-CF₃;
and R^{37e} represents a radical selected from the group consisting of methyl, ethyl, n-propyl, isopropyl, n-butyl, sec-butyl, isobutyl, tert-butyl, n-pentyl, n-hexyl, fluorenyl, fluorenylmethyl, phenyl, benzyl and naphthyl;
optionally in form of one of its stereoisomers, preferably enantiomers or diasteromers, a racemate or in form of a mixture of at least two of its stereoisomers, preferably enantiomers and/or diastereomers, in any mixing ratio, or a salt thereof, or a corresponding solvate thereof.

38. A compound according to one or more of claims 32 to 37, **characterized in that**
R^{1e} represents a hydrogen atom; or a radical selected from the group consisting of methyl, ethyl, n-propyl, n-butyl, n-pentyl and n-hexyl or in general formula le R^{1e} additionally represents a -C(=O)-OR^{37e} moiety;
R^{2e}, R^{3e}, R^{4e} and R^{5e}, independently of one another, each represent a hydrogen atom or -N(R^{11e})-S(=O)₂-R^{12e};
with the proviso that at least one of the substituents R^{2e}, R^{3e}, R^{4e} and R^{5e} represents a -N(R^{11e})-S(=O)₂-R^{12e} moiety;
R^{11e} represents a hydrogen atom, -S(=O)₂-R^{12e} or an alkyl radical selected from the group consisting of methyl, ethyl and n-propyl;
R^{12e} represents a radical selected from the group consisting of benzo[b]thiophenyl and imidazo[2,1-b]thiazolyl, which may be unsubstituted or optionally substituted with 1, 2, 3, 4 or 5 substituent(s) independently selected from the group consisting of methyl, ethyl, n-propyl, isopropyl, F, Cl and Br;
or a pyridinyl radical, which may be unsubstituted or optionally substituted with 1, 2, 3, 4 or 5 substituent(s) independently selected from the group consisting of F, Cl, methyl, ethyl, n-propyl, isopropyl, n-butyl, isobutyl, sec-butyl and tert-butyl;
and R^{37e} represents a radical selected from the group consisting of methyl, ethyl, n-propyl, isopropyl, n-butyl, sec-butyl, isobutyl, tert-butyl, n-pentyl, n-hexyl, fluorenyl, fluorenylmethyl, phenyl, benzyl and naphthyl;
optionally in form of one of its stereoisomers, preferably enantiomers or diasteromers, a racemate or in form of a mixture of at least two of its stereoisomers, preferably enantiomers and/or diastereomers, in any mixing ratio, or a salt thereof, or a corresponding solvate thereof.

39. A compound according to one or more of claims 32 to 38 selected from the group consisting of
[4] 5-chloro-3-methyl-N-(1,2,3,4-tetrahydroisoquinolin-6-yl)benzo[b]thiophene-2-sulfonamide hydrochloride,
[5] 5-chloro-3-methyl-N-(2-methyl-1 ,2,3,4-tetrahydroisoquinolin-6-yl)benzo[b]thiophene-2-sulfonamide hydrochloride,
[10] 6-chloro-N-(1,2,3,4-tetrahydroisoquinolin-6-yl)imidazo[2,1-b]thiazole-5-sulfonamide hydrochloride,
[12] N-(1,2,3,4-tetrahydroisoquinolin-6-yl)pyridine-3-sulfonamide dihydrochloride,
[14] 6-(5-chloro-3-methyl-benzo[b]thiophene-2- sulfonylamino)-3,4-dihydro-1 H-isoquinoline-2-carboxylic acid tert-butyl ester and
[18] 6-(pyridine-3-sulfonylamino)-3,4-dihydro-1 H-isoquinoline-2carboxylic acid tert-butyl ester;
optionally in form of a corresponding solvate thereof.

40. Process for the preparation of a compound according to one or more of claims 32 to 39, **characterised in that** at least one compound of general formula IV, 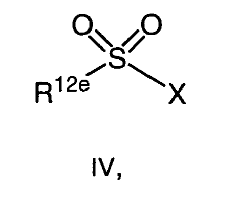 wherein R^{12e} has the meaning according to one or more of claims 32 to 39 and X represents a leaving group, preferably a halogen atom, particularly preferably a chlorine atom, is reacted with at least one compound of general formula V, 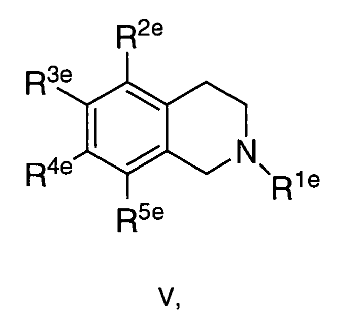 wherein R^{1e} to R^{5e} have the meaning according to one or more of claims 32 to 39, with the proviso that at least one substituent of the group consisting of R^{2e}, R^{3e}, R^{4e} and R^{5e} represents a -N(H)(R^{11e}) moiety, wherein R^{11e} has the meaning according to one or more of claims 32 to 39, or a protected derivative thereof, in a reaction medium, preferably in the presence of at least one base.

41. A salt of a substituted tetrahydroisoquinoline compound of general formula If, 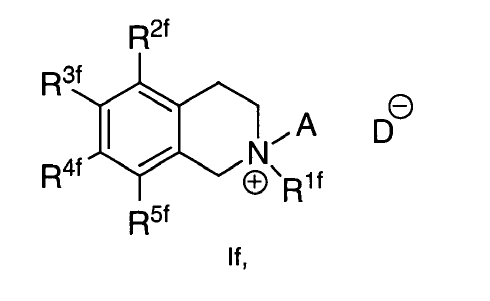 wherein
A represents a hydrogen atom or a radical selected from the group consisting of methyl, ethyl, n-propyl, isopropyl, n-butyl, isobutyl, 2-butyl, tert-butyl and n-pentyl;
D represents an anion selected from the group consisting of chloride, bromide, iodide, fluoride, hydrogensulfate, nitrate, dihydrogenphosphate, thiocyanate, cyanate, acrylate, methanesulfonate, ethanesulfonate, toluenesulfonate, benzenesulfonate, (2,5)-dihydroxy-benzenesulfonate, naphthalene-2-sulfonate, 5-sulfo-napthalene-1-sulfonate, cyclamate, dodecane-1-sulfonate and (7,7)-dimethyl-2-oxo-bicyclo[2.2.1]-hept-1-yl-methanesulfonate;
R^{1f} represents a hydrogen atom; a radical selected from the group consisting of methyl, ethyl, n-propyl, isopropyl, n-butyl, sec-butyl, isobutyl, tert-butyl, n-pentyl, n-hexyl, -CH₂-NH₂, -CH₂-NH-CH₃, -CH₂-N(CH₃)₂, -CH₂-N(C₂H₅)₂, -CH₂-NH-C₂H₅, -CH₂-CH₂-NH₂, -CH₂-CH₂-NH-CH₃, -CH₂-CH₂-N(CH₃)₂, - CH₂-CH₂-N(C₂H₅)₂, -CH₂-CH₂-NH-C₂H₅, -CH₂-CH₂-CH₂-NH-CH₃, -CH₂-CH₂-CH₂-N(CH₃)₂, -CH₂-CH₂-CH₂-N(C₂H₅)₂ and -CH₂-CH₂-CH₂-NH-C₂H₅; or a (hetero)cycloaliphatic radical selected from the group consisting of imidazolidinyl, aziridinyl, azetidinyl, pyrrolidinyl, piperidinyl, morpholinyl, thiomorpholinyl, piperazinyl, pyrazolidinyl and azepanyl, which may be bonded via a -(CH₂)₁, _{2 or 3}- group and which may be unsubstituted or optionally substituted with 1, 2, 3, 4 or 5 substituent(s) independently selected from the group consisting of oxo (=O), thioxo (=S), methyl, ethyl, n-propyl, isopropyl, n-butyl, tert-butyl, sec-butyl, isobutyl, -C(=O)-CH₃, - C(=O)-C₂H₅, -C(=O)-CH₂-CH₂-CH₃, -C(=O)-CH(CH₃)₂, -C(=O)-C(CH₃)₃, - C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃;
R^{2f}, R^{3f}, R^{4f} and R^{5f}, independently of one another, each represent a hydrogen atom; F, Cl, Br, I, -NO₂; -NH₂; -SH; -OH; -CN; -C(=O)-OH; -C(=O)-H; - S(=O)₂-OH; -C(=O)-NH₂; -S(=O)₂-NH₂; -OR^{8f}; -SR^{9f}; -N(R^{11f})-S(=O)₂-R^{12f}; a radical selected from the group consisting of methyl, ethyl, n-propyl, isopropyl, n-butyl, sec-butyl, isobutyl, tert-butyl, vinyl, allyl, ethinyl, -CF₃, - CFH₂, -CF₂H, -CH₂-CF₃ and -CF₂-CF₃; or a radical selected from the group consisting of cyclopropyl, cyclobutyl, cyclopentyl and cyclohexyl; with the proviso that at least one of the substituents R^{2f}, R^{3f}, R^{4f} and R^{5f} represents a -N(R^{11f})-S(=O)₂-R^{12f} moiety;
R^{8f} and R^{9f}, independently of one another, each represent a radical selected from the group consisting of methyl, ethyl, n-propyl, isopropyl, n-butyl, sec-butyl, isobutyl, tert-butyl, -CF₃, -CFH₂, -CF₂H, -CH₂-CF₃ and -CF₂-CF₃; a (hetero)cycloaliphatic radical selected from the group consisting of cyclopropyl, cyclobutyl, cyclopentyl and cyclohexyl; or an aryl or heteroaryl radical selected from the group consisting of phenyl, naphthyl, furyl (furanyl), thienyl (thiophenyl), pyrrolyl, and pyridinyl, which may be bonded via a -(CH₂)₁, _{2 or 3}-group and which may be unsubstituted or optionally substituted with 1, 2 or 3 substituent(s) independently selected from the group consisting of methyl, ethyl, n-propyl, isopropyl, n-butyl, tert-butyl, sec-butyl, isobutyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, F, Cl, Br, I, -CN, -CF₃, -OCF₃, -SCF₃, -OH, -SH, -NH₂, -NO₂, -CHO, -CF₂H and -CFH₂;
R^{11f} represents a hydrogen atom, -S(=O)₂-R^{12f} or an alkyl radical selected from the group consisting of methyl, ethyl, n-propyl, isopropyl, n-butyl, sec-butyl, isobutyl and tert-butyl;
R^{12f} represents a phenyl radical of general formula (Af), 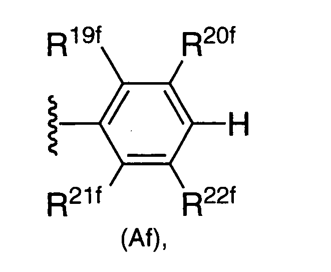 wherein
R^{19f}, R^{20f}, R^{21f} and R^{22f}, independently of one another, each represent a hydrogen atom; F, Cl, Br, I, -NO₂; -NH₂; -SH; -OH; -CN; -C(=O)-OH; -C(=O)-H; -S(=O)₂-OH; -C(=O)-NH₂; -S(=O)₂-NH₂; - C(=O)-R^{23f}; -S(=O)-R^{24f}; -S(=O)₂-R^{24f}; -OR^{25f}; -SR^{26f}; -C(=O)-OR ^{27f}; methyl, ethyl, n-propyl, isopropyl, n-butyl, isobutyl, sec-butyl, tert-butyl, -CF₃, -CF₂H, -CFH₂, -CH₂-CF₃, -CF₂-CF₃, cyclopropyl, cyclobutyl, cyclopentyl and cyclohexyl;
with the proviso that at least one of the substituents R^{19f}, R^{20f}, R^{21f} and R^{22f} is unlike hydrogen;
or a naphthyl radical, which may be unsubstituted or optionally substituted with 1, 2, 3, 4 or 5 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, F, Cl, Br, I, -CN, -CF₃, -OCF₃, -SCF₃, -OH, -SH, -NH₂, -NO₂, -CHO, -CF₂H and -CFH₂;
R^{23f} and R^{27f}, independently of one another, each represent a radical selected from the group consisting of methyl, ethyl, n-propyl, isopropyl, n-butyl, sec-butyl, isobutyl, tert-butyl, n-pentyl, n-hexyl, -CF₃, -CFH₂, -CF₂H, -CH₂-CF₃ and -CF₂-CF₃; or an aryl or heteroaryl radical selected from the group consisting of phenyl, naphthyl, furyl (furanyl), thienyl (thiophenyl), pyrrolyl, and pyridinyl, which may be bonded via a -(CH₂)₁, ₂ or ₃-group and which may be unsubstituted or optionally substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, F, Cl, Br, I, -CN, -CF₃, -OCF₃, -SCF₃, -OH, -SH, -NH₂-NO₂, -CHO, -CF₂H and -CFH₂;
R^{24f} and R^{26f}, independently of one another, each represent a radical selected from the group consisting of methyl, ethyl, n-propyl, isopropyl, n-butyl, sec-butyl, isobutyl, tert-butyl, n-pentyl, n-hexyl, vinyl, allyl, ethinyl, -CF₃, -CFH₂, -CF₂H, -CH₂-CF₃ and -CF₂-CF₃; or an aryl or heteroaryl radical selected from the group consisting of phenyl, naphthyl, furyl (furanyl), thienyl (thiophenyl), pyrrolyl, and pyridinyl, which may be unsubstituted or optionally substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, F, Cl, Br, I, -CN, -CF₃, -OCF₃, -SCF₃, -OH, -SH, - NH₂, -NO₂, -CHO, -CF₂H and -CFH₂;
and R^{25f} represents a radical selected from the group consisting of methyl, ethyl, n-propyl, isopropyl, n-butyl, sec-butyl, isobutyl, tert-butyl, -CF₃, -CFH₂, - CF₂H, -CH₂-CF₃ and -CF₂-CF₃.

42. A salt according to claim 41, **characterized in that**
A represents a hydrogen atom or a radical selected from the group consisting of methyl, ethyl, n-propyl, isopropyl, n-butyl, isobutyl, 2-butyl, tert-butyl and n-pentyl;
D represents an anion selected from the group consisting of chloride, bromide, iodide, fluoride, hydrogensulfate, nitrate, dihydrogenphosphate, thiocyanate, cyanate, acrylate, methanesulfonate, ethanesulfonate, toluenesulfonate, benzenesulfonate, (2,5)-dihydroxy-benzenesulfonate, naphthalene-2-sulfonate, 5-sulfo-napthalene-1-sulfonate, cyclamate, dodecane-1-sulfonate and (7,7)-dimethyl-2-oxo-bicyclo[2.2.1]-hept-1-yl-methanesulfonate;
R^{1f} represents a hydrogen atom; a radical selected from the group consisting of methyl, ethyl, n-propyl, isopropyl, n-butyl, sec-butyl, isobutyl, tert-butyl, n-pentyl, n-hexyl, -CH₂-NH₂, -CH₂-NH-CH₃, -CH₂-N(CH₃)₂, -CH₂-N(C₂H₅)₂, -CH₂-NH-C₂H₅, -CH₂-CH₂-NH₂, -CH₂-CH₂-NH-CH₃, -CH₂-CH₂-N(CH₃)₂, - CH₂-CH₂-N(C₂H₅)₂, -CH₂-CH₂-NH-C₂H₅, -CH₂-CH₂-CH₂-NH-CH₃, -CH₂-CH₂-CH₂-N(CH₃)₂, -CH₂-CH₂-CH₂-N(C₂H₅)₂ and -CH₂-CH₂-CH₂-NH-C₂H₅; or a (hetero)cycloaliphatic radical selected from the group consisting of imidazolidinyl, aziridinyl, azetidinyl, pyrrolidinyl, piperidinyl, morpholinyl, thiomorpholinyl, piperazinyl, pyrazolidinyl and azepanyl, which may be bonded via a -(CH₂)₁, _{2 or 3}- group and which may be unsubstituted or optionally substituted with 1, 2, 3, 4 or 5 substituent(s) independently selected from the group consisting of methyl, ethyl, n-propyl, isopropyl, n-butyl, tert-butyl, sec-butyl and isobutyl;
R^{2f}, R^{3f}, R^{4f} and R^{5f}, independently of one another, each represent a hydrogen atom; F, Cl, Br, I, -NO₂; -O-CH₃; -O-C₂H₅; -O-CF₃; -O-CFH₂; -O-CF₂H; -O-CH₂-CF₃; -O-CF₂-CF₃; -S-CH₃; -S-C₂H₅; -S-CF₃; -S-CFH₂; -S-CF₂H; -S-CH₂-CF₃; -S-CF₂-CF₃; -N(R^{11f})-S(=O)₂-R^{12f}; or a radical selected from the group consisting of methyl, ethyl, n-propyl, isopropyl, tert-butyl, -CF₃, - CFH₂, -CF₂H, -CH₂-CF₃ and -CF₂-CF₃;
with the proviso that at least one of the substituents R^{2f}, R^{3f}, R^{4f} and R^{5f} represents a -N(R^{11f})-S(=O)₂-R^{12f} moiety;
R^{11f} represents a hydrogen atom, -S(=O)₂-R^{12f} or an alkyl radical selected from the group consisting of methyl, ethyl, n-propyl, isopropyl, n-butyl, sec-butyl, isobutyl and tert-butyl;
R^{12f} represents a phenyl radical of general formula (Af), 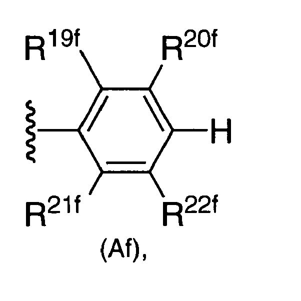 wherein
R^{19f}, R^{20f}, R^{21f} and R^{22f}, independently of one another, each represent a hydrogen atom; F, Cl, Br, I, -NO₂; -NH₂; -SH; -OH; -CN; -C(=O)-OH; -C(=O)-H; -C(=O)-CH₃; -C(=O)-C₂H₅; -O-CH₃; -O-C₂H₅; -O-CF₃; -O-CFH₂; -O-CF₂H; -O-CH₂-CF₃; -O-CF₂-CF₃; -S-CH₃; -S-C₂H₅; -S-CF₃; -S-CFH₂; -S-CF₂H; -S-CH₂-CF₃; -S-CF₂-CF₃; -C(=O)-OCH₃; -C(=O)-OC₂H₅; methyl, ethyl, n-propyl, isopropyl, n-butyl, isobutyl, sec-butyl, tert-butyl, -CF₃, -CF₂H, -CFH₂, -CH₂-CF₃ and - CF₂-CF₃;
with the proviso that at least one of the substituents R^{19f}, R^{20f} , R^{21f} and R^{22f} is unlike hydrogen;
or a naphthyl radical, which may be unsubstituted or optionally substituted with 1, 2, 3, 4 or 5 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, F, Cl, Br, I, -CN, -CF₃, -OCF₃, -SCF₃, - CF₂H and -CFH₂.

43. A salt according to claim 41 or 42, **characterized in that**
R^{1f} represents a hydrogen atom; or a radical selected from the group consisting of methyl, ethyl, n-propyl, n-butyl, n-pentyl and n-hexyl;
R^{2f}, R^{3f}, R^{4f} and R^{5f}, independently of one another, each represent a hydrogen atom or -N(R^{11f})-S(=O)₂-R^{12f};
with the proviso that at least one of the substituents R^{2f}, R^{3f}, R^{4f} and R^{5f} represents a -N(R^{11f})-S(=O)₂-R^{12f} moiety;
R^{11f} represents a hydrogen atom, -S(=O)₂-R^{12f} or an alkyl radical selected from the group consisting of methyl, ethyl and n-propyl;
R^{12f} represents a phenyl radical of general formula (Af), 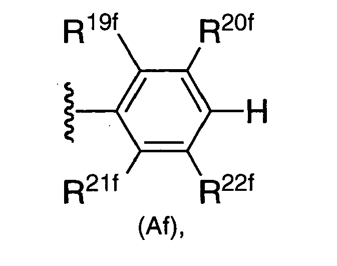 wherein
R^{19f}, R^{20f}, R^{21f} and R^{22f}, independently of one another, each represent a hydrogen atom; F, Cl, Br, I, -O-CH₃; -O-C₂H₅; -O-CF₃; - O-CFH₂; -O-CF₂H; -O-CH₂-CF₃; -O-CF₂-CF₃; methyl, ethyl, n-propyl, isopropyl, n-butyl, isobutyl, sec-butyl and tert-butyl;
with the proviso that at least one of the substituents R^{19f}, R^{20f}, R^{21f} and R^{22f} is unlike hydrogen; or a naphthyl radical, which may be unsubstituted or optionally substituted with 1, 2, 3, 4 or 5 substituent(s) independently selected from the group consisting of methyl, ethyl, n-propyl, isopropyl, F, Cl and Br.

44. A salt according to one or more of claims 41 to 43 selected from the group consisting of
[1] N-(1,2,3,4-tetrahydroisoquinolin-6-yl)naphthalene-1-sulfonamide hydrochloride,
[2] 2,2-dimethyl-6-(N-methylnaphthalene-1-sulfonamido)-1,2,3,4-tetrahydroisoquinolinium iodide,
[3] N-(2-methyl-1,2,3,4-tetrahydroisoquinolin-6-yl)naphthalene-1-sulfonamide hydrochloride,
[6] 4-methyl-N-(1,2,3,4-tetrahydroisoquinolin-6-yl)naphthalene-1-sulfonamide hydrochloride,
[7] 4-methyl-N-(2-methy)-1,2,3,4-tetrahydroisoquinolin-6-yl)naphthalene-1 - sulfonamide hydrochloride,
[8] N-(1,2,3,4-tetrahydroisoquinolin-6-yl)naphthalene-2-sulfonamide hydrochloride,
[9] N-(2-methyl-1,2,3,4-tetrahydroisoquinolin-6-yl)naphthalene-2-sulfonamide hydrochloride and
[11] 2-methoxy-5-methyl-N-(1,2,3,4-tetrahydroisoquinolin-6-yl)benzenesulfonamide hydrochloride,
or a corresponding solvate thereof.

45. Process for the preparation of a salt according to one or more of claims 41 to 44, **characterized in that** at least one compound of general formula VI, 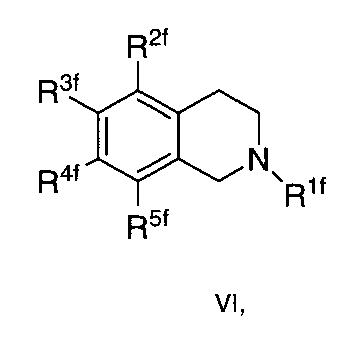 wherein R^{1f} to R^{5f} have the meaning according to one or more of claims 41 to 44, with the proviso that at least one substituent of the group consisting of R^{2f}, R^{3f}, R^{4f} and R^{5f} represents a -NR^{11f}-S(=O)₂-R^{12f} moiety, wherein R^{11f} and R^{12f} have the meaning according to one or more of claims 41 to 44, is reacted with at least one compound of general formula A-D, wherein A and D have the meaning according to one or more of claims 41 to 44, in a reaction medium, to yield at least one salt of general formula If, 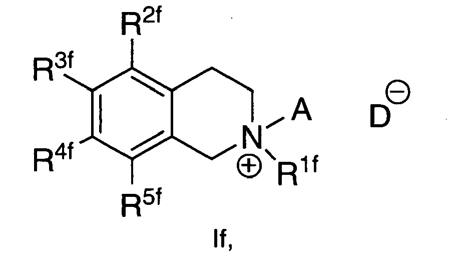 wherein A, D and R^{1f} to R^{5f} have the meaning according to one or more of claims 41 to 44 with the proviso that at least one substituent of the group consisting of R^{2f}, R^{3f}, R^{4f} and R^{5f} represents a -NR^{11f}-S(=O)₂-R^{12f} moiety, wherein R^{11f} and R^{12f} have the meaning according to one or more of claims 41 to 44.

46. A substituted tetrahydroisoquinoline compound of general formula Ig, 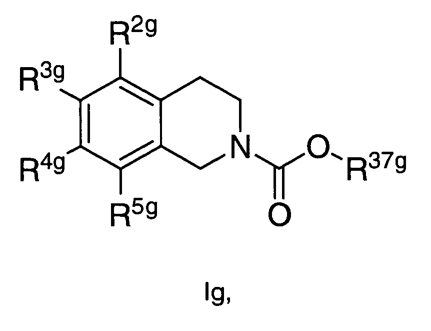 wherein
R^{1g} represents a hydrogen atom; a radical selected from the group consisting of methyl, ethyl, n-propyl, isopropyl, n-butyl, sec-butyl, isobutyl, tert-butyl, n-pentyl, n-hexyl, -CH₂-NH₂, -CH₂-NH-CH₃, -CH₂-N(CH₃)₂, -CH₂-N(C₂H₅)₂, -CH₂-NH-C₂H₅, -CH₂-CH₂-NH₂, -CH₂-CH₂-NH-CH₃, -CH₂-CH₂-N(CH₃)₂, - CH₂-CH₂-N(C₂H₅)₂, -CH₂-CH₂-NH-C₂H₅, -CH₂-CH₂-CH₂-NH-CH₃, -CH₂-CH₂-CH₂-N(CH₃)₂, -CH₂-CH₂-CH₂-N(C₂H₅)₂ and -CH₂-CH₂-CH₂-NH-C₂H₅; or a (hetero)cycloaliphatic radical selected from the group consisting of imidazolidinyl, aziridinyl, azetidinyl, pyrrolidinyl, piperidinyl, morpholinyl, thiomorpholinyl, piperazinyl, pyrazolidinyl and azepanyl, which may be bonded via a -(CH₂)₁, _{2 or 3}- group and which may be unsubstituted or optionally substituted with 1, 2, 3, 4 or 5 substituent(s) independently selected from the group consisting of oxo (=O), thioxo (=S), methyl, ethyl, n-propyl, isopropyl, n-butyl, tert-butyl, sec-butyl, isobutyl, -C(=O)-CH₃, - C(=O)-C₂H₅, -C(=O)-CH₂-CH₂-CH₃, -C(=O)-CH(CH₃)₂, -C(=O)-C(CH₃)₃, - C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃;
R^{2g}, R^{3g}, R^{4g} and R^{5g}, independently of one another, each represent a hydrogen atom; F, Cl, Br, I, -NO₂; -NH₂; -SH; -OH; -CN; -C(=O)-OH; -C(=O)-H; - S(=O)₂-OH; -C(=O)-NH₂; -S(=O)₂-NH₂; -OR^{8g}; -SR^{9g}; -N(R^{11g})-S(=O)₂-R^{12g}; a radical selected from the group consisting of methyl, ethyl, n-propyl, isopropyl, n-butyl, sec-butyl, isobutyl, tert-butyl, vinyl, allyl, ethinyl, -CF₃, - CFH₂, -CF₂H, -CH₂-CF₃ and -CF₂-CF₃; or a radical selected from the group consisting of cyclopropyl, cyclobutyl, cyclopentyl and cyclohexyl;
with the proviso that at least one of the substituents R^{2g}, R^{3g} , R^{4g} and R^{5g} represents a -N(R^{11g})-S(=O)₂-R^{12g} moiety;
R^{8g} and R^{9g}, independently of one another, each represent a radical selected from the group consisting of methyl, ethyl, n-propyl, isopropyl, n-butyl, sec-butyl, isobutyl, tert-butyl, -CF₃, -CFH₂, -CF₂H, -CH₂-CF₃ and -CF₂-CF₃; a (hetero)cycloaliphatic radical selected from the group consisting of cyclopropyl, cyclobutyl, cyclopentyl and cyclohexyl; or an aryl or heteroaryl radical selected from the group consisting of phenyl, naphthyl, furyl (furanyl), thienyl (thiophenyl), pyrrolyl, and pyridinyl, which may be bonded via a -(CH₂)₁, _{2 or 3}-group and which may be unsubstituted or optionally substituted with 1, 2 or 3 substituent(s) independently selected from the group consisting of methyl, ethyl, n-propyl, isopropyl, n-butyl, tert-butyl, sec-butyl, isobutyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, F, Cl, Br, I, -CN, -CF₃, -OCF₃, -SCF₃, -OH, -SH, -NH₂, -NO₂, -CHO, -CF₂H and -CFH₂;
R^{11g} represents a hydrogen atom, -S(=O)₂-R^{12g} or an alkyl radical selected from the group consisting of methyl, ethyl, n-propyl, isopropyl, n-butyl, sec-butyl, isobutyl and tert-butyl;
R^{12g} represents a phenyl radical of general formula (Ag), 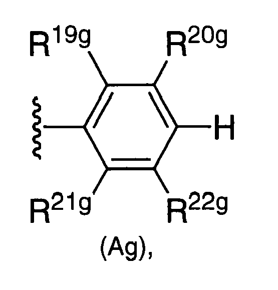 wherein
R^{19g}, R^{20g}, R^{21g} and R^{22g}, independently of one another, each represent a hydrogen atom; F, Cl, Br, I, -NO₂; -NH₂; -SH; -OH; -CN; -C(=O)-OH; -C(=O)-H; -S(=O)₂-OH; -C(=O)-NH₂; -S(=O)₂-NH₂; - C(=O)-R^{23g}; -S(=O)-R^{24g}; -S(=O)₂-R^{24g}; -OR^{25g}; -SR^{26g}; -C(=O)-OR^{27g}; methyl, ethyl, n-propyl, isopropyl, n-butyl, isobutyl, sec-butyl, tert-butyl, -CF₃, -CF₂H, -CFH₂, -CH₂-CF₃, -CF₂-CF₃, cyclopropyl, cyclobutyl, cyclopentyl and cyclohexyl;
with the proviso that at least one of the substituents R^{19g}, R^{20g}, R^{21g} and R^{22g} is unlike hydrogen;
or a naphthyl radical, which may be unsubstituted or optionally substituted with 1, 2, 3, 4 or 5 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH2-CH₃, -S-CH(CH₃)₂, -S-C(CH₃)₃, F, Cl, Br, I, -CN, -CF₃, -OCF₃, -SCF₃, -OH, -SH, -NH₂, -NO₂, -CHO, -CF₂H and -CFH₂;
R^{23g} and R^{27g}, independently of one another, each represent a radical selected from the group consisting of methyl, ethyl, n-propyl, isopropyl, n-butyl, sec-butyl, isobutyl, tert-butyl, n-pentyl, n-hexyl, -CF₃, -CFH₂, -CF₂H, -CH₂-CF₃ and -CF₂-CF₃; or an aryl or heteroaryl radical selected from the group consisting of phenyl, naphthyl, furyl (furanyl), thienyl (thiophenyl), pyrrolyl, and pyridinyl, which may be bonded via a -(CH₂)₁, _{2 or 3}-group and which may be unsubstituted or optionally substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, F, Cl, Br, I, -CN, -CF₃, -OCF₃, -SCF₃, -OH, -SH, -NH₂-NO₂, -CHO, -CF₂H and -CFH₂;
R^{24g} and R^{26g}, independently of one another, each represent a radical selected from the group consisting of methyl, ethyl, n-propyl, isopropyl, n-butyl, sec-butyl, isobutyl, tert-butyl, n-pentyl, n-hexyl, vinyl, allyl, ethinyl, -CF₃, -CFH₂, -CF₂H, -CH₂-CF₃ and -CF₂-CF₃; or an aryl or heteroaryl radical selected from the group consisting of phenyl, naphthyl, furyl (furanyl), thienyl (thiophenyl), pyrrolyl, and pyridinyl, which may be unsubstituted or optionally substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, F, Cl, Br, I, -CN, -CF₃, -OCF₃, -SCF₃, -OH, -SH, - NH₂, -NO₂, -CHO, -CF₂H and -CFH₂;
R^{25g} represents a radical selected from the group consisting of methyl, ethyl, n-propyl, isopropyl, n-butyl, sec-butyl, isobutyl, tert-butyl, -CF₃, -CFH₂, - CF₂H, -CH₂-CF₃ and -CF₂-CF₃
and R^{37g} represents a radical selected from the group consisting of methyl, ethyl, n-propyl, isopropyl, n-butyl, sec-butyl, isobutyl, tert-butyl, n-pentyl, n-hexyl, fluorenyl, fluorenylmethyl, phenyl, benzyl and naphthyl;

47. A compound according to claim 46, **characterized in that**
R^{1g} represents a hydrogen atom; or a radical selected from the group consisting of methyl, ethyl, n-propyl, n-butyl, n-pentyl and n-hexyl;
R^{2g}, R^{3g}, R^{4g} and R^{5g}, independently of one another, each represent a hydrogen atom or -N(R^{11g})-S(=O)₂-R^{12g};
with the proviso that at least one of the substituents R^{2g}, R^{3g}, R^{4g} and R^{5g} represents a -N(R^{11g})-S(=O)₂-R^{12g} moiety;
R^{11g} represents a hydrogen atom, -S(=O)₂-R^{12g} or an alkyl radical selected from the group consisting of methyl, ethyl and n-propyl;
R^{12g} represents a phenyl radical of general formula (Ag), 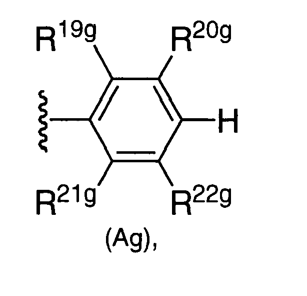 wherein
R^{19g}, R^{20g}, R^{21g} and R^{22g}, independently of one another, each represent a hydrogen atom; F, Cl, Br, I, -O-CH₃; -O-C₂H₅; -O-CF₃; - O-CFH₂; -O-CF₂H; -O-CH₂-CF₃; -O-CF₂-CF₃; methyl, ethyl, n-propyl, isopropyl, n-butyl, isobutyl, sec-butyl and tert-butyl;
with the proviso that at least one of the substituents R^{19g}, R^{20g}, R^{21g} and R^{22g} is unlike hydrogen;
or a naphthyl radical, which may be unsubstituted or optionally substituted with 1, 2, 3, 4 or 5 substituent(s) independently selected from the group consisting of methyl, ethyl, n-propyl, isopropyl, F, Cl and Br;
and R^{37g} represents a radical selected from the group consisting of methyl, ethyl, n-propyl, isopropyl, n-butyl, sec-butyl, isobutyl, tert-butyl, n-pentyl, n-hexyl, fluorenyl, fluorenylmethyl, phenyl, benzyl and naphthyl.

48. A compound according to claim 46 or 47 selected from the group consisting of
[13] 6-(naphthalene-1-sulfonylamino)-3,4-dihydro-1 H-isoquinoline-2-carboxylic acid tert-butyl ester,
[15] 6-(4-methyl-naphthalene-1-sulfonylamino)-3,4 -dihydro-1H-isoquinoline-2-carboxylic acid tert-butyl ester,
[16] 6-(naphthalene-2-sulfonylamino)-3,4-dihydro-1 H-isoquinoline-2-carboxylic acid tert-butyl ester and
[17] 6-(2-methoxy-5-methyl-benzenesulfonylamino)-3,4-dihydro-1H-isoquinoline-2-carboxylic acid tert-butyl ester.
